# EUROPEAN PATENT APPLICATION

(11) **EP 4 835 077 A1**
(43) Date of publication of application: **04.11.2026**
(21) Application number: 25425019.4
(22) Date of filing: 28.04.2025
(51) Int. Cl.: C12N 15/113, C07K 14/435, A01K 67/61

(54) **2GEN SYSTEM**

(71) Applicant: Biocentis S.r.l., 20124 Milan (IT)
(72) Inventor: Hammond, Andrew Marc, 05100 Terni (IT)
(74) Representative: Modiano, Micaela Nadia

(57) **Abstract**

Arthropod pests cause substantial harm to human health, animal health, and agriculture. Many methods of arthropod control have been contemplated, including the use of pesticides, habitat reformation, and various sterile techniques. This disclosure provides methods and compositions for related to the generation of sterile or inviable arthropods.

## Description

### FIELD

The present disclosure relates to methods and compositions for the control of arthropod pests.

### SEQUENCES

A sequence listing contained in the file named "P35676EP00_SL26.xml" which is 17,260,544 bytes (measured in operating system MS-Windows^{®}), created on April 23, 2025, containing a total number of 6850 sequences, from SEQ ID NO: 1 to SEQ ID NO: 6850, is filed electronically herewith and incorporated by reference in its entirety.

A brief description of nucleic acid sequences and amino acid sequences referenced in the present disclosure is provided in Table 1. SEQ ID NOs: 1 to 2902, 4338 to 4340, and 4397 to 6493 correspond to various male-fertility essential gene sequences. SEQ ID NOs: 4250 to 4254, 4258 to 4307, 4309 to 4312, 4386, and 6848 to 6850 correspond to various promoter sequences.

**Table 1. Nucleic acid sequences and amino acid sequences. "NT" refers to nucleic acid sequence and "AA" refers to amino acid sequence. "CDS" refers to coding sequence.**

| **SEQ ID NOs** | **Type** | **Sequence Description** |
|---|---|---|
| 1 to 26 | NT | Ultraconserved sequence from *28S rDNA* from various species |
| 27 to 50 | NT | Various male-fertility essential gene CDS from *Aedes aegypti* |
| 51 to 141 | NT | Various male-fertility essential gene CDS from various arthropod species |
| 142 to 781 | NT | *bel* CDS and transcript sequences from various arthropod species |
| 782 to 784 | NT | Ultraconserved sequence from *bel* in various arthropod species |
| 785 to 798 | NT | Various male-fertility essential gene CDS from various arthropod species |
| 799 to 1112 | NT | *blw* CDS and transcript sequences from various arthropod species |
| 1113 to 1538 | NT | Various male-fertility essential gene CDS from various arthropod species |
| 1539 to 1674 | NT | *knon* CDS and transcript sequences from various arthropod species |
| 1675 to 2208 | NT | Various male-fertility essential gene CDS from various arthropod species |
| 2209 to 2573 | NT | *snky* CDS and transcript sequences from various arthropod species |
| 2574 to 2823 | NT | Various male-fertility essential gene CDS from various arthropod species |
| 2824 to 2878 | NT | *β2-tubulin* CDS from various arthropod species |
| 2879 | NT | *β2-tubulin* synthetic consensus sequence |
| 2880 | AA | β2-tublin protein sequence from *Drosophila suzukii* |
| 2881 | NT | *β2-tubulin* CDS from *Aedes aegypti* |
| 2882 to 2884 | NT | *ary* sequences from *Drosophila melanogaster and D. suzukii* |
| 2885 to 2892 | NT | *kl-2, kl-3,* and *kl-5* sequences from *Drosophila suzukii* and *Aedes aegypti* |
| 2893 to 2898 | NT | *ory* sequences from *Drosophila melanogaster and D. suzukii* |
| 2899 to 2902 | NT | Y-linked male-fertility essential gene sequences from *Drosophila suzukii* |
| 2901 to 3020 | NT | Guide RNA spacer sequences and target site sequences for *28s rDNA* |
| 3021 to 3023 | NT | Target sites for *Aedes aegypti MS1* |
| 3024 to 3330 | NT | Guide RNA spacer sequences for *Aedes aegypti bel* (AAEL004472) |
| 3331 to 3576 | NT | Guide RNA spacer sequences for *Aedes aegypti blw* (AAEL012175) |
| 3577 and 3578 | NT | Ultraconserved *bel* target site sequences |
| 3579 and 3580 | NT | Ultraconserved *bel* guide RNA spacer sequences |
| 3581 to 3601 | NT | Guide RNA spacer sequences and target site sequences for various arthropod genes |
| 3602 to 3744 | NT | Guide RNA spacer sequences for *Aedes aegypti snky* (AAEL002356) |
| 3745 and 3746 | NT | Guide RNA spacer sequences for *Drosophila suzukii zpg* |
| 3747 to 3768 | NT | Guide RNA spacer sequences and target site sequences for *Aedes aegypti β2-tubulin* |
| 3769 | NT | Target site sequence for arthropod *β2-tubulin* |
| 3770 to 3782 | NT | Guide RNA spacer sequences and target site sequences for *Aedes aegypti β2-tubulin* |
| 3783 to 3834 | NT | Guide RNA spacer sequences and target site sequences for *Drosophila suzukii β2-tubulin* |
| 3835 to 4011 | NT | Guide RNA spacer sequences for *Aedes aegypti β2-tubulin* (AAEL019894) |
| 4012 to 4249 | NT | Various plasmid sequences |
| 4250 to 4254 | NT | Various germline-preferred promoter sequences |
| 4255 and 4256 | NT | *nanos* terminator sequences |
| 4257 to 4274 | NT | Various germline-preferred promoter sequences |
| 4275 | NT | 10x UAS inducible promoter sequence |
| 4276 | NT | 15x QUAS inducible promoter sequence |
| 4277 | NT | 15x QUAS-hsp70 inducible promoter sequence |
| 4278 | NT | 15x QUAS-*hsp70-syn21* inducible promoter sequence |
| 4279 | NT | 21x tetO inducible promoter sequence |
| 4280 and 4281 | NT | 5x QUAS and 5xUAS inducible promoter sequences, respectively |
| 4282 | NT | 5x UAS*-hsp70* inducible promoter sequence |
| 4283 and 4284 | NT | 7x tetO inducible promoter sequences |
| 4285 | NT | 7x tetO-hsp70 inducible promoter sequence |
| 4286 | NT | *pUASz* inducible promoter sequence |
| 4287 | NT | QUAS inducible promoter sequence |
| 4288 | NT | tetO inducible promoter sequence |
| 4289 | NT | UAS inducible promoter sequence |
| 4290 | NT | *Drosophila* synthetic core promoter |
| 4291 and 4292 | NT | *hsp70* and *pgd* minimal promoters, respectively |
| 4293 and 4294 | NT | PolIII *7SK* promoter sequences |
| 4295 to 4307 | NT | PolIII *U6* promoter sequences |
| 4308 | NT | 3'-UTR *nanos* sequence from *Drosophila suzukii* |
| 4309 and 4310 | NT | *hsp83* and *nup50* ubiquitous promoter sequences, respectively |
| 4311 and 4312 | NT | *ubiquitin* ubiquitous promoter sequences |
| 4313 and 4314 | NT | *AcrIIA4* anticas CDS sequences |
| 4315 | NT | Expression cassette comprising AcrIIA4 operably linked to a *ubiquitin* promoter |
| 4316 and 4317 | NT | Cas9 CDS |
| 4318 and 4319 | NT | Nuclear localization signal CDS |
| 4320 | NT | Gal4 transactivator CDS |
| 4321 | NT | QF2 transactivator CDS |
| 4322 | NT | QF2^{W} transactivator CDS |
| 4323 | NT | Expression cassette for tTa-based drug repression with modified female-specific splice module for expression in *Aedes aegypti* |
| 4324 and 4325 | NT | tTA and tTAV CDS, respectively |
| 4326 to 4331 | NT | CDS for various female-lethal effectors |
| 4332 to 4334 | NT | Synthetic female-specific splice module sequences |
| 4335 to 4337 | NT | Plasmid sequences for various female-lethal effectors |
| 4338 | NT | CDS for codon-scrambled *ory* |
| 4339 and 4340 | NT | CDS for codon-scrambled *β2-tubulin* |
| 4341 | NT | Expression cassette for codon-scrambled *β2-tubulin* |
| 4342 and 4343 | NT | gRNA scaffold sequences compatible with Cas9 |
| 4344 | NT | Expression cassette for codon-scrambled *β2-tubulin* |
| 4345 to 4385 | NT | Guide RNA spacer sequences and target sequences for various arthropod genes |
| 4386 | NT | *OpIE-2* ubiquitous promoter sequence |
| 4387 | NT | Expression cassette for *ary* |
| 4388 | NT | Expression cassette for codon-scrambled *ory* |
| 4389 and 4390 | NT | PolII terminator sequences |
| 4391 to 4394 | NT | Plasmid sequences comprising QF2 operably linked to a *ubiquitin* promoter |
| 4395 and 4396 | NT | Plasmid sequences comprising codon-scrambled *β2-tubulin* and *piggyBac* repeats |
| 4397 to 5264 | AA | Male-fertility essential genes from *Aedes aegypti* |
| 5265 to 5270 | AA | Male-fertility essential genes from *Drosophila melanogaster* |
| 5271 | AA | Male-fertility essential gene from *Aedes aegypti* |
| 5272 and 5273 | AA | Male-fertility essential genes from *Drosophila melanogaster* |
| 5274 | AA | Male-fertility essential gene from *Aedes aegypti* |
| 5275 to 5283 | AA | Male-fertility essential genes from *Drosophila melanogaster* |
| 5284 | AA | Male-fertility essential gene from *Aedes aegypti* |
| 5285 to 5295 | AA | Male-fertility essential genes from *Drosophila melanogaster* |
| 5296 | AA | Male-fertility essential gene from *Aedes aegypti* |
| 5297 | AA | Male-fertility essential gene from *Drosophila melanogaster* |
| 5298 | AA | Male-fertility essential gene from *Aedes aegypti* |
| 5299 to 5617 | AA | bel sequences from various arthropods |
| 5618 to 5847 | AA | Male-fertility essential genes from *Aedes aegypti* and *Drosophila melanogaster* |
| 5848 to 5914 | AA | knon sequences from various arthropods |
| 5915 to 6160 | AA | Male-fertility essential genes from *Aedes aegypti* and *Drosophila melanogaster* |
| 6161 to 6342 | AA | snky sequences from various arthropods |
| 6343 to 6466 | AA | Male-fertility essential genes from *Aedes aegypti* and *Drosophila melanogaster* |
| 6467 to 6487 | AA | *β2-tubulin* sequences from various arthropods |
| 6488 | AA | Male-fertility essential gene from *Drosophila melanogaster* |
| 6489 and 6490 | AA | Male-fertility essential genes from *Drosophila suzukii* |
| 6491 | AA | Male-fertility essential gene from *Drosophila melanogaster* |
| 6492 and 6493 | AA | Male-fertility essential genes from *Drosophila suzukii* |
| 6494 and 6495 | AA | AcrIIA4 anticas sequences |
| 6496 and 6497 | AA | tTA and tTAV sequences, respectively |
| 6498 to 6504 | AA | Various female-lethal effector sequences from various species |
| 6505 and 6506 | AA | Nuclear localization signal sequences |
| 6507 | AA | Gal4 transactivator sequence |
| 6508 | AA | QF2 transactivator sequence |
| 6509 | AA | QF2^{W} transactivator sequence |
| 6510 | AA | Codon-scrambled ory sequence |
| 6511 to 6831 | AA | blw sequences from various arthropods |
| 6832 to 6834 | NT | miRNA spacer sequences targeting *Aedes aegypti* β2-tubulin |
| 6835 to 6837 | NT | miRNA spacer sequences targeting *Aedes aegypti* β2-tubulin |
| 6838 to 6840 | NT | miRNA spacer sequences targeting *Drosophila suzukii* β2-tubulin |
| 6841 to 6843 | NT | miRNA 5' sequence |
| 6844 to 6845 | NT | miRNA loop structure sequence |
| 6846 and 6847 | NT | miRNA 3' sequence |
| 6848 to 6850 | NT | TRE promoter sequences |

### BACKGROUND

Arthropods cause substantial damage to agricultural productivity, the health of humans and other animals, and ecosystem stability. These organisms have evolved to exploit various ecological niches, often causing extensive damage through direct feeding, egg laying, or transmission of pathogens. The global impact on human health, biodiversity, food security, and global economies is tremendous, and damages may worsen as climate change and globalization drive the spread of invasive harmful arthropod species. Amongst the most devastating are mosquitoes, ticks and sandflies that spread human disease and fruit flies, locusts, aphids, armyworms, bollworms, and rootworms that destroy crops.

In addition to using pesticides and environmental modifications, sterile insect technique (SIT) has been used to attempt to control populations of arthropods. Generally, SIT involves releasing sterile male insects into the wild to outcompete wildtype males in mating, which in turn reduces the population of the target insect. However, SIT requires continuous sorting and release of sterile males, which is both laborious and expensive.

A variety of variations or improvements have been made to SIT in recent years, including precision guided SIT (pgSIT), which utilizes a split CRISPR system to generate sterile males, and female-specific release of insects carrying a dominant lethal (fsRIDL), which provides a repressible female lethal effector that can be switched off during mass rearing and switched on to sex-sort males for release. *See,* for example, Kandul et al., Nature Communications, 10:84 (2019) and Fu et al., Proc. Natl. Acad. Sci. USA, 107:4550-4554 (2010), respectively. Both pgSIT and fsRIDL have drawbacks, however. For example, pgSIT requires the maintenance of two separate insect lines for each planned release and fsRIDL ultimately produces viable females and wildtype males that can contribute to a wild population of the target arthropod.

Self-sustaining gene drive systems (*see,* for example, Hammond et al., Nature Biotechnology, 34:78-83 (2016); Kyrou et al., Nature Biotechnology, 36:1062-1066 (2018); Simoni et al., Nature Biotechnology, 38:1054-1060 (2020); and Morianou et al., bioRxiv 2024.10.21.618489 (October 21, 2024)), Y-linked editor systems that selectively kill or sterilize female progeny of males (*See,* for example, Burt & Deredec, Proc. R. Soc. B, 285: 20180776 (2018)), release of insects carrying a dominant-sterile drive (RIDD) elements that kill or sterilize female progeny of males (*See,* for example, Chen et al. Nat Commun, 15, 8053 (2024)), X-shredding elements that bias progeny towards males *(See,* for example, Galizi et al. Sci Reports, 6:31139 (2016)), X-poisoning elements that produce inviable female progeny and viable male progeny (*See,* for example, Haber et al. Nat Commun, 15, 4983 (2024); and Lawler et al. G3, 14, jkae025 (2024)), Y-shredding elements that bias the progeny towards females (*See,* for example, Vitale et al. PLoS Genetic, 20(6): e1011303 (2924)), and others (*See,* for example, Yan et al. Trends Biotechnol, 43, 826-837 (2025)) have also been explored as related to controlling arthropod pest populations.

Despite these advances, each of the aforementioned methods contains some drawbacks. There exists a need for a safe, cost effective, scalable technology that is both compatible with current technologies and sustainable for long term usage.

The instant application addresses this need by providing compositions and methods related to a "2GEN" system for arthropod population control that is limited to two generations of persistence in the environment, possesses in-built sex-sorting technology, and enables greater than 10x efficiency as compared to previously known sterile male technologies. The 2GEN system also allows a "protected" area to include not only the area of the release, but also where the second generation male progeny of the modified arthropods provided herein travel to and mate as adults.

### SUMMARY

In some aspects, this disclosure provides a male sterile effector comprising: (a) a first promoter operably linked to a first nucleic acid molecule encoding an RNA-guided nuclease; and (b) a second promoter operably linked to a second nucleic acid molecule encoding a guide RNA (gRNA), wherein the gRNA comprises a nucleic acid sequence at least 90% identical or complementary to at least 17 consecutive nucleotides of a first arthropod male-fertility essential gene.

In some aspects, this disclosure provides a male sterile effector comprising a first promoter operably linked to a first nucleic acid molecule encoding a microRNA (miRNA) molecule or a short hairpin RNA (shRNA) molecule comprising a nucleic acid sequence at least 90% identical to at least 17 consecutive nucleotides of a first arthropod male-fertility essential gene messenger RNA molecule.

In some aspects, this disclosure provides one or more isolated nucleic acid molecules encoding any male sterility effector provided herein.

In some aspects, this disclosure provides a sterility system comprising: (a) any male sterile effector provided herein; and (b) a repressible female-lethal effector.

In some aspects, this disclosure provides a sterility system comprising: (a) any male sterile effector provided herein; and (b) a rescue element comprising a promoter operably linked to a nucleic acid molecule encoding at least one of: an anticas protein, a functional copy of the first arthropod male-fertility essential gene, and a functional copy of the second arthropod male-fertility essential gene.

In some aspects, this disclosure provides a sterility system comprising: (a) any male sterile effector provided herein; (b) a repressible female-lethal effector; and (c) a rescue element comprising a fourth promoter operably linked to a nucleic acid molecule encoding at least one of: an anticas protein, a functional copy of the first arthropod male-fertility essential gene, and a functional copy of the second arthropod male-fertility essential gene.

In some aspects, this disclosure provides a sterility system comprising: (a) a male sterile effector comprising a loss-of-function mutation in at least one male-fertility essential gene; and (b) a repressible female-lethal effector.

In some aspects, this disclosure provides a sterility system comprising: (a) a male sterile effector comprising a loss-of-function mutation in at least one male-fertility essential gene; and (b) a rescue element comprising a first promoter operably linked to a functional copy of the at least one male-fertility essential gene.

In some aspects, this disclosure provides a sterility system comprising: (a) a male sterile effector comprising a loss-of-function mutation in at least one male-fertility essential gene; (b) a rescue element comprising a first promoter operably linked to a functional copy of the at least one male-fertility essential gene; and (c) a repressible female-lethal effector.

In some aspects, this disclosure provides one or more isolated nucleic acid molecules encoding any sterility system provided herein.

In some aspects, this disclosure provides an isolated nucleic acid molecule comprising a nucleic acid sequence at least 95% identical to a nucleic acid sequence selected from the group consisting of SEQ ID NOs: 1 to 2879, 2881 to 4396, and 6842 to 6850.

In some aspects, this disclosure provides an isolated nucleic acid molecule comprising a non-naturally occurring codon-scrambled nucleic acid sequence that encodes an amino acid sequence at least 90% identical to an amino acid sequence selected from the group consisting of SEQ ID NOs: 2880 and 4397 to 6493.

In some aspects, this disclosure provides a modified arthropod comprising any isolated nucleic acid molecule provided herein.

In some aspects, this disclosure provides a modified arthropod comprising any male sterile effector provided herein.

In some aspects, this disclosure provides a modified arthropod comprising any sterility system provided herein.

In some aspects, this disclosure provides a modified arthropod comprising a male sterile effector, wherein the male sterile effector comprises a loss-of-function mutation in at least one male-fertility essential gene positioned within a Y chromosome or within an M-locus.

In some aspects, this disclosure provides a modified arthropod, wherein the modified arthropod is an *Aedes aegypti* mosquito.

In some aspects, this disclosure provides a modified arthropod, wherein the modified arthropod is a *Drosophila suzukii* fly.

In some aspects, this disclosure provides a method of generating a population of progeny arthropods, the method comprising: (a) providing a male modified arthropod comprising any sterility system provided herein; and (b) allowing the male modified arthropod to mate with a wildtype female arthropod of the same species to produce the population of progeny arthropods, wherein the population of progeny arthropods comprises: (i) at least one sterile male progeny arthropod, (ii) at least one inviable female progeny arthropod, or (iii) both (i) and (ii).

In some aspects, this disclosure provides a method of generating a population of progeny arthropods, the method comprising: (a) providing a male modified arthropod comprising any sterility system provided herein; and (b) allowing the male modified arthropod to mate with a wildtype female arthropod of the same species to produce the population of progeny arthropods, wherein the population of progeny comprises a subpopulation of male progeny arthropods and a subpopulation of female progeny arthropods, wherein at least 25% of the male progeny arthropods are sterile and wherein at least 25% of the female progeny arthropods are inviable.

In some aspects, this disclosure provides a method of generating a population of progeny arthropods, the method comprising: (a) providing a male modified arthropod comprising any sterility system provided herein; (b) providing a wildtype female arthropod of the same species to the male modified arthropod; and (c) providing conditions which allow the male modified arthropod to mate with the wildtype female arthropod to produce the population of progeny arthropods, wherein the population of progeny comprises: (i) at least one sterile male progeny arthropod, (ii) at least one inviable female progeny arthropod, or (iii) both (i) and (ii).

In some aspects, this disclosure provides a method of generating a population of progeny arthropods, the method comprising: (a) providing a male modified arthropod comprising any sterility system provided herein; (b) providing a wildtype female arthropod of the same species to the male modified arthropod; and (c) providing conditions which allow the male modified arthropod to mate with the wildtype female arthropod to produce the population of progeny arthropods, wherein the population of progeny arthropods comprises a subpopulation of male progeny and a subpopulation of female progeny, wherein at least 25% of the male progeny are sterile and wherein at least 25% of the female progeny are inviable.

In some aspects, this disclosure provides a method comprising: (a) obtaining a male modified arthropod comprising any sterility system provided herein; and (b) releasing the at least one male modified male arthropod outdoors in the vicinity of at least one wildtype female arthropod of the same species.

In some aspects, this disclosure provides a method comprising: (a) obtaining any male modified arthropod provided herein; and (b) releasing the modified arthropod outdoors in the vicinity of at least one wildtype female arthropod of the same species.

In some aspects, this disclosure provides a method comprising: (a) obtaining a male population of any modified arthropod provided herein; and (b) releasing the male population outdoors in the vicinity of a wildtype population of the arthropod that comprises at least one wildtype female arthropod of the same species, wherein at least one member of the male population of the modified arthropod mates with the at least one wildtype female arthropod, and wherein the at least one wildtype female arthropod produces (i) at least one sterile male progeny, (ii) at least one inviable female progeny, or (iii) both (i) and (ii).

In some aspects, this disclosure provides a method comprising: (a) introducing any male sterility effector provided herein to at least one arthropod cell; and (b) obtaining an adult arthropod from the at least one arthropod cell, wherein the adult arthropod comprises the male sterility effector.

In some aspects, this disclosure provides a method comprising: (a) introducing any sterility system provided herein to at least one arthropod cell; and (b) obtaining an adult arthropod from the at least one arthropod cell, wherein the adult arthropod comprises the sterility system.

In some aspects, this disclosure provides a method comprising: (a) introducing any isolated nucleic acid molecule provided herein to at least one arthropod cell; and (b) obtaining an adult arthropod from the at least one arthropod cell, wherein the adult arthropod comprises the sterility system.

In some aspects, this disclosure provides a method comprising: (a) introducing a recoded rescue element into a genome of a first arthropod cell, wherein the recoded rescue element encodes a male-fertility essential gene; (b) further introducing a loss-of-function mutation in an endogenous allele of the male-fertility essential gene into the genome; and (c) obtaining a male arthropod comprising the recoded rescue element and the loss-of-function mutation.

In some aspects, this disclosure provides a method comprising: (a) introducing a loss-of-function mutation in an endogenous allele of a male-fertility essential gene in at least one arthropod cell, wherein the at least one arthropod cell further comprises a recoded recue element encoding a functional copy of the male-fertility essential gene; and (b) obtaining an adult male arthropod from the at least one arthropod cell, wherein the adult male arthropod comprises the loss-of-function mutation and the recoded rescue element.

In some aspects, this disclosure provides a method comprising: (a) obtaining any male modified arthropod provided herein; and (b) allowing the modified male arthropod to mate with a modified female arthropod of the same species, wherein the modified female arthropod is homozygous for the recoded rescue element on an X chromosome or m-locus, to produce a progeny population, wherein the progeny population comprises at least one fertile modified male arthropod comprising the male sterility effector and the recoded rescue element.

In some aspects, this disclosure provides a method comprising: (a) obtaining any male modified arthropod provided herein; (b) allowing the modified male arthropod to mate with at least one wildtype female arthropod of the same species to produce a progeny population; and (c) selecting at least one sterile male arthropod from the progeny population.

In some aspects, this disclosure provides a method comprising: (a) providing any male modified arthropod provided herein and at least one wildtype female arthropod of the same species; (b) obtaining a progeny population produced by the modified male arthropod and the at least one wildtype female arthropod; and (c) selecting at least one sterile male arthropod from the progeny population.

In some aspects, this disclosure provides any modified arthropod produced by any method provided herein.

### BRIEF DESCRIPTION OF THE DRAWINGS

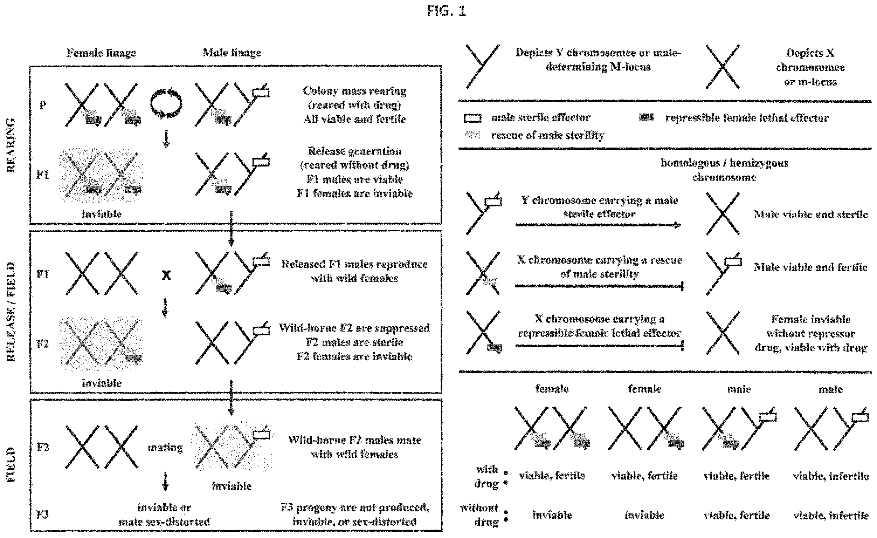

**FIG. 1** depicts the 2GEN system, which allows the mass rearing and release of male sterile and female lethal traits into the environment for two generation-limited genetic control.

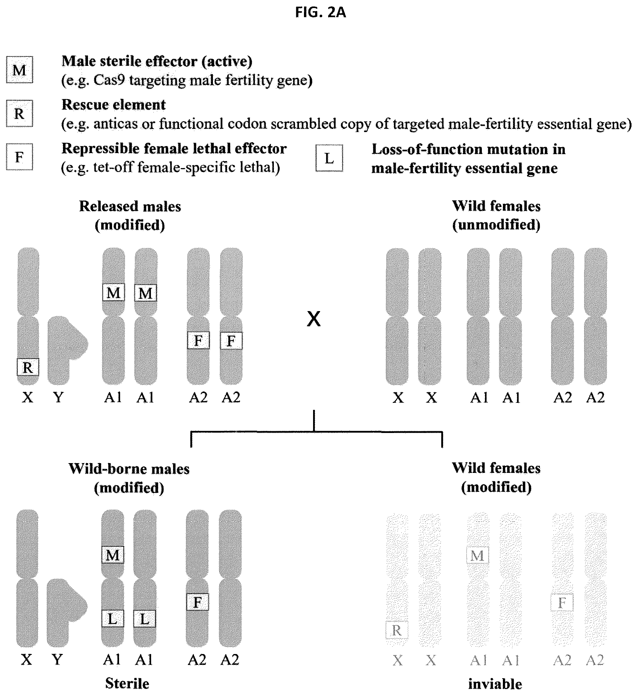

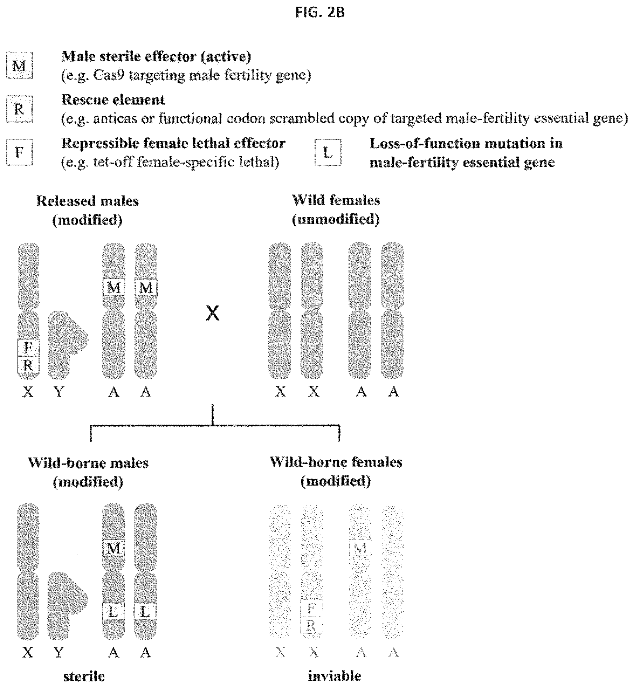

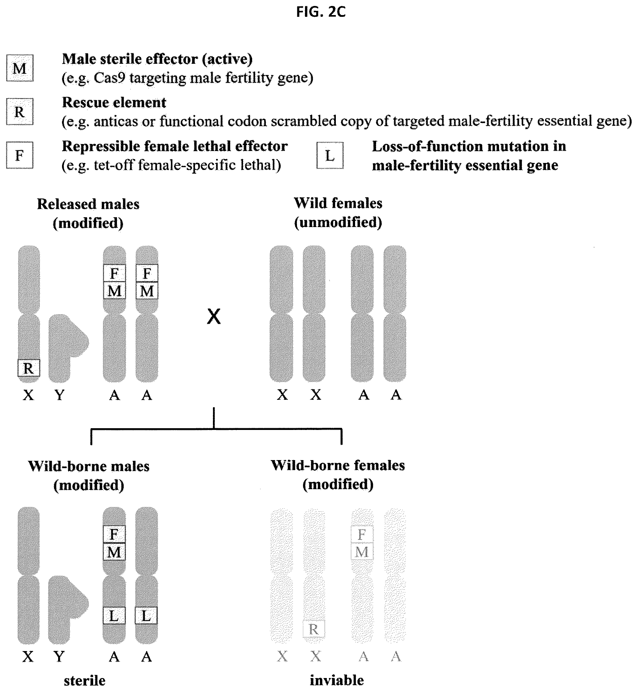

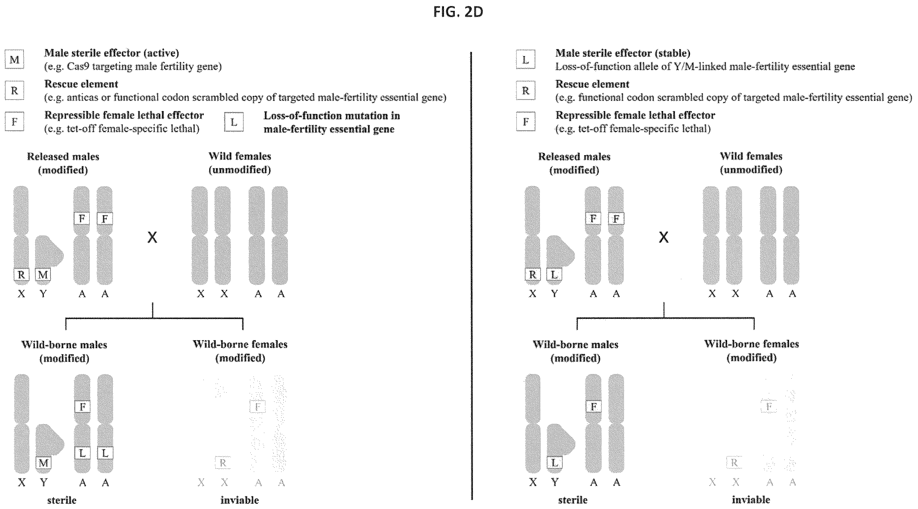

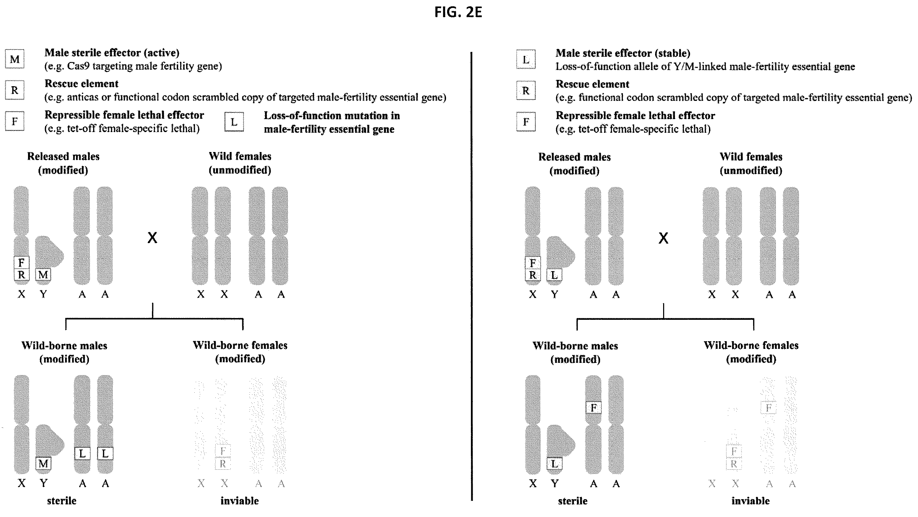

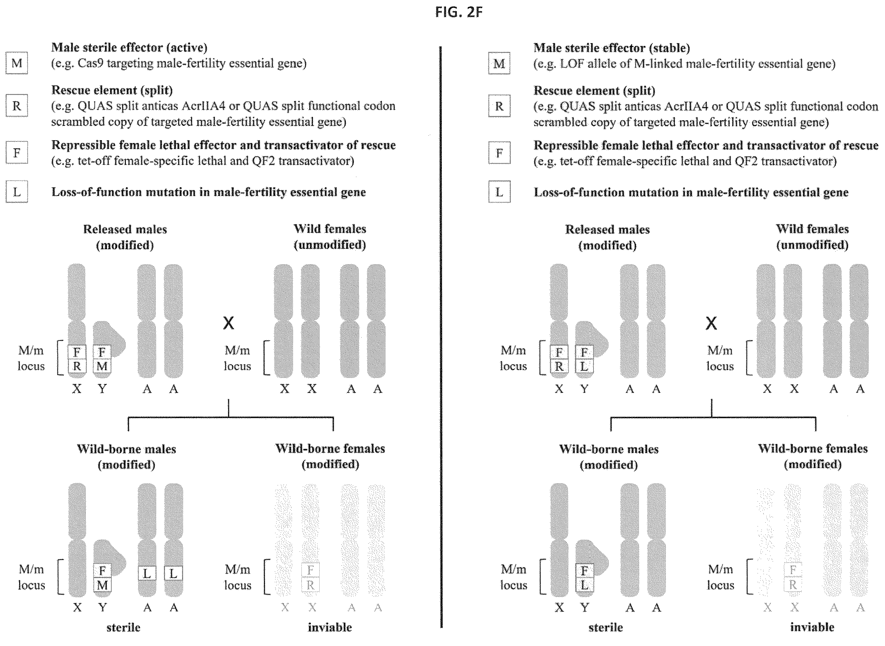

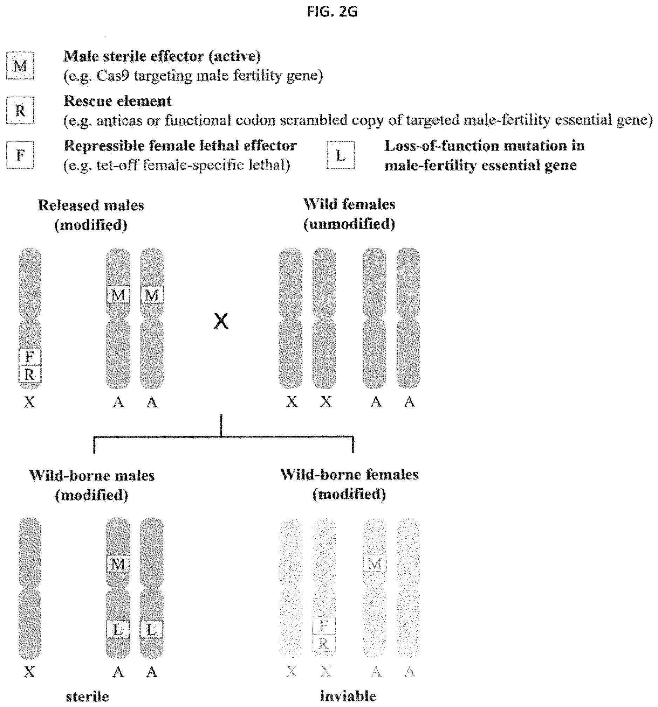

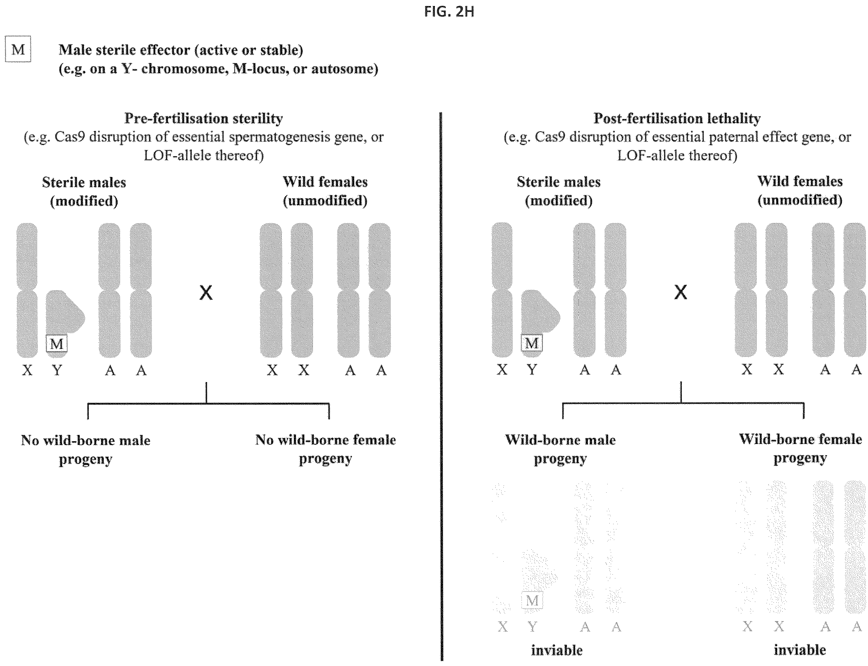

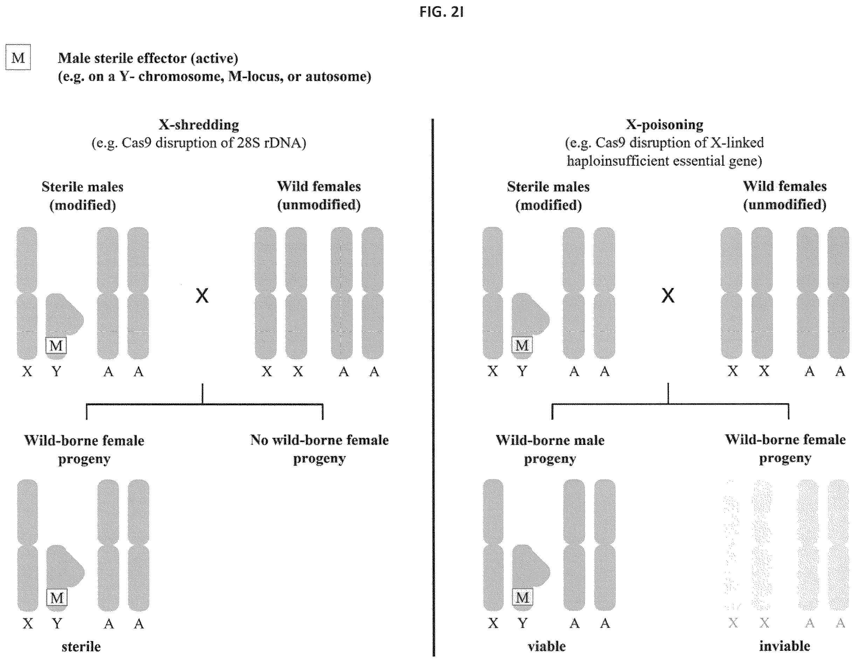

**FIG. 2A****,** **FIG. 2B****,** **FIG. 2C****,** **FIG. 2D****,** **FIG. 2E****,** **FIG. 2F****,** **FIG. 2G****, and** **FIG. 2H****,** depict non-limiting variations of the 2GEN system. **FIG. 2A** depicts a system where a male arthropod comprises a male sterile effector homozygous on an autosome (A1), a female lethal element homozygous on a different autosome (A2), and a rescue element on an X chromosome (or m-locus). The rescue element can prevent the male sterile effector from generating a LOF mutation in a male-fertility essential gene, or compensate for its loss by expressing a codon-scrambled copy the male-fertility essential gene. The male arthropod is mated with wildtype females to produce sterile males that are heterozygous for the male sterile effector and female lethal element, but carry homozygous loss-of-function (LOF) mutations in a male-fertility essential gene located on an autosomal chromosome, and inviable females that are inviable due to inheriting the repressible female lethal effector from the father. In this system the LOF mutation can be located on any chromosome. **FIG. 2B** depicts a system where a male arthropod comprises a repressible female lethal effector and a rescue element on an X chromosome (or m-locus), and a male sterile effector homozygous on an autosome. The rescue element can prevent the male sterile effector from generating a LOF mutation in a male-fertility essential gene, or compensate for its loss by expressing a codon-scrambled copy the male-fertility essential gene. The male arthropod is mated with a wildtype female to produce sterile males that are heterozygous for the male sterile effector, but carry homozygous loss-of-function (LOF) mutations of a male-fertility essential gene located on an autosomal chromosome, and inviable females that are inviable due to inheriting the repressible female lethal effector from the father. In this system the LOF mutation can be located on any chromosome. **FIG. 2C** depicts a system where a male arthropod comprises a rescue element on an X chromosome (or m-locus), and a male sterile effector and repressible female lethal effector homozygous on an autosome. The rescue element can prevent the male sterile effector from generating a LOF mutation in a male-fertility essential gene, or compensate for its loss by expressing a codon-scrambled copy the male-fertility essential gene. The male arthropod is mated with a wildtype female to produce sterile males that are heterozygous for the male sterile effector, but carry homozygous loss-of-function (LOF) mutations of a male-fertility essential gene located on an autosomal chromosome, and inviable females that are inviable due to inheriting the repressible female lethal effector from the father. In this system the LOF mutation can be located on any chromosome. **FIG. 2D** depicts a system where a male arthropod comprises a rescue element on an X chromosome (or m-locus), a repressible female lethal effector homozygous on an autosome, and a male sterile effector on a Y chromosome (or M-locus) that is active (left) or stable (right). The male arthropod comprising the active male sterile effector is mated with a wildtype female to produce sterile males that carry the male sterile effector on a Y chromosome, but carry homozygous loss-of-function (LOF) mutations of a male-fertility essential gene located on an autosomal chromosome, and inviable females that are inviable due to inheriting the repressible female lethal effector from the father (left). In this system the LOF mutation can be located on any chromosome. Alternatively, the male arthropod comprising a stable male sterile effector is mated with a wildtype female to produce sterile males carrying a loss-of-function (LOF) mutation of a male-fertility essential gene located on a Y chromosome, and inviable females that are inviable due to inheriting the repressible female lethal effector from the father (right). In this system the male sterile effector is a LOF mutation on a Y chromosome (or M-locus) and the rescue element is a functional codon scrambled copy of the targeted male-fertility essential gene. **FIG. 2E** depicts a system where a male arthropod comprises a rescue element and a repressible female lethal effector on an X chromosome (or m-locus), and a male sterile effector on a Y chromosome that is active (left) or stable (right). The male arthropod comprising the active male sterile effector is mated with a wildtype female to produce sterile males that carry the male sterile effector on a Y chromosome (or M-locus), but carry homozygous loss-of-function (LOF) mutations of a male-fertility essential gene located on an autosomal chromosome, and inviable females that are inviable due to inheriting the repressible female lethal effector from the father (left). In this system the LOF mutation can be located on any chromosome. Alternatively, the male arthropod comprising a stable male sterile effector is mated with a wildtype female to produce sterile males carrying a loss-of-function (LOF) mutation of a male-fertility essential gene located on a Y chromosome (or M-locus), and inviable females that are inviable due to inheriting the repressible female lethal effector from the father (right). In this system the male sterile effector is a LOF mutation on a Y chromosome (or M-locus) and the rescue element is a functional codon scrambled copy of the targeted male-fertility essential gene. **FIG. 2F** depicts a system where a male arthropod comprises a rescue element and a repressible female lethal effector on an m-locus, and a male sterile effector and a repressible female lethal effector on an M-locus that is active (left) or stable (right). The male arthropod comprising the active male sterile effector is mated with a wildtype female to produce sterile males that carry the male sterile effector and repressible female lethal effector on a M-locus, but carry homozygous loss-of-function (LOF) mutations of a male-fertility essential gene located on an autosomal chromosome, and inviable females that are inviable due to inheriting the repressible female lethal effector from the father (left). In this system the LOF mutation can be located on any chromosome. Alternatively, the male arthropod comprising a stable male sterile effector is mated with a wildtype female to produce sterile males carrying a repressible female lethal effector and a loss-of-function (LOF) mutation of a male-fertility essential gene located on an M-locus), and inviable females that are inviable due to inheriting the repressible female lethal effector from the father (right). In this system the male sterile effector is a LOF mutation on an M-locus and the rescue element is a functional codon scrambled copy of the targeted male-fertility essential gene on an m-locus. **FIG. 2C** depicts a system where a male arthropod is of a species with an XX/XO sex determination system and comprises a rescue element on an X chromosome (or m-locus), and a male sterile effector and repressible female lethal effector homozygous on an autosome. The rescue element can prevent the male sterile effector from generating a LOF mutation in a male-fertility essential gene, or compensate for its loss by expressing a codon-scrambled copy the male-fertility essential gene. The male arthropod is mated with a wildtype female to produce sterile males that are heterozygous for the male sterile effector, but carry homozygous loss-of-function (LOF) mutations of a male-fertility essential gene located on an autosomal chromosome, and inviable females that are inviable due to inheriting the repressible female lethal effector from the father. In this system the LOF mutation can be located on any chromosome. **FIG. 2H** depicts a system where a male arthropod comprises a male sterile effector targeting a gene required for fertility prefertilization (left) or post-fertilization (right). The male arthropod comprising the active male sterile effector is mated with a wildtype female to produce no progeny (left) or inviable progeny (right). **FIG. 2I** depicts a system where a male arthropod comprises a male sterile effector that is an X-shredding system targeting high copy number sequences on the X-chromosome (left) or an X-poisoning system targeting loss-of-function mutation of an X-linked haploinsufficient essential gene (right). The male arthropod comprising the male sterile effector is mated with a wildtype female to produce male biased progeny (left) or progeny comprising inviable females (right).

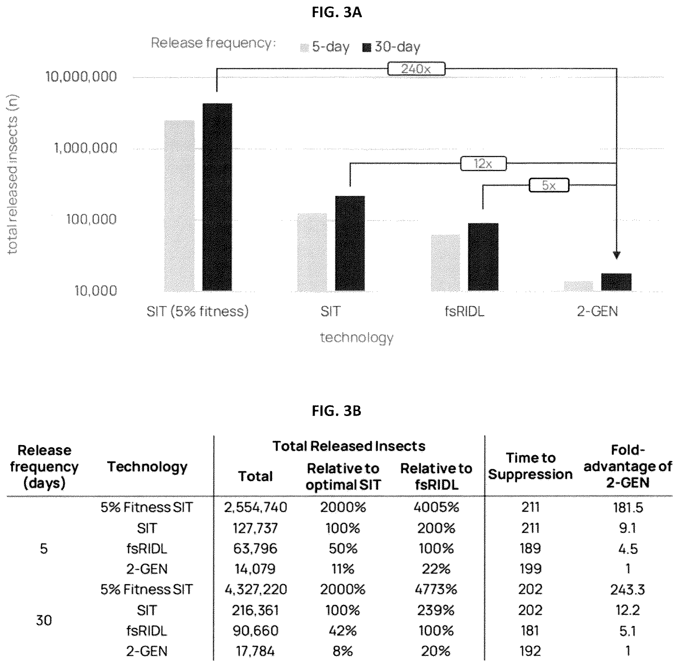

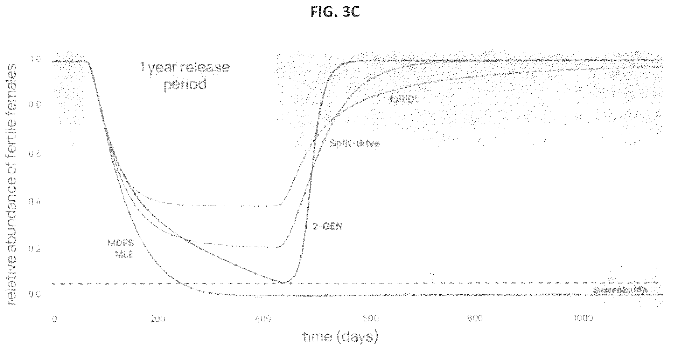

**FIG. 3A, FIG. 3B****,** and **FIG. 3C** depict modeling predictions of the 2GEN system as compared to sterile insect technique (SIT) and female-specific release of insects carrying a dominant lethal (fsRIDL). Each of **FIG. 3A, FIG. 3B****,** and **FIG. 3C** demonstrates the advantages of 2GEN over SIT and fsRIDL regarding the total number of released insects. **FIG. 3B** **and** **FIG. 3C** demonstrate the advantages of 2GEN over SIT and fsRIDL regarding speed of suppression. **FIG. 3B** demonstrates there is a reduced cost for 2GEN compared to SIT and fsRIDL based on less frequent releases. **FIG. 3C** demonstrates the dynamics of suppression, population recovery, and environmental persistence for various systems. Unless otherwise indicated, all simulations assume idealized versions of each technology with full male fitness and full penetrance of sterile and lethal trades. MDFS refers to male-drive female-sterile (alternatively known as Release of Insects carrying a Dominant-sterile Drive, RIDD) that is a homing-based gene drive designed to generate dominant female sterility; MLE refers to a male-linked editor (alternatively known as a Y-linked editor, YLE) that is a nuclease inserted onto the Y-chromosome or M-locus and designed to generate null mutations in an essential haploinsufficient gene; Split-drive refers to a homing gene drive split across two separate loci, including a Cas9 component, and a gRNA component that is inserted in its own recognition sequence to disrupt an essential haplosufficient female fertility gene.

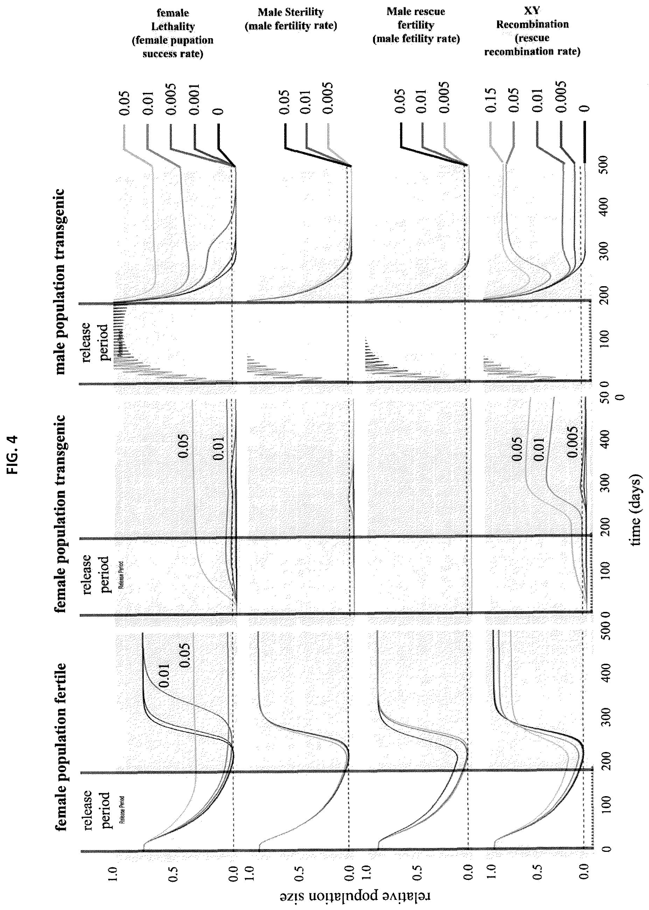

**FIG. 4** depicts modeling simulations of the 2GEN system. A sensitivity analysis is performed to determine the impact on fertile female population (left column), transgenic females (middle column) and transgenic males (right column) for suboptimal rates of female lethality (top row, female pupation success rate 0 to 0.05), male sterility (second row, male fertility rate 0 to 0.05), male fertility in presence of the rescue (third row, male fertility rate 0.7 to 1), and recombination between X/m-locus and Y/M-locus (bottom row, recombination rate 0 to 0.15). In each case only one parameter is changed at a time. Unless indicated, all other parameter values are kept constant, assuming a haplosufficient male fertility gene (Class I), no resistant allele generation, full female lethality caused by the repressible female lethal effector, full male sterility caused by the male sterile effector, full male fertility provided by rescue, a release rate of 0.55 relative to the starting natural population of males, a 6 month release period, and a 7-day release frequency. Parameters are chosen on the basis of a 95% population suppression target that could be achieved using an optimal 2GEN system in which all parameters are set to the maximum. A 95% population suppression threshold is indicated by a dashed line. Simulations are performed using a custom modelling environment based on the MGDriVE framework, assuming life cycle parameters adapted for the *Aedes aegypti* mosquito.

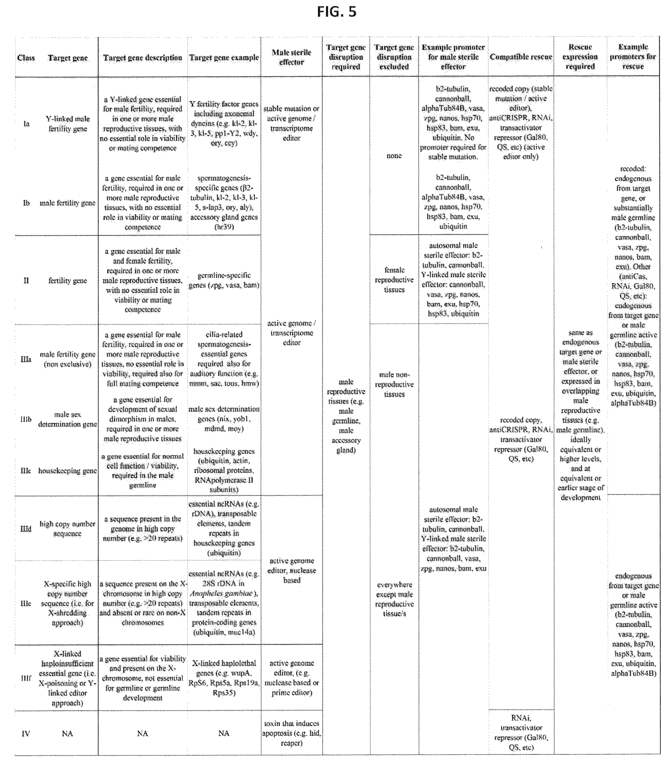

**FIG. 5** provides a table of depicting design criteria and compatibility between male sterile effectors and rescue elements.

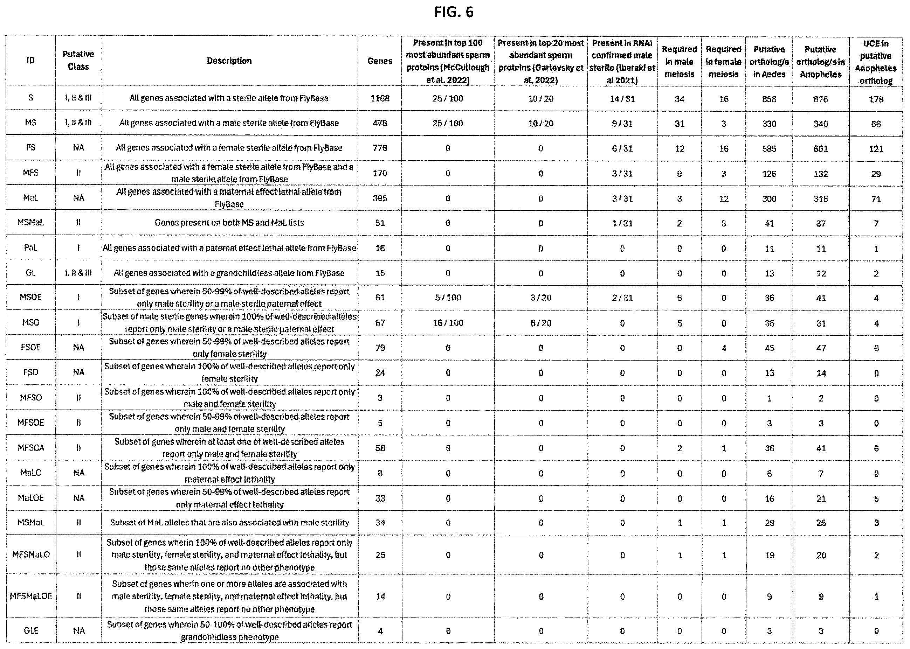

**FIG. 6** provides a table of male fertility genes in *Drosophila melanogaster* and their putative conservation in other arthropod species.

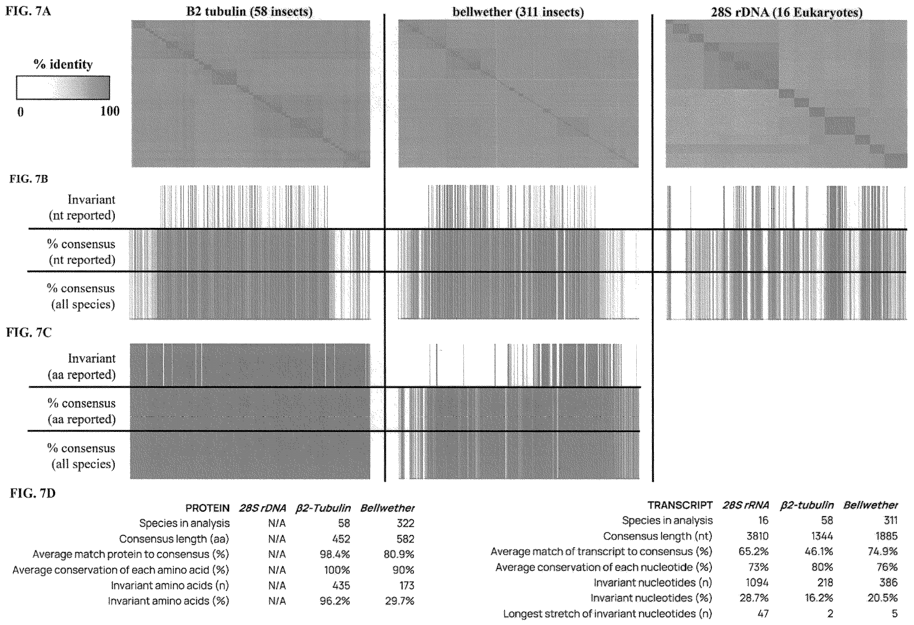

**FIGs. 7A, 7B, 7C, and 7D** depict nucleic acid and/or amino acid sequence conservation for three male sterility targets: *β2-tubulin, bellwether,* and *28S rRNA.* **FIG.** 7A depicts a percentage identity matrix showing pairwise comparisons between transcript sequences belonging to different species for each of, from left to right, *β2-tubulin, bellwether,* and *28S rRNA.* Darker shading indicates higher identity percentage. **FIG. 7B** depicts a nucleotide percentage identity matrix. **FIG. 7C** depicts an amino acid percentage identity matrix. **FIG. 7D** provides statistics regarding nucleotide and amino acid sequence conservation and invariance across the three targets.

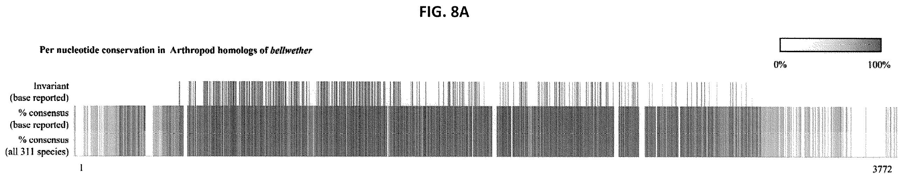

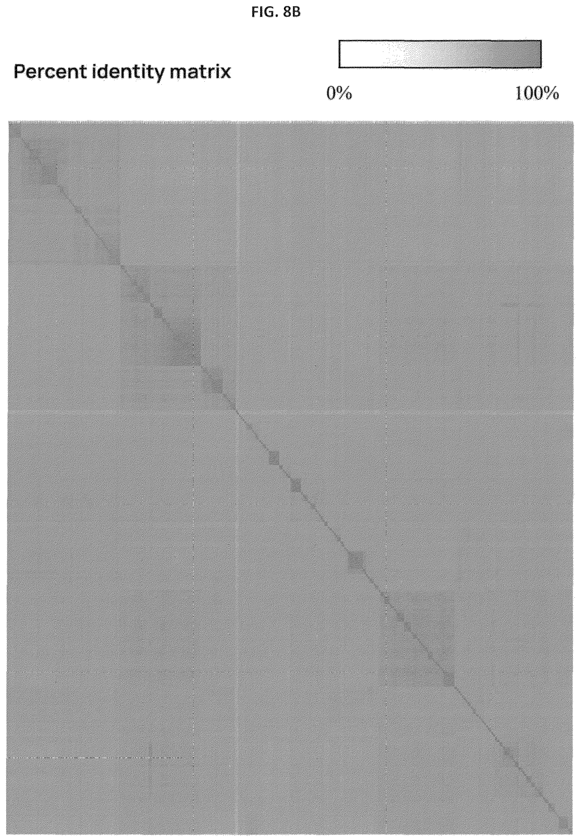

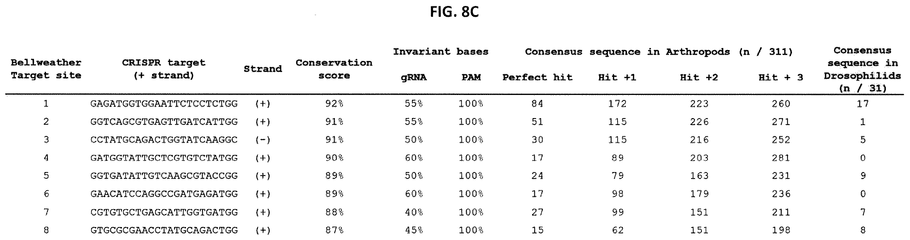

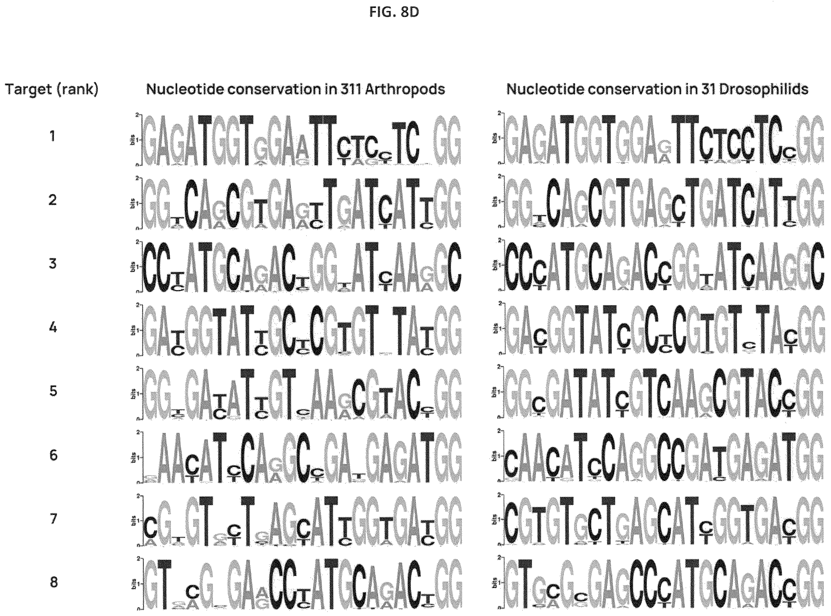

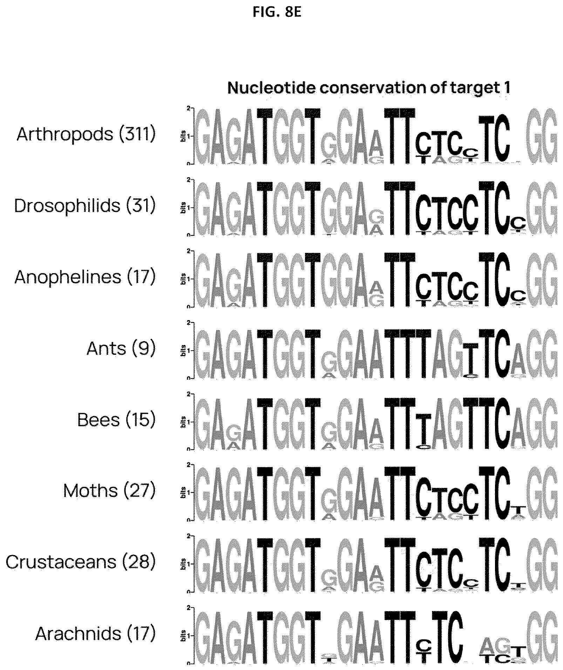

**FIGs. 8A****,** **8B****,** **8C****,** **8D****, and** **8E** depict the conservation of *bellwether* in arthropods. **FIG. 8A** depicts the per nucleotide conservation of *bellwether* transcript sequences across 311 arthropod species. An average of 76% of nucleotides match the consensus sequences across all species, including 385 invariant nucleotides (20.5%). **FIG. 8B** depicts a nucleotide percentage identity matrix for all pairwise comparisons of *bellwether* transcript sequences amongst the 311 species. Darker shading indicates greater percent identity. **FIG. 8C** depicts a table of the most conserved CRISPR target sites identified in *bellwether.* **FIG. 8D** depicts sequence logos for the eight most-conserved target sites in *bellwether* across 311 arthropod species (left) and 31 Drosophilidae species (right). Sequence conservation is represented by the height of individual nucleotides. **FIG. 8E** depicts sequence logos for the most-conserved target site in *bellwether* for different species groups. Sequence conservation is represented by the height of individual nucleotides.

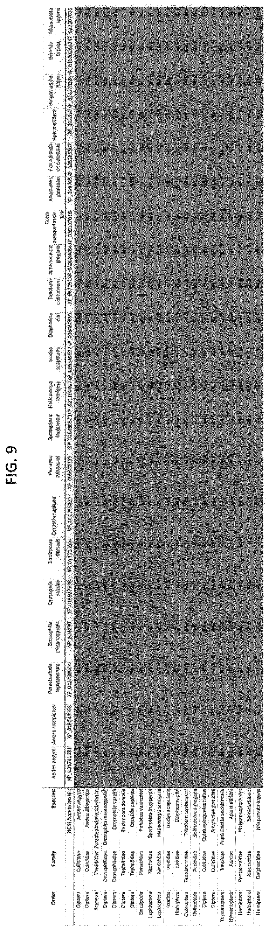

**FIG. 9** provides a table depicting a percentage identity matrix for *β2-tubulin* proteins across arthropod Orders.

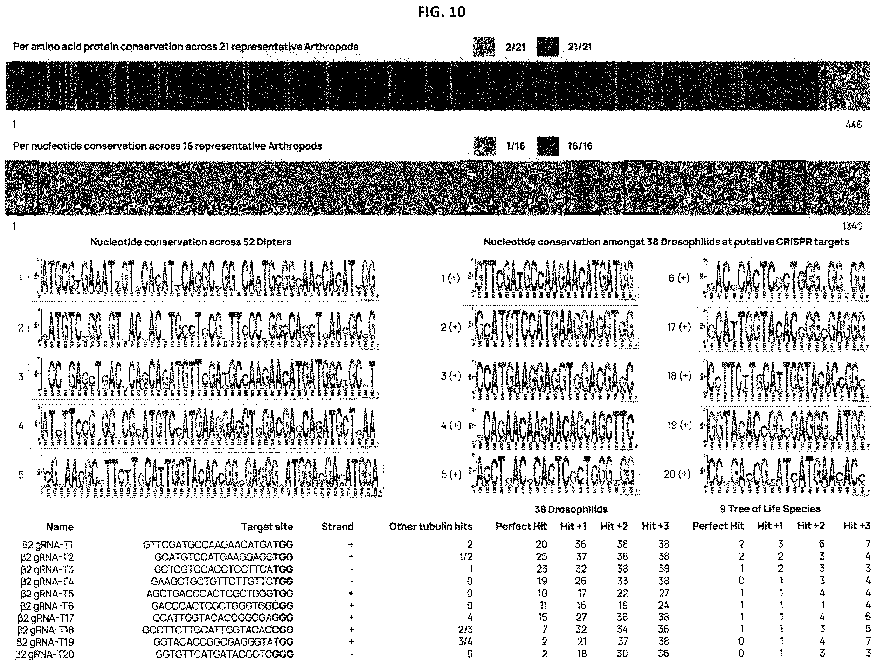

**FIG. 10** depicts amino acid and nucleic acid sequence conservation of *β2-tubulin* in arthropods. The top horizontal bar depicts per amino acid residue conservation, and the bottom horizontal bar depicts per nucleotide conservation. Darker shading indicates greater conservation (measured by calculating the fraction of sequences matching the consensus sequence at a given position). The left sequence logo depicts nucleic acid sequence conservation at five subregions of *β2-tubulin* across 52 dipteran species. The middle and right sequence logos depict nucleic acid sequence conservation amongst 38 Drosophilidae species at 10 putative CRISPR target sites. Sequence conservation is represented by the height of the individual nucleotides in all sequence logos. The table depicts gRNA target sites in Drosophila melanogaster *β2-tubulin,* conservation in other tubulin genes, conservation amongst the 38 Drosophilidae species, and finally conservation amongst *β2-tubulin* genes in nine highly divergent "Tree of Life" species, including *Aedes aegypti, Anopheles coluzii, Anopheles gambiae, Drosophila melanogaster, Drosophila suzukii, Felis catus, Mus musculus, Penaeus vannamei,* and *Plasmodium falciparum.* In each case, perfect and imperfect hits are counted whereby Hit +1 represents a match to the consensus gRNA sequence with only a single nucleotide mismatch.

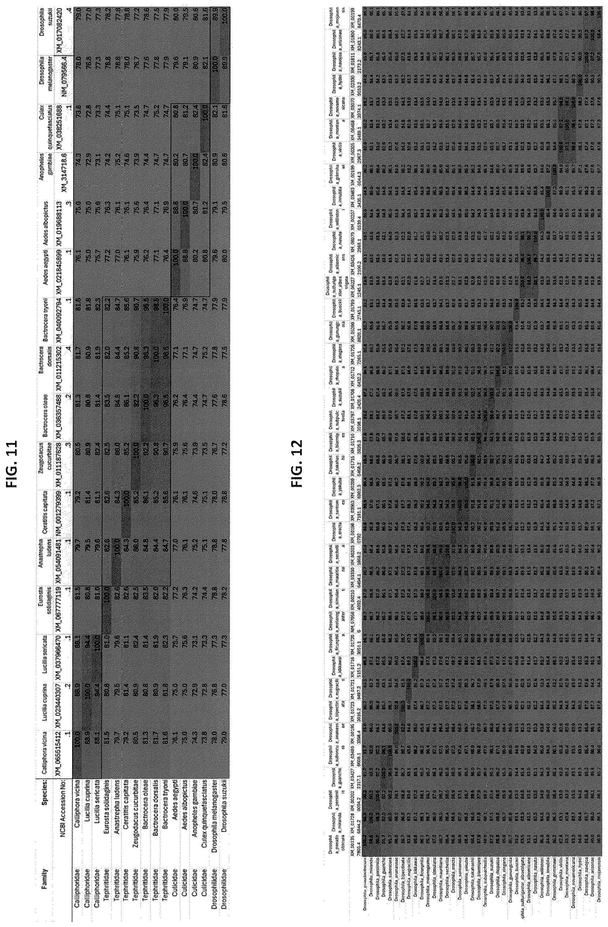

**FIG. 11** provides a table depicting percentage identity matrix for *β2-tubulin* coding (CDS) sequences from diverse Diptera insect species (Calliphoridae, Tephritidae, Culicidae, and Drosophilidae are each boxed). Throughout this disclosure, *"β2-tubulin"* and *"betaTub85D"* are used interchangeably.
**FIG. 12** provides a table depicting percentage identity matrix for *β2-tubulin* coding (CDS) sequences from Drosophilidae species.

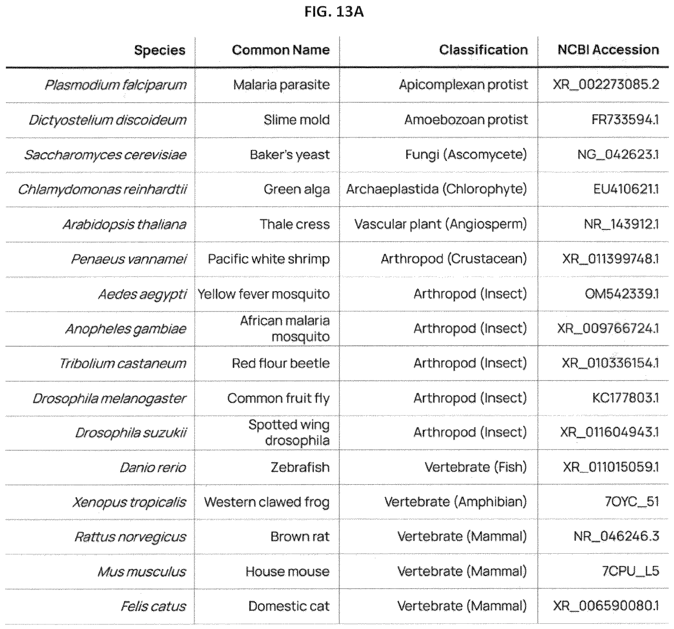

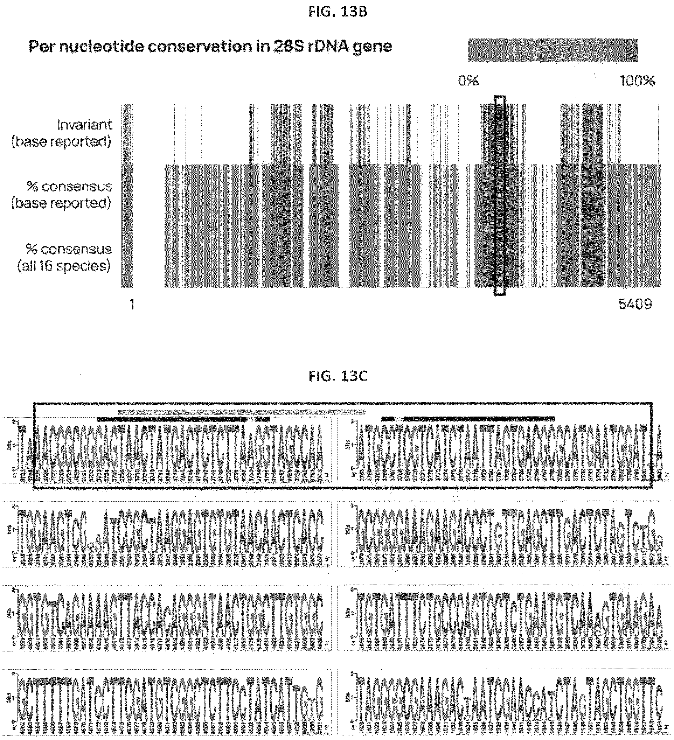

**FIGs. 13A****,** **13B, and 13C** depict the conservation of *28S rDNA* sequences across diverse taxa. **FIG. 13A** depicts a list of species and accession numbers from which *28S rDNA* sequences are obtained. **FIG. 13B** depicts a comparison of *28S rDNA* sequences against a consensus *28SrDNA* sequence. Darker shading indicates a higher percent identity. **FIG. 13C** depicts sequence logos for the most conserved regions of the *28S rDNA* gene. Sequence conservation is represented by the height of the individual nucleotides. The boxed sequence is an ultra-conserved region that comprises the longest invariant sequence identified in Eukaryotes. The bars above the sequence logo within the box indicate potential gRNA target sites.

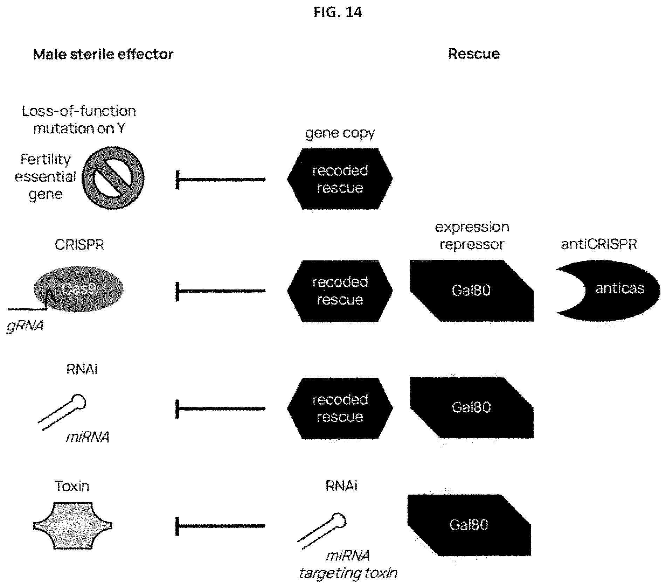

**FIG. 14** depicts non-limiting examples of rescue elements for various male sterile effectors. The figure depicts various rescue elements included a codon-scrambled (recoded) allele of a gene; RNAi that can reduce the expression or activity of a sterilitycausing target (*e.g.,* a toxin); an antiCRISPR protein that prevents a CRISPR nuclease from modifying a target sequence; and repressor proteins (*e.g*., Gal80 or QS) that can prevent transcription of a male sterile effector placed under control of a binary expression system.

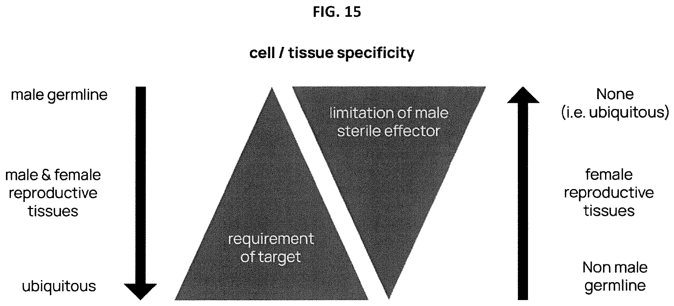

**FIG. 15** depicts the cell and tissue specificity requirements of male sterile effectors and their targets in the genome or transcriptome of an arthropod. The male sterile effector must disrupt a sequence that is essential for male fertility, in a substantial fraction of the cells in which that essential sequence is required, such as the disruption of genes required for spermatogenesis in the male germline. To avoid impacting upon other cells and tissues that may be required for viability or mating competence, expression of the male sterile effector must be restricted, depending upon the requirement of the target. If the target is required only in the germline, then there are no restrictions, however a male sterile effector targeting a gene that is required in the male and female germline should be restricted to males, and a male sterile effector targeting a housekeeping gene should be restricted to the male germline only.

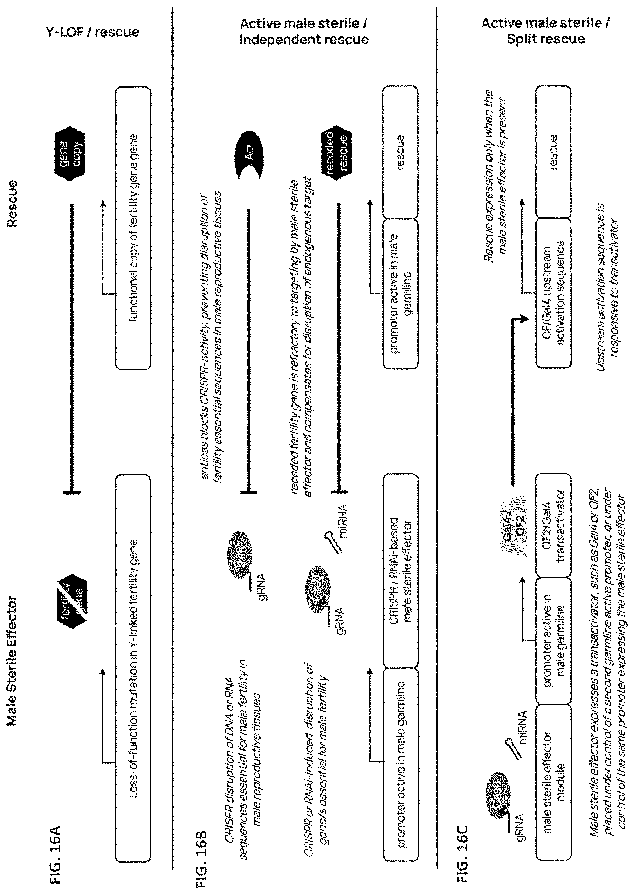

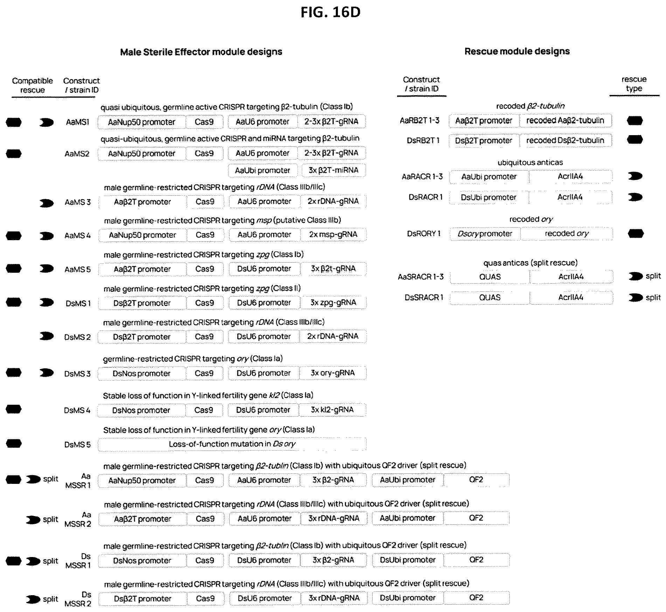

**FIGs. 16A, 16B, 16C****, and** **16D** depict non-limiting examples of male sterile effectors and rescue element designs and compatibility. **FIG. 16A** depicts that a loss of function allele in one or more essential male fertility genes on the Y chromosome can guarantee complete sterility in males, and can be rescued by providing a recoded copy of the gene or genes on the X-chromosome. A stable and fertile strain carrying a sterile allele on the Y chromosome can be created using a targeted genome editing technology (*e.g.*, CRISPR), together with the rescue on the X chromosome. **FIG. 16B** depicts that CRISPR-based effectors can be targeted to disrupt DNA or RNA sequences essential for male fertility in male reproductive tissues such as the male germline, and rescued by expressing antiCRISPR effectors such as AcrIIA4 in the same cells and tissues. Effectors can include Cas9 nuclease programmed to disrupt individual fertility genes or to generate chromosomal instability by targeting high repetitive sequences in the genome. Other CRISPR-based effectors include base editors, prime editors, epigenome editors, CRISPRi, or CRISPRa or technologies that modify the genome or transcriptome at key sequences essential for male fertility. The use of a recoded rescue further expands the list of compatible genome and transcriptome editing technologies to include RNAi (*e.g.*, miRNA, shRNA) and other targeted genome/transcriptome editors, which can be used alone or in combination with CRISPR. These technologies cannot be rescued using antiCRISPR effectors. The use of recoded rescue technology restricts the male sterile effector to those targeting genes that can be recoded, as opposed to those generating genome or transcriptome instability. **FIG. 16C** depicts that a rescue element can be split by placing it under control of an upstream activation sequence (*e.g*., UAS, QUAS) that can be activated by Gal4 or QF/QF2. To express the rescue element, the compatible driver transactivator protein (*e.g.,* Gal4, QF, QF2) is expressed in the cells and tissues where rescue expression is desired. The activator can be linked to the male sterile effector (shown in the figure), a female lethal element, or placed on the Y chromosome/M-locus. **FIG. 16D** depicts non-limiting examples of male sterile effectors and rescue elements designed for use in *Aedes aegypti* (Aa) or *Drosophila suzukii* (Ds). *See also* Example 11.

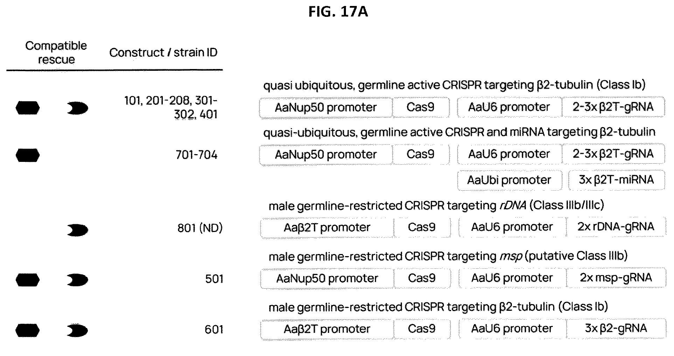

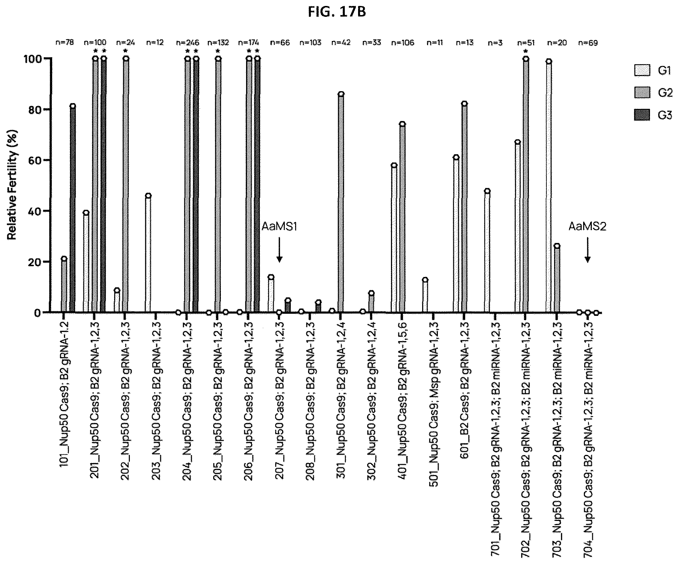

**FIGs. 17A** **and** **17B** depict evaluation and selection of male sterile strains of *Aedes aegypti.* **FIG. 17A** depicts male sterile effector constructs tested in *Aedes aegypti.* **FIG. 17B** depicts the relative percent fertility of *Aedes aegypti* males comprising the constructs depicted in **FIG. 17A** across one (G1), two (G2), or three (G3) generations.

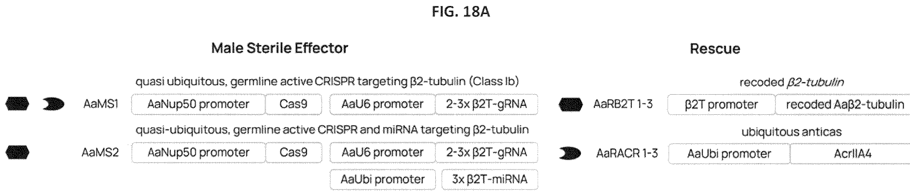

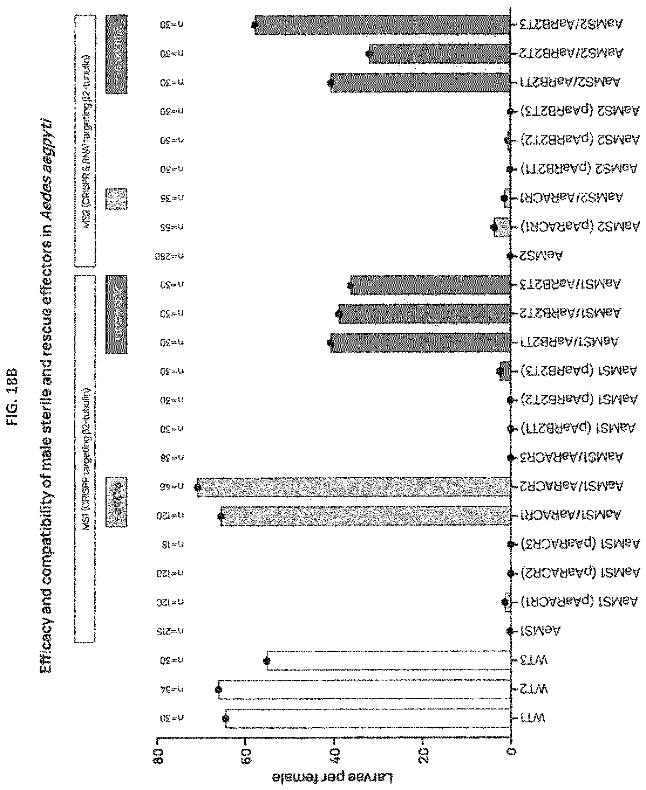

**FIGs. 18A** **and** **18B** depict evaluation of male sterility effectors and rescue elements in *Aedes aegypti.* **FIG. 18A** depicts male sterile effector and rescue element constructs tested in *Aedes aegypti.* **FIG. 18B** depicts the number of larvae per female for evaluated strains. WT refers to wildtype controls.

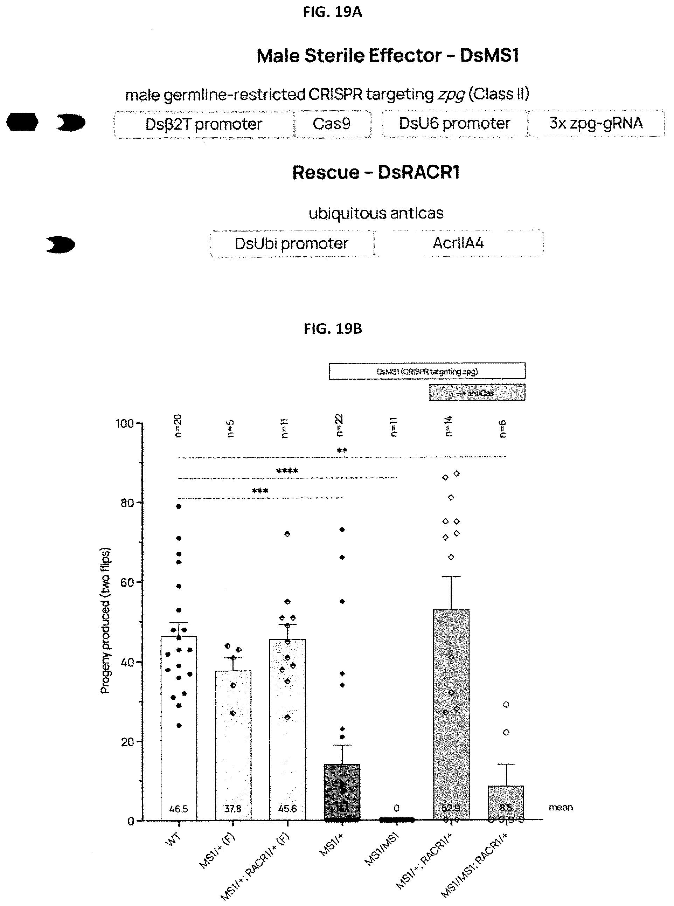

**FIGs. 19A and 19B** depict efficacy and compatibility of a male sterile effector (DsMS1) and a rescue element (DsRACR1) in modified *Drosophila suzukii.* **FIG. 19A** depicts the DsMS1 male sterile effector and the DsRACR1 rescue element. **FIG. 19B** depicts the number of progeny produced by *Drosophila suzukii* comprising various combinations of the constructs depicted in **FIG. 19B****.** WT refers to a wildtype control. Error bars represent standard error of the mean.

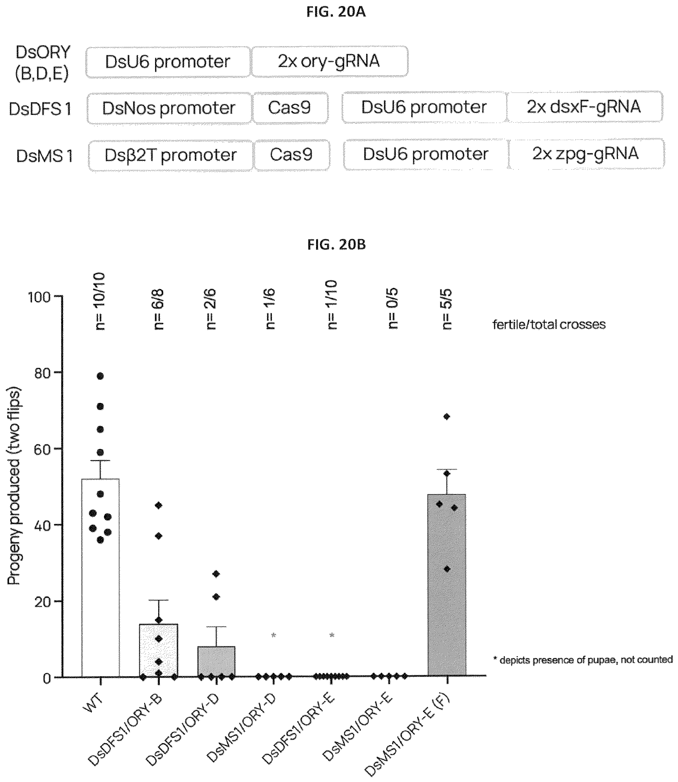

**FIGs. 20A and 20B** depict experimental validation of the Y-linked fertility gene *ory* in *Drosophila suzukii.* **FIG. 20A** depicts male sterility effector constructs tested in *Drosophila suzukii.* **FIG. 20B** depicts the number of progeny produced by *Drosophila suzukii* comprising various combinations of the constructs depicted in **FIG. 20A****.** WT refers to a wildtype control. Error bars represent standard error of the mean. The number of crosses is reported above each bar (n), representing the number of males tested (*e.g.,* n=10 corresponds to a total of 10 independent males mated to a total of 30 females in a 1:3 ratio per tube). Total adult progeny produced across the two flips ("progeny per cross") are reported for each replicate cross as individual datapoints on the graph. Female DsMS1/ORY-E and wild-type controls were set up in the same way (one male mated to three females), and comparisons across the different groups made on the basis of mean total progeny count. In each case, the sex of the transgenic fly is male unless otherwise indicated in parentheses (where (F) refers to female).

### DETAILED DESCRIPTION

Unless defined otherwise, all technical and scientific terms used have the same meaning as commonly understood by one of ordinary skill in the art to which this disclosure belongs. Where a term is provided in the singular, the inventors also contemplate aspects of the disclosure described by the plural of that term. Wherein there are discrepancies in terms and definitions used in references that are incorporated by reference, the terms used in this application shall have the definitions given herein. Other technical terms used have their ordinary meaning in the art in which they are used, as exemplified by various artspecific dictionaries, for example, "The American Heritage® Science Dictionary" (Editors of the American Heritage Dictionaries, 2011, Houghton Mifflin Harcourt, Boston and New York), the "McGraw-Hill Dictionary of Scientific and Technical Terms" (6th edition, 2002, McGraw-Hill, New York), or the "Oxford Dictionary of Biology" (6th edition, 2008, Oxford University Press, Oxford and New York).

Any references cited herein, including, *e.g*., all patents, published patent applications, and non-patent publications, are incorporated herein by reference in their entireties. The disclosure of International Patent Application PCT/EP2024/080870 is incorporated herein by reference.

When a grouping of alternatives is presented, any and all combinations of the members that make up that grouping of alternatives is specifically envisioned. For example, if an item is selected from a group consisting of A, B, C, and D, the inventors specifically envision each alternative individually (*e.g.*, A alone, B alone, etc.), as well as combinations such as A, B, and D; A and C; B and C; etc. The term "and/or" when used in a list of two or more items means any one of the listed items by itself or in combination with any one or more of the other listed items. For example, the expression "A and/or B" is intended to mean either or both of A and B - *e.g.,* A alone, B alone, or A and B in combination. The expression "A, B and/or C" is intended to mean A alone, B alone, C alone, A and B in combination, A and C in combination, B and C in combination, or A, B, and C in combination.

When a range of numbers is provided herein, the range is understood to be inclusive of the edges of the range as well as any number between the defined edges of the range. For example, "between 1 and 10" includes any number between 1 and 10, as well as the number 1 and the number 10.

As used herein, the singular forms "a," "an," and "the" include plural references unless the context clearly dictates otherwise. For example, the term "a compound" or "at least one compound" may include a plurality of compounds.

As used herein, "about" refers to ±10% when modifying a number. For example, and without being limiting, "about 100" refers to between 90 (*e.g.,* 10% less than 100) and 110 (*e.g.,* 10% greater than 100).

Each composition provided herein is specifically envisioned for use in any method provided herein.

This disclosure provides a novel system (including related compositions and methods) to suppress arthropod pests. In general, the entire system is referred to herein as the "2GEN" system because the genetic control elements integral to the system only persist for two generations upon release into the environment. The 2GEN system comprises three main components, which will be described in more detail: a male sterility effector (also referred to interchangeably herein as a male sterile effector), a repressible female-lethal effector, and a rescue element.

The 2GEN system offers a surprising advantage over sterile male and self-limited technologies, most notably, its powerful suppression potential. The perceived wisdom suggests that genetic control elements with greater transgenerational persistence will achieve greater cumulative suppression over time (*e.g.,* considering the area under the curve of suppression for a given number of released males). This trend is generally the case moving through self-limited technologies with increasing persistence and suppression potential from sterile males, to fsRIDL, to split drives, to Y-linked editors (YLE) and finally self-sustaining gene drives. Considering the total release size required to achieve strong suppression over a given period of time, the 2GEN systems is more powerful than fsRIDL and split drives.

Without being limited by any scientific theory, in order to persist in the environment, a genetic control element must not immediately sterilize or kill all carriers and thus, persistence comes at the cost of immediate suppression potential. In this respect, sterile male technologies often achieve faster suppression in the period immediately after release. Self-limited and self-sustaining technologies recover this lost potential in the subsequent generations, wherein second and third generation males continue to reduce successful reproduction by spreading sterile and lethal traits. However, the transgenerational impact wanes quickly for self-limited approaches, as a (desired) contraction in the target population also leads to a reduction in wild-borne, modified males. The next element to consider is the suppression potential of those wild-borne males. For YLE, the element is inherited by all the males and, if the YLE is fully effective, all female progeny will be inviable or sterile. For fsRIDL and split drive however, these elements quickly lose their potency as wild-borne males will be heterozygotes - unable to kill or sterilize all their daughters. Thus, the greatest impact is always in the period immediately after release, and the transgenerational effect quickly becomes redundant and, in some instances where density dependent survival is a factor, can actively promote rebounding of the pest population.

The 2GEN system has the immediate impact of killing all daughters of released males, and wild-borne modified males continue to suppress as a sterile male technology. In this respect, it has the transgenerational advantages of both fsRIDL and split drive, without losing potency, achieving faster suppression with fewer total released insects.

The 2GEN system makes use of several molecular components, including, but not limited to, male sterile effectors, repressible female-lethal effectors, and rescue elements.

In some aspects, this disclosure provides a male sterile effector. As used herein, a "male sterile effector" or a "male sterility effector" refers to any nucleic acid sequence or construct capable of imparting sterility in a male arthropod.

In some aspects, a male sterile effector comprises: (a) a first promoter operably linked to a first nucleic acid molecule encoding an RNA-guided nuclease; and (b) a second promoter operably linked to a second nucleic acid molecule encoding a guide RNA (gRNA), wherein the gRNA comprises a nucleic acid sequence at least 90% identical or complementary to at least 17 consecutive nucleotides of a first arthropod male-fertility essential gene. In some aspects, a male sterile effector further comprises: (c) a third promoter operably linked to a third nucleic acid molecule encoding a microRNA (miRNA) molecule or a short hairpin RNA (shRNA) molecule comprising a sequence at least 90% identical to at least 17 consecutive nucleotides of a second arthropod male-fertility essential gene messenger RNA. a third promoter operably linked to a third nucleic acid molecule encoding a small RNA molecule comprising a sequence at least 90% identical to at least 17 consecutive nucleotides of a second arthropod male-fertility essential gene messenger RNA. In some aspects, a male sterile effector further comprises: (c) a third promoter operably linked to a third nucleic acid molecule encoding a small RNA molecule comprising a sequence at least 80% identical to a second arthropod male-fertility essential gene messenger RNA.

In some aspects, a nucleic acid molecule encoding a gRNA encodes 1 gRNA. In some aspects, a nucleic acid molecule encoding a gRNA encodes 2 gRNAs. In some aspects, a nucleic acid molecule encoding a gRNA encodes 3 gRNAs. In some aspects, a nucleic acid molecule encoding a gRNA encodes 4 gRNAs. In some aspects, a nucleic acid molecule encoding a gRNA encodes 5 gRNAs. In some aspects, a nucleic acid molecule encoding a gRNA encodes 6 gRNAs. In some aspects, a nucleic acid molecule encoding a gRNA encodes 7 gRNAs. In some aspects, a nucleic acid molecule encoding a gRNA encodes 8 gRNAs. In some aspects, a nucleic acid molecule encoding a gRNA encodes 9 gRNAs. In some aspects, a nucleic acid molecule encoding a gRNA encodes 10 gRNAs. In some aspects, a nucleic acid molecule encoding a gRNA encodes between 1 gRNA and 35 gRNAs. In some aspects, a nucleic acid molecule encoding a gRNA encodes between 1 gRNA and 25 gRNAs. In some aspects, a nucleic acid molecule encoding a gRNA encodes between 1 gRNA and 20 gRNAs. In some aspects, a nucleic acid molecule encoding a gRNA encodes between 1 gRNA and 15 gRNAs. In some aspects, a nucleic acid molecule encoding a gRNA encodes between 1 gRNA and 10 gRNAs. In some aspects, a nucleic acid molecule encoding a gRNA encodes between 1 gRNA and 9 gRNAs. In some aspects, a nucleic acid molecule encoding a gRNA encodes between 1 gRNA and 8 gRNAs. In some aspects, a nucleic acid molecule encoding a gRNA encodes between 1 gRNA and 7 gRNAs. In some aspects, a nucleic acid molecule encoding a gRNA encodes between 1 gRNA and 6 gRNAs. In some aspects, a nucleic acid molecule encoding a gRNA encodes between 1 gRNA and 5 gRNAs. In some aspects, a nucleic acid molecule encoding a gRNA encodes between 1 gRNA and 4 gRNAs. In some aspects, a nucleic acid molecule encoding a gRNA encodes between 1 gRNA and 3 gRNAs. In some aspects, a nucleic acid molecule encoding a gRNA encodes between 2 gRNAs and 4 gRNAs. In some aspects, a nucleic acid molecule encoding a gRNA encodes between 2 gRNAs and 5 gRNAs. In some aspects, a nucleic acid molecule encoding a gRNA encodes between 2 gRNAs and 6 gRNAs. In some aspects, a nucleic acid molecule encoding a gRNA encodes between 2 gRNAs and 7 gRNAs. In some aspects, a nucleic acid molecule encoding a gRNA encodes between 2 gRNAs and 8 gRNAs. In some aspects, a nucleic acid molecule encoding a gRNA encodes between 5 gRNAs and 10 gRNAs. In some aspects, a nucleic acid molecule encoding a gRNA encodes at least 1 gRNA. In some aspects, a nucleic acid molecule encoding a gRNA encodes at least 2 gRNAs. In some aspects, a nucleic acid molecule encoding a gRNA encodes at least 3 gRNAs. In some aspects, a nucleic acid molecule encoding a gRNA encodes at least 4 gRNAs. In some aspects, a nucleic acid molecule encoding a gRNA encodes at least 5 gRNAs. In some aspects, a nucleic acid molecule encoding a gRNA encodes at least 6 gRNAs. In some aspects, a nucleic acid molecule encoding a gRNA encodes at least 7 gRNAs. In some aspects, a nucleic acid molecule encoding a gRNA encodes at least 8 gRNAs. In some aspects, a nucleic acid molecule encoding a gRNA encodes at least 9 gRNAs. In some aspects, a nucleic acid molecule encoding a gRNA encodes at least 10 gRNAs. In some aspects, a nucleic acid molecule encoding a gRNA encodes at least 20 gRNAs.

In some aspects, a nucleic acid molecule encoding multiple gRNAs encodes gRNAs that all target the same arthropod male-fertility essential gene. In some aspects, a nucleic acid molecule encoding multiple gRNAs encodes gRNAs that individually target at least two different arthropod male-fertility essential genes.

In some aspects, a male sterile effector comprises a first promoter operably linked to a first nucleic acid molecule encoding a microRNA (miRNA) molecule or a short hairpin RNA (shRNA) molecule comprising a sequence at least 90% identical or complementary to at least 17 consecutive nucleotides of a first arthropod male-fertility essential gene messenger RNA (mRNA).

As used herein, a "promoter" or a "promoter sequence" refers to a DNA sequence that contains an RNA polymerase binding site, transcription start site, initiator sequence, downstream promoter element, upstream activation sequence, and/or TATA box and assists or promotes the transcription and expression of an associated transcribable polynucleotide sequence, for example and without being limiting, a messenger RNA, a ribosomal RNA, a small RNA, a small RNA precursor, or a guide RNA.

In some aspects, a promoter comprises the nucleic acid sequence from about 250 nucleotides to about 3000 nucleotides upstream of the start codon of a gene. In some aspects, a promoter comprises the nucleic acid sequence from about 250 nucleotides to about 2000 nucleotides upstream of the start codon of a gene. In some aspects, a promoter comprises the nucleic acid sequence from about 250 nucleotides to about 1500 nucleotides upstream of the start codon of a gene. In some aspects, a promoter comprises the nucleic acid sequence from about 250 nucleotides to about 1000 nucleotides upstream of the start codon of a gene. In some aspects, a promoter comprises the nucleic acid sequence from about 250 nucleotides to about 750 nucleotides upstream of the start codon of a gene. In some aspects, a promoter comprises the nucleic acid sequence from about 250 nucleotides to about 3000 nucleotides upstream of the start codon of a gene.

Any promoter provided herein is envisioned for use with any transcribable nucleic acid sequence provided herein or with any method provided herein.

In some aspects, a promoter is a germline-preferred promoter. As used herein, a "germline-preferred promoter" refers to a promoter sequence that initiates relatively higher or preferential transcription of operably linked polynucleotides in germline tissues or cells of an arthropod. Such a promoter can, but is not required to, also drive transcription in non-germline tissues or cells at lower levels as compared to germline tissues or cells. In some aspects, a germline-preferred promoter is a male germline-preferred promoter (*e.g*., the promoter is preferentially active in male germline tissues or cells). In some aspects, a germline-preferred promoter is a female germline-preferred promoter (*e.g*., the promoter is preferentially active in female germline tissues or cells). Non-limiting examples of germline tissues or cells include testes, spermatozoa, spermatocytes, germline stem cells, hub cells, spermatogonia, spermatids, gonialblasts, ovaries, ova, oocytes, nurse cells, germarium, egg chamber, and vitellarium.

In some aspects, a germline-preferred promoter is a *β2-tubulin* promoter. In some aspects, a germline-preferred promoter is a *nanos* promoter. In some aspects, a germline-preferred promoter is an *exu* promoter. In some aspects, a germline-preferred promoter is a *zpg* promoter. In some aspects, a germline-preferred promoter is a *vasa* promoter. In some aspects, a germline-preferred promoter is selected from the group consisting of: a *nanos* promoter, a *vasa* promoter, an *exu* promoter, a *zpg* promoter and a *β2-tubulin* promoter.

In some aspects, a germline-preferred promoter comprises a nucleic acid sequence at least 80% identical to a nucleic acid sequence selected from the group consisting of SEQ ID NOs: 4250 to 4254 and 4257 to 4274, or a functional fragment thereof. In some aspects, a germline-preferred promoter comprises a nucleic acid sequence at least 85% identical to a nucleic acid sequence selected from the group consisting of SEQ ID NOs: 4250 to 4254 and 4257 to 4274, or a functional fragment thereof. In some aspects, a germline-preferred promoter comprises a nucleic acid sequence at least 90% identical to a nucleic acid sequence selected from the group consisting of SEQ ID NOs: 4250 to 4254 and 4257 to 4274, or a functional fragment thereof. In some aspects, a germline-preferred promoter comprises a nucleic acid sequence at least 91% identical to a nucleic acid sequence selected from the group consisting of SEQ ID NOs: 4250 to 4254 and 4257 to 4274, or a functional fragment thereof. In some aspects, a germline-preferred promoter comprises a nucleic acid sequence at least 92% identical to a nucleic acid sequence selected from the group consisting of SEQ ID NOs: 4250 to 4254 and 4257 to 4274, or a functional fragment thereof. In some aspects, a germline-preferred promoter comprises a nucleic acid sequence at least 93% identical to a nucleic acid sequence selected from the group consisting of SEQ ID NOs: 4250 to 4254 and 4257 to 4274, or a functional fragment thereof. In some aspects, a germline-preferred promoter comprises a nucleic acid sequence at least 94% identical to a nucleic acid sequence selected from the group consisting of SEQ ID NOs: 4250 to 4254 and 4257 to 4274, or a functional fragment thereof. In some aspects, a germline-preferred promoter comprises a nucleic acid sequence at least 95% identical to a nucleic acid sequence selected from the group consisting of SEQ ID NOs: 4250 to 4254 and 4257 to 4274, or a functional fragment thereof. In some aspects, a germline-preferred promoter comprises a nucleic acid sequence at least 96% identical to a nucleic acid sequence selected from the group consisting of SEQ ID NOs: 4250 to 4254 and 4257 to 4274, or a functional fragment thereof. In some aspects, a germline-preferred promoter comprises a nucleic acid sequence at least 97% identical to a nucleic acid sequence selected from the group consisting of SEQ ID NOs: 4250 to 4254 and 4257 to 4274, or a functional fragment thereof. In some aspects, a germline-preferred promoter comprises a nucleic acid sequence at least 98% identical to a nucleic acid sequence selected from the group consisting of SEQ ID NOs: 4250 to 4254 and 4257 to 4274, or a functional fragment thereof. In some aspects, a germline-preferred promoter comprises a nucleic acid sequence at least 99% identical to a nucleic acid sequence selected from the group consisting of SEQ ID NOs: 4250 to 4254 and 4257 to 4274, or a functional fragment thereof. In some aspects, a germline-preferred promoter comprises a nucleic acid sequence 100% identical to a nucleic acid sequence selected from the group consisting of SEQ ID NOs: 4250 to 4254 and 4257 to 4274, or a functional fragment thereof.

As used herein, a "functional fragment" of a promoter refers to a subsequence (*e.g*., at least one nucleotide shorter than the full-length sequence) of a nucleic acid sequence that is capable of initiating expression of an operably linked nucleic acid in the same spatial and temporal dimensions as the full-length promoter. In some aspects, a functional fragment of a promoter comprises the entire 5'-untranslated region of a corresponding wildtype gene.

For this paragraph, the "A" in a start codon (ATG) of a male-fertility essential gene is referred to as position 0, the "T" in the start codon is referred to as position +1, and the nucleotide positioned immediately upstream of the "A" is referred to as position -1. These positions can thus be used to describe nearby nucleotide positions. For example, positions -10 to -1 would refer to the 10 nucleotide positions immediately upstream (5') from position 0. In some aspects, a functional fragment of a promoter comprises nucleotide positions - 200 to -1 of any male-fertility essential gene. In some aspects, a functional fragment of a promoter comprises nucleotide positions -250 to -1 of any male-fertility essential gene. In some aspects, a functional fragment of a promoter comprises nucleotide positions -300 to -1 of any male-fertility essential gene. In some aspects, a functional fragment of a promoter comprises nucleotide positions -400 to -1 of any male-fertility essential gene. In some aspects, a functional fragment of a promoter comprises nucleotide positions -500 to -1 of any male-fertility essential gene. In some aspects, a functional fragment of a promoter comprises nucleotide positions -600 to -1 of any male-fertility essential gene. In some aspects, a functional fragment of a promoter comprises nucleotide positions -700 to -1 of any male-fertility essential gene. In some aspects, a functional fragment of a promoter comprises nucleotide positions -750 to -1 of any male-fertility essential gene. In some aspects, a functional fragment of a promoter comprises nucleotide positions -800 to -1 of any male-fertility essential gene. In some aspects, a functional fragment of a promoter comprises nucleotide positions -900 to -1 of any male-fertility essential gene. In some aspects, a functional fragment of a promoter comprises nucleotide positions -1000 to -1 of any male-fertility essential gene.

In some aspects, a functional fragment of a promoter comprises the 50 3'-most (*e.g.,* the last 50) nucleotides of a nucleic acid sequence selected from the group consisting of SEQ ID NOs: 4250 to 4254, 4258 to 4307, 4309 to 4312, 4386, and 6848 to 6850. In some aspects, a functional fragment of a promoter comprises the 100 3'-most (*e.g.*, the last 100) nucleotides of a nucleic acid sequence selected from the group consisting of SEQ ID NOs: 4250 to 4254, 4258 to 4307, 4309 to 4312, 4386, and 6848 to 6850. In some aspects, a functional fragment of a promoter comprises the 200 3'-most (*e.g*., the last 200) nucleotides of a nucleic acid sequence selected from the group consisting of SEQ ID NOs: 4250 to 4254, 4258 to 4307, 4309 to 4312, 4386, and 6848 to 6850. In some aspects, a functional fragment of a promoter comprises the 250 3'-most (*e.g*., the last 250) nucleotides of a nucleic acid sequence selected from the group consisting of SEQ ID NOs: 4250 to 4254, 4258 to 4307, 4309 to 4312, 4386, and 6848 to 6850. In some aspects, a functional fragment of a promoter comprises the 300 3'-most (*e.g*., the last 300) nucleotides of a nucleic acid sequence selected from the group consisting of SEQ ID NOs: 4250 to 4254, 4258 to 4307, 4309 to 4312, 4386, and 6848 to 6850. In some aspects, a functional fragment of a promoter comprises the 400 3'-most (*e.g*., the last 400) nucleotides of a nucleic acid sequence selected from the group consisting of SEQ ID NOs: 4250 to 4254, 4258 to 4307, 4309 to 4312, 4386, and 6848 to 6850. In some aspects, a functional fragment of a promoter comprises the 500 3'-most (*e.g*., the last 500) nucleotides of a nucleic acid sequence selected from the group consisting of SEQ ID NOs: 4250 to 4254, 4258 to 4307, 4309 to 4312, 4386, and 6848 to 6850. In some aspects, a functional fragment of a promoter comprises the 600 3'-most (*e.g.,* the last 600) nucleotides of a nucleic acid sequence selected from the group consisting of SEQ ID NOs: 4250 to 4254, 4258 to 4307, 4309 to 4312, 4386, and 6848 to 6850. In some aspects, a functional fragment of a promoter comprises the 700 3'-most (*e.g*., the last 700) nucleotides of a nucleic acid sequence selected from the group consisting of SEQ ID NOs: 4250 to 4254, 4258 to 4307, 4309 to 4312, 4386, and 6848 to 6850. In some aspects, a functional fragment of a promoter comprises the 750 3'-most (*e.g*., the last 750) nucleotides of a nucleic acid sequence selected from the group consisting of SEQ ID NOs: 4250 to 4254, 4258 to 4307, 4309 to 4312, 4386, and 6848 to 6850. In some aspects, a functional fragment of a promoter comprises the 800 3'-most (*e.g*., the last 800) nucleotides of a nucleic acid sequence selected from the group consisting of SEQ ID NOs: 4250 to 4254, 4258 to 4307, 4309 to 4312, 4386, and 6848 to 6850. In some aspects, a functional fragment of a promoter comprises the 900 3'-most (*e.g.,* the last 900) nucleotides of a nucleic acid sequence selected from the group consisting of SEQ ID NOs: 4250 to 4254, 4258 to 4307, 4309 to 4312, 4386, and 6848 to 6850. In some aspects, a functional fragment of a promoter comprises the 1000 3'-most (*e.g*., the last 1000) nucleotides of a nucleic acid sequence selected from the group consisting of SEQ ID NOs: 4250 to 4254, 4258 to 4307, 4309 to 4312, 4386, and 6848 to 6850.

In some aspects, a functional fragment of a promoter consists essentially of the 50 3'-most (*e.g.,* the last 50) nucleotides of a nucleic acid sequence selected from the group consisting of SEQ ID NOs: 4250 to 4254, 4258 to 4307, 4309 to 4312, 4386, and 6848 to 6850. In some aspects, a functional fragment of a promoter consists essentially of the 100 3'-most (*e.g*., the last 100) nucleotides of a nucleic acid sequence selected from the group consisting of SEQ ID NOs: 4250 to 4254, 4258 to 4307, 4309 to 4312, 4386, and 6848 to 6850. In some aspects, a functional fragment of a promoter consists essentially of the 200 3'-most (*e.g*., the last 200) nucleotides of a nucleic acid sequence selected from the group consisting of SEQ ID NOs: 4250 to 4254, 4258 to 4307, 4309 to 4312, 4386, and 6848 to 6850. In some aspects, a functional fragment of a promoter consists essentially of the 250 3'-most (*e.g.,* the last 250) nucleotides of a nucleic acid sequence selected from the group consisting of SEQ ID NOs: 4250 to 4254, 4258 to 4307, 4309 to 4312, 4386, and 6848 to 6850. In some aspects, a functional fragment of a promoter consists essentially of the 300 3'-most (*e.g.*, the last 300) nucleotides of a nucleic acid sequence selected from the group consisting of SEQ ID NOs: 4250 to 4254, 4258 to 4307, 4309 to 4312, 4386, and 6848 to 6850. In some aspects, a functional fragment of a promoter consists essentially of the 400 3'-most (*e.g*., the last 400) nucleotides of a nucleic acid sequence selected from the group consisting of SEQ ID NOs: 4250 to 4254, 4258 to 4307, 4309 to 4312, 4386, and 6848 to 6850. In some aspects, a functional fragment of a promoter consists essentially of the 500 3'-most (*e.g.,* the last 500) nucleotides of a nucleic acid sequence selected from the group consisting of SEQ ID NOs: 4250 to 4254, 4258 to 4307, 4309 to 4312, 4386, and 6848 to 6850. In some aspects, a functional fragment of a promoter consists essentially of the 600 3'-most (*e.g.,* the last 600) nucleotides of a nucleic acid sequence selected from the group consisting of SEQ ID NOs: 4250 to 4254, 4258 to 4307, 4309 to 4312, 4386, and 6848 to 6850. In some aspects, a functional fragment of a promoter consists essentially of the 700 3'-most (*e.g*., the last 700) nucleotides of a nucleic acid sequence selected from the group consisting of SEQ ID NOs: 4250 to 4254, 4258 to 4307, 4309 to 4312, 4386, and 6848 to 6850. In some aspects, a functional fragment of a promoter consists essentially of the 750 3'-most (*e.g*., the last 750) nucleotides of a nucleic acid sequence selected from the group consisting of SEQ ID NOs: 4250 to 4254, 4258 to 4307, 4309 to 4312, 4386, and 6848 to 6850. In some aspects, a functional fragment of a promoter consists essentially of the 800 3'-most (*e.g*., the last 800) nucleotides of a nucleic acid sequence selected from the group consisting of SEQ ID NOs: 4250 to 4254, 4258 to 4307, 4309 to 4312, 4386, and 6848 to 6850. In some aspects, a functional fragment of a promoter consists essentially of the 900 3'-most (*e.g*., the last 900) nucleotides of a nucleic acid sequence selected from the group consisting of SEQ ID NOs: 4250 to 4254, 4258 to 4307, 4309 to 4312, 4386, and 6848 to 6850. In some aspects, a functional fragment of a promoter consists essentially of the 1000 3'-most (*e.g*., the last 1000) nucleotides of a nucleic acid sequence selected from the group consisting of SEQ ID NOs: 4250 to 4254, 4258 to 4307, 4309 to 4312, 4386, and 6848 to 6850.

In some aspects, a promoter comprises at least a functional fragment of the endogenous promoter of a male-fertility essential gene. In some aspects, a promoter comprises the endogenous promoter of a male-fertility essential gene.

When a gene name is used to describe a promoter (*e.g., a nanos* promoter), it will be understood that this refers to the endogenous promoter of the gene. If specific promoter sequences are unknown in publicly available databases (*e.g*., FlyBase, GenBank^{®}, Eukaryotic Promoter Database (*see* Dreos et al., Nucleic Acids Research, 45:D51-D55 (2017))), identification of promoters can be performed using techniques standard in the art. *See,* for example, Solovyev et al., "Identification of Promoter Regions and Regulatory Sites." In: Ladunga, I. (eds) Computational Biology of Transcription Factor Binding. Methods in Molecular Biology, Vol. 674 (2010).

In some aspects, a promoter is a ubiquitous promoter. As used herein, a "ubiquitous promoter" refers to a promoter sequence that initiates transcription of operably linked polynucleotides in all, or nearly all, tissues or cells of an arthropod.

In some aspects, a ubiquitous promoter is a *Nup50* promoter. In some aspects, a ubiquitous promoter is a *ubiquitin* promoter. In some aspects, a ubiquitous promoter is an *hsp83* promoter. In some aspects, a ubiquitous promoter is selected from the group consisting of: a *Nup50* promoter, an *hsp83* promoter, and a *ubiquitin* promoter.

In some aspects, a ubiquitous promoter comprises a nucleic acid sequence at least 80% identical to a nucleic acid sequence selected from the group consisting of SEQ ID NOs: 4309 to 4312 and 4386, or a functional fragment thereof. In some aspects, a ubiquitous promoter comprises a nucleic acid sequence at least 85% identical to a nucleic acid sequence selected from the group consisting of SEQ ID NOs: 4309 to 4312 and 4386, or a functional fragment thereof. In some aspects, a ubiquitous promoter comprises a nucleic acid sequence at least 90% identical to a nucleic acid sequence selected from the group consisting of SEQ ID NOs: 4309 to 4312 and 4386, or a functional fragment thereof. In some aspects, a ubiquitous promoter comprises a nucleic acid sequence at least 91% identical to a nucleic acid sequence selected from the group consisting of SEQ ID NOs: 4309 to 4312 and 4386, or a functional fragment thereof. In some aspects, a ubiquitous promoter comprises a nucleic acid sequence at least 92% identical to a nucleic acid sequence selected from the group consisting of SEQ ID NOs: 4309 to 4312 and 4386, or a functional fragment thereof. In some aspects, a ubiquitous promoter comprises a nucleic acid sequence at least 93% identical to a nucleic acid sequence selected from the group consisting of SEQ ID NOs: 4309 to 4312 and 4386, or a functional fragment thereof. In some aspects, a ubiquitous promoter comprises a nucleic acid sequence at least 94% identical to a nucleic acid sequence selected from the group consisting of SEQ ID NOs: 4309 to 4312 and 4386, or a functional fragment thereof. In some aspects, a ubiquitous promoter comprises a nucleic acid sequence at least 95% identical to a nucleic acid sequence selected from the group consisting of SEQ ID NOs: 4309 to 4312 and 4386, or a functional fragment thereof. In some aspects, a ubiquitous promoter comprises a nucleic acid sequence at least 96% identical to a nucleic acid sequence selected from the group consisting of SEQ ID NOs: 4309 to 4312 and 4386, or a functional fragment thereof. In some aspects, a ubiquitous promoter comprises a nucleic acid sequence at least 97% identical to a nucleic acid sequence selected from the group consisting of SEQ ID NOs: 4309 to 4312 and 4386, or a functional fragment thereof. In some aspects, a ubiquitous promoter comprises a nucleic acid sequence at least 98% identical to a nucleic acid sequence selected from the group consisting of SEQ ID NOs: 4309 to 4312 and 4386, or a functional fragment thereof. In some aspects, a ubiquitous promoter comprises a nucleic acid sequence at least 99% identical to a nucleic acid sequence selected from the group consisting of SEQ ID NOs: 4309 to 4312 and 4386, or a functional fragment thereof. In some aspects, a ubiquitous promoter comprises a nucleic acid sequence 100% identical to a nucleic acid sequence selected from the group consisting of SEQ ID NOs: 4309 to 4312 and 4386, or a functional fragment thereof.

In some aspects, a promoter is an inducible promoter. As used herein, an "inducible promoter" refers to a promoter that initiates transcription in response to an environmental stimulus such as, without being limiting, temperature, light, and chemical application. In some aspects, an inducible promoter comprises a response element that is useful for enhancing or inducing expression of an operably linked promoter. Non-limiting examples of such response elements include UAS, QUAS, tetO, and LexO.

In some aspects, an inducible promoter comprises: (a) a response element selected from the group consisting of UAS, QUAS, tetO , and LexO; (b) *Hs70Bb;* or (c) both (a) and (b). In some aspects, an inducible promoter is *Hs70Bb.*

In some aspects, an inducible promoter comprises a nucleic acid sequence at least 80% identical to a nucleic acid sequence selected from the group consisting of SEQ ID NOs: 4275 to 4289, or a functional fragment thereof. In some aspects, an inducible promoter comprises a nucleic acid sequence at least 85% identical to a nucleic acid sequence selected from the group consisting of SEQ ID NOs: 4275 to 4289, or a functional fragment thereof. In some aspects, an inducible promoter comprises a nucleic acid sequence at least 90% identical to a nucleic acid sequence selected from the group consisting of SEQ ID NOs: 4275 to 4289, or a functional fragment thereof. In some aspects, an inducible promoter comprises a nucleic acid sequence at least 91% identical to a nucleic acid sequence selected from the group consisting of SEQ ID NOs: 4275 to 4289, or a functional fragment thereof. In some aspects, an inducible promoter comprises a nucleic acid sequence at least 92% identical to a nucleic acid sequence selected from the group consisting of SEQ ID NOs: 4275 to 4289, or a functional fragment thereof. In some aspects, an inducible promoter comprises a nucleic acid sequence at least 93% identical to a nucleic acid sequence selected from the group consisting of SEQ ID NOs: 4275 to 4289, or a functional fragment thereof. In some aspects, an inducible promoter comprises a nucleic acid sequence at least 94% identical to a nucleic acid sequence selected from the group consisting of SEQ ID NOs: 4275 to 4289, or a functional fragment thereof. In some aspects, an inducible promoter comprises a nucleic acid sequence at least 95% identical to a nucleic acid sequence selected from the group consisting of SEQ ID NOs: 4275 to 4289, or a functional fragment thereof. In some aspects, an inducible promoter comprises a nucleic acid sequence at least 96% identical to a nucleic acid sequence selected from the group consisting of SEQ ID NOs: 4275 to 4289, or a functional fragment thereof. In some aspects, an inducible promoter comprises a nucleic acid sequence at least 97% identical to a nucleic acid sequence selected from the group consisting of SEQ ID NOs: 4275 to 4289, or a functional fragment thereof. In some aspects, an inducible promoter comprises a nucleic acid sequence at least 98% identical to a nucleic acid sequence selected from the group consisting of SEQ ID NOs: 4275 to 4289, or a functional fragment thereof. In some aspects, an inducible promoter comprises a nucleic acid sequence at least 99% identical to a nucleic acid sequence selected from the group consisting of SEQ ID NOs: 4275 to 4289, or a functional fragment thereof. In some aspects, an inducible promoter comprises a nucleic acid sequence 100% identical to a nucleic acid sequence selected from the group consisting of SEQ ID NOs: 4275 to 4289, or a functional fragment thereof.

In some aspects, a promoter is selected from the group consisting of a germline-preferred promoter, a ubiquitous promoter, and an inducible promoter.

In some aspects, a promoter is selected from the group consisting of: a *β2-tubulin* promoter, an *Nup50* promoter, a *nanos* promoter, a *ubiquitin* promoter, a *vasa* promoter, and an *exu* promoter, or a functional fragment thereof.

In some aspects, a promoter is selected from the group consisting of: a *β2-tubulin* promoter, a *protamine* promoter, a *Dnah3* promoter, a *Loopin-1* promoter, an *S-Lap1* promoter, an *S-Lap2* promoter, an *S-Lap3* promoter, an *S-Lap4* promoter, an *S-Lap5* promoter, an *S-Lap7* promoter, and an *S-Lap8* promoter, or a functional fragment thereof.

In some aspects, a promoter is selected from the group consisting of: *a β2-tubulin* promoter, a *protamine* promoter, a *dnah3* promoter, a *loopin-1* promoter, an *s-lap1* promoter, an *s-lap2* promoter, an *s-lap3* promoter, an *s-lap4* promoter, an *s-lap5* promoter, an *s-lap7* promoter, an *s-lap8* promoter, a *vasa* promoter, a *nanos* promoter, an *exu* promoter, a *fas3* promoter, a *squid* promoter, a *belle* promoter, a *geminin* promoter, an *sds3* promoter, a *rαcGAP1* promoter, an *aurora kinase* promoter, a *shu* promoter, a *zpg* promoter, an *argonaute 3* promoter, an *aubergine* promoter, an *eif4E-5* promoter, a *bgcn* promoter, a *bam* promoter, a *piwi* promoter, a *pumilio* promoter and a *spol* promoter, or a functional fragment thereof.

In some aspects, a promoter is selected from the group consisting of: a *β2-tubulin* promoter, an *exu* promoter, a *nanos* promoter, and a *vasa* promoter, or a functional fragment thereof. In some aspects, a promoter is selected from the group consisting of: a *U6* promoter, a *7SK* promoter, a *U3* promoter, an *H1* promoter, a *ubiquitin* promoter, and a *tRNA* promoter, or a functional fragment thereof.

In some aspects, a *β2-tubulin* promoter comprises a nucleic acid sequence at least 80% identical to a nucleic acid sequence selected from the group consisting of SEQ ID NOs: 4271 to 4274, or a functional fragment thereof. In some aspects, a *β2-tubulin* promoter comprises a nucleic acid sequence at least 85% identical to a nucleic acid sequence selected from the group consisting of SEQ ID NOs: 4271 to 4274, or a functional fragment thereof. In some aspects, a *β2-tubulin* promoter comprises a nucleic acid sequence at least 90% identical to a nucleic acid sequence selected from the group consisting of SEQ ID NOs: 4271 to 4274, or a functional fragment thereof. In some aspects, a *β2-tubulin* promoter comprises a nucleic acid sequence at least 91% identical to a nucleic acid sequence selected from the group consisting of SEQ ID NOs: 4271 to 4274, or a functional fragment thereof. In some aspects, a *β2-tubulin* promoter comprises a nucleic acid sequence at least 92% identical to a nucleic acid sequence selected from the group consisting of SEQ ID NOs: 4271 to 4274, or a functional fragment thereof. In some aspects, a *β2-tubulin* promoter comprises a nucleic acid sequence at least 93% identical to a nucleic acid sequence selected from the group consisting of SEQ ID NOs: 4271 to 4274, or a functional fragment thereof. In some aspects, a *β2-tubulin* promoter comprises a nucleic acid sequence at least 94% identical to a nucleic acid sequence selected from the group consisting of SEQ ID NOs: 4271 to 4274, or a functional fragment thereof. In some aspects, a *β2-tubulin* promoter comprises a nucleic acid sequence at least 95% identical to a nucleic acid sequence selected from the group consisting of SEQ ID NOs: 4271 to 4274, or a functional fragment thereof. In some aspects, a *β2-tubulin* promoter comprises a nucleic acid sequence at least 96% identical to a nucleic acid sequence selected from the group consisting of SEQ ID NOs: 4271 to 4274, or a functional fragment thereof. In some aspects, a *β2-tubulin* promoter comprises a nucleic acid sequence at least 97% identical to a nucleic acid sequence selected from the group consisting of SEQ ID NOs: 4271 to 4274, or a functional fragment thereof. In some aspects, a *β2-tubulin* promoter comprises a nucleic acid sequence at least 98% identical to a nucleic acid sequence selected from the group consisting of SEQ ID NOs: 4271 to 4274, or a functional fragment thereof. In some aspects, a *β2-tubulin* promoter comprises a nucleic acid sequence at least 99% identical to a nucleic acid sequence selected from the group consisting of SEQ ID NOs: 4271 to 4274, or a functional fragment thereof. In some aspects, a *β2-tubulin* promoter comprises a nucleic acid sequence 100% identical to a nucleic acid sequence selected from the group consisting of SEQ ID NOs: 4271 to 4274, or a functional fragment thereof.

In some aspects, a *Nup50* promoter comprises a nucleic acid sequence at least 80% identical to the nucleic acid sequence of SEQ ID NO: 4310, or a functional fragment thereof. In some aspects, a *Nup50* promoter comprises a nucleic acid sequence at least 85% identical to the nucleic acid sequence of SEQ ID NO: 4310, or a functional fragment thereof. In some aspects, a *Nup50* promoter comprises a nucleic acid sequence at least 90% identical to the nucleic acid sequence of SEQ ID NO: 4310, or a functional fragment thereof. In some aspects, a *Nup50* promoter comprises a nucleic acid sequence at least 91% identical to the nucleic acid sequence of SEQ ID NO: 4310, or a functional fragment thereof. In some aspects, a *Nup50* promoter comprises a nucleic acid sequence at least 92% identical to the nucleic acid sequence of SEQ ID NO: 4310, or a functional fragment thereof. In some aspects, a *Nup50* promoter comprises a nucleic acid sequence at least 93% identical to the nucleic acid sequence of SEQ ID NO: 4310, or a functional fragment thereof. In some aspects, a *Nup50* promoter comprises a nucleic acid sequence at least 94% identical to the nucleic acid sequence of SEQ ID NO: 4310, or a functional fragment thereof. In some aspects, a *Nup50* promoter comprises a nucleic acid sequence at least 95% identical to the nucleic acid sequence of SEQ ID NO: 4310, or a functional fragment thereof. In some aspects, a *Nup50* promoter comprises a nucleic acid sequence at least 96% identical to the nucleic acid sequence of SEQ ID NO: 4310, or a functional fragment thereof. In some aspects, a *Nup50* promoter comprises a nucleic acid sequence at least 97% identical to the nucleic acid sequence of SEQ ID NO: 4310, or a functional fragment thereof. In some aspects, a *Nup50* promoter comprises a nucleic acid sequence at least 98% identical to the nucleic acid sequence of SEQ ID NO: 4310, or a functional fragment thereof. In some aspects, a *Nup50* promoter comprises a nucleic acid sequence at least 99% identical to the nucleic acid sequence of SEQ ID NO: 4310, or a functional fragment thereof. In some aspects, a *Nup50* promoter comprises a nucleic acid sequence 100% identical to the nucleic acid sequence of SEQ ID NO: 4310, or a functional fragment thereof.

In some aspects, a *U6* promoter comprises a nucleic acid sequence at least 80% identical to a nucleic acid sequence selected from the group consisting of SEQ ID NOs: 4295 to 4307, or a functional fragment thereof. In some aspects, a *U6* promoter comprises a nucleic acid sequence at least 85% identical to a nucleic acid sequence selected from the group consisting of SEQ ID NOs: 4295 to 4307, or a functional fragment thereof. In some aspects, a *U6* promoter comprises a nucleic acid sequence at least 90% identical to a nucleic acid sequence selected from the group consisting of SEQ ID NOs: 4295 to 4307, or a functional fragment thereof. In some aspects, a *U6* promoter comprises a nucleic acid sequence at least 91% identical to a nucleic acid sequence selected from the group consisting of SEQ ID NOs: 4295 to 4307, or a functional fragment thereof. In some aspects, a *U6* promoter comprises a nucleic acid sequence at least 92% identical to a nucleic acid sequence selected from the group consisting of SEQ ID NOs: 4295 to 4307, or a functional fragment thereof. In some aspects, a *U6* promoter comprises a nucleic acid sequence at least 93% identical to a nucleic acid sequence selected from the group consisting of SEQ ID NOs: 4295 to 4307, or a functional fragment thereof. In some aspects, a *U6* promoter comprises a nucleic acid sequence at least 94% identical to a nucleic acid sequence selected from the group consisting of SEQ ID NOs: 4295 to 4307, or a functional fragment thereof. In some aspects, a *U6* promoter comprises a nucleic acid sequence at least 95% identical to a nucleic acid sequence selected from the group consisting of SEQ ID NOs: 4295 to 4307, or a functional fragment thereof. In some aspects, a *U6* promoter comprises a nucleic acid sequence at least 96% identical to a nucleic acid sequence selected from the group consisting of SEQ ID NOs: 4295 to 4307, or a functional fragment thereof. In some aspects, a *U6* promoter comprises a nucleic acid sequence at least 97% identical to a nucleic acid sequence selected from the group consisting of SEQ ID NOs: 4295 to 4307, or a functional fragment thereof. In some aspects, a *U6* promoter comprises a nucleic acid sequence at least 98% identical to a nucleic acid sequence selected from the group consisting of SEQ ID NOs: 4295 to 4307, or a functional fragment thereof. In some aspects, a *U6* promoter comprises a nucleic acid sequence at least 99% identical to a nucleic acid sequence selected from the group consisting of SEQ ID NOs: 4295 to 4307, or a functional fragment thereof. In some aspects, a *U6* promoter comprises a nucleic acid sequence 100% identical to a nucleic acid sequence selected from the group consisting of SEQ ID NOs: 4295 to 4307, or a functional fragment thereof.

In some aspects, a *nanos* promoter comprises a nucleic acid sequence at least 80% identical to a nucleic acid sequence selected from the group consisting of SEQ ID NOs: 4253, 4254, and 4258, or a functional fragment thereof. In some aspects, a *nanos* promoter comprises a nucleic acid sequence at least 85% identical to a nucleic acid sequence selected from the group consisting of SEQ ID NOs: 4253, 4254, and 4258, or a functional fragment thereof. In some aspects, a *nanos* promoter comprises a nucleic acid sequence at least 90% identical to a nucleic acid sequence selected from the group consisting of SEQ ID NOs: 4253, 4254, and 4258, or a functional fragment thereof. In some aspects, a *nanos* promoter comprises a nucleic acid sequence at least 91% identical to a nucleic acid sequence selected from the group consisting of SEQ ID NOs: 4253, 4254, and 4258, or a functional fragment thereof. In some aspects, a *nanos* promoter comprises a nucleic acid sequence at least 92% identical to a nucleic acid sequence selected from the group consisting of SEQ ID NOs: 4253, 4254, and 4258, or a functional fragment thereof. In some aspects, a *nanos* promoter comprises a nucleic acid sequence at least 93% identical to a nucleic acid sequence selected from the group consisting of SEQ ID NOs: 4253, 4254, and 4258, or a functional fragment thereof. In some aspects, a *nanos* promoter comprises a nucleic acid sequence at least 94% identical to a nucleic acid sequence selected from the group consisting of SEQ ID NOs: 4253, 4254, and 4258, or a functional fragment thereof. In some aspects, a *nanos* promoter comprises a nucleic acid sequence at least 95% identical to a nucleic acid sequence selected from the group consisting of SEQ ID NOs: 4253, 4254, and 4258, or a functional fragment thereof. In some aspects, a *nanos* promoter comprises a nucleic acid sequence at least 96% identical to a nucleic acid sequence selected from the group consisting of SEQ ID NOs: 4253, 4254, and 4258, or a functional fragment thereof. In some aspects, a *nanos* promoter comprises a nucleic acid sequence at least 97% identical to a nucleic acid sequence selected from the group consisting of SEQ ID NOs: 4253, 4254, and 4258, or a functional fragment thereof. In some aspects, a *nanos* promoter comprises a nucleic acid sequence at least 98% identical to a nucleic acid sequence selected from the group consisting of SEQ ID NOs: 4253, 4254, and 4258, or a functional fragment thereof. In some aspects, a *nanos* promoter comprises a nucleic acid sequence at least 99% identical to a nucleic acid sequence selected from the group consisting of SEQ ID NOs: 4253, 4254, and 4258, or a functional fragment thereof. In some aspects, a *nanos* promoter comprises a nucleic acid sequence 100% identical to a nucleic acid sequence selected from the group consisting of SEQ ID NOs: 4253, 4254, and 4258, or a functional fragment thereof.

In some aspects, an *exu* promoter comprises a nucleic acid sequence at least 80% identical to a nucleic acid sequence selected from the group consisting of SEQ ID NOs: 4251 and 4252, or a functional fragment thereof. In some aspects, an *exu* promoter comprises a nucleic acid sequence at least 85% identical to a nucleic acid sequence selected from the group consisting of SEQ ID NOs: 4251 and 4252, or a functional fragment thereof. In some aspects, an *exu* promoter comprises a nucleic acid sequence at least 90% identical to a nucleic acid sequence selected from the group consisting of SEQ ID NOs: 4251 and 4252, or a functional fragment thereof. In some aspects, an *exu* promoter comprises a nucleic acid sequence at least 91% identical to a nucleic acid sequence selected from the group consisting of SEQ ID NOs: 4251 and 4252, or a functional fragment thereof. In some aspects, an *exu* promoter comprises a nucleic acid sequence at least 92% identical to a nucleic acid sequence selected from the group consisting of SEQ ID NOs: 4251 and 4252, or a functional fragment thereof. In some aspects, an *exu* promoter comprises a nucleic acid sequence at least 93% identical to a nucleic acid sequence selected from the group consisting of SEQ ID NOs: 4251 and 4252, or a functional fragment thereof. In some aspects, an *exu* promoter comprises a nucleic acid sequence at least 94% identical to a nucleic acid sequence selected from the group consisting of SEQ ID NOs: 4251 and 4252, or a functional fragment thereof. In some aspects, an *exu* promoter comprises a nucleic acid sequence at least 95% identical to a nucleic acid sequence selected from the group consisting of SEQ ID NOs: 4251 and 4252, or a functional fragment thereof. In some aspects, an *exu* promoter comprises a nucleic acid sequence at least 96% identical to a nucleic acid sequence selected from the group consisting of SEQ ID NOs: 4251 and 4252, or a functional fragment thereof. In some aspects, an *exu* promoter comprises a nucleic acid sequence at least 97% identical to a nucleic acid sequence selected from the group consisting of SEQ ID NOs: 4251 and 4252, or a functional fragment thereof. In some aspects, an *exu* promoter comprises a nucleic acid sequence at least 98% identical to a nucleic acid sequence selected from the group consisting of SEQ ID NOs: 4251 and 4252, or a functional fragment thereof. In some aspects, an *exu* promoter comprises a nucleic acid sequence at least 99% identical to a nucleic acid sequence selected from the group consisting of SEQ ID NOs: 4251 and 4252, or a functional fragment thereof. In some aspects, an *exu* promoter comprises a nucleic acid sequence 100% identical to a nucleic acid sequence selected from the group consisting of SEQ ID NOs: 4251 and 4252, or a functional fragment thereof.

In some aspects, an *hsp83* promoter comprises a nucleic acid sequence at least 80% identical to the nucleic acid sequence of SEQ ID NO: 4309, or a functional fragment thereof. In some aspects, an *hsp83* promoter comprises a nucleic acid sequence at least 85% identical to the nucleic acid sequence of SEQ ID NO: 4309, or a functional fragment thereof. In some aspects, an *hsp83* promoter comprises a nucleic acid sequence at least 90% identical to the nucleic acid sequence of SEQ ID NO: 4309, or a functional fragment thereof. In some aspects, an *hsp83* promoter comprises a nucleic acid sequence at least 91% identical to the nucleic acid sequence of SEQ ID NO: 4309, or a functional fragment thereof. In some aspects, an *hsp83* promoter comprises a nucleic acid sequence at least 92% identical to the nucleic acid sequence of SEQ ID NO: 4309, or a functional fragment thereof. In some aspects, an *hsp83* promoter comprises a nucleic acid sequence at least 93% identical to the nucleic acid sequence of SEQ ID NO: 4309, or a functional fragment thereof. In some aspects, an *hsp83* promoter comprises a nucleic acid sequence at least 94% identical to the nucleic acid sequence of SEQ ID NO: 4309, or a functional fragment thereof. In some aspects, an *hsp83* promoter comprises a nucleic acid sequence at least 95% identical to the nucleic acid sequence of SEQ ID NO: 4309, or a functional fragment thereof. In some aspects, an *hsp83* promoter comprises a nucleic acid sequence at least 96% identical to the nucleic acid sequence of SEQ ID NO: 4309 or a functional fragment thereof. In some aspects, an *hsp83* promoter comprises a nucleic acid sequence at least 97% identical to the nucleic acid sequence of SEQ ID NO: 4309, or a functional fragment thereof. In some aspects, an *hsp83* promoter comprises a nucleic acid sequence at least 98% identical to the nucleic acid sequence of SEQ ID NO: 4309, or a functional fragment thereof. In some aspects, an *hsp83* promoter comprises a nucleic acid sequence at least 99% identical to the nucleic acid sequence of SEQ ID NO: 4309, or a functional fragment thereof. In some aspects, an *hsp83* promoter comprises a nucleic acid sequence 100% identical to the nucleic acid sequence of SEQ ID NO: 4309, or a functional fragment thereof.

In some aspects, a *vasa* promoter comprises a nucleic acid sequence at least 80% identical to a nucleic acid sequence selected from the group consisting of SEQ ID NOs: 4263 and 4267, or a functional fragment thereof. In some aspects, a *vasa* promoter comprises a nucleic acid sequence at least 85% identical to a nucleic acid sequence selected from the group consisting of SEQ ID NOs: 4263 and 4267, or a functional fragment thereof. In some aspects, a *vasa* promoter comprises a nucleic acid sequence at least 90% identical to a nucleic acid sequence selected from the group consisting of SEQ ID NOs: 4263 and 4267, or a functional fragment thereof. In some aspects, a *vasa* promoter comprises a nucleic acid sequence at least 91% identical to a nucleic acid sequence selected from the group consisting of SEQ ID NOs: 4263 and 4267, or a functional fragment thereof. In some aspects, a *vasa* promoter comprises a nucleic acid sequence at least 92% identical to a nucleic acid sequence selected from the group consisting of SEQ ID NOs: 4263 and 4267, or a functional fragment thereof. In some aspects, a *vasa* promoter comprises a nucleic acid sequence at least 93% identical to a nucleic acid sequence selected from the group consisting of SEQ ID NOs: 4263 and 4267, or a functional fragment thereof. In some aspects, a *vasa* promoter comprises a nucleic acid sequence at least 94% identical to a nucleic acid sequence selected from the group consisting of SEQ ID NOs: 4263 and 4267, or a functional fragment thereof. In some aspects, a *vasa* promoter comprises a nucleic acid sequence at least 95% identical to a nucleic acid sequence selected from the group consisting of SEQ ID NOs: 4263 and 4267, or a functional fragment thereof. In some aspects, a *vasa* promoter comprises a nucleic acid sequence at least 96% identical to a nucleic acid sequence selected from the group consisting of SEQ ID NOs: 4263 and 4267, or a functional fragment thereof. In some aspects, a *vasa* promoter comprises a nucleic acid sequence at least 97% identical to a nucleic acid sequence selected from the group consisting of SEQ ID NOs: 4263 and 4267, or a functional fragment thereof. In some aspects, a *vasa* promoter comprises a nucleic acid sequence at least 98% identical to a nucleic acid sequence selected from the group consisting of SEQ ID NOs: 4263 and 4267, or a functional fragment thereof. In some aspects, a *vasa* promoter comprises a nucleic acid sequence at least 99% identical to a nucleic acid sequence selected from the group consisting of SEQ ID NOs: 4263 and 4267, or a functional fragment thereof. In some aspects, a *vasa* promoter comprises a nucleic acid sequence 100% identical to a nucleic acid sequence selected from the group consisting of SEQ ID NOs: 4263 and 4267, or a functional fragment thereof.

In some aspects, a *7SK* promoter comprises a nucleic acid sequence at least 80% identical to the nucleic acid sequence of SEQ ID NO: 4293 or 4294. In some aspects, a *7SK* promoter comprises a nucleic acid sequence at least 85% identical to the nucleic acid sequence of SEQ ID NO: 4293 or 4294. In some aspects, a *7SK* promoter comprises a nucleic acid sequence at least 90% identical to the nucleic acid sequence of SEQ ID NO: 4293 or 4294. In some aspects, a *7SK* promoter comprises a nucleic acid sequence at least 91% identical to the nucleic acid sequence of SEQ ID NO: 4293 or 4294. In some aspects, a *7SK* promoter comprises a nucleic acid sequence at least 92% identical to the nucleic acid sequence of SEQ ID NO: 4293 or 4294. In some aspects, a *7SK* promoter comprises a nucleic acid sequence at least 93% identical to the nucleic acid sequence of SEQ ID NO: 4293 or 4294. In some aspects, a *7SK* promoter comprises a nucleic acid sequence at least 94% identical to the nucleic acid sequence of SEQ ID NO: 4293 or 4294. In some aspects, a *7SK* promoter comprises a nucleic acid sequence at least 95% identical to the nucleic acid sequence of SEQ ID NO: 4293 or 4294. In some aspects, a *7SK* promoter comprises a nucleic acid sequence at least 96% identical to the nucleic acid sequence of SEQ ID NO: 4293 or 4294. In some aspects, a *7SK* promoter comprises a nucleic acid sequence at least 97% identical to the nucleic acid sequence of SEQ ID NO: 4293 or 4294. In some aspects, a *7SK* promoter comprises a nucleic acid sequence at least 98% identical to the nucleic acid sequence of SEQ ID NO: 4293 or 4294. In some aspects, a *7SK* promoter comprises a nucleic acid sequence at least 99% identical to the nucleic acid sequence of SEQ ID NO: 4293 or 4294. In some aspects, a *7SK* promoter comprises a nucleic acid sequence 100% identical to the nucleic acid sequence of SEQ ID NO: 4293 or 4294.

In some aspects, a promoter comprises a nucleic acid sequence at least 80% identical to a nucleic acid sequence selected from the group consisting of SEQ ID NOs: 4250 to 4254, 4258 to 4307, 4309 to 4312, 4386, and 6848 to 6850, or a functional fragment thereof. In some aspects, a promoter comprises a nucleic acid sequence at least 85% identical to a nucleic acid sequence selected from the group consisting of SEQ ID NOs: 4250 to 4254, 4258 to 4307, 4309to 4312, 4386, and 6848 to 6850, or a functional fragment thereof. In some aspects, a promoter comprises a nucleic acid sequence at least 90% identical to a nucleic acid sequence selected from the group consisting of SEQ ID NOs: 4250 to 4254, 4258 to 4307, 4309 to 4312, 4386, and 6848 to 6850, or a functional fragment thereof. In some aspects, a promoter comprises a nucleic acid sequence at least 91% identical to a nucleic acid sequence selected from the group consisting of SEQ ID NOs: 4250 to 4254, 4258 to 4307, 4309 to 4312, 4386, and 6848 to 6850, or a functional fragment thereof. In some aspects, a promoter comprises a nucleic acid sequence at least 92% identical to a nucleic acid sequence selected from the group consisting of SEQ ID NOs: 4250 to 4254, 4258 to 4307, 4309 to 4312, 4386, and 6848 to 6850, or a functional fragment thereof. In some aspects, a promoter comprises a nucleic acid sequence at least 93% identical to a nucleic acid sequence selected from the group consisting of SEQ ID NOs: 4250 to 4254, 4258 to 4307, 4309 to 4312, 4386, and 6848 to 6850, or a functional fragment thereof. In some aspects, a promoter comprises a nucleic acid sequence at least 94% identical to a nucleic acid sequence selected from the group consisting of SEQ ID NOs: 4250 to 4254, 4258 to 4307, 4309 to 4312, 4386, and 6848 to 6850, or a functional fragment thereof. In some aspects, a promoter comprises a nucleic acid sequence at least 95% identical to a nucleic acid sequence selected from the group consisting of SEQ ID NOs: 4250 to 4254, 4258 to 4307, 4309 to 4312, 4386, and 6848 to 6850, or a functional fragment thereof. In some aspects, a promoter comprises a nucleic acid sequence at least 96% identical to a nucleic acid sequence selected from the group consisting of SEQ ID NOs: 4250 to 4254, 4258 to 4307, 4309 to 4312, 4386, and 6848 to 6850, or a functional fragment thereof. In some aspects, a promoter comprises a nucleic acid sequence at least 97% identical to a nucleic acid sequence selected from the group consisting of SEQ ID NOs: 4250 to 4254, 4258 to 4307, 4309 to 4312, 4386, and 6848 to 6850, or a functional fragment thereof. In some aspects, a promoter comprises a nucleic acid sequence at least 98% identical to a nucleic acid sequence selected from the group consisting of SEQ ID NOs: 4250 to 4254, 4258 to 4307, 4309 to 4312, 4386, and 6848 to 6850, or a functional fragment thereof. In some aspects, a promoter comprises a nucleic acid sequence at least 99% identical to a nucleic acid sequence selected from the group consisting of SEQ ID NOs: 4250 to 4254, 4258 to 4307, 4309 to 4312, 4386, and 6848 to 6850, or a functional fragment thereof. In some aspects, a promoter comprises a nucleic acid sequence 100% identical to a nucleic acid sequence selected from the group consisting of SEQ ID NOs: 4250 to 4254, 4258 to 4307, 4309 to 4312, 4386, and 6848 to 6850, or a functional fragment thereof.

In some aspects of this disclosure, a composition or method refers multiple promoters, nucleic acid molecules, arthropod male-fertility essential genes, etc. For clarity, the components recited in these circumstances are often described using ordinal numbers (*e.g.,* a first promoter, a second promoter, a third promoter, etc.), although such ordinal numbers are not intended to be limiting.

In some aspects, a promoter is a PolII promoter. A "PolII promoter" refers to a promoter that is bound by an RNA polymerase II.

In some aspects, a promoter is a PolIII promoter. A "PolII promoter" refers to a promoter that is bound by an RNA polymerase III. Non-limiting examples of PolIII promoters include a *U6* promoter, a *7SK* promoter, a *U3* promoter, and a *tRNA* promoter. In some aspects, a PolIII promoter is selected from the group consisting of a *U6* promoter, *a 7SK* promoter, a *U3* promoter, and a *tRNA* promoter. In some aspects, a promoter is selected from the group consisting of a *U6* promoter, a *7SK* promoter, a *U3* promoter, and a *tRNA* promoter.

In some aspects, a promoter is selected from the group consisting of a PolII promoter and a PolIII promoter.

In some aspects, an upstream activation sequence for a transactivator is operably linked to a promoter. In some aspects, an upstream activation sequence for a transactivator is operably linked to a male sterility effector. In some aspects, an upstream activation sequence for a transactivator is operably linked to a rescue element. In some aspects, an upstream activation sequence for a transactivator is operably linked to a repressible female-lethal effector. In some aspects, an upstream activation sequence for a transactivator is positioned within a Y chromosome or M-locus. In some aspects, an upstream activation sequence for a transactivator is positioned within an X chromosome or m-locus. In some aspects, an upstream activation sequence for a transactivator is positioned within an autosome.

In some aspects, a promoter, a rescue element, a male sterility effector, or a repressible female-lethal effector is operably linked to at least 1 copy of an upstream activation sequence for a transactivator. In some aspects, a promoter, a rescue element, a male sterility effector, or a repressible female-lethal effector is operably linked to at least 2 copies of an upstream activation sequences for a transactivator. In some aspects, a promoter, a rescue element, a male sterility effector, or a repressible female-lethal effector is operably linked to at least 3 copies of an upstream activation sequences for a transactivator. In some aspects, a promoter, a rescue element, a male sterility effector, or a repressible female-lethal effector is operably linked to at least 4 copies of an upstream activation sequences for a transactivator. In some aspects, a promoter, a rescue element, a male sterility effector, or a repressible female-lethal effector is operably linked to at least 5 copies of an upstream activation sequences for a transactivator. In some aspects, a promoter, a rescue element, a male sterility effector, or a repressible female-lethal effector is operably linked to at least 6 copies of an upstream activation sequences for a transactivator. In some aspects, a promoter, a rescue element, a male sterility effector, or a repressible female-lethal effector is operably linked to at least 7 copies of an upstream activation sequences for a transactivator. In some aspects, a promoter, a rescue element, a male sterility effector, or a repressible female-lethal effector is operably linked to at least 8 copies of an upstream activation sequences for a transactivator. In some aspects, a promoter, a rescue element, a male sterility effector, or a repressible female-lethal effector is operably linked to at least 9 copies of an upstream activation sequences for a transactivator. In some aspects, a promoter, a rescue element, a male sterility effector, or a repressible female-lethal effector is operably linked to at least 10 copies of an upstream activation sequences for a transactivator. In some aspects, a promoter, a rescue element, a male sterility effector, or a repressible female-lethal effector is operably linked to at least 20 copies of an upstream activation sequences for a transactivator. In some aspects, a promoter, a rescue element, a male sterility effector, or a repressible female-lethal effector is operably linked to at least 30 copies of an upstream activation sequences for a transactivator. In some aspects, a promoter, a rescue element, a male sterility effector, or a repressible female-lethal effector is operably linked to at least 40 copies of an upstream activation sequences for a transactivator. In some aspects, a promoter, a rescue element, a male sterility effector, or a repressible female-lethal effector is operably linked to between 1 and 40 copies of an upstream activation sequences for a transactivator. In some aspects, a promoter, a rescue element, a male sterility effector, or a repressible female-lethal effector is operably linked to between 1 and 35 copies of an upstream activation sequences for a transactivator. In some aspects, a promoter, a rescue element, a male sterility effector, or a repressible female-lethal effector is operably linked to between 1 and 30 copies of an upstream activation sequences for a transactivator. In some aspects, a promoter, a rescue element, a male sterility effector, or a repressible female-lethal effector is operably linked to between 1 and 20 copies of an upstream activation sequences for a transactivator. In some aspects, a promoter, a rescue element, a male sterility effector, or a repressible female-lethal effector is operably linked to between 1 and 15 copies of an upstream activation sequences for a transactivator. In some aspects, a promoter, a rescue element, a male sterility effector, or a repressible female-lethal effector is operably linked to between 1 and 10 copies of an upstream activation sequences for a transactivator. In some aspects, a promoter, a rescue element, a male sterility effector, or a repressible female-lethal effector is operably linked to between 1 and 8 copies of an upstream activation sequences for a transactivator. In some aspects, a promoter, a rescue element, a male sterility effector, or a repressible female-lethal effector is operably linked to between 2 and 20 copies of an upstream activation sequences for a transactivator. In some aspects, a promoter, a rescue element, a male sterility effector, or a repressible female-lethal effector is operably linked to between 2 and 15 copies of an upstream activation sequences for a transactivator. In some aspects, a promoter, a rescue element, a male sterility effector, or a repressible female-lethal effector is operably linked to between 5 and 20 copies of an upstream activation sequences for a transactivator. In some aspects, a promoter, a rescue element, a male sterility effector, or a repressible female-lethal effector is operably linked to between 5 and 10 copies of an upstream activation sequences for a transactivator.

Without being limited by any theory, transactivators are transcription factors that recognize and bind to specific upstream activation sequences and initiate transcription. By changing the number of upstream activation sequences present at a given locus the amount of expression can be fine-tuned. Generally, the more upstream activation sequences present, the stronger (or higher) the expression.

In some aspects, a transactivator is a Gal4 transactivator. In some aspects, a transactivator is a QF transactivator. In some aspects, a transactivator is a QF2 transactivator. In some aspects, a transactivator is a QF2^{W} transactivator. In some aspects, a transactivator is selected from the group consisting of Gal4, QF, QF2, and QF2^{w}. *See,* for example, Brand and Perrimon, Development, 118(2):401-415 (1993) and Duffy, Genesis, 34:1-15 (2002) for information regarding Gal4; Potter et al., Cell, 141(3):536-548 (2010) and Riabinina et al., Nature Methods, 12(3):219-222 (2015) for information regarding the Q-system (e.g., QF, QF2, QF2^{W}).

In some aspects, a Gal4 transactivator is encoded by the nucleic acid sequence of SEQ ID NO: 4320. In some aspects, a QF2 transactivator is encoded by the nucleic acid sequence of SEQ ID NO: 4321. In some aspects, a QF2^{W} transactivator is encoded by the nucleic acid sequence of SEQ ID NO: 4322.

In some aspects, a Gal4 transactivator comprises the amino acid sequence of SEQ ID NO: 6507. In some aspects, a QF2 transactivator comprises the amino acid sequence of SEQ ID NO: 6508. In some aspects, a QF2^{W} transactivator comprises the amino acid sequence of SEQ ID NO: 6509.

In some aspects, an upstream activation sequence is UAS. In some aspects, an upstream activation sequence is QUAS. In some aspects, an upstream activation sequence is selected from the group consisting of UAS and QUAS. Typically, Gal4 recognizes (or is compatible with) UAS and QF, QF2, and QF2^{W} recognize (or are compatible with) QUAS.

A UAS sequence is provided as SEQ ID NO: 4289. Concatenated versions of UAS are provided as SEQ ID NOs: 4275 (10 copies of UAS) and 4281 (5 copies of UAS).

In some aspects, a nucleic acid sequence comprises at least 1 copy of UAS. In some aspects, a nucleic acid sequence comprises at least 2 copies of UAS. In some aspects, a nucleic acid sequence comprises at least 3 copies of UAS. In some aspects, a nucleic acid sequence comprises,at least 4 copies of UAS. In some aspects, a nucleic acid sequence comprises at least 5 copies of UAS. In some aspects, a nucleic acid sequence comprises at least 7 copies of UAS. In some aspects, a nucleic acid sequence comprises at least 10 copies of UAS. In some aspects, a nucleic acid sequence comprises at least 15 copies of UAS. In some aspects, a nucleic acid sequence comprises 1 copy of UAS. In some aspects, a nucleic acid sequence comprises 2 copies of UAS. In some aspects, a nucleic acid sequence comprises 3 copies of UAS. In some aspects, a nucleic acid sequence comprises 4 copies of UAS. In some aspects, a nucleic acid sequence comprises 5 copies of UAS. In some aspects, a nucleic acid sequence comprises 6 copies of UAS. In some aspects, a nucleic acid sequence comprises 7 copies of UAS. In some aspects, a nucleic acid sequence comprises 8 copies of UAS. In some aspects, a nucleic acid sequence comprises 9 copies of UAS. In some aspects, a nucleic acid sequence comprises 10 copies of UAS. In some aspects, a nucleic acid sequence comprises 15 copies of UAS.

A QUAS sequence is provided as SEQ ID NO: 4287. Concatenated versions of QUAS are provided as SEQ ID NOs: 4276 (15 copies of QUAS) and 4280 (5 copies of QUAS).

In some aspects, a nucleic acid sequence comprises at least 1 copy of QUAS. In some aspects, a nucleic acid sequence comprises at least 2 copies of QUAS. In some aspects, a nucleic acid sequence comprises at least 3 copies of QUAS. In some aspects, a nucleic acid sequence comprises at least 4 copies of QUAS. In some aspects, a nucleic acid sequence comprises at least 5 copies of QUAS. In some aspects, a nucleic acid sequence comprises at least 7 copies of QUAS. In some aspects, a nucleic acid sequence comprises at least 10 copies of QUAS. In some aspects, a nucleic acid sequence comprises at least 15 copies of QUAS. In some aspects, a nucleic acid sequence comprises 1 copy of QUAS. In some aspects, a nucleic acid sequence comprises 2 copies of QUAS. In some aspects, a nucleic acid sequence comprises 3 copies of QUAS. In some aspects, a nucleic acid sequence comprises 4 copies of QUAS. In some aspects, a nucleic acid sequence comprises 5 copies of QUAS. In some aspects, a nucleic acid sequence comprises 6 copies of QUAS. In some aspects, a nucleic acid sequence comprises 7 copies of QUAS. In some aspects, a nucleic acid sequence comprises 8 copies of QUAS. In some aspects, a nucleic acid sequence comprises 9 copies of QUAS. In some aspects, a nucleic acid sequence comprises 10 copies of QUAS. In some aspects, a nucleic acid sequence comprises 15 copies of QUAS.

As used herein, "operably linked" refers to a functional linkage between two or more elements. For example, an operable linkage between a polynucleotide of interest and a regulatory sequence (e.g., a promoter) is a functional link that allows for expression of the polynucleotide of interest. Operably linked elements may be contiguous or non-contiguous. In some aspects, a promoter provided herein is operably linked to a heterologous nucleic acid molecule.

As used herein, a "heterologous" sequence refers to a sequence (nucleic acid or amino acid) that originates from a foreign species, or, if from the same species, is modified sufficiently to be distinct from its native form in composition and/or genomic locus by deliberate human intervention. As a non-limiting example, a native fruit fly ubiquitin promoter operably linked to a native fruit fly actin-encoding sequence is considered a heterologous sequence even when present in a fruit fly because the two sequences do not naturally occur together in operable linkage.

The use of the terms "polynucleotide" are "nucleic acid molecule" (which are used interchangeably herein) are not intended to limit the present disclosure to polynucleotides comprising deoxyribonucleic acid (DNA). For example, ribonucleic acid (RNA) molecules are also envisioned. Those of ordinary skill in the art will recognize that polynucleotides and nucleic acid molecules can comprise ribonucleotides and combinations of ribonucleotides and deoxyribonucleotides. Such deoxyribonucleotides and ribonucleotides include both naturally occurring molecules and synthetic analogues. The polynucleotides of the present disclosure also encompass all forms of sequences including, but not limited to, single-stranded forms, double-stranded forms, hairpins, stem-and-loop structures, and the like. In some aspects, a nucleic acid molecule is a DNA molecule. In some aspects, a nucleic acid molecule is an RNA molecule. In some aspects, a nucleic acid molecule is single-stranded. In some aspects, a nucleic acid molecule is double-stranded. In some aspects, a nucleic acid molecule encodes a polypeptide. In some aspects, a nucleic acid molecule encodes a gRNA. In some aspects, a nucleic acid molecule encodes a small RNA. In some aspects, a nucleic acid molecule encodes a miRNA. In some aspects, a nucleic acid molecule encodes a shRNA. In some aspects, a nucleic acid molecule comprises a promoter sequence.

Nucleic acids can be isolated using techniques routine in the art. For example, nucleic acids can be isolated using any method including, without limitation, recombinant nucleic acid technology, and/or the polymerase chain reaction (PCR). General PCR techniques are described, for example in PCR Primer: A Laboratory Manual, Dieffenbach & Dveksler, Eds., Cold Spring Harbor Laboratory Press, 1995. Recombinant nucleic acid techniques include, for example, restriction enzyme digestion and ligation, which can be used to isolate a nucleic acid. Isolated nucleic acids also can be chemically synthesized, either as a single nucleic acid molecule or as a series of oligonucleotides.

As used herein, an "isolated nucleic acid molecule" refers to a nucleic acid molecule that is at least partially separated from other nucleic acid molecules it is typically associated with in its natural (e.g. wildtype) state. For example, an isolated nucleic acid molecule is one that has been separated from the nucleic acid molecules that normally flank it in its natural state. Thus, an isolated nucleic acid molecule encoding a male sterile effector, a rescue element, a repressible female-lethal effector, or a sterility system is considered isolated because they comprise nucleic acid elements with which they are not associated with in nature. Such isolated nucleic acid molecules are considered isolated even when integrated into a chromosome of an arthropod cell or when present in a nucleic acid solution with other nucleic acid molecules. In some aspects, this disclosure provides any combination of any number of isolated nucleic acid molecules provided herein.

In some aspects, this disclosure provides an isolated nucleic acid molecule encoding any amino acid sequence provided herein. In some aspects, this disclosure provides an isolated nucleic acid molecule encoding an amino acid sequence at least 80% identical to an amino acid sequence selected from the group consisting of SEQ ID NOs: 2880 and 4397 to 6831. In some aspects, this disclosure provides an isolated nucleic acid molecule encoding an amino acid sequence at least 85% identical to an amino acid sequence selected from the group consisting of SEQ ID NOs: 2880 and 4397 to 6831. In some aspects, this disclosure provides an isolated nucleic acid molecule encoding an amino acid sequence at least 90% identical to an amino acid sequence selected from the group consisting of SEQ ID NOs: 2880 and 4397 to 6831. In some aspects, this disclosure provides an isolated nucleic acid molecule encoding an amino acid sequence at least 91% identical to an amino acid sequence selected from the group consisting of SEQ ID NOs: 2880 and 4397 to 6831. In some aspects, this disclosure provides an isolated nucleic acid molecule encoding an amino acid sequence at least 92% identical to an amino acid sequence selected from the group consisting of SEQ ID NOs: 2880 and 4397 to 6831. In some aspects, this disclosure provides an isolated nucleic acid molecule encoding an amino acid sequence at least 93% identical to an amino acid sequence selected from the group consisting of SEQ ID NOs: 2880 and 4397 to 6831. In some aspects, this disclosure provides an isolated nucleic acid molecule encoding an amino acid sequence at least 94% identical to an amino acid sequence selected from the group consisting of SEQ ID NOs: 2880 and 4397 to 6831. In some aspects, this disclosure provides an isolated nucleic acid molecule encoding an amino acid sequence at least 95% identical to an amino acid sequence selected from the group consisting of SEQ ID NOs: 2880 and 4397 to 6831. In some aspects, this disclosure provides an isolated nucleic acid molecule encoding an amino acid sequence at least 96% identical to an amino acid sequence selected from the group consisting of SEQ ID NOs: 2880 and 4397 to 6831. In some aspects, this disclosure provides an isolated nucleic acid molecule encoding an amino acid sequence at least 97% identical to an amino acid sequence selected from the group consisting of SEQ ID NOs: 2880 and 4397 to 6831. In some aspects, this disclosure provides an isolated nucleic acid molecule encoding an amino acid sequence at least 98% identical to an amino acid sequence selected from the group consisting of SEQ ID NOs: 2880 and 4397 to 6831. In some aspects, this disclosure provides an isolated nucleic acid molecule encoding an amino acid sequence at least 99% identical to an amino acid sequence selected from the group consisting of SEQ ID NOs: 2880 and 4397 to 6831. In some aspects, this disclosure provides an isolated nucleic acid molecule encoding an amino acid sequence 100% identical to an amino acid sequence selected from the group consisting of SEQ ID NOs: 2880 and 4397 to 6831.

In some aspects, this disclosure provides an isolated nucleic acid molecule encoding any amino acid sequence provided herein. In some aspects, this disclosure provides an isolated nucleic acid molecule encoding an amino acid sequence at least 80% similar to an amino acid sequence selected from the group consisting of SEQ ID NOs: 2880 and 4397 to 6831. In some aspects, this disclosure provides an isolated nucleic acid molecule encoding an amino acid sequence at least 85% similar to an amino acid sequence selected from the group consisting of SEQ ID NOs: 2880 and 4397 to 6831. In some aspects, this disclosure provides an isolated nucleic acid molecule encoding an amino acid sequence at least 90% similar to an amino acid sequence selected from the group consisting of SEQ ID NOs: 2880 and 4397 to 6831. In some aspects, this disclosure provides an isolated nucleic acid molecule encoding an amino acid sequence at least 91% similar to an amino acid sequence selected from the group consisting of SEQ ID NOs: 2880 and 4397 to 6831. In some aspects, this disclosure provides an isolated nucleic acid molecule encoding an amino acid sequence at least 92% similar to an amino acid sequence selected from the group consisting of SEQ ID NOs: 2880 and 4397 to 6831. In some aspects, this disclosure provides an isolated nucleic acid molecule encoding an amino acid sequence at least 93% similar to an amino acid sequence selected from the group consisting of SEQ ID NOs: 2880 and 4397 to 6831. In some aspects, this disclosure provides an isolated nucleic acid molecule encoding an amino acid sequence at least 94% similar to an amino acid sequence selected from the group consisting of SEQ ID NOs: 2880 and 4397 to 6831. In some aspects, this disclosure provides an isolated nucleic acid molecule encoding an amino acid sequence at least 95% similar to an amino acid sequence selected from the group consisting of SEQ ID NOs: 2880 and 4397 to 6831. In some aspects, this disclosure provides an isolated nucleic acid molecule encoding an amino acid sequence at least 96% similar to an amino acid sequence selected from the group consisting of SEQ ID NOs: 2880 and 4397 to 6831. In some aspects, this disclosure provides an isolated nucleic acid molecule encoding an amino acid sequence at least 97% similar to an amino acid sequence selected from the group consisting of SEQ ID NOs: 2880 and 4397 to 6831. In some aspects, this disclosure provides an isolated nucleic acid molecule encoding an amino acid sequence at least 98% similar to an amino acid sequence selected from the group consisting of SEQ ID NOs: 2880 and 4397 to 6831. In some aspects, this disclosure provides an isolated nucleic acid molecule encoding an amino acid sequence at least 99% similar to an amino acid sequence selected from the group consisting of SEQ ID NOs: 2880 and 4397 to 6831. In some aspects, this disclosure provides an isolated nucleic acid molecule encoding an amino acid sequence 100% similar to an amino acid sequence selected from the group consisting of SEQ ID NOs: 2880 and 4397 to 6831.

In some aspects, this disclosure provides an isolated nucleic acid molecule comprising any nucleic acid molecule provided herein. In some aspects, this disclosure provides an isolated nucleic acid molecule comprising a nucleic acid sequence at least 80% identical to a nucleic acid sequence selected from the group consisting of SEQ ID NOs: 1 to 2879 , 2881 to 4396, and 6842 to 6850. In some aspects, this disclosure provides an isolated nucleic acid molecule comprising a nucleic acid sequence at least 85% identical to a nucleic acid sequence selected from the group consisting of SEQ ID NOs: 1 to 2879 , 2881 to 4396, and 6842 to 6850. In some aspects, this disclosure provides an isolated nucleic acid molecule comprising a nucleic acid sequence at least 90% identical to a nucleic acid sequence selected from the group consisting of SEQ ID NOs: 1 to 2879 , 2881 to 4396, and 6842 to 6850. In some aspects, this disclosure provides an isolated nucleic acid molecule comprising a nucleic acid sequence at least 91% identical to a nucleic acid sequence selected from the group consisting of SEQ ID NOs: 1 to 2879 , 2881 to 4396, and 6842 to 6850. In some aspects, this disclosure provides an isolated nucleic acid molecule comprising a nucleic acid sequence at least 92% identical to a nucleic acid sequence selected from the group consisting of SEQ ID NOs: 1 to 2879 , 2881 to 4396, and 6842 to 6850. In some aspects, this disclosure provides an isolated nucleic acid molecule comprising a nucleic acid sequence at least 93% identical to a nucleic acid sequence selected from the group consisting of SEQ ID NOs: 1 to 2879 , 2881 to 4396, and 6842 to 6850. In some aspects, this disclosure provides an isolated nucleic acid molecule comprising a nucleic acid sequence at least 94% identical to a nucleic acid sequence selected from the group consisting of SEQ ID NOs: 1 to 2879 , 2881 to 4396, and 6842 to 6850. In some aspects, this disclosure provides an isolated nucleic acid molecule comprising a nucleic acid sequence at least 95% identical to a nucleic acid sequence selected from the group consisting of SEQ ID NOs: 1 to 2879 , 2881 to 4396, and 6842 to 6850. In some aspects, this disclosure provides an isolated nucleic acid molecule comprising a nucleic acid sequence at least 96% identical to a nucleic acid sequence selected from the group consisting of SEQ ID NOs: 1 to 2879 , 2881 to 4396, and 6842 to 6850. In some aspects, this disclosure provides an isolated nucleic acid molecule comprising a nucleic acid sequence at least 97% identical to a nucleic acid sequence selected from the group consisting of SEQ ID NOs: 1 to 2879 , 2881 to 4396, and 6842 to 6850. In some aspects, this disclosure provides an isolated nucleic acid molecule comprising a nucleic acid sequence at least 98s% identical to a nucleic acid sequence selected from the group consisting of SEQ ID NOs: 1 to 2879 , 2881 to 4396, and 6842 to 6850. In some aspects, this disclosure provides an isolated nucleic acid molecule comprising a nucleic acid sequence at least 99% identical to a nucleic acid sequence selected from the group consisting of SEQ ID NOs: 1 to 2879 , 2881 to 4396, and 6842 to 6850. In some aspects, this disclosure provides an isolated nucleic acid molecule comprising a nucleic acid sequence 100% identical to a nucleic acid sequence selected from the group consisting of SEQ ID NOs: 1 to 2879 , 2881 to 4396, and 6842 to 6850.

In some aspects, this disclosure provides one or more plasmids comprising any isolated nucleic acid molecule provided herein. In some aspects, this disclosure provides two or more plasmids comprising any isolated nucleic acid molecule provided herein. In some aspects, this disclosure provides three or more plasmids comprising any isolated nucleic acid molecule provided herein. In some aspects, this disclosure provides one or more plasmids encoding any male sterility effector provided herein. In some aspects, this disclosure provides one or more plasmids encoding any repressible female-lethal effector provided herein. In some aspects, this disclosure provides one or more plasmids encoding any rescue element provided herein. In some aspects, this disclosure provides an arthropod comprising any plasmid provided herein.

In some aspects, a plasmid comprises a nucleic acid sequence at least 80% identical to a nucleic acid sequence selected from the group consisting of SEQ ID NOs: 4012 to 4249, 4335 to 4337, and 4391 to 4396. In some aspects, a plasmid comprises a nucleic acid sequence at least 85% identical to a nucleic acid sequence selected from the group consisting of SEQ ID NOs: 4012 to 4249, 4335 to 4337, and 4391 to 4396. In some aspects, a plasmid comprises a nucleic acid sequence at least 90% identical to a nucleic acid sequence selected from the group consisting of SEQ ID NOs: 4012 to 4249, 4335 to 4337, and 4391 to 4396. In some aspects, a plasmid comprises a nucleic acid sequence at least 91% identical to a nucleic acid sequence selected from the group consisting of SEQ ID NOs: 4012 to 4249, 4335 to 4337, and 4391 to 4396. In some aspects, a plasmid comprises a nucleic acid sequence at least 92% identical to a nucleic acid sequence selected from the group consisting of SEQ ID NOs: 4012 to 4249, 4335 to 4337, and 4391 to 4396. In some aspects, a plasmid comprises a nucleic acid sequence at least 93% identical to a nucleic acid sequence selected from the group consisting of SEQ ID NOs: 4012 to 4249, 4335 to 4337, and 4391 to 4396. In some aspects, a plasmid comprises a nucleic acid sequence at least 94% identical to a nucleic acid sequence selected from the group consisting of SEQ ID NOs: 4012 to 4249, 4335 to 4337, and 4391 to 4396. In some aspects, a plasmid comprises a nucleic acid sequence at least 95% identical to a nucleic acid sequence selected from the group consisting of SEQ ID NOs: 4012 to 4249, 4335 to 4337, and 4391 to 4396. In some aspects, a plasmid comprises a nucleic acid sequence at least 96% identical to a nucleic acid sequence selected from the group consisting of SEQ ID NOs: 4012 to 4249, 4335 to 4337, and 4391 to 4396. In some aspects, a plasmid comprises a nucleic acid sequence at least 97% identical to a nucleic acid sequence selected from the group consisting of SEQ ID NOs: 4012 to 4249, 4335 to 4337, and 4391 to 4396. In some aspects, a plasmid comprises a nucleic acid sequence at least 98% identical to a nucleic acid sequence selected from the group consisting of SEQ ID NOs: 4012 to 4249, 4335 to 4337, and 4391 to 4396. In some aspects, a plasmid comprises a nucleic acid sequence at least 99% identical to a nucleic acid sequence selected from the group consisting of SEQ ID NOs: 4012 to 4249, 4335 to 4337, and 4391 to 4396. In some aspects, a plasmid comprises a nucleic acid sequence 100% identical to a nucleic acid sequence selected from the group consisting of SEQ ID NOs: 4012 to 4249, 4335 to 4337, and 4391 to 4396.

In some aspects, this disclosure provides a modified arthropod comprising any plasmid provided herein. In some aspects, this disclosure provides a modified arthropod comprising any 2 plasmids provided herein. In some aspects, this disclosure provides a modified arthropod comprising any 3 plasmids provided herein. In some aspects, this disclosure provides a modified arthropod comprising a plasmid comprising a nucleic acid sequence selected from the group consisting of SEQ ID NOs: 4012 to 4249, 4335 to 4337, and 4391 to 4396. In some aspects, this disclosure provides a modified arthropod comprising any combination of plasmids, wherein the plasmids each comprise a nucleic acid sequence selected from the group consisting of SEQ ID NOs: 4012 to 4249, 4335 to 4337, and 4391 to 4396.

In some aspects, this disclosure provides an isolated nucleic acid molecule comprising any plasmid provided herein.

In some aspects, an isolated nucleic acid molecule comprises at least 1 promoter. In some aspects, an isolated nucleic acid molecule comprises at least 2 promoters. In some aspects, an isolated nucleic acid molecule comprises at least 3 promoters. In some aspects, an isolated nucleic acid molecule comprises at least 4 promoters. In some aspects, an isolated nucleic acid molecule comprises at least 5 promoters. In some aspects, an isolated nucleic acid molecule comprises at least 6 promoters. In some aspects, an isolated nucleic acid molecule comprises at least 7 promoters. In some aspects, an isolated nucleic acid molecule comprises at least 8 promoters.

In some aspects, this disclosure provides an isolated nucleic acid molecule encoding any male sterile effector provided herein. In some aspects, this disclosure provides one or more isolated nucleic acid molecules encoding any male sterile effector provided herein. In some aspects, this disclosure provides two or more isolated nucleic acid molecules encoding any male sterile effector provided herein. In some aspects, this disclosure provides two isolated nucleic acid molecules encoding any male sterile effector provided herein. In some aspects, this disclosure provides three isolated nucleic acid molecules encoding any male sterile effector provided herein.

The terms "percent identity" or "percent identical" as used herein in reference to two or more nucleotide or amino acid sequences is calculated by (i) comparing two optimally aligned sequences (nucleotide or amino acid) over a window of comparison (the "alignable" region or regions), (ii) determining the number of positions at which the identical nucleic acid base (for nucleotide sequences) or amino acid residue (for proteins and polypeptides) occurs in both sequences to yield the number of matched positions, (iii) dividing the number of matched positions by the total number of positions in the window of comparison, and then (iv) multiplying this quotient by 100% to yield the percent identity. If the "percent identity" is being calculated in relation to a reference sequence without a particular comparison window being specified, then the percent identity is determined by dividing the number of matched positions over the region of alignment by the total length *(e.g.,* full-length) of the reference sequence *(e.g.,* a SEQ ID NO provided in the instant disclosure). Accordingly, for purposes of the present application, when two sequences (query and subject) are optimally aligned (with allowance for gaps in their alignment), the "percent identity" for the query sequence is equal to the number of identical positions between the two sequences divided by the total number of positions in the query sequence over its full-length (or a comparison window, if specified), which is then multiplied by 100%.

As a non-limiting example, if a query sequence having a length of 50 nucleotides is compared to a reference sequence having a full-length of 100 nucleotides, the maximum percent identify for the query sequence compared to the reference sequence is 50%.

The terms "percent sequence complementarity" or "percent complementarity" as used herein in reference to two nucleotide sequences is similar to the concept of percent identity but refers to the percentage of nucleotides of a query sequence that optimally base-pair or hybridize to nucleotides a subject sequence when the query and subject sequences are linearly arranged and optimally base paired without secondary folding structures, such as loops, stems or hairpins. Such a percent complementarity can be between two DNA strands, two RNA strands, or a DNA strand and a RNA strand. The "percent complementarity" can be calculated by (i) optimally base-pairing or hybridizing the two nucleotide sequences in a linear and fully extended arrangement (e.g., without folding or secondary structures) over a window of comparison, (ii) determining the number of positions that base-pair between the two sequences over the window of comparison to yield the number of complementary positions, (iii) dividing the number of complementary positions by the total number of positions in the window of comparison, and (iv) multiplying this quotient by 100% to yield the percent complementarity of the two sequences. Optimal base pairing of two sequences can be determined based on the known pairings of nucleotide bases, such as G-C, A-T, and A-U, through hydrogen binding. If the "percent complementarity" is being calculated in relation to a reference sequence without specifying a particular comparison window, then the percent identity is determined by dividing the number of complementary positions between the two linear sequences by the total length of the reference sequence (e.g., a SEQ ID NO provided herein). Thus, for purposes of the present application, when two sequences (query and subject) are optimally base-paired (with allowance for mismatches or non-base-paired nucleotides), the "percent complementarity" for the query sequence is equal to the number of base-paired positions between the two sequences divided by the total number of positions in the query sequence over its length, which is then multiplied by 100%.

When percentage of sequence identity is used in reference to amino acids it is recognized that residue positions which are not identical often differ by conservative amino acid substitutions, where amino acid residues are substituted for other amino acid residues with similar chemical properties (e.g., charge or hydrophobicity) and therefore do not change the functional properties of the molecule. When sequences differ in conservative substitutions, the percent sequence identity can be adjusted upwards to correct for the conservative nature of the substitution. Sequences that differ by such conservative substitutions are said to have "sequence similarity" or "similarity."

Without being limiting, an aliphatic (e.g., glycine, alanine, valine, leucine, isoleucine) amino acid residue can be substituted for another aliphatic amino acid residue in a conservative substitution; a hydroxyl (e.g., serine, cysteine, threonine, methionine) amino acid residue can be substituted for another hydroxyl amino acid residue in a conservative substitution; an aromatic (e.g., phenylalanine, tyrosine, tryptophan) amino acid residue can be substituted for another aromatic amino acid residue in a conservative substitution; a basic (e.g., histidine, lysine, arginine) amino acid residue can be substituted for another basic amino acid residue in a conservative substitution; and an acidic (e.g., aspartate, glutamate, asparagine, glutamine) amino acid residue can be substituted for another acidic amino acid residue in a conservative substitution.

As a non-limiting example, the amino acid sequence (glycine-serine-glycine) would have 66.67% sequence identity and 100% sequence similarity with the amino acid sequence (glycine-serine-alanine) because glycine and alanine are aliphatic amino acid residues that can be conservatively substituted for each other.

For optimal alignment of sequences to calculate their percent identity, various pair-wise or multiple sequence alignment algorithms and programs are known in the art, such as ClustalW or Basic Local Alignment Search Tool^{®} (BLAST^{™}), etc., that can be used to compare the sequence identity or similarity between two or more nucleotide or amino acid sequences. Although other alignment and comparison methods are known in the art, the alignment and percent identity between two sequences (including the percent identity ranges described above) can be as determined by the ClustalW algorithm, *see, e.g.,* Chenna et al., "Multiple sequence alignment with the Clustal series of programs," Nucleic Acids Research 31: 3497-3500 (2003); Thompson et al., "Clustal W: Improving the sensitivity of progressive multiple sequence alignment through sequence weighting, position-specific gap penalties and weight matrix choice," Nucleic Acids Research 22: 4673-4680 (1994); Larkin MA et al., "Clustal W and Clustal X version 2.0," Bioinformatics 23: 2947-48 (2007); and Altschul et al. "Basic local alignment search tool." J. Mol. Biol. 215:403-410 (1990), the entire contents and disclosures of which are incorporated herein by reference.

As used herein, an "RNA-guided nuclease" refers to a nuclease that binds to an RNA molecule, where the RNA molecule then guides the nuclease to a specific *(e.g.,* complementary) sequence. RNA-guided nucleases can cleave one or two strands of a targeted nucleic acid molecule to introduce one or more insertions, deletions, substitutions, and/or base substitutions. If an RNA-guided nuclease cleaves only a single strand of a target nucleic acid molecule it is termed a "nickase." In some aspects, an RNA-guided nuclease is a nickase.

A non-limiting example of RNA-guided nucleases are the CRISPR (Clustered Regularly Interspaced Short Palindromic Repeats) RNA-guided nucleases. In some aspects an RNA-guided nuclease is a CRISPR RNA-guided nuclease. In some aspects an RNA-guided nuclease is selected from the group consisting of a Type I CRISPR RNA-guided nuclease, a Type II CRISPR RNA-guided nuclease, a Type III CRISPR RNA-guided nuclease, a Type IV CRISPR RNA-guided nuclease, a Type V CRISPR RNA-guided nuclease, and a Type VI CRISPR RNA-guided nuclease. In some aspects, an RNA-guided nuclease is selected from the group consisting of Casl, CaslB, Cas2, Cas3, Cas4, Cas5, Cas6, Cas7, Cas8, Cas9 (also known as Csnl and Csxl2), CaslO, Cas12a (also known as Cpf1), Csy1, Csy2, Csy3, Csel, Cse2, Cscl, Csc2, Csa5, Csn2, Csm2, Csm3, Csm4, Csm5, Csm6, Cmr1, Cmr3, Cmr4, Cmr5, Cmr6, Csb1, Csb2, Csb3, Csxl7, Csxl4, CsxlO, Csxl6, CsaX, Csx3, Csxl, Csxl5, Csf1, Csf2, Csf3, Csf4, CasX, CasY, and Mad7. In some aspects, an RNA-guided nuclease is a Cas9. In some aspects, a Cas9 is a *Streptococcus pyogenes* Cas9. In some aspects, Cas9 is a *Staphylococcus aureus* Cas9.

In some aspects, a Cas9 is encoded by a nucleic acid sequence at least 80% identical to the nucleic acid sequence of SEQ ID NO: 4316 or 4317. In some aspects, a Cas9 is encoded by a nucleic acid sequence at least 85% identical to the nucleic acid sequence of SEQ ID NO: 4316 or 4317. In some aspects, a Cas9 is encoded by a nucleic acid sequence at least 90% identical to the nucleic acid sequence of SEQ ID NO: 4316 or 4317. In some aspects, a Cas9 is encoded by a nucleic acid sequence at least 91% identical to the nucleic acid sequence of SEQ ID NO: 4316 or 4317. In some aspects, a Cas9 is encoded by a nucleic acid sequence at least 92% identical to the nucleic acid sequence of SEQ ID NO: 4316 or 4317. In some aspects, a Cas9 is encoded by a nucleic acid sequence at least 93% identical to the nucleic acid sequence of SEQ ID NO: 4316 or 4317. In some aspects, a Cas9 is encoded by a nucleic acid sequence at least 94% identical to the nucleic acid sequence of SEQ ID NO: 4316 or 4317. In some aspects, a Cas9 is encoded by a nucleic acid sequence at least 95% identical to the nucleic acid sequence of SEQ ID NO: 4316 or 4317. In some aspects, a Cas9 is encoded by a nucleic acid sequence at least 96% identical to the nucleic acid sequence of SEQ ID NO: 4316 or 4317. In some aspects, a Cas9 is encoded by a nucleic acid sequence at least 97% identical to the nucleic acid sequence of SEQ ID NO: 4316 or 4317. In some aspects, a Cas9 is encoded by a nucleic acid sequence at least 98% identical to the nucleic acid sequence of SEQ ID NO: 4316 or 4317. In some aspects, a Cas9 is encoded by a nucleic acid sequence at least 99% identical to the nucleic acid sequence of SEQ ID NO: 4316 or 4317. In some aspects, a Cas9 is encoded by a nucleic acid sequence 100% identical to the nucleic acid sequence of SEQ ID NO: 4316 or 4317.

In some aspects, an RNA-guided nuclease comprises at least one nuclear localization signal (NLS). In some aspects, an RNA-guided nuclease comprises a nuclear localization signal on its carboxyl terminus. In some aspects, an RNA-guided nuclease comprises a nuclear localization signal on its amino terminus. In some aspects, an RNA-guided nuclease comprises a nuclear localization signal on both its carboxyl terminus and its amino terminus.

In some aspects, an NLS is encoded by a nucleic acid sequence at least 80% identical to the nucleic acid sequence of SEQ ID NO: 4318 or 4319. In some aspects, an NLS is encoded by a nucleic acid sequence at least 85% identical to the nucleic acid sequence of SEQ ID NO: 4318 or 4319. In some aspects, an NLS is encoded by a nucleic acid sequence at least 90% identical to the nucleic acid sequence of SEQ ID NO: 4318 or 4319. In some aspects, an NLS is encoded by a nucleic acid sequence at least 91% identical to the nucleic acid sequence of SEQ ID NO: 4318 or 4319. In some aspects, an NLS is encoded by a nucleic acid sequence at least 92% identical to the nucleic acid sequence of SEQ ID NO: 4318 or 4319. In some aspects, an NLS is encoded by a nucleic acid sequence at least 93% identical to the nucleic acid sequence of SEQ ID NO: 4318 or 4319. In some aspects, an NLS is encoded by a nucleic acid sequence at least 94% identical to the nucleic acid sequence of SEQ ID NO: 4318 or 4319. In some aspects, an NLS is encoded by a nucleic acid sequence at least 95% identical to the nucleic acid sequence of SEQ ID NO: 4318 or 4319. In some aspects, an NLS is encoded by a nucleic acid sequence at least 96% identical to the nucleic acid sequence of SEQ ID NO: 4318 or 4319. In some aspects, an NLS is encoded by a nucleic acid sequence at least 97% identical to the nucleic acid sequence of SEQ ID NO: 4318 or 4319. In some aspects, an NLS is encoded by a nucleic acid sequence at least 98% identical to the nucleic acid sequence of SEQ ID NO: 4318 or 4319. In some aspects, an NLS is encoded by a nucleic acid sequence at least 99% identical to the nucleic acid sequence of SEQ ID NO: 4318 or 4319. In some aspects, an NLS is encoded by a nucleic acid sequence 100% identical to the nucleic acid sequence of SEQ ID NO: 4318 or 4319.

In some aspects, an NLS comprises an amino acid sequence at least 80% identical or similar to the amino acid sequence of SEQ ID NO: 6505 or 6506. In some aspects, an NLS comprises an amino acid sequence at least 85% identical or similar to the amino acid sequence of SEQ ID NO: 6505 or 6506. In some aspects, an NLS comprises an amino acid sequence at least 90% identical or similar to the amino acid sequence of SEQ ID NO: 6505 or 6506. In some aspects, an NLS comprises an amino acid sequence at least 91% identical or similar to the amino acid sequence of SEQ ID NO: 6505 or 6506. In some aspects, an NLS comprises an amino acid sequence at least 92% identical or similar to the amino acid sequence of SEQ ID NO: 6505 or 6506. In some aspects, an NLS comprises an amino acid sequence at least 93% identical or similar to the amino acid sequence of SEQ ID NO: 6505 or 6506. In some aspects, an NLS comprises an amino acid sequence at least 94% identical or similar to the amino acid sequence of SEQ ID NO: 6505 or 6506. In some aspects, an NLS comprises an amino acid sequence at least 95% identical or similar to the amino acid sequence of SEQ ID NO: 6505 or 6506. In some aspects, an NLS comprises an amino acid sequence at least 96% identical or similar to the amino acid sequence of SEQ ID NO: 6505 or 6506. In some aspects, an NLS comprises an amino acid sequence at least 97% identical or similar to the amino acid sequence of SEQ ID NO: 6505 or 6506. In some aspects, an NLS comprises an amino acid sequence at least 98% identical or similar to the amino acid sequence of SEQ ID NO: 6505 or 6506. In some aspects, an NLS comprises an amino acid sequence at least 99% identical or similar to the amino acid sequence of SEQ ID NO: 6505 or 6506. In some aspects, an NLS comprises an amino acid sequence 100% identical or similar to the amino acid sequence of SEQ ID NO: 6505 or 6506.

As used herein, a "guide RNA" or "gRNA" refers to an RNA that recognizes a target DNA sequence and directs, or "guides," an RNA-guided nuclease to a target DNA sequence. A guide RNA for Cas9 is comprised of a region that is complementary to a target DNA (referred to as the crRNA) and a region that binds the Cas9 (referred to as the tracrRNA). In some aspects, a gRNA is a single-chain guide RNA. A "single-chain guide RNA" (or "sgRNA") refers to an RNA molecule comprising a crRNA covalently linked a tracrRNA by a linker sequence, which may be expressed as a single RNA transcript or molecule. A guide RNA may be a single RNA molecule (sgRNA) or two separate RNAs molecules (a 2-piece gRNA). Although this disclosure uses the terms "guide RNA" and "gRNA" throughout, it will be appreciated that this disclosure also provides for the guide molecule being be a single-stranded DNA molecule in place of an RNA molecule.

Without being limiting, a prerequisite for cleavage of the target site by a CRIPSR RNA-guided nuclease in the presence of a conserved protospacer-adjacent motif (PAM) adjacent to the target sequence. For Cas9, the PAM is positioned downstream (e.g., 3' to) of the target site and typically has the sequence 5'-NGG-3' or 5'-CCN-3' or, less frequently, 5'-NAG-3'. Specificity is provided by a "seed sequence" approximately 12 nucleotides upstream of the PAM, which must match between the gRNA and target nucleic acid molecule.

In some aspects, a guide RNA comprises a guide sequence (also referred to as a spacer sequence) that binds to a complementary sequence of an arthropod male-fertility essential gene. It will be appreciated that perfect complementarity is not required for binding of a gRNA to a target sequence. In some aspects, a guide sequence comprises 1, 2, 3, 4, 5, or 6 mismatches when compared to a target sequence of an arthropod male-fertility essential gene.

As used herein, a "target sequence" refers to a selected sequence or region of a DNA molecule in which a modification (e.g., cleavage, site-directed integration) is desired. A target sequence comprises a target site.

As used herein, a "target site" refers to the portion of a target sequence that is cleaved by an RNA-guided nuclease (e.g., a CRISPR enzyme coupled with a gRNA; an RNA-induced silencing complex coupled with a small RNA *(e.g.,* a miRNA or shRNA)). In contrast to a non-target nucleic acid or non-target region, a target site comprises significant complementarity to a guide nucleic acid or a small RNA molecule (e.g., without being limiting, a gRNA, a miRNA, an shRNA).

In some aspects, a gRNA guide sequence comprises 15 nucleotides. In some aspects, a gRNA guide sequence comprises 16 nucleotides. In some aspects, a gRNA guide sequence comprises 17 nucleotides. In some aspects, a gRNA guide sequence comprises 18 nucleotides. In some aspects, a gRNA guide sequence comprises 19 nucleotides. In some aspects, a gRNA guide sequence comprises 20 nucleotides. In some aspects, a gRNA guide sequence comprises 21 nucleotides. In some aspects, a gRNA guide sequence comprises 22 nucleotides. In some aspects, a gRNA guide sequence comprises 23 nucleotides. In some aspects, a gRNA guide sequence comprises 24 nucleotides. In some aspects, a gRNA guide sequence comprises 25 nucleotides. In some aspects, a gRNA guide sequence comprises 26 nucleotides. In some aspects, a gRNA guide sequence comprises 27 nucleotides.

In some aspects, a gRNA guide sequence comprises between 15 nucleotides and 30 nucleotides. In some aspects, a gRNA guide sequence comprises between 15 nucleotides and 28 nucleotides. In some aspects, a gRNA guide sequence comprises between 15 nucleotides and 26 nucleotides. In some aspects, a gRNA guide sequence comprises between 15 nucleotides and 24 nucleotides. In some aspects, a gRNA guide sequence comprises between 15 nucleotides and 22 nucleotides. In some aspects, a gRNA guide sequence comprises between 15 nucleotides and 20 nucleotides. In some aspects, a gRNA guide sequence comprises between 17 nucleotides and 30 nucleotides. In some aspects, a gRNA guide sequence comprises between 17 nucleotides and 28 nucleotides. In some aspects, a gRNA guide sequence comprises between 17 nucleotides and 26 nucleotides. In some aspects, a gRNA guide sequence comprises between 17 nucleotides and 24 nucleotides. In some aspects, a gRNA guide sequence comprises between 17 nucleotides and 22 nucleotides. In some aspects, a gRNA guide sequence comprises between 17 nucleotides and 20 nucleotides. In some aspects, a gRNA guide sequence comprises between 20 nucleotides and 25 nucleotides.

In some aspects, a gRNA comprises at least 15 nucleotides. In some aspects, a gRNA comprises at least 16 nucleotides. In some aspects, a gRNA comprises at least 17 nucleotides. In some aspects, a gRNA comprises at least 18 nucleotides. In some aspects, a gRNA comprises at least 19 nucleotides. In some aspects, a gRNA comprises at least 20 nucleotides. In some aspects, a gRNA comprises at least 21 nucleotides. In some aspects, a gRNA comprises at least 22 nucleotides. In some aspects, a gRNA comprises at least 23 nucleotides. In some aspects, a gRNA comprises at least 24 nucleotides. In some aspects, a gRNA comprises at least 25 nucleotides. In some aspects, a gRNA comprises at least 26 nucleotides. In some aspects, a gRNA comprises at least 27 nucleotides. In some aspects, a gRNA comprises at least 28 nucleotides. In some aspects, a gRNA comprises at least 29 nucleotides. In some aspects, a gRNA comprises at least 30 nucleotides. In some aspects, a gRNA comprises at least 35 nucleotides. In some aspects, a gRNA comprises at least 40 nucleotides. In some aspects, a gRNA comprises at least 45 nucleotides. In some aspects, a gRNA comprises at least 50 nucleotides. In some aspects, a gRNA comprises at least 75 nucleotides. In some aspects, a gRNA comprises at least 100 nucleotides. In some aspects, a gRNA comprises at least 125 nucleotides. In some aspects, a gRNA comprises at least 150 nucleotides. In some aspects, a gRNA comprises at least 200 nucleotides.

In some aspects, a gRNA comprises between 15 nucleotides and 250 nucleotides. In some aspects, a gRNA comprises between 15 nucleotides and 200 nucleotides. In some aspects, a gRNA comprises between 15 nucleotides and 150 nucleotides. In some aspects, a gRNA comprises between 15 nucleotides and 125 nucleotides. In some aspects, a gRNA comprises between 15 nucleotides and 100 nucleotides. In some aspects, a gRNA comprises between 15 nucleotides and 75 nucleotides. In some aspects, a gRNA comprises between 20 nucleotides and 50 nucleotides. In some aspects, a gRNA comprises between 20 nucleotides and 250 nucleotides. In some aspects, a gRNA comprises between 20 nucleotides and 200 nucleotides. In some aspects, a gRNA comprises between 20 nucleotides and 150 nucleotides. In some aspects, a gRNA comprises between 20 nucleotides and 125 nucleotides. In some aspects, a gRNA comprises between 20 nucleotides and 100 nucleotides. In some aspects, a gRNA comprises between 50 nucleotides and 250 nucleotides. In some aspects, a gRNA comprises between 50 nucleotides and 200 nucleotides. In some aspects, a gRNA comprises between 50 nucleotides and 150 nucleotides. In some aspects, a gRNA comprises between 50 nucleotides and 125 nucleotides. In some aspects, a gRNA comprises between 50 nucleotides and 100 nucleotides. In some aspects, a gRNA comprises between 75 nucleotides and 250 nucleotides. In some aspects, a gRNA comprises between 75 nucleotides and 200 nucleotides. In some aspects, a gRNA comprises between 75 nucleotides and 150 nucleotides. In some aspects, a gRNA comprises between 75 nucleotides and 125 nucleotides. In some aspects, a gRNA comprises between 75 nucleotides and 100 nucleotides. In some aspects, a gRNA comprises between 100 nucleotides and 250 nucleotides. In some aspects, a gRNA comprises between 100 nucleotides and 200 nucleotides. In some aspects, a gRNA comprises between 100 nucleotides and 150 nucleotides. In some aspects, a gRNA comprises between 100 nucleotides and 125 nucleotides.

In some aspects, a gRNA comprises a nucleic acid sequence at least 90% identical or complementary to at least 17 consecutive nucleotides of an arthropod male-fertility essential gene. In some aspects, a gRNA comprises a nucleic acid sequence 100% identical or complementary to at least 17 consecutive nucleotides of an arthropod male-fertility essential gene. In some aspects, a gRNA comprises a nucleic acid sequence at least 90% identical or complementary to at least 18 consecutive nucleotides of an arthropod male-fertility essential gene. In some aspects, a gRNA comprises a nucleic acid sequence at least 95% identical or complementary to at least 18 consecutive nucleotides of an arthropod male-fertility essential gene. In some aspects, a gRNA comprises a nucleic acid sequence 100% identical or complementary to at least 18 consecutive nucleotides of an arthropod male-fertility essential gene. In some aspects, a gRNA comprises a nucleic acid sequence at least 90% identical or complementary to at least 19 consecutive nucleotides of an arthropod male-fertility essential gene. In some aspects, a gRNA comprises a nucleic acid sequence 100% identical or complementary to at least 19 consecutive nucleotides of an arthropod male-fertility essential gene. In some aspects, a gRNA comprises a nucleic acid sequence at least 90% identical or complementary to at least 20 consecutive nucleotides of an arthropod male-fertility essential gene. In some aspects, a gRNA comprises a nucleic acid sequence at least 95% identical or complementary to at least 20 consecutive nucleotides of an arthropod male-fertility essential gene. In some aspects, a gRNA comprises a nucleic acid sequence 100% identical or complementary to at least 20 consecutive nucleotides of an arthropod male-fertility essential gene. In some aspects, a gRNA comprises a nucleic acid sequence at least 90% identical or complementary to at least 21 consecutive nucleotides of an arthropod male-fertility essential gene. In some aspects, a gRNA comprises a nucleic acid sequence at least 95% identical or complementary to at least 21 consecutive nucleotides of an arthropod male-fertility essential gene. In some aspects, a gRNA comprises a nucleic acid sequence 100% identical or complementary to at least 21 consecutive nucleotides of an arthropod male-fertility essential gene. In some aspects, a gRNA comprises a nucleic acid sequence at least 90% identical or complementary to at least 22 consecutive nucleotides of an arthropod male-fertility essential gene. In some aspects, a gRNA comprises a nucleic acid sequence at least 95% identical or complementary to at least 22 consecutive nucleotides of an arthropod male-fertility essential gene. In some aspects, a gRNA comprises a nucleic acid sequence 100% identical or complementary to at least 22 consecutive nucleotides of an arthropod male-fertility essential gene. In some aspects, a gRNA comprises a nucleic acid sequence at least 90% identical or complementary to at least 23 consecutive nucleotides of an arthropod male-fertility essential gene. In some aspects, a gRNA comprises a nucleic acid sequence at least 95% identical or complementary to at least 23 consecutive nucleotides of an arthropod male-fertility essential gene. In some aspects, a gRNA comprises a nucleic acid sequence 100% identical or complementary to at least 23 consecutive nucleotides of an arthropod male-fertility essential gene. In some aspects, a gRNA comprises a nucleic acid sequence at least 80% identical or complementary to at least 24 consecutive nucleotides of an arthropod male-fertility essential gene. In some aspects, a gRNA comprises a nucleic acid sequence at least 85% identical or complementary to at least 24 consecutive nucleotides of an arthropod male-fertility essential gene. In some aspects, a gRNA comprises a nucleic acid sequence at least 90% identical or complementary to at least 24 consecutive nucleotides of an arthropod male-fertility essential gene. In some aspects, a gRNA comprises a nucleic acid sequence at least 95% identical or complementary to at least 24 consecutive nucleotides of an arthropod male-fertility essential gene. In some aspects, a gRNA comprises a nucleic acid sequence 100% identical or complementary to at least 24 consecutive nucleotides of an arthropod male-fertility essential gene.

In some aspects, a gRNA guide sequence is binds to an exon sequence of an arthropod male-fertility essential gene. In some aspects, a gRNA guide sequence is binds to an intron sequence of an arthropod male-fertility essential gene. In some aspects, a gRNA guide sequence is binds to a promoter sequence of an arthropod male-fertility essential gene. In some aspects, a gRNA guide sequence is binds to a 5'-untranslated region (UTR) sequence of an arthropod male-fertility essential gene. In some aspects, a gRNA guide sequence is binds to a 3'-UTR sequence of an arthropod male-fertility essential gene. In some aspects, a gRNA guide sequence is binds to an intron/exon boundary of an arthropod male-fertility essential gene.

In some aspects a gRNA comprises a nucleic acid sequence at least 80% identical to a nucleic acid sequence selected from the group consisting of SEQ ID NOs: 1 to 2879 and 2881 to 2902. In some aspects a gRNA comprises a nucleic acid sequence at least 85% identical to a nucleic acid sequence selected from the group consisting of SEQ ID NOs: 1 to 2879 and 2881 to 2902. In some aspects a gRNA comprises a nucleic acid sequence at least 90% identical to a nucleic acid sequence selected from the group consisting of SEQ ID NOs: 1 to 2879 and 2881 to 2902. In some aspects a gRNA comprises a nucleic acid sequence at least 91% identical to a nucleic acid sequence selected from the group consisting of SEQ ID NOs: 1 to 2879 and 2881 to 2902. In some aspects a gRNA comprises a nucleic acid sequence at least 92% identical to a nucleic acid sequence selected from the group consisting of SEQ ID NOs: 1 to 2879 and 2881 to 2902. In some aspects a gRNA comprises a nucleic acid sequence at least 93% identical to a nucleic acid sequence selected from the group consisting of SEQ ID NOs: 1 to 2879 and 2881 to 2902. In some aspects a gRNA comprises a nucleic acid sequence at least 94% identical to a nucleic acid sequence selected from the group consisting of SEQ ID NOs: 1 to 2879 and 2881 to 2902. In some aspects a gRNA comprises a nucleic acid sequence at least 95% identical to a nucleic acid sequence selected from the group consisting of SEQ ID NOs: 1 to 2879 and 2881 to 2902. In some aspects a gRNA comprises a nucleic acid sequence at least 96% identical to a nucleic acid sequence selected from the group consisting of SEQ ID NOs: 1 to 2879 and 2881 to 2902. In some aspects a gRNA comprises a nucleic acid sequence at least 97% identical to a nucleic acid sequence selected from the group consisting of SEQ ID NOs: 1 to 2879 and 2881 to 2902. In some aspects a gRNA comprises a nucleic acid sequence at least 98% identical to a nucleic acid sequence selected from the group consisting of SEQ ID NOs: 1 to 2879 and 2881 to 2902. In some aspects a gRNA comprises a nucleic acid sequence at least 99% identical to a nucleic acid sequence selected from the group consisting of SEQ ID NOs: 1 to 2879 and 2881 to 2902. In some aspects a gRNA comprises a nucleic acid sequence 100% identical to a nucleic acid sequence selected from the group consisting of SEQ ID NOs: 1 to 2879 and 2881 to 2902.

In some aspects, a gRNA comprises a nucleic acid sequence at least 80% identical or complementary to a nucleic acid sequence selected from the group consisting of SEQ ID NOs: 2903 to 4011, 4342, 4343, and 4345 to 4385. In some aspects, a gRNA comprises a nucleic acid sequence at least 85% identical or complementary to a nucleic acid sequence selected from the group consisting of SEQ ID NOs: 2903 to 4011, 4342, 4343, and 4345 to 4385. In some aspects, a gRNA comprises a nucleic acid sequence at least 90% identical or complementary to a nucleic acid sequence selected from the group consisting of SEQ ID NOs: 2903 to 4011, 4342, 4343, and 4345 to 4385. In some aspects, a gRNA comprises a nucleic acid sequence at least 91% identical or complementary to a nucleic acid sequence selected from the group consisting of SEQ ID NOs: 2903 to 4011, 4342, 4343, and 4345 to 4385. In some aspects, a gRNA comprises a nucleic acid sequence at least 92% identical or complementary to a nucleic acid sequence selected from the group consisting of SEQ ID NOs: 2903 to 4011, 4342, 4343, and 4345 to 4385. In some aspects, a gRNA comprises a nucleic acid sequence at least 93% identical or complementary to a nucleic acid sequence selected from the group consisting of SEQ ID NOs: 2903 to 4011, 4342, 4343, and 4345 to 4385. In some aspects, a gRNA comprises a nucleic acid sequence at least 94% identical or complementary to a nucleic acid sequence selected from the group consisting of SEQ ID NOs: 2903 to 4011, 4342, 4343, and 4345 to 4385. In some aspects, a gRNA comprises a nucleic acid sequence at least 95% identical or complementary to a nucleic acid sequence selected from the group consisting of SEQ ID NOs: 2903 to 4011, 4342, 4343, and 4345 to 4385. In some aspects, a gRNA comprises a nucleic acid sequence at least 96% identical or complementary to a nucleic acid sequence selected from the group consisting of SEQ ID NOs: 2903 to 4011, 4342, 4343, and 4345 to 4385. In some aspects, a gRNA comprises a nucleic acid sequence at least 97% identical or complementary to a nucleic acid sequence selected from the group consisting of SEQ ID NOs: 2903 to 4011, 4342, 4343, and 4345 to 4385. In some aspects, a gRNA comprises a nucleic acid sequence at least 98% identical or complementary to a nucleic acid sequence selected from the group consisting of SEQ ID NOs: 2903 to 4011, 4342, 4343, and 4345 to 4385. In some aspects, a gRNA comprises a nucleic acid sequence at least 99% identical or complementary to a nucleic acid sequence selected from the group consisting of SEQ ID NOs: 2903 to 4011, 4342, 4343, and 4345 to 4385. In some aspects, a gRNA comprises a nucleic acid sequence at least 100% identical or complementary to a nucleic acid sequence selected from the group consisting of SEQ ID NOs: 2903 to 4011, 4342, 4343, and 4345 to 4385.

In some aspects, a male sterile effector comprises a first promoter operably linked to a first nucleic acid molecule encoding a small RNA molecule comprising a nucleic acid sequence at least 90% identical to at least 17 consecutive nucleotides of a first arthropod male-fertility essential gene messenger RNA molecule. In some aspects, a male sterile effector comprises a first promoter operably linked to a first nucleic acid molecule encoding a microRNA (miRNA) molecule or a short hairpin RNA (shRNA) molecule comprising a nucleic acid sequence at least 90% identical to at least 17 consecutive nucleotides of a first arthropod male-fertility essential gene messenger RNA molecule. In some aspects, a male sterile effector comprises a first promoter operably linked to a first nucleic acid molecule encoding a small RNA molecule comprising a nucleic acid sequence at least 80% identical to a first arthropod male-fertility essential gene messenger RNA molecule.

It will be appreciated that perfect complementarity is not required for binding of a small RNA molecule to a target sequence. In some aspects, a small RNA molecule comprises 1, 2, 3, 4, 5, or 6 mismatches when compared to a target sequence of an arthropod male-fertility essential gene.

As used herein, the term "small RNA molecule" refers to any non-coding RNA molecule less than 200 nucleotides in length. In addition to providing small RNA molecules, this disclosure also provides a small RNA precursor molecule for any small RNA molecule.

In some aspects, a small RNA molecule comprises between 18 nucleotides and 30 nucleotides in length. In another aspect, a small RNA molecule comprises between 18 nucleotides and 24 nucleotides in length. In another aspect, a small RNA molecule comprises between 18 nucleotides and 22 nucleotides in length.

In another aspect, a small RNA molecule is 18 nucleotides in length. In another aspect, a small RNA molecule is 19 nucleotides in length. In another aspect, a small RNA molecule is 20 nucleotides in length. In another aspect, a small RNA molecule is 21 nucleotides in length. In another aspect, a small RNA molecule is 22 nucleotides in length. In another aspect, a small RNA molecule is 23 nucleotides in length. In another aspect, a small RNA molecule is 24 nucleotides in length. In another aspect, a small RNA molecule is 25 nucleotides in length. In another aspect, a small RNA molecule is 26 nucleotides in length. In another aspect, a small RNA molecule is 27 nucleotides in length. In another aspect, a small RNA molecule is 28 nucleotides in length.

In another aspect, a small RNA molecule is at least 18 nucleotides in length. In another aspect, a small RNA molecule is at least 19 nucleotides in length. In another aspect, a small RNA molecule is at least 20 nucleotides in length. In another aspect, a small RNA molecule is at least 21 nucleotides in length. In another aspect, a small RNA molecule is at least 22 nucleotides in length. In another aspect, a small RNA molecule is at least 23 nucleotides in length. In another aspect, a small RNA molecule is at least 24 nucleotides in length. In another aspect, a small RNA molecule is at least 25 nucleotides in length. In another aspect, a small RNA molecule is at least 26 nucleotides in length. In another aspect, a small RNA molecule is at least 27 nucleotides in length. In another aspect, a small RNA molecule is at least 28 nucleotides in length.

In some aspects, a small RNA molecule comprises between 18 nucleotides and 30 nucleotides. In another aspect, a small RNA molecule comprises between 18 nucleotides and 24 nucleotides. In another aspect, a small RNA molecule comprises between 18 nucleotides and 21 nucleotides. In another aspect, a small RNA molecule comprises between 21 nucleotides and 24 nucleotides. In another aspect, a small RNA molecule comprises between 21 nucleotides and 30 nucleotides.

In some aspects, a small RNA molecule provided herein is a microRNA (miRNA) molecule. In some aspects, a small RNA molecule provided herein is an artificial miRNA molecule. In some aspects, a small RNA molecule provided herein is a short hairpin RNA (shRNA) molecule. In another aspect, a small RNA molecule provided herein is a small interfering RNA (siRNA) molecule. In another aspect, a small RNA molecule provided herein is a heterochromatic siRNA (hc-siRNA) molecule. In another aspect, a small RNA molecule provided herein is a Piwi-interacting RNA (piRNA) molecule. In some aspects, a small RNA molecule provided herein is a double-stranded RNA (dsRNA) molecule. In another aspect, a small RNA molecule provided herein is a hairpin double-stranded RNA (hp-dsRNA) molecule. In another aspect, a small RNA molecule provided herein is a *trans*acting siRNA (ta-siRNA) molecule. In another aspect, a small RNA molecule provided herein is a naturally occurring antisense siRNA (nat-siRNA) molecule. In another aspect, a small RNA molecule provided herein is a Cas9-guide RNA (gRNA). In another aspect, a small RNA molecule provided herein is a gRNA molecule

Without being limiting, a miRNA or shRNA molecule typically comprises, from 5' to 3', a 5' sequence, a loop structure, a miRNA spacer sequence (which binds to a target site), and a 3' sequence. In some aspects, a miRNA or shRNA molecule comprises, from 5' to 3', a 5' sequence *(e.g.,* SEQ ID NO: 6841 or 6842), a loop structure *(e.g.,* SEQ ID NO: 6844), a miRNA spacer sequence (e.g., one of SEQ ID NOs: 6832 to 6840), and a 3' sequence (e.g., SEQ ID NO: 6846). In some aspects, a miRNA or shRNA molecule comprises, from 5' to 3', a 5' sequence *(e.g.,* SEQ ID NO: 6843), a loop structure *(e.g.,* SEQ ID NO: 6845), a miRNA spacer sequence (e.g., one of SEQ ID NOs: 6832 to 6840), and a 3' sequence (e.g., SEQ ID NO: 6847).

In some aspects, a small RNA molecule is selected from the group consisting of a miRNA molecule and an shRNA molecule. In some aspects, a small RNA molecule is selected from the group consisting of a dsRNA molecule, a siRNA molecule, an shRNA molecule, and a miRNA molecule. In another aspect, a small RNA molecule is selected from the group consisting of a miRNA molecule, an siRNA molecule, an shRNA molecule, an hc-siRNA molecule, a piRNA molecule, a dsRNA molecule, an hp-dsRNA molecule, a ta-siRNA molecule, a nat-siRNA molecule, and a gRNA molecule.

In some aspects, a small RNA molecule comprises a nucleic acid sequence having at least 80% identity or complementarity with an arthropod male-fertility essential gene mRNA molecule. In some aspects, a small RNA molecule comprises a nucleic acid sequence having at least 85% identity or complementarity with an arthropod male-fertility essential gene mRNA molecule. In some aspects, a small RNA molecule comprises a nucleic acid sequence having at least 90% identity or complementarity with an arthropod male-fertility essential gene mRNA molecule. In some aspects, a small RNA molecule comprises a nucleic acid sequence having at least 91% identity or complementarity with an arthropod male-fertility essential gene mRNA molecule. In some aspects, a small RNA molecule comprises a nucleic acid sequence having at least 92% identity or complementarity with an arthropod male-fertility essential gene mRNA molecule. In some aspects, a small RNA molecule comprises a nucleic acid sequence having at least 93% identity or complementarity with an arthropod male-fertility essential gene mRNA molecule. In some aspects, a small RNA molecule comprises a nucleic acid sequence having at least 94% identity or complementarity with an arthropod male-fertility essential gene mRNA molecule. In some aspects, a small RNA molecule comprises a nucleic acid sequence having at least 95% identity or complementarity with an arthropod male-fertility essential gene mRNA molecule. In some aspects, a small RNA molecule comprises a nucleic acid sequence having at least 96% identity or complementarity with an arthropod male-fertility essential gene mRNA molecule. In some aspects, a small RNA molecule comprises a nucleic acid sequence having at least 97% identity or complementarity with an arthropod male-fertility essential gene mRNA molecule. In some aspects, a small RNA molecule comprises a nucleic acid sequence having at least 98% identity or complementarity with an arthropod male-fertility essential gene mRNA molecule. In some aspects, a small RNA molecule comprises a nucleic acid sequence having at least 99% identity or complementarity with an arthropod male-fertility essential gene mRNA molecule. In some aspects, a small RNA molecule comprises a nucleic acid sequence having 100% identity or complementarity with an arthropod male-fertility essential gene mRNA molecule.

In some aspects, a miRNA molecule or shRNA molecule comprises a nucleic acid sequence having at least 80% identity or complementarity with an arthropod male-fertility essential gene mRNA molecule. In some aspects, a miRNA molecule or shRNA molecule comprises a nucleic acid sequence having at least 85% identity or complementarity with an arthropod male-fertility essential gene mRNA molecule. In some aspects, a miRNA molecule or shRNA molecule comprises a nucleic acid sequence having at least 90% identity or complementarity with an arthropod male-fertility essential gene mRNA molecule. In some aspects, a miRNA molecule or shRNA molecule comprises a nucleic acid sequence having at least 91% identity or complementarity with an arthropod male-fertility essential gene mRNA molecule. In some aspects, a miRNA molecule or shRNA molecule comprises a nucleic acid sequence having at least 92% identity or complementarity with an arthropod male-fertility essential gene mRNA molecule. In some aspects, a miRNA molecule or shRNA molecule comprises a nucleic acid sequence having at least 93% identity or complementarity with an arthropod male-fertility essential gene mRNA molecule. In some aspects, a miRNA molecule or shRNA molecule comprises a nucleic acid sequence having at least 94% identity or complementarity with an arthropod male-fertility essential gene mRNA molecule. In some aspects, a miRNA molecule or shRNA molecule comprises a nucleic acid sequence having at least 95% identity or complementarity with an arthropod male-fertility essential gene mRNA molecule. In some aspects, a miRNA molecule or shRNA molecule comprises a nucleic acid sequence having at least 96% identity or complementarity with an arthropod male-fertility essential gene mRNA molecule. In some aspects, a miRNA molecule or shRNA molecule comprises a nucleic acid sequence having at least 97% identity or complementarity with an arthropod male-fertility essential gene mRNA molecule. In some aspects, a miRNA molecule or shRNA molecule comprises a nucleic acid sequence having at least 98% identity or complementarity with an arthropod male-fertility essential gene mRNA molecule. In some aspects, a miRNA molecule or shRNA molecule comprises a nucleic acid sequence having at least 99% identity or complementarity with an arthropod male-fertility essential gene mRNA molecule. In some aspects, a miRNA molecule or shRNA molecule comprises a nucleic acid sequence having 100% identity or complementarity with an arthropod male-fertility essential gene mRNA molecule.

In some aspects, a small RNA molecule comprises a nucleic acid sequence at least 90% identical to at least 17 consecutive nucleotides of an arthropod male-fertility essential gene. In some aspects, a small RNA molecule comprises a nucleic acid sequence 100% identical to at least 17 consecutive nucleotides of an arthropod male-fertility essential gene. In some aspects, a small RNA molecule comprises a nucleic acid sequence at least 90% identical to at least 18 consecutive nucleotides of an arthropod male-fertility essential gene. In some aspects, a small RNA molecule comprises a nucleic acid sequence at least 95% identical to at least 18 consecutive nucleotides of an arthropod male-fertility essential gene. In some aspects, a small RNA molecule comprises a nucleic acid sequence 100% identical to at least 18 consecutive nucleotides of an arthropod male-fertility essential gene. In some aspects, a small RNA molecule comprises a nucleic acid sequence at least 90% identical to at least 19 consecutive nucleotides of an arthropod male-fertility essential gene. In some aspects, a small RNA molecule comprises a nucleic acid sequence 100% identical to at least 19 consecutive nucleotides of an arthropod male-fertility essential gene. In some aspects, a small RNA molecule comprises a nucleic acid sequence at least 85% identical to at least 20 consecutive nucleotides of an arthropod male-fertility essential gene. In some aspects, a small RNA molecule comprises a nucleic acid sequence at least 90% identical to at least 20 consecutive nucleotides of an arthropod male-fertility essential gene. In some aspects, a small RNA molecule comprises a nucleic acid sequence at least 95% identical to at least 20 consecutive nucleotides of an arthropod male-fertility essential gene. In some aspects, a small RNA molecule comprises a nucleic acid sequence 100% identical to at least 20 consecutive nucleotides of an arthropod male-fertility essential gene. In some aspects, a small RNA molecule comprises a nucleic acid sequence at least 85% identical to at least 21 consecutive nucleotides of an arthropod male-fertility essential gene. In some aspects, a small RNA molecule comprises a nucleic acid sequence at least 90% identical to at least 21 consecutive nucleotides of an arthropod male-fertility essential gene. In some aspects, a small RNA molecule comprises a nucleic acid sequence at least 95% identical to at least 21 consecutive nucleotides of an arthropod male-fertility essential gene. In some aspects, a small RNA molecule comprises a nucleic acid sequence 100% identical to at least 21 consecutive nucleotides of an arthropod male-fertility essential gene. In some aspects, a small RNA molecule comprises a nucleic acid sequence at least 85% identical to at least 22 consecutive nucleotides of an arthropod male-fertility essential gene. In some aspects, a small RNA molecule comprises a nucleic acid sequence at least 90% identical to at least 22 consecutive nucleotides of an arthropod male-fertility essential gene. In some aspects, a small RNA molecule comprises a nucleic acid sequence at least 95% identical to at least 22 consecutive nucleotides of an arthropod male-fertility essential gene. In some aspects, a small RNA molecule comprises a nucleic acid sequence 100% identical to at least 22 consecutive nucleotides of an arthropod male-fertility essential gene. In some aspects, a small RNA molecule comprises a nucleic acid sequence at least 85% identical to at least 23 consecutive nucleotides of an arthropod male-fertility essential gene. In some aspects, a small RNA molecule comprises a nucleic acid sequence at least 90% identical to at least 23 consecutive nucleotides of an arthropod male-fertility essential gene. In some aspects, a small RNA molecule comprises a nucleic acid sequence at least 95% identical to at least 23 consecutive nucleotides of an arthropod male-fertility essential gene. In some aspects, a small RNA molecule comprises a nucleic acid sequence 100% identical to at least 23 consecutive nucleotides of an arthropod male-fertility essential gene. In some aspects, a small RNA molecule comprises a nucleic acid sequence at least 85% identical to at least 24 consecutive nucleotides of an arthropod male-fertility essential gene. In some aspects, a small RNA molecule comprises a nucleic acid sequence at least 90% identical to at least 24 consecutive nucleotides of an arthropod male-fertility essential gene. In some aspects, a small RNA molecule comprises a nucleic acid sequence at least 95% identical to at least 24 consecutive nucleotides of an arthropod male-fertility essential gene. In some aspects, a small RNA molecule comprises a nucleic acid sequence 100% identical to at least 24 consecutive nucleotides of an arthropod male-fertility essential gene.

In some aspects, a miRNA molecule or an shRNA molecule comprises a nucleic acid sequence at least 90% identical to at least 19 consecutive nucleotides of an arthropod male-fertility essential gene. In some aspects, a miRNA molecule or an shRNA molecule comprises a nucleic acid sequence 100% identical to at least 19 consecutive nucleotides of an arthropod male-fertility essential gene. In some aspects, a miRNA molecule or an shRNA molecule comprises a nucleic acid sequence at least 85% identical to at least 20 consecutive nucleotides of an arthropod male-fertility essential gene. In some aspects, a miRNA molecule or an shRNA molecule comprises a nucleic acid sequence at least 90% identical to at least 20 consecutive nucleotides of an arthropod male-fertility essential gene. In some aspects, a miRNA molecule or an shRNA molecule comprises a nucleic acid sequence at least 95% identical to at least 20 consecutive nucleotides of an arthropod male-fertility essential gene. In some aspects, a miRNA molecule or an shRNA molecule comprises a nucleic acid sequence 100% identical to at least 20 consecutive nucleotides of an arthropod male-fertility essential gene. In some aspects, a miRNA molecule or an shRNA molecule comprises a nucleic acid sequence at least 85% identical to at least 21 consecutive nucleotides of an arthropod male-fertility essential gene. In some aspects, a miRNA molecule or an shRNA molecule comprises a nucleic acid sequence at least 90% identical to at least 21 consecutive nucleotides of an arthropod male-fertility essential gene. In some aspects, a miRNA molecule or an shRNA molecule comprises a nucleic acid sequence at least 95% identical to at least 21 consecutive nucleotides of an arthropod male-fertility essential gene. In some aspects, a miRNA molecule or an shRNA molecule comprises a nucleic acid sequence 100% identical to at least 21 consecutive nucleotides of an arthropod male-fertility essential gene. In some aspects, a miRNA molecule or an shRNA molecule comprises a nucleic acid sequence at least 85% identical to at least 22 consecutive nucleotides of an arthropod male-fertility essential gene. In some aspects, a miRNA molecule or an shRNA molecule comprises a nucleic acid sequence at least 90% identical to at least 22 consecutive nucleotides of an arthropod male-fertility essential gene. In some aspects, a miRNA molecule or an shRNA molecule comprises a nucleic acid sequence at least 95% identical to at least 22 consecutive nucleotides of an arthropod male-fertility essential gene. In some aspects, a miRNA molecule or an shRNA molecule comprises a nucleic acid sequence 100% identical to at least 22 consecutive nucleotides of an arthropod male-fertility essential gene. In some aspects, a miRNA molecule or an shRNA molecule comprises a nucleic acid sequence at least 85% identical to at least 23 consecutive nucleotides of an arthropod male-fertility essential gene. In some aspects, a miRNA molecule or an shRNA molecule comprises a nucleic acid sequence at least 90% identical to at least 23 consecutive nucleotides of an arthropod male-fertility essential gene. In some aspects, a miRNA molecule or an shRNA molecule comprises a nucleic acid sequence at least 95% identical to at least 23 consecutive nucleotides of an arthropod male-fertility essential gene. In some aspects, a miRNA molecule or an shRNA molecule comprises a nucleic acid sequence 100% identical to at least 23 consecutive nucleotides of an arthropod male-fertility essential gene. In some aspects, a miRNA molecule or an shRNA molecule comprises a nucleic acid sequence at least 85% identical to at least 24 consecutive nucleotides of an arthropod male-fertility essential gene. In some aspects, a miRNA molecule or an shRNA molecule comprises a nucleic acid sequence at least 90% identical to at least 24 consecutive nucleotides of an arthropod male-fertility essential gene. In some aspects, a miRNA molecule or an shRNA molecule comprises a nucleic acid sequence at least 95% identical to at least 24 consecutive nucleotides of an arthropod male-fertility essential gene. In some aspects, a miRNA molecule or an shRNA molecule comprises a nucleic acid sequence 100% identical to at least 24 consecutive nucleotides of an arthropod male-fertility essential gene.

In some aspects, a miRNA molecule or an shRNA molecule comprises a nucleic acid sequence at least 80% identical to a nucleic acid sequence selected from the group consisting of SEQ ID NOs: 1 to 2879 and 2881 to 2902. In some aspects, a miRNA molecule or an shRNA molecule comprises a nucleic acid sequence at least 85% identical to a nucleic acid sequence selected from the group consisting of SEQ ID NOs: 1 to 2879 and 2881 to 2902. In some aspects, a miRNA molecule or an shRNA molecule comprises a nucleic acid sequence at least 90% identical to a nucleic acid sequence selected from the group consisting of SEQ ID NOs: 1 to 2879 and 2881 to 2902. In some aspects, a miRNA molecule or an shRNA molecule comprises a nucleic acid sequence at least 91% identical to a nucleic acid sequence selected from the group consisting of SEQ ID NOs: 1 to 2879 and 2881 to 2902. In some aspects, a miRNA molecule or an shRNA molecule comprises a nucleic acid sequence at least 92% identical to a nucleic acid sequence selected from the group consisting of SEQ ID NOs: 1 to 2879 and 2881 to 2902. In some aspects, a miRNA molecule or an shRNA molecule comprises a nucleic acid sequence at least 93% identical to a nucleic acid sequence selected from the group consisting of SEQ ID NOs: 1 to 2879 and 2881 to 2902. In some aspects, a miRNA molecule or an shRNA molecule comprises a nucleic acid sequence at least 94% identical to a nucleic acid sequence selected from the group consisting of SEQ ID NOs: 1 to 2879 and 2881 to 2902. In some aspects, a miRNA molecule or an shRNA molecule comprises a nucleic acid sequence at least 95% identical to a nucleic acid sequence selected from the group consisting of SEQ ID NOs: 1 to 2879 and 2881 to 2902. In some aspects, a miRNA molecule or an shRNA molecule comprises a nucleic acid sequence at least 96% identical to a nucleic acid sequence selected from the group consisting of SEQ ID NOs: 1 to 2879 and 2881 to 2902. In some aspects, a miRNA molecule or an shRNA molecule comprises a nucleic acid sequence at least 97% identical to a nucleic acid sequence selected from the group consisting of SEQ ID NOs: 1 to 2879 and 2881 to 2902. In some aspects, a miRNA molecule or an shRNA molecule comprises a nucleic acid sequence at least 98% identical to a nucleic acid sequence selected from the group consisting of SEQ ID NOs: 1 to 2879 and 2881 to 2902. In some aspects, a miRNA molecule or an shRNA molecule comprises a nucleic acid sequence at least 99% identical to a nucleic acid sequence selected from the group consisting of SEQ ID NOs: 1 to 2879 and 2881 to 2902. In some aspects, a miRNA molecule or an shRNA molecule comprises a nucleic acid sequence 100% identical to a nucleic acid sequence selected from the group consisting of SEQ ID NOs: 1 to 2879 and 2881 to 2902.

In some aspects, a miRNA molecule comprises a nucleic acid sequence at least 80% identical to a nucleic acid sequence selected from the group consisting of SEQ ID NOs: 6832 to 6847. In some aspects, a miRNA molecule comprises a nucleic acid sequence at least 85% identical to a nucleic acid sequence selected from the group consisting of SEQ ID NOs: 6832 to 6847. In some aspects, a miRNA molecule comprises a nucleic acid sequence at least 90% identical to a nucleic acid sequence selected from the group consisting of SEQ ID NOs: 6832 to 6847. In some aspects, a miRNA molecule comprises a nucleic acid sequence at least 91% identical to a nucleic acid sequence selected from the group consisting of SEQ ID NOs: 6832 to 6847. In some aspects, a miRNA molecule comprises a nucleic acid sequence at least 92% identical to a nucleic acid sequence selected from the group consisting of SEQ ID NOs: 6832 to 6847. In some aspects, a miRNA molecule comprises a nucleic acid sequence at least 93% identical to a nucleic acid sequence selected from the group consisting of SEQ ID NOs: 6832 to 6847. In some aspects, a miRNA molecule comprises a nucleic acid sequence at least 94% identical to a nucleic acid sequence selected from the group consisting of SEQ ID NOs: 6832 to 6847. In some aspects, a miRNA molecule comprises a nucleic acid sequence at least 95% identical to a nucleic acid sequence selected from the group consisting of SEQ ID NOs: 6832 to 6847. In some aspects, a miRNA molecule comprises a nucleic acid sequence at least 96% identical to a nucleic acid sequence selected from the group consisting of SEQ ID NOs: 6832 to 6847. In some aspects, a miRNA molecule comprises a nucleic acid sequence at least 97% identical to a nucleic acid sequence selected from the group consisting of SEQ ID NOs: 6832 to 6847. In some aspects, a miRNA molecule comprises a nucleic acid sequence at least 98% identical to a nucleic acid sequence selected from the group consisting of SEQ ID NOs: 6832 to 6847. In some aspects, a miRNA molecule comprises a nucleic acid sequence at least 99% identical to a nucleic acid sequence selected from the group consisting of SEQ ID NOs: 6832 to 6847. In some aspects, a miRNA molecule comprises a nucleic acid sequence 100% identical to a nucleic acid sequence selected from the group consisting of SEQ ID NOs: 6832 to 6847.In some aspects, a miRNA molecule comprises a 5' sequence comprising a nucleic acid sequence selected from the group consisting of SEQ ID NOs: 6841 and 6842; a loop sequence comprising the nucleic acid sequence of SEQ ID NO: 6844; a miRNA spacer comprising a nucleic acid sequence selected from the group consisting of SEQ ID NOs: 6832 to 6840; and a 3' sequence comprising the nucleic acid sequence of SEQ ID NO: 6846. In some aspects, a miRNA molecule comprises a 5' sequence comprising the nucleic acid sequence of SEQ ID NO: 6843; a loop sequence comprising the nucleic acid sequence of SEQ ID NO: 6845; a miRNA spacer comprising a nucleic acid sequence selected from the group consisting of SEQ ID NOs: 6832 to 6840; and a 3' sequence comprising the nucleic acid sequence of SEQ ID NO: 6847.

In some aspects, this disclosure provides a sterility system. As used herein, a "sterility system" refers to a group of individual components that cause sterility or inviability in arthropods when appropriately expressed. Sterility systems comprise any male sterile effector provided herein and either a repressible female-lethal effector, a rescue element, or both a repressible female-lethal effector and a rescue element.

In some aspects, a sterility system comprises: (a) a male sterile effector; and (b) a repressible female-lethal effector. In some aspects, a sterility system comprises: (a) a male sterile effector; and (b) a rescue element comprising a fourth promoter operably linked to a nucleic acid molecule encoding at least one of: an anticas protein, and a functional copy of one or more arthropod male-fertility essential genes. In some aspects, a sterility system comprises: (a) a male sterile effector; and (b) a repressible female-lethal effector; and (c) a rescue element comprising a fourth promoter operably linked to a nucleic acid molecule encoding at least one of: an anticas protein, and a functional copy of one or more arthropod male-fertility essential genes.

In some aspects, a sterility system comprises: (a) a male sterile effector comprising a loss-of-function mutation in at least one male-fertility essential gene; and (b) a repressible female-lethal effector. In some aspects, a sterility system comprises: (a) a male sterile effector comprising a loss-of-function mutation in at least one male-fertility essential gene; and (b) a rescue element comprising a first promoter operably linked to a functional copy of the at least one male-fertility essential gene. In some aspects, a sterility system comprises: (a) a male sterile effector comprising a loss-of-function mutation in at least one male-fertility essential gene; (b) a rescue element comprising a first promoter operably linked to a functional copy of the at least one male-fertility essential gene; and (c) a repressible female-lethal effector.

In some aspects, this disclosure provides an isolated nucleic acid molecule encoding any sterility system provided herein. In some aspects, this disclosure provides at least one isolated nucleic acid molecule encoding any sterility system provided herein. In some aspects, this disclosure provides at least two isolated nucleic acid molecules encoding any sterility system provided herein. In some aspects, this disclosure provides at least three isolated nucleic acid molecules encoding any sterility system provided herein. In some aspects, this disclosure provides at least four isolated nucleic acid molecules encoding any sterility system provided herein. In some aspects, this disclosure provides at least five isolated nucleic acid molecules encoding any sterility system provided herein. In some aspects, this disclosure provides at least six isolated nucleic acid molecules encoding any sterility system provided herein.

As used herein, a "repressible female-lethal effector" refers to a nucleic acid sequence that encodes a nucleic acid sequence or protein that causes lethality when expressed in female arthropods operably linked to a repressible promoter. A "repressible promoter" is kept in an inactive state by providing an appropriate repressor (e.g., a suitable temperature, a drug or other chemical such as, without being limiting, tetracycline), which prevents the female-lethal effector from being expressed. However, in the absence of the repressor the repressible promoter initiates transcription of the female-lethal effector and causes lethality in females.

In some aspects, a repressible promoter is a drug-repressible promoter. As used herein, a "drug-repressible promoter" refers to a promoter that is inactive in the presence of a particular drug and active when the particular drug is absent. In some aspects, a drug-repressible promoter is a tetracycline-repressible promoter. Such promoters are inactive in the presence of tetracycline and active when tetracycline is absent.

Throughout this disclosure, doxycycline and other analogs of tetracycline can be used in place of tetracycline whenever tetracycline is referenced herein. Non-limiting examples of analogs of tetracycline or doxycycline include: chlortetracycline, oxytetracycline, demeclocylcline, lymecycline, meclocycline, methacycline, minocycline, rolitetracycline, tigecycline, eravacycline, sarecycline, and omadacycline.

Non-limiting examples of female-lethal effectors include: *Hid, grim, reaper, NipplDm,* calmodulin, *Michelob-X,* tTA, and tTAV (a variant of tTA). Such female-lethal effectors can be utilized with female-specific/male-specific splicing constructs (e.g., those originating from the *doublesex* gene) to ensure females are inviable while males are left unharmed. *See,* for example, WO 2019/243840. Without being limiting, one example of such a system includes a tetracycline response element (TRE) comprising seven repeats of the tetO sequence separated by short spacer sequences positioned upstream (to the 5'-end of) a minimal promoter operably linked to a female-specific splice module and a tTAV (a synthetic sequence comprising a tetracycline binding domain and a transactivator). tTAV can bind to the TRE element to enhance activity of the minimal promoter. When tetracycline is present, the tetracycline binds to any tTAV produced by the minimal promoter and prevents tTAV from binding to the TRE, and both male and female insects survive. However, when tetracycline is not present tTAV binds to the TRE to produce more tTAV which creates a positive feedback loop that creates enough tTAV to become cytotoxic and cause death only in females.

In some aspects, a tTA comprises an amino acid sequence of SEQ ID NO: 6496. In some aspects a tTAV comprises an amino acid sequence of SEQ ID NO: 6497.

In some aspects, a tTA is encoded by the nucleic acid sequence of SEQ ID NO: 4324. In some aspects, a tTAV is encoded by the nucleic acid sequence of SEQ ID NO: 4325.

In some aspects, a female-lethal effector comprises a nucleic acid sequence at least 80% identical to a nucleic acid sequence selected from the group consisting of SEQ ID NOs: 4324 to 4331. In some aspects, a female-lethal effector comprises a nucleic acid sequence at least 85% identical to a nucleic acid sequence selected from the group consisting of SEQ ID NOs: 4324 to 4331. In some aspects, a female-lethal effector comprises a nucleic acid sequence at least 90% identical to a nucleic acid sequence selected from the group consisting of SEQ ID NOs: 4324 to 4331. In some aspects, a female-lethal effector comprises a nucleic acid sequence at least 91% identical to a nucleic acid sequence selected from the group consisting of SEQ ID NOs: 4324 to 4331. In some aspects, a female-lethal effector comprises a nucleic acid sequence at least 92% identical to a nucleic acid sequence selected from the group consisting of SEQ ID NOs: 4324 to 4331. In some aspects, a female-lethal effector comprises a nucleic acid sequence at least 93% identical to a nucleic acid sequence selected from the group consisting of SEQ ID NOs: 4324 to 4331. In some aspects, a female-lethal effector comprises a nucleic acid sequence at least 94% identical to a nucleic acid sequence selected from the group consisting of SEQ ID NOs: 4324 to 4331. In some aspects, a female-lethal effector comprises a nucleic acid sequence at least 95% identical to a nucleic acid sequence selected from the group consisting of SEQ ID NOs: 4324 to 4331. In some aspects, a female-lethal effector comprises a nucleic acid sequence at least 96% identical to a nucleic acid sequence selected from the group consisting of SEQ ID NOs: 4324 to 4331. In some aspects, a female-lethal effector comprises a nucleic acid sequence at least 97% identical to a nucleic acid sequence selected from the group consisting of SEQ ID NOs: 4324 to 4331. In some aspects, a female-lethal effector comprises a nucleic acid sequence at least 98% identical to a nucleic acid sequence selected from the group consisting of SEQ ID NOs: 4324 to 4331. In some aspects, a female-lethal effector comprises a nucleic acid sequence at least 99% identical to a nucleic acid sequence selected from the group consisting of SEQ ID NOs: 4324 to 4331. In some aspects, a female-lethal effector comprises a nucleic acid sequence 100% identical to a nucleic acid sequence selected from the group consisting of SEQ ID NOs: 4324 to 4331.

In some aspects, a female-lethal effector comprises an amino acid sequence at least 80% identical or similar to an amino acid sequence selected from the group consisting of SEQ ID NOs: 6496 to 6504. In some aspects, a female-lethal effector comprises an amino acid sequence at least 85% identical or similar to an amino acid sequence selected from the group consisting of SEQ ID NOs: 6496 to 6504. In some aspects, a female-lethal effector comprises an amino acid sequence at least 90% identical or similar to an amino acid sequence selected from the group consisting of SEQ ID NOs: 6496 to 6504. In some aspects, a female-lethal effector comprises an amino acid sequence at least 91% identical or similar to an amino acid sequence selected from the group consisting of SEQ ID NOs: 6496 to 6504. In some aspects, a female-lethal effector comprises an amino acid sequence at least 92% identical or similar to an amino acid sequence selected from the group consisting of SEQ ID NOs: 6496 to 6504. In some aspects, a female-lethal effector comprises an amino acid sequence at least 93% identical or similar to an amino acid sequence selected from the group consisting of SEQ ID NOs: 6496 to 6504. In some aspects, a female-lethal effector comprises an amino acid sequence at least 94% identical or similar to an amino acid sequence selected from the group consisting of SEQ ID NOs: 6496 to 6504. In some aspects, a female-lethal effector comprises an amino acid sequence at least 95% identical or similar to an amino acid sequence selected from the group consisting of SEQ ID NOs: 6496 to 6504. In some aspects, a female-lethal effector comprises an amino acid sequence at least 96% identical or similar to an amino acid sequence selected from the group consisting of SEQ ID NOs: 6496 to 6504. In some aspects, a female-lethal effector comprises an amino acid sequence at least 97% identical or similar to an amino acid sequence selected from the group consisting of SEQ ID NOs: 6496 to 6504. In some aspects, a female-lethal effector comprises an amino acid sequence at least 98% identical or similar to an amino acid sequence selected from the group consisting of SEQ ID NOs: 6496 to 6504. In some aspects, a female-lethal effector comprises an amino acid sequence at least 99% identical or similar to an amino acid sequence selected from the group consisting of SEQ ID NOs: 6496 to 6504. In some aspects, a female-lethal effector comprises an amino acid sequence 100% identical or similar to an amino acid sequence selected from the group consisting of SEQ ID NOs: 6496 to 6504.

In some aspects, a repressible female-lethal effector is selected from the group consisting of: *Hid, grim, reaper, NipplDm,* calmodulin, *Michelob-X,* tTA, and tTAV, or variants thereof. In some aspects, a repressible female-lethal effector is a Class IV effector. In some aspects, a repressible female-lethal effector is a proapoptotic gene.

As used herein, a "female-specific splice module" refers to a sequence that is spliced in females, but not in males. In some aspects, a repressible female-lethal effector comprises a female-specific splice module. In some aspects, a female-specific splice module is selected from the group consisting of a *tra* female-specific splice module (SEQ ID NO: 4333 and 4334), a *dsx* female-specific splice module (SEQ ID NO: 4332), and an *actin-4* female-specific splice module. *See* Tan et al., Proc. Natl. Acad. Sci. USA, 110(17):6766-6770 (2013); Spinner et al., Front Bioeng Biotechnol., 10:975786 (2022); Liu et al., bioRxiv doi:10.1101/2023.08.11.553026 (August 2023); Li et al., Pest Manag Sci., 77(11):4915-4922 (2021); and Fu et al., Proc. Natl. Acad. Sci. USA, 107(10):4550-4554 (2010).

In some aspects, a female-specific splice module comprises a nucleic acid sequence at least 80% identical to a nucleic acid sequence selected from the group consisting of SEQ ID NOs: 4332 to 4334. In some aspects, a female-specific splice module comprises a nucleic acid sequence at least 85% identical to a nucleic acid sequence selected from the group consisting of SEQ ID NOs: 4332 to 4334. In some aspects, a female-specific splice module comprises a nucleic acid sequence at least 90% identical to a nucleic acid sequence selected from the group consisting of SEQ ID NOs: 4332 to 4334. In some aspects, a female-specific splice module comprises a nucleic acid sequence at least 91% identical to a nucleic acid sequence selected from the group consisting of SEQ ID NOs: 4332 to 4334. In some aspects, a female-specific splice module comprises a nucleic acid sequence at least 92% identical to a nucleic acid sequence selected from the group consisting of SEQ ID NOs: 4332 to 4334. In some aspects, a female-specific splice module comprises a nucleic acid sequence at least 93% identical to a nucleic acid sequence selected from the group consisting of SEQ ID NOs: 4332 to 4334. In some aspects, a female-specific splice module comprises a nucleic acid sequence at least 94% identical to a nucleic acid sequence selected from the group consisting of SEQ ID NOs: 4332 to 4334. In some aspects, a female-specific splice module comprises a nucleic acid sequence at least 95% identical to a nucleic acid sequence selected from the group consisting of SEQ ID NOs: 4332 to 4334. In some aspects, a female-specific splice module comprises a nucleic acid sequence at least 96% identical to a nucleic acid sequence selected from the group consisting of SEQ ID NOs: 4332 to 4334. In some aspects, a female-specific splice module comprises a nucleic acid sequence at least 97% identical to a nucleic acid sequence selected from the group consisting of SEQ ID NOs: 4332 to 4334. In some aspects, a female-specific splice module comprises a nucleic acid sequence at least 98% identical to a nucleic acid sequence selected from the group consisting of SEQ ID NOs: 4332 to 4334. In some aspects, a female-specific splice module comprises a nucleic acid sequence at least 99% identical to a nucleic acid sequence selected from the group consisting of SEQ ID NOs: 4332 to 4334. In some aspects, a female-specific splice module comprises a nucleic acid sequence 100% identical to a nucleic acid sequence selected from the group consisting of SEQ ID NOs: 4332 to 4334.

In some aspects, a repressible female-lethal effector is operably linked to a promoter. In some aspects, a repressible female-lethal effector is operably linked to a TRE promoter. In some aspects, a repressible female-lethal effector is operatively linked to an *hsp83* promoter. In some aspects, a repressible female-lethal effector is operatively linked to a *ubiquitin* promoter.

In some aspects, a repressible female-lethal effector is operably linked to a TRE promoter and the female-lethal effector is selected from the group consisting of tTA and tTAV, or variants thereof. In some aspects, a repressible female-lethal effector is operably linked to an *hsp83* promoter and the female lethal effector is selected from the group consisting of tTA and tTAV, or variants thereof.

In some aspects, a TRE promoter comprises at least 1 copy of a tetO sequence operably linked to a minimal promoter. In some aspects, a TRE promoter comprises at least 2 copies of a tetO sequence operably linked to a minimal promoter. In some aspects, a TRE promoter comprises at least 3 copies of a tetO sequence operably linked to a minimal promoter. In some aspects, a TRE promoter comprises at least 5 copies of a tetO sequence operably linked to a minimal promoter. In some aspects, a TRE promoter comprises at least 7 copies of a tetO sequence operably linked to a minimal promoter. In some aspects, a TRE promoter comprises at least 10 copies of a tetO sequence operably linked to a minimal promoter. In some aspects, a TRE promoter comprises at least 15 copies of a tetO sequence operably linked to a minimal promoter. In some aspects, a TRE promoter comprises at least 21 copies of a tetO sequence operably linked to a minimal promoter. In some aspects, a spacer is present between any two copies of tetO. In some aspects, there is no spacer present between any two copies of tetO.

In some aspects, a TRE promoter comprises 1 copy of a tetO sequence operably linked to a minimal promoter. In some aspects, a TRE promoter comprises 2 copies of a tetO sequence operably linked to a minimal promoter. In some aspects, a TRE promoter comprises 3 copies of a tetO sequence operably linked to a minimal promoter. In some aspects, a TRE promoter comprises 7 copies of a tetO sequence operably linked to a minimal promoter. In some aspects, a TRE promoter comprises 21 copies of a tetO sequence operably linked to a minimal promoter. In some aspects, a TRE promoter comprises between 1 copy and 21 copies of a tetO sequence operably linked to a minimal promoter.

In some aspects, a tetO sequence comprises the nucleic acid sequence of SEQ ID NO: 4288. Examples of concatenated tetO sequences are provided as SEQ ID NOs: 4279 (21 copies of tetO), 4283 (7 copies of tetO), and 4284 (7 copies of tetO).

In some aspects, a TRE promoter comprises a nucleic acid sequence at least 80% identical to a nucleic acid sequence selected from the group consisting of SEQ ID NOs: 4279, 4283 to 4285, 4288, and 6848 to 6850. In some aspects, a TRE promoter comprises a nucleic acid sequence at least 85% identical to a nucleic acid sequence selected from the group consisting of SEQ ID NOs: 4279, 4283 to 4285, 4288, and 6848 to 6850. In some aspects, a TRE promoter comprises a nucleic acid sequence at least 90% identical to a nucleic acid sequence selected from the group consisting of SEQ ID NOs: 4279, 4283 to 4285, 4288, and 6848 to 6850. In some aspects, a TRE promoter comprises a nucleic acid sequence at least 91% identical to a nucleic acid sequence selected from the group consisting of SEQ ID NOs: 4279, 4283 to 4285, 4288, and 6848 to 6850. In some aspects, a TRE promoter comprises a nucleic acid sequence at least 92% identical to a nucleic acid sequence selected from the group consisting of SEQ ID NOs: 4279, 4283 to 4285, 4288, and 6848 to 6850. In some aspects, a TRE promoter comprises a nucleic acid sequence at least 93% identical to a nucleic acid sequence selected from the group consisting of SEQ ID NOs: 4279, 4283 to 4285, 4288, and 6848 to 6850. In some aspects, a TRE promoter comprises a nucleic acid sequence at least 94% identical to a nucleic acid sequence selected from the group consisting of SEQ ID NOs: 4279, 4283 to 4285, 4288, and 6848 to 6850. In some aspects, a TRE promoter comprises a nucleic acid sequence at least 95% identical to a nucleic acid sequence selected from the group consisting of SEQ ID NOs: 4279, 4283 to 4285, 4288, and 6848 to 6850. In some aspects, a TRE promoter comprises a nucleic acid sequence at least 96% identical to a nucleic acid sequence selected from the group consisting of SEQ ID NOs: 4279, 4283 to 4285, 4288, and 6848 to 6850. In some aspects, a TRE promoter comprises a nucleic acid sequence at least 97% identical to a nucleic acid sequence selected from the group consisting of SEQ ID NOs: 4279, 4283 to 4285, 4288, and 6848 to 6850. In some aspects, a TRE promoter comprises a nucleic acid sequence at least 98% identical to a nucleic acid sequence selected from the group consisting of SEQ ID NOs: 4279, 4283 to 4285, 4288, and 6848 to 6850. In some aspects, a TRE promoter comprises a nucleic acid sequence at least 99% identical to a nucleic acid sequence selected from the group consisting of SEQ ID NOs: 4279, 4283 to 4285, 4288, and 6848 to 6850. In some aspects, a TRE promoter comprises a nucleic acid sequence 100% identical to a nucleic acid sequence selected from the group consisting of SEQ ID NOs: 4279, 4283 to 4285, 4288, and 6848 to 6850.As used herein, a "minimal promoter" refers to a promoter that is typically inactive on its own, but can initiate gene transcription when combined with other transcription regulatory elements. In some aspects, a minimal promoter is an *hsp70* minimal promoter. In some aspects, a minimal promoter is a *DSCP* minimal promoter. In some aspects, a minimal promoter is a *pgd* minimal promoter. In some aspects, a minimal promoter is an *sry-a* minimal promoter. In some aspects, a minimal promoter is selected from the group consisting of an *hsp70* minimal promoter, a *DSCP* minimal promoter, a*pgd* minimal promoter, and an *sry-a* minimal promoter.

In some aspects, a minimal promoter comprises a nucleic acid sequence at least 80% identical to a nucleic acid sequence selected from the group consisting of SEQ ID NOs: 4290 to 4292, or a functional fragment thereof. In some aspects, a minimal promoter comprises a nucleic acid sequence at least 85% identical to a nucleic acid sequence selected from the group consisting of SEQ ID NOs: 4290 to 4292, or a functional fragment thereof. In some aspects, a minimal promoter comprises a nucleic acid sequence at least 90% identical to a nucleic acid sequence selected from the group consisting of SEQ ID NOs: 4290 to 4292, or a functional fragment thereof. In some aspects, a minimal promoter comprises a nucleic acid sequence at least 91% identical to a nucleic acid sequence selected from the group consisting of SEQ ID NOs: 4290 to 4292, or a functional fragment thereof. In some aspects, a minimal promoter comprises a nucleic acid sequence at least 92% identical to a nucleic acid sequence selected from the group consisting of SEQ ID NOs: 4290 to 4292, or a functional fragment thereof. In some aspects, a minimal promoter comprises a nucleic acid sequence at least 93% identical to a nucleic acid sequence selected from the group consisting of SEQ ID NOs: 4290 to 4292, or a functional fragment thereof. In some aspects, a minimal promoter comprises a nucleic acid sequence at least 94% identical to a nucleic acid sequence selected from the group consisting of SEQ ID NOs: 4290 to 4292, or a functional fragment thereof. In some aspects, a minimal promoter comprises a nucleic acid sequence at least 95% identical to a nucleic acid sequence selected from the group consisting of SEQ ID NOs: 4290 to 4292, or a functional fragment thereof. In some aspects, a minimal promoter comprises a nucleic acid sequence at least 96% identical to a nucleic acid sequence selected from the group consisting of SEQ ID NOs: 4290 to 4292, or a functional fragment thereof. In some aspects, a minimal promoter comprises a nucleic acid sequence at least 97% identical to a nucleic acid sequence selected from the group consisting of SEQ ID NOs: 4290 to 4292, or a functional fragment thereof. In some aspects, a minimal promoter comprises a nucleic acid sequence at least 98% identical to a nucleic acid sequence selected from the group consisting of SEQ ID NOs: 4290 to 4292, or a functional fragment thereof. In some aspects, a minimal promoter comprises a nucleic acid sequence at least 99% identical to a nucleic acid sequence selected from the group consisting of SEQ ID NOs: 4290 to 4292, or a functional fragment thereof. In some aspects, a minimal promoter comprises a nucleic acid sequence 100% identical to a nucleic acid sequence selected from the group consisting of SEQ ID NOs: 4290 to 4292, or a functional fragment thereof.

In some aspects, a sterility system provided herein further comprises a second female-lethal effector. In some aspects, a sterility system provided herein further comprises a third female-lethal effector.

In some aspects, this disclosure provides a method comprising: (a) obtaining a population of modified arthropods comprising any rescue element provided herein and any repressible female-lethal effector provided herein; and (b) rearing the population in the presence of a repressor of the repressible female-lethal effector. In some aspects, the method further comprises: (c) allowing the population to produce a progeny population. In some aspects, the method further comprises: (c) selecting at least one male modified arthropod from the population of modified arthropods. In some aspects, the method further comprises: (c) selecting at least one male modified arthropod from the population of modified arthropods; and (d) releasing the at least one male modified arthropod outdoors. In some aspects, the method further comprises: (c) removing the repressor of the repressible female-lethal effector from the population. In some aspects, the method further comprises: (c) removing the repressor of the repressible female-lethal effector from the population; and selecting at least one male modified arthropod from the population of modified arthropods. In some aspects, the method further comprises: (c) removing the repressor of the repressible female-lethal effector from the population; (d) selecting at least one male modified arthropod from the population of modified arthropods; and (e) releasing the at least one male modified arthropod outdoors.

As used herein, a "rescue element" refers to a nucleic acid molecule encoding a protein that counteracts the actions of a corresponding male sterile effector when expressed in a spatial and temporal overlap with the male sterile effector. As a non-limiting example, when a male sterile effector comprises a Cas9 protein, a rescue element encoding an anticas protein such as AcrIIA4 suppresses Cas9 and prevents or lessens the activity of the male sterile effector, thus allowing fertility to persist.

In some aspects, a rescue element comprises an anticas protein or a nucleic acid sequence encoding an anticas protein. Anticas proteins block the activity of CRISPR RNA-guided nucleases and prevent the nucleases from cleaving target sequences. *See,* for example, Yang and Patel, Mol. Cell., 67(1):117-127 (2017). In some aspects, an anticas protein is an AcrIIA4 protein. In some aspects, an anticas protein is an AcrIIA2 protein.

In some aspects, an AcrIIA4 protein comprises an amino acid sequence at least 80% identical or similar to SEQ ID NO: 6494 or 6495. In some aspects, an AcrIIA4 protein comprises an amino acid sequence at least 85% identical or similar to SEQ ID NO: 6494 or 6495. In some aspects, an AcrIIA4 protein comprises an amino acid sequence at least 90% identical or similar to SEQ ID NO: 6494 or 6495. In some aspects, an AcrIIA4 protein comprises an amino acid sequence at least 91% identical or similar to SEQ ID NO: 6494 or 6495. In some aspects, an AcrIIA4 protein comprises an amino acid sequence at least 92% identical or similar to SEQ ID NO: 6494 or 6495. In some aspects, an AcrIIA4 protein comprises an amino acid sequence at least 93% identical or similar to SEQ ID NO: 6494 or 6495. In some aspects, an AcrIIA4 protein comprises an amino acid sequence at least 94% identical or similar to SEQ ID NO: 6494 or 6495. In some aspects, an AcrIIA4 protein comprises an amino acid sequence at least 95% identical or similar to SEQ ID NO: 6494 or 6495. In some aspects, an AcrIIA4 protein comprises an amino acid sequence at least 96% identical or similar to SEQ ID NO: 6494 or 6495. In some aspects, an AcrIIA4 protein comprises an amino acid sequence at least 97% identical or similar to SEQ ID NO: 6494 or 6495. In some aspects, an AcrIIA4 protein comprises an amino acid sequence at least 98% identical or similar to SEQ ID NO: 6494 or 6495. In some aspects, an AcrIIA4 protein comprises an amino acid sequence at least 99% identical or similar to SEQ ID NO: 6494 or 6495. In some aspects, an AcrIIA4 protein comprises an amino acid sequence 100% identical or similar to SEQ ID NO: 6494 or 6495.

In some aspects, a nucleic acid sequence encoding an AcrIIA4 protein comprises a nucleic acid sequence at least 80% identical to SEQ ID NO: 4313 or 4314. In some aspects, a nucleic acid sequence encoding an AcrIIA4 protein comprises a nucleic acid sequence at least 85% identical to SEQ ID NO: 4313 or 4314. In some aspects, a nucleic acid sequence encoding an AcrIIA4 protein comprises a nucleic acid sequence at least 90% identical to SEQ ID NO: 4313 or 4314. In some aspects, a nucleic acid sequence encoding an AcrIIA4 protein comprises a nucleic acid sequence at least 91% identical to SEQ ID NO: 4313 or 4314. In some aspects, a nucleic acid sequence encoding an AcrIIA4 protein comprises a nucleic acid sequence at least 92% identical to SEQ ID NO: 4313 or 4314. In some aspects, a nucleic acid sequence encoding an AcrIIA4 protein comprises a nucleic acid sequence at least 93% identical to SEQ ID NO: 4313 or 4314. In some aspects, a nucleic acid sequence encoding an AcrIIA4 protein comprises a nucleic acid sequence at least 94% identical to SEQ ID NO: 4313 or 4314. In some aspects, a nucleic acid sequence encoding an AcrIIA4 protein comprises a nucleic acid sequence at least 95% identical to SEQ ID NO: 4313 or 4314. In some aspects, a nucleic acid sequence encoding an AcrIIA4 protein comprises a nucleic acid sequence at least 96% identical to SEQ ID NO: 4313 or 4314. In some aspects, a nucleic acid sequence encoding an AcrIIA4 protein comprises a nucleic acid sequence at least 97% identical to SEQ ID NO: 4313 or 4314. In some aspects, a nucleic acid sequence encoding an AcrIIA4 protein comprises a nucleic acid sequence at least 98% identical to SEQ ID NO: 4313 or 4314. In some aspects, a nucleic acid sequence encoding an AcrIIA4 protein comprises a nucleic acid sequence at least 99% identical to SEQ ID NO: 4313 or 4314. In some aspects, a nucleic acid sequence encoding an AcrIIA4 protein comprises a nucleic acid sequence 100% identical to SEQ ID NO: 4313 or 4314.

In some aspects, a rescue element is a functional copy of a an arthropod male-fertility essential gene. As a non-limiting example, if an arthropod is homozygous for a loss-of-function mutation in a male-fertility essential gene, a wildtype allele of the male-fertility essential gene *(e.g.,* a functional copy) can be inserted at a non-native locus in the genome to rescue the loss-of-function mutation.

As used herein, a "functional copy" or "functional allele" of a gene refers to an allele of a gene that encodes a protein with wildtype function.

In some aspects, a functional copy of a gene comprises a wildtype allele of the male-fertility essential gene that is being rescued. In some aspects, a wildtype allele of a male-fertility essential gene is from the same species as the male-fertility essential gene that is being rescued. For example, if gene X is being rescued in *Drosophila suzukii,* a wildtype allele of gene X from *Drosophila suzukii* can serve as a functional copy of the gene.

In some aspects, a wildtype allele of a male-fertility essential gene is from a different species as the male-fertility essential gene that is being rescued. For example, if gene X is being rescued in *Drosophila suzukii,* a wildtype allele of gene X from *Drosophila melanogaster* can serve as a functional copy of the gene.

In some aspects, a functional copy of a male-fertility essential gene comprises a naturally-occurring ortholog of the male-fertility essential gene from a different arthropod as the male-fertility essential gene that is being rescued. In some aspects, the naturally-occurring ortholog is from a different arthropod species in the same taxonomic Genus as the arthropod being rescued by the functional copy. In some aspects, the naturally-occurring ortholog is from a different arthropod species in the same taxonomic Family as the arthropod being rescued by the functional copy. In some aspects, the naturally-occurring ortholog is from a different arthropod species in the same taxonomic Order as the arthropod being rescued by the functional copy.

In some aspects, a functional copy of a gene comprises a codon-scrambled allele of the male-fertility essential gene that is being rescued. As used herein, a "codon-scrambled allele" (or a "codon-rescued allele" or a "recoded rescue element") refers to an allele that encodes an amino acid sequence at least 90% identical or similar (or at least 91%, at least 92%, at least 93%, at least 94%, at least 95%, at least 96%, at least 97%, at least 98%, at least 99%, or 100% identical or similar to) to a wildtype allele of the same gene *(e.g.,* the codon-scrambled allele and the wildtype allele "correspond" to each other), but utilizes different amino acid codons as compared to the wildtype allele. Without being limited by any theory, codon scrambling allows an allele to escape the deleterious effects caused by RNA-guided nucleases or miRNAs targeting a corresponding wildtype allele. As a non-limiting example of these aspects, Gene A from Species X is desired to be codon-scrambled, so it is codon-scrambled and introduced into Species X.

In some aspects, a small RNA molecule designed to target a wildtype allele is unable to bind to a corresponding codon-scrambled allele. In some aspects, a gRNA molecule designed to target a wildtype allele is unable to bind to a corresponding codon-scrambled allele. In some aspects, a miRNA molecule designed to target a wildtype allele is unable to bind to a corresponding codon-scrambled allele. In some aspects, an shRNA molecule designed to target a wildtype allele is unable to bind to a corresponding codon-scrambled allele. In some aspects, a codon-scrambled allele is a wildtype allele from a different arthropod species. For example, gene X from *Drosophila melanogaster* could serve as a codon-scrambled allele for gene X in *Drosophila suzukii* if the *Drosophila melanogaster* allele encodes an amino acid sequence at least 90% identical or similar to the wildtype allele for gene X in *Drosophila suzukii.*

In some aspects, a codon-scrambled allele comprises a codon-scrambled allele of an orthologous gene. As a non-limiting example for these aspects, Gene A from Species X is desired to be codon-scrambled, so the orthologous GeneA^{O} from Species Y is codon-scrambled and introduced into Species X.

Because amino acid codons are well-understood in the art and because male-fertility essential gene sequences are known in the art, it is within the purview of one of ordinary skill in the art to design and utilize suitable codon-scrambled alleles at least for any male-fertility essential gene provided herein.

In some aspects, a codon-scrambled allele of a male-fertility essential gene comprises a nucleic acid sequence at least 80% identical to a nucleic acid sequence selected from the group consisting of SEQ ID NOs: 4338 to 4340. In some aspects, a codon-scrambled allele of a male-fertility essential gene comprises a nucleic acid sequence at least 85% identical to a nucleic acid sequence selected from the group consisting of SEQ ID NOs: 4338 to 4340. In some aspects, a codon-scrambled allele of a male-fertility essential gene comprises a nucleic acid sequence at least 90% identical to a nucleic acid sequence selected from the group consisting of SEQ ID NOs: 4338 to 4340. In some aspects, a codon-scrambled allele of a male-fertility essential gene comprises a nucleic acid sequence at least 91% identical to a nucleic acid sequence selected from the group consisting of SEQ ID NOs: 4338 to 4340. In some aspects, a codon-scrambled allele of a male-fertility essential gene comprises a nucleic acid sequence at least 92% identical to a nucleic acid sequence selected from the group consisting of SEQ ID NOs: 4338 to 4340. In some aspects, a codon-scrambled allele of a male-fertility essential gene comprises a nucleic acid sequence at least 93% identical to a nucleic acid sequence selected from the group consisting of SEQ ID NOs: 4338 to 4340. In some aspects, a codon-scrambled allele of a male-fertility essential gene comprises a nucleic acid sequence at least 94% identical to a nucleic acid sequence selected from the group consisting of SEQ ID NOs: 4338 to 4340. In some aspects, a codon-scrambled allele of a male-fertility essential gene comprises a nucleic acid sequence at least 95% identical to a nucleic acid sequence selected from the group consisting of SEQ ID NOs: 4338 to 4340. In some aspects, a codon-scrambled allele of a male-fertility essential gene comprises a nucleic acid sequence at least 96% identical to a nucleic acid sequence selected from the group consisting of SEQ ID NOs: 4338 to 4340. In some aspects, a codon-scrambled allele of a male-fertility essential gene comprises a nucleic acid sequence at least 97% identical to a nucleic acid sequence selected from the group consisting of SEQ ID NOs: 4338 to 4340. In some aspects, a codon-scrambled allele of a male-fertility essential gene comprises a nucleic acid sequence at least 98% identical to a nucleic acid sequence selected from the group consisting of SEQ ID NOs: 4338 to 4340. In some aspects, a codon-scrambled allele of a male-fertility essential gene comprises a nucleic acid sequence at least 99% identical to a nucleic acid sequence selected from the group consisting of SEQ ID NOs: 4338 to 4340. In some aspects, a codon-scrambled allele of a male-fertility essential gene comprises a nucleic acid sequence 100% identical to a nucleic acid sequence selected from the group consisting of SEQ ID NOs: 4338 to 4340.

In some aspects, this disclosure provides an isolated nucleic acid molecule encoding any sterility system provided herein. In some aspects, this disclosure provides an isolated nucleic acid molecule encoding any repressible female-lethal effector provided herein. In some aspects, this disclosure provides an isolated nucleic acid molecule encoding any rescue element provided herein.

In some aspects, this disclosure provides an isolated nucleic acid molecule comprising a non-naturally occurring codon-scrambled nucleic acid sequence that encodes an amino acid sequence at least 90% identical or similar to an amino acid sequence selected from the group consisting of SEQ ID NOs: 2880 and 4397 to 6493. In some aspects, this disclosure provides an isolated nucleic acid molecule comprising a non-naturally occurring codon-scrambled nucleic acid sequence that encodes an amino acid sequence at least 91% identical or similar to an amino acid sequence selected from the group consisting of SEQ ID NOs: 2880 and 4397 to 6493. In some aspects, this disclosure provides an isolated nucleic acid molecule comprising a non-naturally occurring codon-scrambled nucleic acid sequence that encodes an amino acid sequence at least 92% identical or similar to an amino acid sequence selected from the group consisting of SEQ ID NOs: 2880 and 4397 to 6493. In some aspects, this disclosure provides an isolated nucleic acid molecule comprising a non-naturally occurring codon-scrambled nucleic acid sequence that encodes an amino acid sequence at least 93% identical or similar to an amino acid sequence selected from the group consisting of SEQ ID NOs: 2880 and 4397 to 6493. In some aspects, this disclosure provides an isolated nucleic acid molecule comprising a non-naturally occurring codon-scrambled nucleic acid sequence that encodes an amino acid sequence at least 94% identical or similar to an amino acid sequence selected from the group consisting of SEQ ID NOs: 2880 and 4397 to 6493. In some aspects, this disclosure provides an isolated nucleic acid molecule comprising a non-naturally occurring codon-scrambled nucleic acid sequence that encodes an amino acid sequence at least 95% identical or similar to an amino acid sequence selected from the group consisting of SEQ ID NOs: 2880 and 4397 to 6493. In some aspects, this disclosure provides an isolated nucleic acid molecule comprising a non-naturally occurring codon-scrambled nucleic acid sequence that encodes an amino acid sequence at least 96% identical or similar to an amino acid sequence selected from the group consisting of SEQ ID NOs: 2880 and 4397 to 6493. In some aspects, this disclosure provides an isolated nucleic acid molecule comprising a non-naturally occurring codon-scrambled nucleic acid sequence that encodes an amino acid sequence at least 97% identical or similar to an amino acid sequence selected from the group consisting of SEQ ID NOs: 2880 and 4397 to 6493. In some aspects, this disclosure provides an isolated nucleic acid molecule comprising a non-naturally occurring codon-scrambled nucleic acid sequence that encodes an amino acid sequence at least 98% identical or similar to an amino acid sequence selected from the group consisting of SEQ ID NOs: 2880 and 4397 to 6493. In some aspects, this disclosure provides an isolated nucleic acid molecule comprising a non-naturally occurring codon-scrambled nucleic acid sequence that encodes an amino acid sequence at least 99% identical or similar to an amino acid sequence selected from the group consisting of SEQ ID NOs: 2880 and 4397 to 6493. In some aspects, this disclosure provides an isolated nucleic acid molecule comprising a non-naturally occurring codon-scrambled nucleic acid sequence that encodes an amino acid sequence 100% identical or similar to an amino acid sequence selected from the group consisting of SEQ ID NOs: 2880 and 4397 to 6493.

As used herein, a "male-fertility essential gene" refers to any locus whose normal function is required for the development of at least one component of the male reproductive system (e.g., without being limiting, testes, sperm, spermatocytes, germline stem cells, hub cells, spermatogonia, spermatids, gonialblasts, seminal vesicles, ejaculatory ducts, ejaculatory bulbs) or for the sperm-dependent survival of an embryo. If the expression pattern or level of a male-fertility essential gene is altered from its wildtype expression pattern or level, fertility can be reduced or entirely eliminated. Similarly, if the coding sequence of a male-fertility essential gene is altered, the protein encoded by the altered coding sequence may be unable to perform its wildtype function and fertility can be reduced or entirely eliminated. In some aspects, a locus comprises a gene. In some aspects, a locus comprises a chromosome. In some aspects, a locus comprises multiple genes. In some aspects, a locus comprises repetitive DNA.

Non-limiting examples of male-fertility essential genes includes the following genes, with gene names based on those used in *Drosophila melanogaster* unless a GenBank^{®} accession number *(e.g.,* as for AAEL028070) is provided instead of a gene name *(see* Öztürk-Çolak et al., Genetics, 227(1):iyad211 (2024); Jenkins et al., Methods Mol. Biol., 2540:1-34 (2022)): *betaTub85D*, *ory, zpg, 28S rDNA, S-Lap3, blw, ary, kl-2, kl-3, kl-5, snky, knon, blanks, sa, ddbt, WDY, S-Lap1, S-Lap2, S-Lap4, S-Lap5, S-Lap7, S-Lap8, fzo, exu, bel, shu, CG12069, Cyt-clL, Hsp60B, Kpcl, Kpc2, loopin-1, mmm, sac, Red7, Rsbp15, Rsph1, Rsph3, Rsph4a, Rsph9, wam, bam, bb8, bond, wupA, RpS6, RpS10, RpS19a, RpL35, RpL37, muc14a,* AAEL028070, *atlas, aust, Mst27D, nht, run, spag4, pp1-Y1, ppl-Y2, soti, eIF4E5, Dic61B, maca, can, bgcn, Dpy-30L2, ms(2)34Fe, ms(2)35Ci, ms(3)76Ba, ms(3)76Cc, ms(3)K81, nsr, Ntl, Pif1A, Prosalpha4T1, Prosalpha6T, PRY, shps, salto, tbrd-2, ttm2, xmas, ATPsynbetaL, 14-3-3epsilon, ab, abd-A, Abd-B, ac, Ack, Acly, Acn, ago, alphaTub84B, aly, aop, ap, Apl, ara, Arf6, asl, asp, asun, Atg8b, ATP7, ATPsyngamma, aub, aux, ball, betaTub60D, Bin1, bol, bor, Brca2, Brr2, brul, brun, Bsg, Btk, Bug22, Bx, ca, Cad99C, Cap-D3, Cap-H2, caup, Cdc42, Cds, Cepl35, Cep290, CG13202, CG18675, CG31759, CG8236, CG9313, CG9920, chb, Chc, Chdl, chic, CIAPIN1, cic, clu, cmb, cn, cnn, Cog7, COX4L, Cpes, crm, CSN5, csul, ct, ctp, cue, Cul3, CycB, Cyp40, Cyt-b5, Cyt-c-d, Dab, dally, dany, Dark, dbf, Dcr-2, DCTN5-p25, Ddxl, del, Dhc16F, dia, Diap1, didum, Diedel, Dis3l2, dj, Dlc90F, Dnaaf3, Dnaaf5, Dnaaf6, Dnah3, Doa, dor, Dronc, dsx, dtr, Duba, dwg, dx, EcR, eff, eIF2A, eIF3m, eIF4E1, eIF4E3, eIF4G2, emc, EndoGI, endos, eRF3, Erk7, eya, f, Fas2, Fas3, fbl, Fmr1, fru, fry, Fsn, fwd, fws, fz2, g, Gas8, gdrd, gish, Gld2, Gorab, Grip128, Grip75, Grip84, Grip91, gskt, GstS1, gudu, H, hay, HBS1, Hel89B, heph, hfp, HipHop, hmw, hop, HP1e, Hr39, Hr51, Hsp60C, Hsp83, HtrA2, hts, hyd, Ibf2, ifc, Inos, InR, Iswi, jar, JYalpha, Kap-alpha1, Kdm5, klhll0, Klp3A, Klp67A, l(2)dtl, l(2)gl, Lam, larp, Lasp, lds, Liprin-alpha, Lis-1, lncRNA:CR45362, lncRNA:Hsromega, IncRNA:iab4, IncRNA:iab8, lost, lovit, lz, m, mael, mago, mal, mam, MAN1, mei-P26, mei-S332, Mer, mfr, mfrn, Mgat1, mgr, Mhc, mia, mil, mino, mip120, mip40, mle, mlt, Mob4, mod, mod(mdg4), msl-3, Mst77F, mt:CoI, mt:Cyt-b, mtsh, mxc, Myc, Mytl, Naa20A, nAChRbeta1, nclb, ND-B17, neb, nej, Nle, nmd, noi, Nop60B, Npc1a, ntc, Nup154, nw, orb2, Orc1, Osbp, osk, otu, ox, p24-1, pAbp, Pall, papi, PAPLA1, park, pbl, Pdelc, Pdkl, pea, pelo, Pen, Pex10, Pex12, Pex16, Pex2, Pfas, Phb1, Pih1D1, Pink1, piwi, Pkd2, Pkn, Pngl, poe, PolQ, Polr2A, pont, Pop1, porin, Poxn, prd, pros, Prp8, psidin, PSMG1 puf, qm, Rab30, Rae1 Rala, Ran, rau, Rb97D, Rdl, ref(2)P, rept, rho-7, Rm62, rn, Rnp4F, Roclb, Root, RpL36, Rpp30, RpS27A, RpS3, rst, rt, Rtca, rux, SAK, Sas-4, sau, sbr, sc, SCaMC, scat, scny, ScsbetaA, sd, Sf3a1 She, shop, shv, sif, sim, sina, slmo, sm, SmB, Smg5, snRNA:U7, sns, Sodl, spag, spd-2, spn-E, SRPK, ss, ssp3, stai, Stat92E, stau, stet, stg, sti, Strip, su(Hw), su(sable), Su(var)3-3, sub, Sxl, Sym, Syp, Syx1, Syx5, Taf6, Taz, tbrd-1, TER94, Tfb5, t-Grip84, Theg, thoc5, tilB, tj, tomb, Top2, Top3alpha, topi, Topors, Torsin, tous, tra, tra2, Trel, Trf, Trl, tRNA:Leu-CAA-1-1, tRNA:Tyr-GTA-1-3, Trxrl, tsr, Tssk, TTLL3B, tud, tut, twe, twy, U2A, Ubi-p63E, Ulp1, upd1, upd3, Upj2, Uspl4, v, vg, VhaPPA1-2, vib, vls, Vmat, vn, Vps28, vret, Vrpl, Vtld, WASp, Wdr92, wls, wnd, Wnt2, wts, wuho, y, Yipldl, YME1L, zip, Zmyndl0,* and *Zw10,* or an ortholog thereof.

In some aspects, a male germline-essential gene is selected from the group consisting of: *betaTub85D, ory, zpg, 28S rDNA, S-Lap3, blw, ary, kl-2, kl-3, kl-5, snky, knon, blanks, sa, ddbt, WDY, S-Lap1, S-Lap2, S-Lap4, S-Lap5, S-Lap7, S-Lap8, fzo, exu, bel, shu, CG12069, Cyt-clL, Hsp60B, Kpc1, Kpc2, loopin-1, mmm, sac, Rcd7, Rsbp15, Rsphl, Rsph3, Rsph4a, Rsph9, wam, bam, bb8, bond, wupA, RpS6, RpS10, RpS19a, RpL35, RpL37, muc14a,* AAEL028070, *atlas, aust, Mst27D, nht, run, spag4, ppl-Y1, ppl-Y2, soti, eIF4E5, Dic6lB, maca, can, bgcn, Dpy-30L2, ms(2)34Fe, ms(2)35Ci, ms(3)76Ba, ms(3)76Cc, ms(3)K81, nsr, Ntl, Pif1A, Prosalpha4T1, Prosalpha6T, PRY, shps, salto, tbrd-2, ttm2, xmas, ATPsynbetaL, 14-3-3epsilon, ab, abd-A, Abd-B, ac, Ack, Acly, Acn, ago, alphaTub84B, aly, aop, ap, Apl, ara, Arf6, asl, asp, asun, Atg8b, ATP7, ATPsyngamma, aub, aux, ball, betaTub60D, Bin1, bol, bor, Brca2, Brr2, brul, brun, Bsg, Btk, Bug22, Bx, ca, Cad99C, Cap-D3, Cap-H2, caup, Cdc42, Cds, Cep135, Cep290, CG13202, CG18675, CG31759, CG8236, CG9313, CG9920, chb, Chc, Chdl, chic, CIAPIN1, cic, clu, cmb, cn, cnn, Cog7, COX4L, Cpes, crm, CSN5, csul, ct, ctp, cue, Cul3, CycB, Cyp40, Cyt-b5, Cyt-c-d, Dab, dally, dany, Dark, dbf, Dcr-2, DCTN5-p25, Ddxl, del, Dhc16F, dia, Diapl, didum, Diedel, Dis3l2, dj, Dlc90F, Dnaaf3, Dnaaf5, Dnaaf6, Dnah3, Doa, dor, Dronc, dsx, dtr, Duba, dwg, dx, EcR, eff, eIF2A, eIF3m, eIF4E1, elF4E3, eIF4G2, emc, EndoGI, endos, eRF3, Erk7, eya, f, Fas2, Fas3, fbl, Fmrl, fru, fry, Fsn, fwd, fws, fz2, g, Gas8, gdrd, gish, Gld2, Gorab, Grip128, Grip75, Grip84, Grip91, gskt, GstS1, gudu, H, hay, HBS1, Hel89B, heph, hfp, HipHop, hmw, hop, HPle, Hr39, Hr51, Hsp60C, Hsp83, HtrA2, hts, hyd, Ibj2, ifc, Inos, InR, Iswi, jar, JYalpha, Kap-alpha1, Kdm5, klhll0, Klp3A, Klp67A, l(2)dtl, l(2)gl, Lam, larp, Lasp, lds, Liprin-alpha, Lis-1, IncRNA:CR45362, lncRNA:Hsromega, IncRNA:iab4, IncRNA:iab8, lost, lovit, lz, m, mael, mago, mal, mam, MAN1, mei-P26, mei-S332, Mer, mfr, mfrn, Mgatl, mgr, Mhc, mia, mil, mino, mip120, mip40, mle, mlt, Mob4, mod, mod(mdg4), msl-3, Mst77F, mt:Col, mt:Cyt-b, mtsh, mxc, Myc, Mytl, Naa20A, nAChRbeta1, nclb, ND-B17, neb, nej, Nle, nmd, noi, Nop60B, Npc1a, ntc, Nup154, nw, orb2, Orc1, Osbp, osk, otu, ox, p24-1, pAbp, Pall, papi, PAPLA1, park, pbl, Pdelc, Pdkl, pea, pelo, Pen, Pex10, Pex12, Pex16, Pex2, Pfas, Phbl, Pih1D1, Pink1, piwi, Pkd2, Pkn, Pngl, poe, PolQ, Polr2A, pont, Pop1, porin, Poxn, prd, pros, Prp8, psidin, PSMG1, puf, qm, Rab30, Rae1, Rala, Ran, rau, Rb97D, Rdl, ref(2)P, rept, rho-7, Rm62, rn, Rnp4F, Roclb, Root, RpL36, Rpp30, RpS27A, RpS3, rst, rt, Rtca, rux, SAK, Sas-4, sau, sbr, sc, SCaMC, scat, scny, ScsbetaA, sd, Sf3a1, Shc, shop, shv, sif, sim, sina, slmo, sm, SmB, Smg5, snRNA:U7, sns, Sod1, spag, spd-2, spn-E, SRPK, ss, ssp3, stai, Stat92E, stau, stet, stg, sti, Strip, su(Hw), su(sable), Su(var)3-3, sub, Sxl, Sym, Syp, Syx13, Syx5, Taf6, Taz, tbrd-1, TER94, Tfb5, t-Grip84, Theg, thoc5, tilB, tj, tomb, Top2, Top3alpha, topi, Topors, Torsin, tous, tra, tra2, Trel, Trf, Trl, tRNA:Leu-CAA-1-1, tRNA:Tyr-GTA-1-3, Trxrl, tsr, Tssk, TTLL3B, tud, tut, twe, twy, U2A, Ubi-p63E, Ulpl, upd1, upd3, Upj2, Uspl4, v, vg, VhaPPAl-2, vib, vls, Vmat, vn, Vps28, vret, Vrpl, Vtld, WASp, Wdr92, wls, wnd, Wnt2, wts, wuho, y, Yipldl, YME1L, zip, Zmynd10,* and *Zw10,* or an ortholog thereof.

In some aspects, a male-fertility essential gene is selected from the group consisting of *betaTub85D, ory, zpg, 28S rDNA, S-Lap3, blw, ary, kl-2, kl-3, kl-5, snky, knon, blanks, sa, ddbt, WDY, S-Lap1, S-Lap2, S-Lap4, S-Lap5, S-Lap7, S-Lap8, fzo, exu, bel, shu, CG12069, Cyt-c1L, Hsp60B, Kpc1, Kpc2, loopin-1, mmm, sac, Red7, Rsbp15, Rsph1, Rsph3, Rsph4a, Rsph9, wam, bam, bb8, bond, wupA, RpS6, RpS10, RpS19a, RpL35, RpL37, muc14α,* AAEL028070, *atlas, aust, Mst27D, nht, run, spag4, pp1-Y1, ppl-Y2, soti, eIF4E5, Dic61B, maca, can, bgcn, Dpy-30L2, ms(2)34Fe, ms(2)35Ci, ms(3)76Ba, ms(3)76Cc, ms(3)K81, nsr, Ntl, Pif1A, Prosalpha4T1, Prosalpha6T, PRY, shps, salto, tbrd-2, ttm2, xmas, and ATPsynbetaL,* or an ortholog thereof.

In some aspects, a male-fertility essential gene is *betaTub85D.* In some aspects, a male-fertility essential gene is *S-Lap1.* In some aspects, a male-fertility essential gene is *S-Lap2.* In some aspects, a male-fertility essential gene is *S-Lap3.* In some aspects, a male-fertility essential gene is *S-Lap4.* In some aspects, a male-fertility essential gene is *S-Lap5.* In some aspects, a male-fertility essential gene is *S-Lap7.* In some aspects, a male-fertility essential gene is *S-Lap8.* In some aspects, a male-fertility essential gene is *zpg.* In some aspects, a male-fertility essential gene is *ary.* In some aspects, a male-fertility essential gene is *msp.* In some aspects, a male-fertility essential gene is *bel.* In some aspects, a male-fertility essential gene is *blw.* In some aspects, a male-fertility essential gene is *28s rDNA.* In some aspects, a male-fertility essential gene is *sa.* In some aspects, a male-fertility essential gene is *ory.* In some aspects, a male-fertility essential gene is *kl-2.* In some aspects, a male-fertility essential gene is *kl-3.* In some aspects, a male-fertility essential gene is AAEL028070. AAEL028070 refers to a GenBank^{®} accession number. In some aspects, a male-fertility essential gene is *kl-5.* In some aspects, a male-fertility essential gene is *snky.* In some aspects, a male-fertility essential gene is *knon.* In some aspects, a male-fertility essential gene is *blanks.* In some aspects, a male-fertility essential gene is *ddbt.* In some aspects, a male-fertility essential gene is *WDY.* In some aspects, a male-fertility essential gene is *fzo.* In some aspects, a male-fertility essential gene is *exu.* In some aspects, a male-fertility essential gene is *shu.* In some aspects, a male-fertility essential gene is *CG12069.* In some aspects, a male-fertility essential gene is *Cyt-c1L.* In some aspects, a male-fertility essential gene is *Hsp60B.* In some aspects, a male-fertility essential gene is *Kpcl.* In some aspects, a male-fertility essential gene is *Kpc2.* In some aspects, a male-fertility essential gene is *loopin-1.* In some aspects, a male-fertility essential gene is *mmm.* In some aspects, a male-fertility essential gene is *sac.* In some aspects, a male-fertility essential gene is *Rcd7.* In some aspects, a male-fertility essential gene is *Rsbpl5.* In some aspects, a male-fertility essential gene is *Rsphl.* In some aspects, a male-fertility essential gene is *Rsph3.* In some aspects, a male-fertility essential gene is *Rsph4a.* In some aspects, a male-fertility essential gene is *Rsph9.* In some aspects, a male-fertility essential gene is *wam.* In some aspects, a male-fertility essential gene is *bam.* In some aspects, a male-fertility essential gene is *bb8.* In some aspects, a male-fertility essential gene is *bond.* In some aspects, a male-fertility essential gene is *wupA.* In some aspects, a male-fertility essential gene is *RpS6.* In some aspects, a male-fertility essential gene is *RpS10.* In some aspects, a male-fertility essential gene is *RpS19a.* In some aspects, a male-fertility essential gene is *RpL35.* In some aspects, a male-fertility essential gene is *RpL37.* In some aspects, a male-fertility essential gene is *muc14a.* In some aspects, a male-fertility essential gene is *atlas.* In some aspects, a male-fertility essential gene is *aust.* In some aspects, a male-fertility essential gene is *Mst27D.* In some aspects, a male-fertility essential gene is *nht.* In some aspects, a male-fertility essential gene is *run.* In some aspects, a male-fertility essential gene is *spag4.* In some aspects, a male-fertility essential gene is *pp1-Y1.* In some aspects, a male-fertility essential gene is *ppl-Y2.* In some aspects, a male-fertility essential gene is *soti.* In some aspects, a male-fertility essential gene is *eIF4E5.* In some aspects, a male-fertility essential gene is *Dic61B.* In some aspects, a male-fertility essential gene is *maca.* In some aspects, a male-fertility essential gene is *can.* In some aspects, a male-fertility essential gene is *bgcn.* In some aspects, a male-fertility essential gene is *Dpy-30L2.* In some aspects, a male-fertility essential gene is *ms(2)34Fe.* In some aspects, a male-fertility essential gene is *ms(2)35Ci.* In some aspects, a male-fertility essential gene is *ms(3)76Ba.* In some aspects, a male-fertility essential gene is *ms(3)76Cc.* In some aspects, a male-fertility essential gene is *ms(3)K81.* In some aspects, a male-fertility essential gene is *nsr.* In some aspects, a male-fertility essential gene is *Ntl.* In some aspects, a male-fertility essential gene is *Pif14.* In some aspects, a male-fertility essential gene is *Prosalpha4T1.* In some aspects, a male-fertility essential gene is *Prosalpha6T.* In some aspects, a male-fertility essential gene is *PRY.* In some aspects, a male-fertility essential gene is *shps.* In some aspects, a male-fertility essential gene is *salto.* In some aspects, a male-fertility essential gene is *tbrd-2.* In some aspects, a male-fertility essential gene is *ttm2.* In some aspects, a male-fertility essential gene is *xmas.* In some aspects, a male-fertility essential gene is *ATPsynbetaL.*

As used herein, an "orthologue" of a gene refers to a gene that encodes a protein comprising an amino acid sequence least 75% identical or similar to the amino acid sequence of the corresponding reference gene. In some aspects, an orthologous gene encodes a protein comprising an amino acid sequence at least 80% identical or similar to the amino acid sequence of the corresponding reference gene. In some aspects, an orthologous gene encodes a protein comprising an amino acid sequence at least 85% identical or similar to the amino acid sequence of the corresponding reference gene. In some aspects, an orthologous gene encodes a protein comprising an amino acid sequence at least 90% identical or similar to the amino acid sequence of the corresponding reference gene. In some aspects, an orthologous gene encodes a protein comprising an amino acid sequence at least 92.5% identical or similar to the amino acid sequence of the corresponding reference gene. In some aspects, an orthologous gene encodes a protein comprising an amino acid sequence at least 95% identical or similar to the amino acid sequence of the corresponding reference gene. In some aspects, an orthologous gene encodes a protein comprising an amino acid sequence at least 97.5% identical or similar to the amino acid sequence of the corresponding reference gene. In some aspects, an orthologous gene encodes a protein comprising an amino acid sequence at least 99% identical or similar to the amino acid sequence of the corresponding reference gene. The reference gene can be any gene or protein whose sequence is known. In some aspects, a reference gene is any one of SEQ ID NOs: 1 to 2879 and 2881 to 2902. In some aspects, a reference gene encodes the amino acid sequence of any one of SEQ ID NOs: 2880 or 4397 to 6493. Unless otherwise specified, a reference gene is a *Drosophila melanogaster* gene. However, in some aspects, a reference gene is an *Anopheles gambiae* gene, an *Aedes aegypti* gene, a *Ceratitis capitata* gene, a *Drosophila suzukii* gene, or a *Bombyx mori* gene.

Non-limiting examples of orthologs of *Drosophila melanogaster* male-fertility essential genes in *Drosophila suzukii* include the following GenBank^{®} Accession Nos: LOC108019987 (ortholog of abd-A), LOC108016728 (ortholog of ac), LOC108020196 (ortholog of nAChRbeta1), LOC118879029 (ortholog of Pfas), LOC118879022 (ortholog of Pfas), LOC118879507 (ortholog of Pfas), LOC108018579 (ortholog of Pfas), LOC118879498 (ortholog of Pfas), LOC108020954 (ortholog of aop), LOC108014052 (ortholog of bru1), LOC108021469 (ortholog of aub), LOC108021436 (ortholog of aub), LOC108007897 (ortholog of bam), LOC108015486 (ortholog of ca), LOC118878848 (ortholog of chic), LOC108016424 (ortholog of chic), LOC108019093 (ortholog of Chc), LOC108021272 (ortholog of eya), LOC108019136 (ortholog of crm), LOC108020468 (ortholog of CycB), LOC108010245 (ortholog of CycB), LOC108006684 (ortholog of Dab), LOC108016929 (ortholog of dor), LOC108015388 (ortholog of dx), LOC108019790 (ortholog of EcR), LOC108013509 (ortholog of emc), LOC108009852 (ortholog of exu), LOC108013709 (ortholog of Fas2), LOC108018875 (ortholog of Fas3), LOC108021577 (ortholog of tj), LOC108013724 (ortholog of g), LOC108017079 (ortholog of H), LOC108013265 (ortholog of hay), LOC108007143 (ortholog of Hsp83), LOC108013721 (ortholog of Grip91), LOC108015801 (ortholog of Grip91), LOC108017670 (ortholog of ifc), LOC108019389 (ortholog of ifc), LOC108021410 (ortholog of CIAPIN1), LOC108021101 (ortholog of stc), LOC108007492 (ortholog of ND-B17), LOC108013403 (ortholog of Phb1), LOC108004974 (ortholog of l(2)gl), LOC108010587 (ortholog of hyd), LOC108011737 (ortholog of sti), LOC108012756 (ortholog of Lam), LOC108014965 (ortholog of lz), LOC108005492 (ortholog of m), LOC108004661 (ortholog of RpL36), LOC108015832 (ortholog of RpS3), LOC108015418 (ortholog of mal), LOC108009712 (ortholog of mam), LOC108006539 (ortholog of twe), LOC108009086 (ortholog of mei-S332), LOC108009820 (ortholog of mago), LOC108005870 (ortholog of msl-3), LOC108006370 (ortholog of ms(3)K81), LOC108014882 (ortholog of PolQ), LOC108004763 (ortholog of otu), LOC108006869 (ortholog of pbl), LOC108017885 (ortholog of Poxn), LOC108007690 (ortholog of prd), LOC108021128 (ortholog of ref(2)P), LOC108015817 (ortholog of Rm62), LOC108016784 (ortholog of Polr2A), LOC108019217 (ortholog of rst), LOC108016393 (ortholog of rt), LOC108014951 (ortholog of run), LOC108011117 (ortholog of rux), LOC108012169 (ortholog of sbr), LOC108019094 (ortholog of sd), LOC108008450 (ortholog of shu), LOC108020005 (ortholog of sina), LOC108009181 (ortholog of sm), LOC108014577 (ortholog of spn-E), LOC108019439 (ortholog of Btk), LOC108009368 (ortholog of stau), LOC108007241 (ortholog of stg), LOC108008428 (ortholog of sub), LOC108015132 (ortholog of su(sable)), LOC108005620 (ortholog of tra), LOC108015852 (ortholog of betaTub85D), LOC108009285 (ortholog of tud), LOC108021494 (ortholog of RpS27A), LOC108021836 (ortholog of v), LOC108018247 (ortholog of vg), LOC108021336 (ortholog of vls), LOC108011413 (ortholog of vn), LOC108016638 (ortholog of y), LOC108016700 (ortholog of sc), LOC108004712 (ortholog of ct), LOC108012887 (ortholog of Rdl), LOC108008603 (ortholog of Wnt2), LOC108021279 (ortholog of porin), LOC108012634 (ortholog of aly), LOC108013032 (ortholog of fwd), LOC108019338 (ortholog of neb), LOC108013528 (ortholog of Klp67A), LOC108009878 (ortholog of bgen), LOC108016922 (ortholog of zw10), LOC108020297 (ortholog of fru), LOC108018462 (ortholog of sim), LOC108016885 (ortholog of hop), LOC108021354 (ortholog of piwi), LOC108006357 (ortholog of Rb97D), LOC108010412 (ortholog of upd1), LOC108006241 (ortholog of nmd), LOC108008276 (ortholog of GstS1), LOC108011937 (ortholog of Trf), LOC108013676 (ortholog of Cdc42), LOC108012849 (ortholog of Cds), LOC108017142 (ortholog of Sas-4), LOC108021202 (ortholog of dia), LOC108005820 (ortholog of fbl), LOC108012996 (ortholog of bol), LOC108020335 (ortholog of pelo), LOC108009960 (ortholog of blw), LOC108015612 (ortholog of eff), LOC108010811 (ortholog of heph), LOC108019822 (ortholog of ox), LOC108011941 (ortholog of poe), LOC108021484 (ortholog of scat), LOC108011242 (ortholog of ms(2)35Ci), LOC108006847 (ortholog of Hsp60B), LOC108012256 (ortholog of can), LOC108019888 (ortholog of Iswi), LOC108016966 (ortholog of Klp3A), LOC108021518 (ortholog of Syx5), LOC108008480 (ortholog of tsr), LOC108012158 (ortholog of wts), LOC108016951 (ortholog of ctp), LOC108021445 (ortholog of Ance), LOC108021501 (ortholog of Ance), LOC108013132 (ortholog of Trl), LOC108009171 (ortholog of protA), LOC108008365 (ortholog of l(2)dtl), LOC108017773 (ortholog of Arf6), LOC108019729 (ortholog of cnn), LOC108004699 (ortholog of Rnp4F), LOC108016254 (ortholog of mia), LOC108016279 (ortholog of noi), LOC108005087 (ortholog of tilB), LOC108012363 (ortholog of eIF4E1), LOC108019198 (ortholog of Rala), LOC108012978 (ortholog of Shc), LOC108008374 (ortholog of spag), LOC108009779 (ortholog of Lis-1), LOC108007279 (ortholog of ara), LOC108009345 (ortholog of csul), LOC108007671 (ortholog of mael), LOC108012860 (ortholog of fry), LOC118876780 (ortholog of fz2), LOC118877518 (ortholog of fz2), LOC108007806 (ortholog of Stat92E), LOC108005908 (ortholog of qm), LOC108014110 (ortholog of dj), LOC108020857 (ortholog of Smg5), LOC118878112 (ortholog of ATPsyngamma), LOC108012817 (ortholog of stet), LOC108004754 (ortholog of Ran), LOC108013038 (ortholog of Pdk1), LOC118879005 (ortholog of asun), LOC108007378 (ortholog of asun), LOC108014291 (ortholog of eRF3), LOC108009167 (ortholog of Pkn), LOC108019124 (ortholog of Trxr1), LOC108013005 (ortholog of chb), LOC108021073 (ortholog of Nup154), LOC108019807 (ortholog of Vps28), LOC108021301 (ortholog of Nle), LOC108014574 (ortholog of Hel89B), LOC108021683 (ortholog of dtr), LOC108019176 (ortholog of mnt), LOC108013401 (ortholog of zpg), LOC108019577 (ortholog of sns), LOC108020980 (ortholog of Npcla), LOC118877724 (ortholog of Dlc90F), LOC108020290 (ortholog of D1c90F), LOC108014829 (ortholog of Bin1), LOC108021675 (ortholog of fws), LOC108005857 (ortholog of Kap-alphal), LOC108008341 (ortholog of Cpes), LOC108016612 (ortholog of Torsin), LOC108016752 (ortholog of Rtca), LOC108018201 (ortholog of Inos), LOC108014969 (ortholog of mei-P26), LOC108006913 (ortholog of SAK), LOC108013531 (ortholog of Drone), LOC108015345 (ortholog of Grip84), LOC108021282 (ortholog of Grip75), LOC108005361 (ortholog of Grip128), LOC108019819 (ortholog of Taz), LOC108015308 (ortholog of Pop1), LOC108017495 (ortholog of mino), LOC108005682 (ortholog of ball), LOC108008336 (ortholog of Kpc2), LOC108020068 (ortholog of Ack), LOC108005388 (ortholog of EndoGI), LOC108012815 (ortholog of hfp), LOC108013881 (ortholog of Fmrl), LOC108014608 (ortholog of slmo), LOC108018986 (ortholog of Gas8), LOC108015376 (ortholog of wuho), LOC108015346 (ortholog of Pink1), LOC108019070 (ortholog of Upf2), LOC108005175 (ortholog of p24-1), LOC108005319 (ortholog of ATP7), LOC108011218 (ortholog of shop), LOC108015149 (ortholog of Naa20A), LOC108020967 (ortholog of shv), LOC108021497 (ortholog of Pex12), LOC108021474 (ortholog of papi), LOC108005286 (ortholog of tomb), LOC108007064 (ortholog of Hsp60C), LOC108006372 (ortholog of rau), LOC108016098 (ortholog of Kdm5), LOC108021736 (ortholog of TTLL3B), LOC108021598 (ortholog of Rab30), LOC108008912 (ortholog of gudu), LOC108014560 (ortholog of PAPLA1), LOC108012692 (ortholog of aust), LOC108005589 (ortholog of Usp14), LOC108005108 (ortholog of Bug22), LOC108020972 (ortholog of spag4), LOC108014289 (ortholog of Pih1D1), LOC108011979 (ortholog of Yipldl), LOC108011962 (ortholog of Rsph1), LOC108010589 (ortholog of Prosalpha6T), LOC108020923 (ortholog of ssp3), LOC108015231 (ortholog of COX4L), LOC108010970 (ortholog of Pngl), LOC118878886 (ortholog of Pngl), LOC108018210 (ortholog of U2A), LOC108008177 (ortholog of CG13202), LOC118876905 (ortholog of CG13202), LOC118876896 (ortholog of CG13202), LOC108008476 (ortholog of rho-7), LOC108009102 (ortholog of Prp8), LOC108019739 (ortholog of mip120), LOC108019650 (ortholog of S-Lap5), LOC108019638 (ortholog of S-Lap7), LOC108010107 (ortholog of eIF3m), LOC108017686 (ortholog of Hr51), LOC108009095 (ortholog of clu), LOC108008555 (ortholog of S-Lap8), LOC108009639 (ortholog of Dcr-2), LOC108018245 (ortholog of Dnaaf3), LOC108017878 (ortholog of tbrd-2), LOC108008622 (ortholog of mip40), LOC108010121 (ortholog of Mgat1), LOC108009939 (ortholog of CG9313), LOC108008353 (ortholog of Rael), LOC108008320 (ortholog of nsr), LOC108010273 (ortholog of tous), LOC108010161 (ortholog of YME1L), LOC108008871 (ortholog of atlas), LOC108010211 (ortholog of thoc5), LOC108009132 (ortholog of Rsph4a), LOC108010099 (ortholog of MAN1), LOC108013046 (ortholog of Dis312), LOC108013618 (ortholog of ttm2), LOC108013625 (ortholog of Cep290), LOC108012541 (ortholog of Pex10), LOC108007162 (ortholog of Strip), LOC108011871 (ortholog of Dpy-30L2), LOC108014446 (ortholog of Dnah3), LOC108005769 (ortholog of mmm), LOC108005770 (ortholog of t-Grip84), LOC108012609 (ortholog of Pex2), LOC108012503 (ortholog of S-Lap1), LOC108012863 (ortholog of Cyp40), LOC108020137 (ortholog of wls), LOC108014501 (ortholog of Duba), LOC108014984 (ortholog of twy), LOC108011264 (ortholog of Syx13), LOC108013235 (ortholog of cmb), LOC108013165 (ortholog of Dnaaf6), LOC108013170 (ortholog of gdrd), LOC108011741 (ortholog of Cep135), LOC108014633 (ortholog of ATPsynbetaL), LOC108018641 (ortholog of Wdr92), LOC108018868 (ortholog of ms(3)76Ba), LOC118876984 (ortholog of ms(3)76Ba), LOC108010646 (ortholog of ms(3)76Ba), LOC108019343 (ortholog of ms(3)76Cc), LOC108010678 (ortholog of wnd), LOC108011064 (ortholog of Vtld), LOC108012839 (ortholog of Pex16), LOC108017624 (ortholog of eIF2A), LOC108011636 (ortholog of aux), LOC118877826 (ortholog of aux), LOC108004873 (ortholog of Sym), LOC108014355 (ortholog of PSMG1), LOC108006989 (ortholog of Ibf2), LOC108010938 (ortholog of Cap-H2), LOC108019282 (ortholog of Rsbp15), LOC108010884 (ortholog of HtrA2), LOC108012042 (ortholog of Atg8b), LOC108020286 (ortholog of hmw), LOC108017518 (ortholog of hmw), LOC108020302 (ortholog of Tssk), LOC118877818 (ortholog of Syp), LOC108010574 (ortholog of Syp), LOC108011343 (ortholog of Theg), LOC108006447 (ortholog of Gld2), LOC108014863 (ortholog of wam), LOC108014860 (ortholog of bb8), LOC108011894 (ortholog of tbrd-1), LOC108017496 (ortholog of mfrn), LOC108012213 (ortholog of Diedel), LOC108012212 (ortholog of Diedel), LOC108015501 (ortholog of Cad99C), LOC108008047 (ortholog of CG12069), LOC108019450 (ortholog of klhl10), LOC118877514 (ortholog of rept), LOC108020451 (ortholog of rept), LOC108012039 (ortholog of pont), LOC108006777 (ortholog of pont), LOC108006441 (ortholog of DCTN5-p25), LOC108021588 (ortholog of Top3alpha), LOC108012807 (ortholog of Roclb), LOC108011452 (ortholog of Mytl), LOC108020036 (ortholog of CG18675), LOC108013175 (ortholog of park), LOC108012319 (ortholog of nht), LOC108020053 (ortholog of ago), LOC108007551 (ortholog of Pkd2), LOC108005619 (ortholog of Apl), LOC108013243 (ortholog of HBS1), LOC108019798 (ortholog of Fsn), LOC108013633 (ortholog of S-Lap3), LOC108015377 (ortholog of Trel), LOC108020989 (ortholog of Liprin-alpha), LOC108010275 (ortholog of Brca2), LOC108008844 (ortholog of dany), LOC108014093 (ortholog of CG31759), LOC108013864 (ortholog of Rsph9), LOC108020240 (ortholog of Mst27D), LOC108015405 (ortholog of Cap-D3), LOC108012941 (ortholog of S-Lap4), LOC108013241 (ortholog of SCaMC), LOC108013572 (ortholog of S-Lap2), LOC108012240 (ortholog of tut), LOC108012474 (ortholog of Rsph3), LOC108015431 (ortholog of Erk7), LOC108012555 (ortholog of Gorab), LOC108010411 (ortholog of upd3), LOC108008494 (ortholog of salto), LOC108013126 (ortholog of endos), LOC108006797 (ortholog of Lasp), LOC108011409 (ortholog of sif), LOC108021454 (ortholog of del), LOC108021216 (ortholog of Myo31DF), LOC108014990 (ortholog of Mer), LOC108009413 (ortholog of pea), LOC108014680 (ortholog of Cyt-c-d), LOC108012886 (ortholog of Mst77F), LOC108006151 (ortholog of snky), LOC118877681 (ortholog of psidin), LOC108005058 (ortholog of psidin), LOC108009082 (ortholog of Vrp1), LOC108018466 (ortholog of Chd1), LOC108007342 (ortholog of gish), LOC108018187 (ortholog of Mob4), LOC108008533 (ortholog of loopin-1), LOC108010208 (ortholog of Nop60B), LOC108006230 (ortholog of Su(var)3-3), LOC108010672 (ortholog of Rcd7), LOC108014025 (ortholog of Diap1), LOC108015294 (ortholog of mxc), LOC108019735 (ortholog of Vmat), LOC108006747 (ortholog of asl), LOC108021039 (ortholog of Hr39), LOC108020993 (ortholog of Cul3), LOC108018059 (ortholog of didum), LOC108021702 (ortholog of ms(2)34Fe), LOC108014960 (ortholog of nej), LOC108021023 (ortholog of brun), LOC108011955 (ortholog of Bsg), LOC108004671 (ortholog of f), LOC108015727 (ortholog of VhaPPA1-2), LOC108006127 (ortholog of cic), LOC108014325 (ortholog of cic), LOC108020987 (ortholog of mtsh), LOC108021303 (ortholog of SmB), LOC108019170 (ortholog of Myc), LOC108021249 (ortholog of Acn), LOC108007982 (ortholog of bel), LOC108009301 (ortholog of hts), LOC108010043 (ortholog of nclb), LOC108011640 (ortholog of lost), LOC108013774 (ortholog of Brr2), LOC108009795 (ortholog of Dark), LOC108012245 (ortholog of dally), LOC108013202 (ortholog of Dic61B), LOC108016832 (ortholog of Sxl), LOC108018123 (ortholog of Cyt-b5), LOC108018617 (ortholog of ab), LOC108006027 (ortholog of mgr), LOC108014236 (ortholog of Mhc), LOC108011393 (ortholog of Pde1c), LOC108006181 (ortholog of pAbp), LOC108009283 (ortholog of mlt), LOC108010419 (ortholog of Bx), LOC108010582 (ortholog of Prosalpha4T1), LOC108007657 (ortholog of Tfb5), LOC108020086 (ortholog of stai), LOC108006708 (ortholog of Zmynd10), LOC108013546 (ortholog of mfr), LOC108020243 (ortholog of Sf3a1), LOC118878586 (ortholog of Sf3a1), LOC108020445 (ortholog of Ntl), LOC108017094 (ortholog of rn), LOC108018110 (ortholog of Topors), LOC108020430 (ortholog of JYalpha), LOC108012022 (ortholog of mil), LOC108021470 (ortholog of sau), LOC118879521 (ortholog of lovit), LOC108010756 (ortholog of vib), LOC108005093 (ortholog of ap), LOC108016798 (ortholog of Dhc16F), LOC108014870 (ortholog ofInR), LOC108016422 (ortholog of InR), LOC108010325 (ortholog of nw), LOC108009511 (ortholog of Pall), LOC108016756 (ortholog of Rpp30), LOC108020395 (ortholog of Top2), LOC108013034 (ortholog of ddbt), LOC108008611 (ortholog of knon), LOC108020645 (ortholog of shps), LOC108009876 (ortholog of TER94), LOC108018128 (ortholog of Orc1), LOC108005400 (ortholog of xmas), LOC108017993 (ortholog of SRPK), LOC108018224 (ortholog of dbf), LOC108021640 (ortholog of Pen), LOC108010141 (ortholog of betaTub60D), LOC108016868 (ortholog of wupA), and LOC108004706 (ortholog of RpS6).

Non-limiting examples of orthologs of *Drosophila melanogaster* male-fertility essential genes in *Aedes aegypti* include the following GenBank^{®} Accession Nos: AAEL009742 (ortholog of abd-A), AAEL025694 (ortholog of Abd-B), AAEL008350 (ortholog of ac), AAEL008346 (ortholog of ac), AAEL023131 (ortholog of ac), AAEL010570 (ortholog of nAChRbeta1), AAEL005384 (ortholog of Pfas), AAEL010488 (ortholog of aop), AAEL019474 (ortholog of bru1), AAEL005791 (ortholog of asp), AAEL013692 (ortholog of aub), AAEL013233 (ortholog of aub), AAEL013227 (ortholog of aub), AAEL021425 (ortholog of aub), AAEL006287 (ortholog of aub), AAEL008098 (ortholog of aub), AAEL007698 (ortholog of aub), AAEL006116 (ortholog of ca), AAEL026095 (ortholog of ca), AAEL013353 (ortholog of chic), AAEL022819 (ortholog of Chc), AAEL019952 (ortholog of eya), AAEL020151 (ortholog of crm), AAEL010094 (ortholog of CycB), AAEL024936 (ortholog of Dab), AAEL004237 (ortholog of dor), AAEL001459 (ortholog of dx), AAEL019431 (ortholog of EcR), AAEL002721 (ortholog of emc), AAEL010097 (ortholog of exu), AAEL009173 (ortholog of Fas2), AAEL007686 (ortholog of tj), AAEL008462 (ortholog of g), AAEL008617 (ortholog of H), AAEL013205 (ortholog of hay), AAEL011708 (ortholog of Hsp83), AAEL014843 (ortholog of Hsp83), AAEL007903 (ortholog of kl-2), AAEL008465 (ortholog of Grip91), AAEL013047 (ortholog of ifc), AAEL001501 (ortholog of CIAPIN1), AAEL001636 (ortholog of stc), AAEL009066 (ortholog of ND-B17), AAEL009345 (ortholog of Phb1), AAEL009932 (ortholog of l(2)gl), AAEL020992 (ortholog of hyd), AAEL026474 (ortholog of sti), AAEL000335 (ortholog of Lam), AAEL003415 (ortholog of Lam), AAEL018118 (ortholog of lds), AAEL007040 (ortholog of lz), AAEL022989 (ortholog of lz), AAEL021126 (ortholog of m), AAEL002639 (ortholog of RpL36), AAEL000010 (ortholog of RpL36), AAEL008192 (ortholog of RpS3), AAEL011727 (ortholog of mal), AAEL022615 (ortholog of mal), AAEL019475 (ortholog of mam), AAEL014408 (ortholog of twe), AAEL010453 (ortholog of mago), AAEL027919 (ortholog of mle), AAEL004859 (ortholog of mle), AAEL010054 (ortholog of msl-3), AAEL023907 (ortholog of mod), AAEL022577 (ortholog of mod), AAEL027737 (ortholog of mod), AAEL007013 (ortholog of mod), AAEL005888 (ortholog of PolQ), AAEL000442 (ortholog of osk), AAEL018263 (ortholog of otu), AAEL000984 (ortholog of pbl), AAEL022262 (ortholog of Poxn), AAEL026751 (ortholog of ref(2)P), AAEL017363 (ortholog of Polr2A), AAEL002754 (ortholog of rst), AAEL002745 (ortholog of rst), AAEL000855 (ortholog of rt), AAEL006167 (ortholog of run), AAEL001007 (ortholog of sbr), AAEL024396 (ortholog of sd), AAEL011632 (ortholog of shu), AAEL009614 (ortholog of sina), AAEL025268 (ortholog of sm), AAEL019937 (ortholog of Sod1), AAEL023716 (ortholog of spn-E), AAEL019480 (ortholog of Btk), AAEL011825 (ortholog of ss), AAEL014408 (ortholog of stg), AAEL022590 (ortholog of sub), AAEL010074 (ortholog of su(sable)), AAEL024455 (ortholog of su(sable)), AAEL027148 (ortholog of tra2), AAEL004293 (ortholog of tra2), AAEL009224 (ortholog of tra2), AAEL006416 (ortholog of tra2), AAEL006642 (ortholog of alphaTub84B), AAEL013229 (ortholog of alphaTub84B), AAEL019894 (ortholog of betaTub85D), AAEL021145 (ortholog of tud), AAEL013536 (ortholog of RpS27A), AAEL003888 (ortholog of Ubip63E), AAEL000428 (ortholog of v), AAEL007554 (ortholog of vls), AAEL019712 (ortholog of vn), AAEL006830 (ortholog of y), AAEL006985 (ortholog of y), AAEL019970 (ortholog of ct), AAEL008354 (ortholog of Rdl), AAEL026367 (ortholog of Wnt2), AAEL022304 (ortholog of porin), AAEL023176 (ortholog of porin), AAEL005631 (ortholog of aly), AAEL009294 (ortholog of fwd), AAEL001986 (ortholog of neb), AAEL009227 (ortholog of Klp67A), AAEL002769 (ortholog of pros), AAEL011103 (ortholog of zw10), AAEL024283 (ortholog of fru), AAEL011013 (ortholog of sim), AAEL012553 (ortholog of hop), AAEL013692 (ortholog of piwi), AAEL013233 (ortholog of piwi), AAEL013227 (ortholog of piwi), AAEL021425 (ortholog of piwi), AAEL006287 (ortholog of piwi), AAEL008098 (ortholog of piwi), AAEL007698 (ortholog of piwi), AAEL005454 (ortholog of nmd), AAEL011741 (ortholog of GstS1), AAEL002079 (ortholog of Trf), AAEL008543 (ortholog of Cdc42), AAEL001940 (ortholog of Cds), AAEL006988 (ortholog of Taf6), AAEL026780 (ortholog of Sas-4), AAEL008183 (ortholog of Sas-4), AAEL014868 (ortholog of dia), AAEL012409 (ortholog of fbl), AAEL019836 (ortholog of bol), AAEL007854 (ortholog of pelo), AAEL012175 (ortholog of blw), AAEL003103 (ortholog of eff), AAEL013723 (ortholog of heph), AAEL004227 (ortholog of jar), AAEL000182 (ortholog of ox), AAEL008107 (ortholog of poe), AAEL001497 (ortholog of scat), AAEL011584 (ortholog of Hsp60B), AAEL003968 (ortholog of Iswi), AAEL010568 (ortholog of Klp3A), AAEL013541 (ortholog of Syx5), AAEL003957 (ortholog of tsr), AAEL013826 (ortholog of wts), AAEL017451 (ortholog ofAnce), AAEL017460 (ortholog of Ance), AAEL000970 (ortholog of Trl), AAEL010877 (ortholog of l(2)dtl), AAEL004913 (ortholog of Arf6), AAEL019814 (ortholog of cnn), AAEL019945 (ortholog of Rnp4F), AAEL019944 (ortholog of Rnp4F), AAEL026327 (ortholog of noi), AAEL021751 (ortholog of noi), AAEL004711 (ortholog of tilB), AAEL002488 (ortholog of Ddx1), AAEL014749 (ortholog of Rala), AAEL008739 (ortholog of Shc), AAEL000770 (ortholog of Lis-1), AAEL007505 (ortholog of ara), AAEL007505 (ortholog of caup), AAEL021474 (ortholog of csul), AAEL007578 (ortholog of csul), AAEL008595 (ortholog of mael), AAEL018151 (ortholog of mael), AAEL019632 (ortholog of fry), AAEL019704 (ortholog of fz2), AAEL020559 (ortholog of Stat92E), AAEL004900 (ortholog of qm), AAEL004986 (ortholog of Smg5), AAEL008848 (ortholog of ATPsyngamma), AAEL001749 (ortholog of stet), AAEL009287 (ortholog of Ran), AAEL007284 (ortholog of Pdkl), AAEL006440 (ortholog of Pdkl), AAEL027882 (ortholog of asun), AAEL012059 (ortholog of asun), AAEL017368 (ortholog of eRF3), AAEL001549 (ortholog of Pkn), AAEL018354 (ortholog of Osbp), AAEL002886 (ortholog of Trxrl), AAEL005211 (ortholog of chb), AAEL026104 (ortholog of Nup154), AAEL002324 (ortholog of Vps28), AAEL004063 (ortholog of Nle), AAEL019648 (ortholog of Hel89B), AAEL020552 (ortholog of dtr), AAEL010772 (ortholog of dtr), AAEL006567 (ortholog of mnt), AAEL006726 (ortholog of zpg), AAEL003853 (ortholog of sns), AAEL019883 (ortholog of Npc1a), AAEL006647 (ortholog of Dlc90F), AAEL014368 (ortholog of Bin1), AAEL005180 (ortholog of fws), AAEL010900 (ortholog of Kap-alphal), AAEL014158 (ortholog of Cpes), AAEL004448 (ortholog of Torsin), AAEL009623 (ortholog of Rtca), AAEL002834 (ortholog of Inos), AAEL017329 (ortholog of mei-P26), AAEL009360 (ortholog of SAK), AAEL011562 (ortholog of Drone), AAEL013903 (ortholog of Grip84), AAEL003546 (ortholog of Grip75), AAEL006653 (ortholog of Grip128), AAEL001564 (ortholog of Taz), AAEL009542 (ortholog of Pop1), AAEL022505 (ortholog of CSN5), AAEL021734 (ortholog of mino), AAEL021382 (ortholog of mino), AAEL007080 (ortholog of mino), AAEL015216 (ortholog of ball), AAEL008115 (ortholog of Kpc2), AAEL021353 (ortholog of Ack), AAEL004415 (ortholog of hfp), AAEL009326 (ortholog of Fmrl), AAEL019482 (ortholog of slmo), AAEL004231 (ortholog of Gas8), AAEL004558 (ortholog of wuho), AAEL011594 (ortholog of Pink1), AAEL010598 (ortholog of Upf2), AAEL003433 (ortholog of ATP7), AAEL014252 (ortholog of shop), AAEL009373 (ortholog of Naa20A), AAEL009946 (ortholog of shv), AAEL006463 (ortholog of Pex12), AAEL013072 (ortholog of papi), AAEL011584 (ortholog of Hsp60C), AAEL001832 (ortholog of rau), AAEL011092 (ortholog of Kdm5), AAEL000578 (ortholog of TTLL3B), AAEL010682 (ortholog of gudu), AAEL021625 (ortholog of PAPLA1), AAEL010496 (ortholog of Usp14), AAEL013151 (ortholog of Bug22), AAEL011754 (ortholog of Yip1d1), AAEL023977 (ortholog of Yip1d1), AAEL010639 (ortholog of Rsph1), AAEL015007 (ortholog of Prosalpha6T), AAEL019916 (ortholog of ssp3), AAEL005170 (ortholog of COX4L), AAEL014507 (ortholog of Pngl), AAEL012656 (ortholog of U2A), AAEL003257 (ortholog of CG13202), AAEL013749 (ortholog of rho-7), AAEL009959 (ortholog of Prp8), AAEL005893 (ortholog of mip120), AAEL020020 (ortholog of mip120), AAEL006975 (ortholog of S-Lap5), AAEL006975 (ortholog of S-Lap7), AAEL004347 (ortholog of eIF3m), AAEL020847 (ortholog of Hr51), AAEL027504 (ortholog of clu), AAEL006975 (ortholog of S-Lap8), AAEL006794 (ortholog of Dcr-2), AAEL010556 (ortholog of Dnaaf3), AAEL006859 (ortholog of mip40), AAEL014276 (ortholog of mip40), AAEL002329 (ortholog of Mgat1), AAEL027332 (ortholog of CG9313), AAEL022963 (ortholog of CG9313), AAEL007066 (ortholog of Rael), AAEL005358 (ortholog of nsr), AAEL005967 (ortholog of tous), AAEL021307 (ortholog of YME1L), AAEL011637 (ortholog of thoc5), AAEL001416 (ortholog of MAN1), AAEL001397 (ortholog of Dis312), AAEL006124 (ortholog of ttm2), AAEL025983 (ortholog of Cep290), AAEL020542 (ortholog of Pex10), AAEL007865 (ortholog of Strip), AAEL008036 (ortholog of Dnah3), AAEL012239 (ortholog of mmm), AAEL005470 (ortholog of Pex2), AAEL000108 (ortholog of S-Lap1), AAEL023987 (ortholog of S-Lap1), AAEL000013 (ortholog of Cyp40), AAEL007842 (ortholog of wls), AAEL000448 (ortholog of Duba), AAEL006760 (ortholog of twy), AAEL009398 (ortholog of Syx13), AAEL010869 (ortholog of cmb), AAEL014067 (ortholog of Dnaaf6), AAEL003087 (ortholog of Wdr92), AAEL010835 (ortholog of wnd), AAEL023327 (ortholog of Pex16), AAEL010461 (ortholog of Pex16), AAEL008248 (ortholog of eIF2A), AAEL005425 (ortholog of aux), AAEL025809 (ortholog of sac), AAEL002437 (ortholog of Sym), AAEL007351 (ortholog of PSMG1), AAEL004705 (ortholog of CG8236), AAEL011746 (ortholog of ScsbetaA), AAEL005838 (ortholog of topi), AAEL006924 (ortholog of Rsbp15), AAEL013161 (ortholog of CG9920), AAEL001052 (ortholog of CG9920), AAEL006373 (ortholog of HtrA2), AAEL007227 (ortholog of Kpc1), AAEL023907 (ortholog of maca), AAEL022577 (ortholog of maca), AAEL027737 (ortholog of maca), AAEL007013 (ortholog of maca), AAEL005777 (ortholog of hmw), AAEL024137 (ortholog of Tssk), AAEL002879 (ortholog of Syp), AAEL010149 (ortholog of Theg), AAEL006249 (ortholog of Gld2), AAEL003757 (ortholog of bb8), AAEL025958 (ortholog of bb8), AAEL022554 (ortholog of Root), AAEL009915 (ortholog of Root), AAEL022105 (ortholog of puf), AAEL006564 (ortholog of mfrn), AAEL011871 (ortholog of Cyt-c1L), AAEL008421 (ortholog of Cad99C), AAEL001976 (ortholog of CG12069), AAEL019763 (ortholog of klhl10), AAEL010341 (ortholog of rept), AAEL004686 (ortholog of pont), AAEL007566 (ortholog of DCTN5-p25), AAEL007683 (ortholog of Top3alpha), AAEL006140 (ortholog of Mytl), AAEL019701 (ortholog of CG18675), AAEL018318 (ortholog of park), AAEL026220 (ortholog of ago), AAEL010698 (ortholog of Apl), AAEL020913 (ortholog of HBS1), AAEL000825 (ortholog of HBS1), AAEL008758 (ortholog of Fsn), AAEL006975 (ortholog of S-Lap3), AAEL019988 (ortholog of Trel), AAEL008058 (ortholog of Liprin-alpha), AAEL007307 (ortholog of Cog7), AAEL026452 (ortholog of CG31759), AAEL007706 (ortholog of Rsph9), AAEL011548 (ortholog of Cap-D3), AAEL006975 (ortholog of S-Lap4), AAEL004589 (ortholog of SCaMC), AAEL000108 (ortholog of S-Lap2), AAEL023987 (ortholog of S-Lap2), AAEL001374 (ortholog of Rsph3), AAEL011765 (ortholog of Gorab), AAEL007717 (ortholog of endos), AAEL022750 (ortholog of Lasp), AAEL019674 (ortholog of sif), AAEL025570 (ortholog of Myo31DF), AAEL022373 (ortholog of Mer), AAEL026040 (ortholog of pea), AAEL019906 (ortholog of Cyt-c-d), AAEL004457 (ortholog of Cyt-c-d), AAEL002356 (ortholog of snky), AAEL004142 (ortholog of psidin), AAEL008154 (ortholog of Vrp1), AAEL004351 (ortholog of gish), AAEL003454 (ortholog of Mob4), AAEL006975 (ortholog of loopin-1), AAEL003514 (ortholog of Nop60B), AAEL011415 (ortholog of Su(var)3-3), AAEL013249 (ortholog of eIF4G2), AAEL009074 (ortholog of Diap1), AAEL002662 (ortholog of bond), AAEL026276 (ortholog of Vmat), AAEL012031 (ortholog of asl), AAEL019418 (ortholog of Pif1A), AAEL023349 (ortholog of Pif1A),AAEL001304 (ortholog of Hr39), AAEL007187 (ortholog of Cul3), AAEL024528 (ortholog of didum), AAEL017391 (ortholog of nej), AAEL009070 (ortholog of larp), AAEL026852 (ortholog of brun), AAEL012262 (ortholog of Bsg), AAEL018112 (ortholog of f), AAEL019500 (ortholog of cic), AAEL022986 (ortholog of mtsh), AAEL002377 (ortholog of SmB), AAEL012233 (ortholog of Myc), AAEL020359 (ortholog of vret), AAEL004472 (ortholog of bel), AAEL021134 (ortholog of hts), AAEL011105 (ortholog of hts), AAEL012851 (ortholog of nclb), AAEL002178 (ortholog of lost), AAEL022923 (ortholog of Brr2), AAEL011187 (ortholog of Brr2), AAEL000874 (ortholog of Dark), AAEL000993 (ortholog of dally), AAEL006541 (ortholog of Dic61B), AAEL010017 (ortholog of Cyt-b5), AAEL004427 (ortholog of mgr), AAEL026217 (ortholog of Mhc), AAEL021838 (ortholog of Mhc), AAEL005656 (ortholog of Mhc), AAEL010318 (ortholog of pAbp), AAEL004440 (ortholog of mlt), AAEL005830 (ortholog of Prosalpha4T1), AAEL019792 (ortholog of Tfb5), AAEL008078 (ortholog of Doa), AAEL012840 (ortholog of stai), AAEL010315 (ortholog of Zmynd10), AAEL023908 (ortholog of mfr), AAEL013818 (ortholog of Sf3a1), AAEL019554 (ortholog of Ntl), AAEL008683 (ortholog of Topors), AAEL000432 (ortholog of mil), AAEL013951 (ortholog of mil), AAEL004973 (ortholog of sau), AAEL012109 (ortholog of lovit), AAEL007225 (ortholog of kl-3), AAEL002485 (ortholog of WDY), AAEL019489 (ortholog of ap), AAEL000512 (ortholog of Dhc16F), AAEL002317 (ortholog of InR), AAEL001565 (ortholog of Pal1), AAEL002232 (ortholog of Rpp30), AAEL018244 (ortholog of Top2), AAEL010585 (ortholog of TER94), AAEL024003 (ortholog of Orc1), AAEL023619 (ortholog of SRPK), AAEL009883 (ortholog of bor), AAEL001518 (ortholog of dbf), AAEL012960 (ortholog of Pen), AAEL001411 (ortholog of zip), and AAEL002848 (ortholog of betaTub60D).

Non-limiting examples of orthologs of *Drosophila melanogaster* male-fertility essential genes in *Anopheles gambiae* include the following GenBank^{®} Accession Nos: AGAP004662 (ortholog of abd-A), AGAP004664 (ortholog of Abd-B), AGAP000966 (ortholog of nAChRbetal), AGAP002091 (ortholog of Pfas), AGAP029575 (ortholog of aop), AGAP003703 (ortholog of asp), AGAP009509 (ortholog of aub), AGAP011204 (ortholog of aub), AGAP004229 (ortholog of ca), AGAP009861 (ortholog of chic), AGAP003021 (ortholog of Chc), AGAP008726 (ortholog of eya), AGAP001029 (ortholog of crm), AGAP004963 (ortholog of CycB), AGAP004962 (ortholog of CycB), AGAP006528 (ortholog of Dab), AGAP000983 (ortholog of dor), AGAP005269 (ortholog of dwg), AGAP000903 (ortholog of dx), AGAP029539 (ortholog of EcR), AGAP007696 (ortholog of emc), AGAP007944 (ortholog of emc), AGAP007365 (ortholog of exu), AGAP007364 (ortholog of exu), AGAP002802 (ortholog of Fas2), AGAP029564 (ortholog of Fas3), AGAP012693 (ortholog of Fas3), AGAP010030 (ortholog of tj), AGAP000161 (ortholog of g), AGAP028632 (ortholog of hay), AGAP006958 (ortholog of Hsp83), AGAP006961 (ortholog of Hsp83), AGAP006959 (ortholog of Hsp83), AGAP009550 (ortholog of kl-2), AGAP003865 (ortholog of Grip91), AGAP012111 (ortholog of ifc), AGAP011402 (ortholog of ifc), AGAP008883 (ortholog of CIAPIN1), AGAP008728 (ortholog of stc), AGAP009602 (ortholog of ND-B17), AGAP009323 (ortholog of Phb1), AGAP004483 (ortholog of l(2)gl), AGAP001780 (ortholog of hyd), AGAP006932 (ortholog of sti), AGAP011938 (ortholog of Lam), AGAP008015 (ortholog of Lam), AGAP011966 (ortholog of Ids), AGAP002506 (ortholog of lz), AGAP000786 (ortholog of m), AGAP002921 (ortholog of RpL36), AGAP001910 (ortholog of RpS3), AGAP000555 (ortholog of mal), AGAP010410 (ortholog of mam), AGAP010755 (ortholog of mago), AGAP006599 (ortholog of mle), AGAP001917 (ortholog of msl-3), AGAP007533 (ortholog of PolQ), AGAP003545 (ortholog of osk), AGAP004973 (ortholog of otu), AGAP004972 (ortholog of otu), AGAP000835 (ortholog of otu), AGAP005796 (ortholog of pbl), AGAP008818 (ortholog of Poxn), AGAP029708 (ortholog of ref(2)P), AGAP029707 (ortholog of ref(2)P), AGAP012045 (ortholog of Rm62), AGAP007810 (ortholog of Polr2A), AGAP000218 (ortholog of rst), AGAP010784 (ortholog of rt), AGAP001094 (ortholog of run), AGAP002193 (ortholog of sbr), AGAP000512 (ortholog of sd), AGAP011458 (ortholog of shu), AGAP006127 (ortholog of sina), AGAP029616 (ortholog of sm), AGAP002829 (ortholog of spn-E), AGAP008317 (ortholog of Btk), AGAP010259 (ortholog of ss), AGAP003301 (ortholog of stg), AGAP006989 (ortholog of sub), AGAP027985 (ortholog of su(Hw)), AGAP028107 (ortholog of su(Hw)), AGAP012027 (ortholog of su(Hw)), AGAP028668 (ortholog of su(Hw)), AGAP028673 (ortholog of su(Hw)), AGAP029740 (ortholog of su(Hw)), AGAP004729 (ortholog of su(Hw)), AGAP028625 (ortholog of su(Hw)), AGAP011127 (ortholog of su(sable)), AGAP013051 (ortholog of tra2), AGAP029421 (ortholog of tra2), AGAP006798 (ortholog of tra2), AGAP010971 (ortholog of alphaTub84B), AGAP001218 (ortholog of alphaTub84B), AGAP001219 (ortholog of alphaTub84B), AGAP003237 (ortholog of betaTub85D), AGAP013742 (ortholog of RpS27A), AGAP002721 (ortholog of v), AGAP009164 (ortholog of vg), AGAP009782 (ortholog of vls), AGAP007110 (ortholog of vn), AGAP000879 (ortholog of y), AGAP000876 (ortholog of sc), AGAP001187 (ortholog of ct), AGAP006028 (ortholog of Rdl), AGAP010283 (ortholog of Wnt2), AGAP009833 (ortholog of porin), AGAP028005 (ortholog of aly), AGAP007512 (ortholog of fwd), AGAP008035 (ortholog of neb), AGAP006803 (ortholog of Klp67A), AGAP004052 (ortholog of pros), AGAP004467 (ortholog of Zw10), AGAP000080 (ortholog of fru), AGAP000773 (ortholog of sim), AGAP008354 (ortholog of hop), AGAP002374 (ortholog of Rb97D), AGAP009625 (ortholog of nmd), AGAP010404 (ortholog of GstS1), AGAP008084 (ortholog of Trf), AGAP002440 (ortholog of Cdc42), AGAP007175 (ortholog of Cds), AGAP004254 (ortholog of Taf6), AGAP001897 (ortholog of Sas-4), AGAP009643 (ortholog of dia), AGAP029654 (ortholog of fbl), AGAP005506 (ortholog of bol), AGAP008269 (ortholog of pelo), AGAP005134 (ortholog of blw), AGAP000145 (ortholog of eff), AGAP003945 (ortholog of heph), AGAP000776 (ortholog of jar), AGAP004710 (ortholog of ox), AGAP001157 (ortholog of poe), AGAP008353 (ortholog of scat), AGAP010700 (ortholog of Iswi), AGAP000575 (ortholog of Klp3A), AGAP012471 (ortholog of Klp3A), AGAP008756 (ortholog of Syx5), AGAP012056 (ortholog of tsr), AGAP003618 (ortholog of wts), AGAP001335 (ortholog of ctp), AGAP009754 (ortholog of Ance), AGAP009755 (ortholog of Ance), AGAP009756 (ortholog of Ance), AGAP028646 (ortholog of Ance), AGAP009751 (ortholog of Ance), AGAP011583 (ortholog of Trl), AGAP007166 (ortholog of l(2)dtl), AGAP006890 (ortholog of Arf6), AGAP002434 (ortholog of cnn), AGAP002959 (ortholog of Rnp4F), AGAP003085 (ortholog of noi), AGAP008927 (ortholog of tilB), AGAP004765 (ortholog of Ddx1), AGAP007172 (ortholog of eIF4E1), AGAP012108 (ortholog of Rala), AGAP011463 (ortholog of Shc), AGAP011849 (ortholog of Lis-1), AGAP005010 (ortholog of ara), AGAP009048 (ortholog of csul), AGAP002022 (ortholog of mael), AGAP004892 (ortholog of fry), AGAP010442 (ortholog of fz2), AGAP010423 (ortholog of Stat92E), AGAP000099 (ortholog of Stat92E), AGAP006894 (ortholog of qm), AGAP008181 (ortholog of Smg5), AGAP007966 (ortholog of ATPsyngamma), AGAP005058 (ortholog of stet), AGAP007699 (ortholog of Ran), AGAP001471 (ortholog of Pdkl), AGAP002295 (ortholog of asun), AGAP009310 (ortholog of eRF3), AGAP007587 (ortholog of Pkn), AGAP010893 (ortholog of Osbp), AGAP000565 (ortholog of Trxr-1), AGAP007623 (ortholog of chb), AGAP007999 (ortholog of Nup154), AGAP005344 (ortholog of Vps28), AGAP002221 (ortholog of Nle), AGAP001820 (ortholog of Hel89B), AGAP009594 (ortholog of dtr), AGAP013301 (ortholog of Mnt), AGAP006241 (ortholog of zpg), AGAP029636 (ortholog of sns), AGAP029471 (ortholog of sns), AGAP008137 (ortholog of Npcla), AGAP001229 (ortholog of Dlc90F), AGAP003036 (ortholog of Bin1), AGAP005853 (ortholog of fws), AGAP005401 (ortholog of Kap-alphal), AGAP029921 (ortholog of Torsin), AGAP004820 (ortholog of Rtca), AGAP011830 (ortholog of Inos), AGAP002344 (ortholog of mei-P26), AGAP012499 (ortholog of SAK), AGAP004754 (ortholog of Drone), AGAP005575 (ortholog of Grip84), AGAP008847 (ortholog of Grip75), AGAP001227 (ortholog of Grip128), AGAP007599 (ortholog of Taz), AGAP012125 (ortholog of Pop1), AGAP002880 (ortholog of CSN5), AGAP004825 (ortholog of spd-2), AGAP003687 (ortholog of mino), AGAP001933 (ortholog of ball), AGAP002436 (ortholog of Kpc2), AGAP011776 (ortholog of Ack), AGAP010553 (ortholog of hfp), AGAP008027 (ortholog of slmo), AGAP005850 (ortholog of Gas8), AGAP001406 (ortholog of wuho), AGAP004315 (ortholog of Pink1), AGAP000337 (ortholog of Upf2), AGAP029519 (ortholog of ATP7), AGAP010877 (ortholog of shop), AGAP001878 (ortholog of Naa20A), AGAP008327 (ortholog of shv), AGAP010497 (ortholog of Pex12), AGAP010171 (ortholog of papi), AGAP004002 (ortholog of Hsp60C), AGAP008705 (ortholog of rau), AGAP004854 (ortholog of Kdm5), AGAP009959 (ortholog of TTLL3B), AGAP002092 (ortholog of Rab30), AGAP008223 (ortholog of gudu), AGAP002379 (ortholog of PAPLA1), AGAP009348 (ortholog of Usp14), AGAP009680 (ortholog of Bug22), AGAP000948 (ortholog of Yip1d1), AGAP010199 (ortholog of Rsph1), AGAP010253 (ortholog of Prosalpha6T), AGAP029493 (ortholog of ssp3), AGAP008727 (ortholog of COX4L), AGAP007390 (ortholog of Pngl), AGAP007670 (ortholog of U2A), AGAP005268 (ortholog of CG13202), AGAP002626 (ortholog of rho-7), AGAP006885 (ortholog of Prp8), AGAP010380 (ortholog of mip120), AGAP007904 (ortholog of S-Lap5), AGAP012281 (ortholog of eIF3m), AGAP012600 (ortholog of Hr51), AGAP009890 (ortholog of Hr51), AGAP029760 (ortholog of clu), AGAP012289 (ortholog of Dcr-2), AGAP007214 (ortholog of Dnaaf3), AGAP003410 (ortholog of mip40), AGAP005347 (ortholog of Mgat1), AGAP008689 (ortholog of CG9313), AGAP012373 (ortholog of Rael), AGAP003356 (ortholog of nsr), AGAP008095 (ortholog of tous), AGAP009973 (ortholog of YMEI1L), AGAP004746 (ortholog of thoc5), AGAP007422 (ortholog of MAN1), AGAP007395 (ortholog of Dis312), AGAP001108 (ortholog of ttm2), AGAP005101 (ortholog of Cep290), AGAP000928 (ortholog of Pex10), AGAP007902 (ortholog of Strip), AGAP002009 (ortholog of Dnah3), AGAP001028 (ortholog of blanks), AGAP000990 (ortholog of mmm), AGAP009027 (ortholog of mmm), AGAP010836 (ortholog of Pex2), AGAP001952 (ortholog of S-Lap1), AGAP003265 (ortholog of Cyp40), AGAP007896 (ortholog of wls), AGAP002086 (ortholog of Duba), AGAP010444 (ortholog of twy), AGAP011727 (ortholog of Syx13), AGAP028638 (ortholog of cmb), AGAP009084 (ortholog of CG5048), AGAP006359 (ortholog of Wdr92), AGAP002283 (ortholog of Pex16), AGAP002525 (ortholog of eIF2A), AGAP003715 (ortholog of aux), AGAP008745 (ortholog of sac), AGAP002618 (ortholog of Sym), AGAP002405 (ortholog of PSMG1), AGAP006406 (ortholog of CG8236), AGAP013127 (ortholog of CG8236), AGAP004744 (ortholog of ScsbetaA), AGAP001502 (ortholog of CG9920), AGAP000240 (ortholog of HtrA2), AGAP009295 (ortholog of Kpc1), AGAP001419 (ortholog of Syp), AGAP007785 (ortholog of Theg), AGAP001130 (ortholog of Gld2), AGAP003314 (ortholog of Gld2), AGAP004621 (ortholog of wam), AGAP004622 (ortholog of bb8), AGAP003449 (ortholog of Root), AGAP003643 (ortholog of puf), AGAP006030 (ortholog of mfrn), AGAP006936 (ortholog of Cyt-c1L), AGAP002310 (ortholog of Cad99C), AGAP008039 (ortholog of CG12069), AGAP003895 (ortholog of klhl10), AGAP009746 (ortholog of rept), AGAP013747 (ortholog of DCTN5-p25), AGAP010028 (ortholog of Top3alpha), AGAP001950 (ortholog of Mytl), AGAP029939 (ortholog of CG18675), AGAP006580 (ortholog of park), AGAP005359 (ortholog of ago), AGAP006942 (ortholog of Apl), AGAP002603 (ortholog of HBS1), AGAP012460 (ortholog of Fsn), AGAP004930 (ortholog of Trel), AGAP001469 (ortholog of Liprin-alpha), AGAP004439 (ortholog of Cog7), AGAP009079 (ortholog of CG31759), AGAP011032 (ortholog of Rsph9), AGAP004751 (ortholog of Cap-D3), AGAP006508 (ortholog of SCaMC), AGAP005880 (ortholog of Rsph3), AGAP004931 (ortholog of Gorab), AGAP010476 (ortholog of endos), AGAP006526 (ortholog of Lasp), AGAP006590 (ortholog of sif), AGAP010160 (ortholog of Myo31DF), AGAP010346 (ortholog of Mer), AGAP007212 (ortholog of pea), AGAP005349 (ortholog of snky), AGAP001954 (ortholog of psidin), AGAP001886 (ortholog of Vrp1), AGAP008698 (ortholog of Chd1), AGAP007306 (ortholog of Mob4), AGAP004739 (ortholog of Nop60B), AGAP011661 (ortholog of Su(var)3-3), AGAP028093 (ortholog of eIF4G2), AGAP002502 (ortholog of eIF4G2), AGAP007294 (ortholog of Diap1), AGAP007521 (ortholog of scny), AGAP008805 (ortholog of scny), AGAP008781 (ortholog of bond), AGAP004476 (ortholog of Vmat), AGAP003526 (ortholog of asl), AGAP029052 (ortholog of Pif1A),AGAP009399 (ortholog of Hr39), AGAP009400 (ortholog of Hr39), AGAP008105 (ortholog of Cul3), AGAP012905 (ortholog of Cul3), AGAP006479 (ortholog of didum), AGAP000029 (ortholog of nej), AGAP005291 (ortholog of larp), AGAP009487 (ortholog of brun), AGAP008408 (ortholog of Bsg), AGAP001061 (ortholog of f), AGAP029510 (ortholog of cic), AGAP008112 (ortholog of mtsh), AGAP005523 (ortholog of SmB), AGAP000646 (ortholog of Myc), AGAP010722 (ortholog of vret), AGAP009237 (ortholog of Acn), AGAP003047 (ortholog of bel), AGAP004852 (ortholog of hts), AGAP004768 (ortholog of nclb), AGAP000622 (ortholog of lost), AGAP001519 (ortholog of Brr2), AGAP010737 (ortholog of Dark), AGAP005799 (ortholog of dally), AGAP006036 (ortholog of Dic61B), AGAP007121 (ortholog of Cyt-b5), AGAP029774 (ortholog of ab), AGAP011165 (ortholog of mgr), AGAP010147 (ortholog of Mhc), AGAP011092 (ortholog of pAbp), AGAP011141 (ortholog of mlt), AGAP005423 (ortholog of Prosalpha4T1), AGAP013775 (ortholog of Tfb5), AGAP000847 (ortholog of Doa), AGAP013457 (ortholog of Doa), AGAP009940 (ortholog of stai), AGAP011078 (ortholog of Zmynd10), AGAP004862 (ortholog of mfr), AGAP011328 (ortholog of Sf3al), AGAP008467 (ortholog of Ntl), AGAP005831 (ortholog of Topors), AGAP009639 (ortholog of mil), AGAP012606 (ortholog of sau), AGAP012632 (ortholog of sau), AGAP000137 (ortholog of lovit), AGAP007675 (ortholog of kl-3), AGAP001672 (ortholog of kl-5), AGAP008980 (ortholog of ap), AGAP003226 (ortholog of Dhc16F), AGAP012424 (ortholog of InR), AGAP005784 (ortholog of Pall), AGAP007606 (ortholog of Pall), AGAP008481 (ortholog of Rpp30), AGAP029408 (ortholog of Rpp30), AGAP008917 (ortholog of Top2), AGAP005630 (ortholog of TER94), AGAP004957 (ortholog of Orc1), AGAP005322 (ortholog of SRPK), AGAP001792 (ortholog of bor), AGAP011573 (ortholog of dbf), AGAP009792 (ortholog of Pen), AGAP007523 (ortholog of zip), and AGAP010510 (ortholog of betaTub60D).

Non-limiting examples of orthologs of *Drosophila melanogaster* male-fertility essential genes in *Bactrocera tyroni* include the following GenBank^{®} Accession Nos: LOC120766601 (ortholog of abd-A), LOC120766752 (ortholog of Abd-B), LOC120775568 (ortholog of ac), LOC120768304 (ortholog of nAChRbetal), LOC120769154 (ortholog of Pfas), LOC120769069 (ortholog of aop), LOC120767613 (ortholog of bru1), LOC120773471 (ortholog of asp), LOC120771071 (ortholog of aub), LOC120767784 (ortholog of ca), LOC120781394 (ortholog of chic), LOC120774405 (ortholog of Chc), LOC120769784 (ortholog of eya), LOC120769496 (ortholog of eya), LOC120774429 (ortholog of crm), LOC120772751 (ortholog of CycB), LOC120779124 (ortholog of Dab), LOC120773439 (ortholog of dor), LOC120775216 (ortholog of dx), LOC120770309 (ortholog of EcR), LOC120778232 (ortholog of emc), LOC120770577 (ortholog of exu), LOC120776153 (ortholog of Fas2), LOC120781664 (ortholog of tj), LOC120773469 (ortholog of g), LOC120778428 (ortholog of H), LOC120777101 (ortholog of hay), LOC120776832 (ortholog of Hsp83), LOC120773901 (ortholog of kl-2), LOC120773470 (ortholog of Grip91), LOC120781615 (ortholog of ifc), LOC120768374 (ortholog of CIAPIN1), LOC120767390 (ortholog of stc), LOC120768259 (ortholog of ND-B17), LOC120769402 (ortholog of Phb1), LOC120767690 (ortholog of l(2)gl), LOC120780503 (ortholog of hyd), LOC120776779 (ortholog of sti), LOC120769730 (ortholog of Lam), LOC120782127 (ortholog of lds), LOC120774819 (ortholog of lz), LOC120775048 (ortholog of m), LOC120775941 (ortholog of RpL36), LOC120766629 (ortholog of RpS3), LOC120775040 (ortholog of mal), LOC120772570 (ortholog of mam), LOC120770379 (ortholog of mago), LOC120775297 (ortholog of mle), LOC120776833 (ortholog of msl-3), LOC120775429 (ortholog of mod), LOC120773549 (ortholog of mod), LOC120767237 (ortholog of mod), LOC120771314 (ortholog of ms(3)K81), LOC120769309 (ortholog of PolQ), LOC120782358 (ortholog of osk), LOC120775340 (ortholog of otu), LOC120777503 (ortholog of pbl), LOC120771307 (ortholog of Poxn), LOC120769172 (ortholog of prd), LOC120767379 (ortholog of ref(2)P), LOC120767078 (ortholog of Rm62), LOC120773459 (ortholog of Polr2A), LOC120774415 (ortholog of rst), LOC120776743 (ortholog of rt), LOC120775598 (ortholog of run), LOC120775237 (ortholog of sbr), LOC120775003 (ortholog of sd), LOC120772453 (ortholog of shu), LOC120776637 (ortholog of sina), LOC120771104 (ortholog of sm), LOC120776552 (ortholog of spn-E), LOC120769686 (ortholog of Btk), LOC120772771 (ortholog of ss), LOC120770311 (ortholog of stau), LOC120774290 (ortholog of stg), LOC120772726 (ortholog of sub), LOC120782332 (ortholog of su(Hw)), LOC120774159 (ortholog of su(sable)), LOC120771621 (ortholog of tra2), LOC120766628 (ortholog of alphaTub84B), LOC120766987 (ortholog of betaTub85D), LOC120772968 (ortholog of tud), LOC120781550 (ortholog of RpS27A), LOC120774823 (ortholog of v), LOC120772550 (ortholog of vg), LOC120768040 (ortholog of vls), LOC120777987 (ortholog of vn), LOC120776163 (ortholog of y), LOC120776162 (ortholog of y), LOC120775255 (ortholog of sc), LOC120774364 (ortholog of ct), LOC120776837 (ortholog of Rdl), LOC120772225 (ortholog of Wnt2), LOC120768367 (ortholog of porin), LOC120776391 (ortholog of fwd), LOC120767373 (ortholog of neb), LOC120776412 (ortholog of Klp67A), LOC120770217 (ortholog of bgen), LOC120766805 (ortholog of pros), LOC120767768 (ortholog of Zw10), LOC120766984 (ortholog of fru), LOC120766388 (ortholog of sim), LOC120773541 (ortholog of hop), LOC120775926 (ortholog of piwi), LOC120782336 (ortholog of Rb97D), LOC120773816 (ortholog of upd1), LOC120769201 (ortholog of nmd), LOC120771007 (ortholog of GstS1), LOC120768181 (ortholog of Trf), LOC120774156 (ortholog of Cdc42), LOC120777218 (ortholog of Cds), LOC120777098 (ortholog of Taf6), LOC120766168 (ortholog of Sas-4), LOC120781425 (ortholog of dia), LOC120776333 (ortholog of cue), LOC120776728 (ortholog of fbl), LOC120777714 (ortholog of bol), LOC120777570 (ortholog of pelo), LOC120772750 (ortholog of blw), LOC120774903 (ortholog of eff), LOC120766250 (ortholog of heph), LOC120781355 (ortholog of jar), LOC120770238 (ortholog of ox), LOC120768217 (ortholog of poe), LOC120768232 (ortholog of scat), LOC120779324 (ortholog of Hsp60B), LOC120773003 (ortholog of Iswi), LOC120773990 (ortholog of Klp3A), LOC120768054 (ortholog of Syx5), LOC120772394 (ortholog of tsr), LOC120767022 (ortholog of wts), LOC120774369 (ortholog of ctp), LOC120767596 (ortholog of Ance), LOC120777621 (ortholog of Trl), LOC120774136 (ortholog of ProtA), LOC120773008 (ortholog of l(2)dtl), LOC120771926 (ortholog of Arf6), LOC120770117 (ortholog of cnn), LOC120773953 (ortholog of Rnp4F), LOC120782406 (ortholog of noi), LOC120774502 (ortholog of tilB), LOC120776914 (ortholog of Ddx1), LOC120777123 (ortholog of eIF4E1), LOC120773915 (ortholog of Rala), LOC120772097 (ortholog of Shc), LOC120772215 (ortholog of spag), LOC120770050 (ortholog of Lis-1), LOC120770052 (ortholog of Lis-1), LOC120778817 (ortholog of ara), LOC120772383 (ortholog of csul), LOC120775122 (ortholog of mael), LOC120778467 (ortholog of fry), LOC120777480 (ortholog of fz2), LOC120767083 (ortholog of Stat92E), LOC120777331 (ortholog of qm), LOC120767352 (ortholog of Smg5), LOC120775537 (ortholog of ATPsyngamma), LOC120776334 (ortholog of stet), LOC120775742 (ortholog of Ran), LOC120776946 (ortholog of Pdkl), LOC120782434 (ortholog of asun), LOC120767590 (ortholog of eRF3), LOC120770954 (ortholog of Pkn), LOC120767075 (ortholog of Osbp), LOC120775995 (ortholog of Trxr-1), LOC120778859 (ortholog of chb), LOC120768016 (ortholog of Nup154), LOC120772674 (ortholog of Vps28), LOC120775833 (ortholog of Nle), LOC120769980 (ortholog of Hel89B), LOC120769349 (ortholog of dtr), LOC120774006 (ortholog of Mnt), LOC120779054 (ortholog of zpg), LOC120770318 (ortholog of sns), LOC120767380 (ortholog of Npc1a), LOC120766289 (ortholog of Dlc90F), LOC120766377 (ortholog of Bin1), LOC120768977 (ortholog of fws), LOC120777522 (ortholog of Kap-alphal), LOC120771022 (ortholog of Cpes), LOC120775497 (ortholog of Torsin), LOC120773345 (ortholog of Rtca), LOC120771002 (ortholog of Inos), LOC120774069 (ortholog of mei-P26), LOC120778287 (ortholog of SAK), LOC120778475 (ortholog of Dronc), LOC120774970 (ortholog of Grip84), LOC120767467 (ortholog of Grip75), LOC120775116 (ortholog of Grip128), LOC120770234 (ortholog of Taz), LOC120776726 (ortholog of Pop1), LOC120766449 (ortholog of CSN5), LOC120777159 (ortholog of mino), LOC120782749 (ortholog of ball), LOC120772661 (ortholog of Kpc2), LOC120770496 (ortholog of Ack), LOC120774028 (ortholog of hfp), LOC120775088 (ortholog of Fmrl), LOC120769200 (ortholog of slmo), LOC120776031 (ortholog of Gas8), LOC120773743 (ortholog of wuho), LOC120775264 (ortholog of Pink1), LOC120774723 (ortholog of Upf2), LOC120774704 (ortholog of ATP7), LOC120774108 (ortholog of shop), LOC120775542 (ortholog of Naa20A), LOC120767233 (ortholog of shv), LOC120768932 (ortholog of Pex12), LOC120769508 (ortholog of papi), LOC120769104 (ortholog of rau), LOC120781865 (ortholog of Kdm5), LOC120769683 (ortholog of TTLL3B), LOC120767777 (ortholog of Rab30), LOC120768268 (ortholog of gudu), LOC120775130 (ortholog of PAPLA1), LOC120769248 (ortholog of Usp14), LOC120781389 (ortholog of Bug22), LOC120769440 (ortholog of Yipldl), LOC120769245 (ortholog of Rsph1), LOC120768533 (ortholog of Prosalpha6T), LOC120767460 (ortholog of ssp3), LOC120775378 (ortholog of COX4L), LOC120772103 (ortholog of Pngl), LOC120772129 (ortholog of U2A), LOC120772164 (ortholog of CG13202), LOC120772329 (ortholog of rho-7), LOC120771309 (ortholog of Prp8), LOC120770448 (ortholog of mip120), LOC120775795 (ortholog of S-Lap7), LOC120771442 (ortholog of eIF3m), LOC120771513 (ortholog of Hr51), LOC120773119 (ortholog of clu), LOC120770472 (ortholog of Dcr-2), LOC120772032 (ortholog of Dnaaf3), LOC120771506 (ortholog of mip40), LOC120771144 (ortholog of Mgat1), LOC120771037 (ortholog of CG9313), LOC120772115 (ortholog of Rael), LOC120770182 (ortholog of nsr), LOC120770197 (ortholog of nsr), LOC120774996 (ortholog of nsr), LOC120772871 (ortholog of tous), LOC120770252 (ortholog of YME1L), LOC120767219 (ortholog of thoc5), LOC120776403 (ortholog of Rsph4a), LOC120776668 (ortholog of Rsph4a), LOC120771521 (ortholog of MAN1), LOC120777215 (ortholog of Dis312), LOC120774859 (ortholog of ttm2), LOC120777005 (ortholog of Cep290), LOC120776362 (ortholog of Pex10), LOC120777242 (ortholog of Strip), LOC120768230 (ortholog of Dpy-30L2), LOC120777998 (ortholog of Dnah3), LOC120776042 (ortholog of mmm), LOC120779150 (ortholog of Pex2), LOC120777608 (ortholog of S-Lap1), LOC120779005 (ortholog of S-Lap1), LOC120777741 (ortholog of Cyp40), LOC120777740 (ortholog of Cyp40), LOC120778242 (ortholog of wls), LOC120777581 (ortholog of Duba), LOC120778678 (ortholog of twy), LOC120778458 (ortholog of Syx13), LOC120776356 (ortholog of cmb), LOC120776644 (ortholog of CG5048), LOC120782348 (ortholog of ATPsynbetaL), LOC120777257 (ortholog of Wdr92), LOC120771314 (ortholog of HipHop), LOC120777952 (ortholog ofms(3)76Cc), LOC120776339 (ortholog of wnd), LOC120776777 (ortholog of ms(3)76Ca), LOC120777624 (ortholog of Pex16), LOC120778351 (ortholog of eIF2A), LOC120773877 (ortholog of aux), LOC120773169 (ortholog of Sym), LOC120782430 (ortholog of PSMG1), LOC120774976 (ortholog of CG8236), LOC120766821 (ortholog of ScsbetaA), LOC120766872 (ortholog of topi), LOC120782831 (ortholog of Rsbp15), LOC120771467 (ortholog of CG9920), LOC120782786 (ortholog of HtrA2), LOC120780510 (ortholog of Kpc1), LOC120775429 (ortholog of maca), LOC120766965 (ortholog of CG14305), LOC120766367 (ortholog of Syp), LOC120774111 (ortholog of Gld2), LOC120782516 (ortholog of wam), LOC120782662 (ortholog of wam), LOC120772906 (ortholog of wam), LOC120769241 (ortholog of wam), LOC120767571 (ortholog of bb8), LOC120775159 (ortholog of tbrd-1), LOC120769237 (ortholog of Root), LOC120766470 (ortholog of puf), LOC120766595 (ortholog of mfrn), LOC120778269 (ortholog of Cyt-c1L), LOC120777560 (ortholog of Cad99C), LOC120766274 (ortholog of CG12069), LOC120769805 (ortholog of CG12069), LOC120769657 (ortholog of klhl10), LOC120776803 (ortholog of rept), LOC120782232 (ortholog of pont), LOC120767987 (ortholog of DCTN5-p25), LOC120781400 (ortholog of Top3alpha), LOC120775789 (ortholog of Roclb), LOC120778209 (ortholog of Mytl), LOC120776971 (ortholog of CG18675), LOC120778774 (ortholog of park), LOC120776474 (ortholog of ago), LOC120767360 (ortholog of Pkd2), LOC120779126 (ortholog of Ap1), LOC120776895 (ortholog of HBS1), LOC120772001 (ortholog of Fsn), LOC120775795 (ortholog of S-Lap3), LOC120774710 (ortholog of Trel), LOC120769153 (ortholog of Liprin-alpha), LOC120772200 (ortholog of Cog7), LOC120774483 (ortholog of CG31759), LOC120769606 (ortholog of Rsph9), LOC120775795 (ortholog of S-Lap4), LOC120769681 (ortholog of SCaMC), LOC120776713 (ortholog of SCaMC), LOC120776640 (ortholog of SCaMC), LOC120777608 (ortholog of S-Lap2), LOC120779005 (ortholog of S-Lap2), LOC120778348 (ortholog of tut), LOC120777875 (ortholog of Rsph3), LOC120775865 (ortholog of Erk7), LOC120777261 (ortholog of Gorab), LOC120776438 (ortholog of endos), LOC120777107 (ortholog of Lasp), LOC120777048 (ortholog of sif), LOC120769758 (ortholog of Myo31DF), LOC120774148 (ortholog of Mer), LOC120770970 (ortholog of pea), LOC120767791 (ortholog of Cyt-c-d), LOC120780632 (ortholog of snky), LOC120770724 (ortholog of psidin), LOC120770138 (ortholog of Vrp1), LOC120782369 (ortholog of gish), LOC120770061 (ortholog of Mob4), LOC120771089 (ortholog of Nop60B), LOC120777897 (ortholog of Su(var)3-3), LOC120776472 (ortholog of Rcd7), LOC120766974 (ortholog of eIF4G2), LOC120779073 (ortholog of Diap1), LOC120773935 (ortholog of mxc), LOC120766278 (ortholog of bond), LOC120771429 (ortholog of Vmat), LOC120781260 (ortholog of asl), LOC120782664 (ortholog of Pif1A), LOC120769137 (ortholog of Hr39), LOC120767724 (ortholog of Cul3), LOC120771810 (ortholog of didum), LOC120776323 (ortholog of ms(2)34Fe), LOC120774974 (ortholog of nej), LOC120782454 (ortholog of larp), LOC120768012 (ortholog of brun), LOC120767946 (ortholog of Bsg), LOC120775616 (ortholog of f), LOC120774485 (ortholog of f), LOC120766849 (ortholog of cic), LOC120769723 (ortholog of mtsh), LOC120775954 (ortholog of SmB), LOC120776071 (ortholog of Myc), LOC120775203 (ortholog of vret), LOC120768278 (ortholog of Acn), LOC120769018 (ortholog of bel), LOC120772466 (ortholog of hts), LOC120771991 (ortholog of nclb), LOC120777451 (ortholog of lost), LOC120776648 (ortholog of Brr2), LOC120778051 (ortholog of dally), LOC120776013 (ortholog of Dic61B), LOC120774740 (ortholog of Dic61B), LOC120773681 (ortholog of Dic61B), LOC120772230 (ortholog of Cyt-b5), LOC120770046 (ortholog of Cyt-b5), LOC120781558 (ortholog of ab), LOC120782811 (ortholog of mgr), LOC120768282 (ortholog of Mhc), LOC120772808 (ortholog of pAbp), LOC120770102 (ortholog of mlt), LOC120774021 (ortholog of Prosalpha4Tl), LOC120771047 (ortholog of Tfb5), LOC120776707 (ortholog of Doa), LOC120767746 (ortholog of stai), LOC120777237 (ortholog of Zmynd10), LOC120777796 (ortholog of mfr), LOC120775011 (ortholog of Sf3a1), LOC120767206 (ortholog of Ntl), LOC120770934 (ortholog of Topors), LOC120782451 (ortholog of mil), LOC120768541 (ortholog of sau), LOC120780173 (ortholog of lovit), LOC120769587 (ortholog of kl-3), LOC120766410 (ortholog of kl-5), LOC120767446 (ortholog of WDY), LOC120771973 (ortholog of ap), LOC120774925 (ortholog of Dhc16F), LOC120782635 (ortholog of InR), LOC120767322 (ortholog of Pall), LOC120769822 (ortholog of Rpp30), LOC120768061 (ortholog of Top2), LOC120772986 (ortholog of knon), LOC120781693 (ortholog of shps), LOC120770943 (ortholog of TER94), LOC120770952 (ortholog of Orc1), LOC120772990 (ortholog of SRPK), LOC120782435 (ortholog of bor), LOC120775913 (ortholog of dbf), LOC120769001 (ortholog of Pen), LOC120770998 (ortholog of zip), and LOC120771294 (ortholog of betaTub60D).

Non-limiting examples of orthologs of *Drosophila melanogaster* male-fertility essential genes in *Bactrocera dorsalis* include the following GenBank^{®} Accession Nos: LOC105226091 (ortholog of abd-A), LOC105226106 (ortholog of Abd-B), LOC105234201 (ortholog of ac), LOC105230833 (ortholog of nAChRbetal), LOC105228744 (ortholog of aop), LOC115066591 (ortholog of asp), LOC105230567 (ortholog of aub), LOC105233229 (ortholog of ca), LOC105232075 (ortholog of Chc), LOC105224219 (ortholog of crm), LOC105229679 (ortholog of CycB), LOC105223426 (ortholog of Dab), LOC105222881 (ortholog of dor), LOC105234061 (ortholog of dx), LOC105226312 (ortholog of EcR), LOC105224429 (ortholog of emc), LOC105225443 (ortholog of exu), LOC105227884 (ortholog of tj), LOC105225508 (ortholog of g), LOC105233250 (ortholog of H), LOC105223929 (ortholog of hay), LOC105226434 (ortholog of Hsp83), LOC109580057 (ortholog of kl-2), LOC105233317 (ortholog of kl-2), LOC105225507 (ortholog of Grip91), LOC105226394 (ortholog of ifc), LOC105225681 (ortholog of stc), LOC105229084 (ortholog of ND-B17), LOC105222253 (ortholog of Phb1), LOC105233546 (ortholog of l(2)gl), LOC105230161 (ortholog of hyd), LOC105233087 (ortholog of sti), LOC105234127 (ortholog of Lam), LOC105226635 (ortholog of lds), LOC105224371 (ortholog of m), LOC105226559 (ortholog of RpL36), LOC105231498 (ortholog of RpS3), LOC105229732 (ortholog of mal), LOC105228185 (ortholog of mam), LOC105223862 (ortholog of mago), LOC105225000 (ortholog of mle), LOC105226432 (ortholog of msl-3), LOC105232053 (ortholog of mod), LOC105233405 (ortholog of ms(3)K81), LOC105226327 (ortholog of PolQ), LOC105232054 (ortholog of osk), LOC105222775 (ortholog of otu), LOC105222852 (ortholog of Poxn), LOC105224398 (ortholog of prd), LOC105232320 (ortholog of ref(2)P), LOC105232490 (ortholog of Rm62), LOC105227498 (ortholog of Polr2A), LOC105227401 (ortholog of rt), LOC105226934 (ortholog of run), LOC105230383 (ortholog of sbr), LOC105230740 (ortholog of sd), LOC105228650 (ortholog of shu), LOC105233714 (ortholog of sina), LOC105230469 (ortholog of spn-E), LOC105229269 (ortholog of Btk), LOC105230895 (ortholog of ss), LOC105229849 (ortholog of stg), LOC105228975 (ortholog of sub), LOC105225855 (ortholog of su(Hw)), LOC105228593 (ortholog of su(sable)), LOC105222300 (ortholog of tra2), LOC105231497 (ortholog of alphaTub84B), LOC105233263 (ortholog of betaTub85D), LOC105229969 (ortholog oftud), LOC 1 05230070 (ortholog of RpS27A), LOC105229692 (ortholog of vg), LOC105223740 (ortholog of vls), LOC105234192 (ortholog of y), LOC105234199 (ortholog of sc), LOC105228617 (ortholog of ct), LOC105227682 (ortholog of Wnt2), LOC105233273 (ortholog of porin), LOC105226463 (ortholog of fwd), LOC105225141 (ortholog of Klp67A), LOC105226919 (ortholog of bgcn), LOC105226639 (ortholog of pros), LOC105224207 (ortholog of Zw10), LOC105224493 (ortholog of fru), LOC105234151 (ortholog of sim), LOC105225518 (ortholog of hop), LOC105225888 (ortholog of piwi), LOC105222093 (ortholog ofRb97D), LOC105223634 (ortholog of upd1), LOC105233576 (ortholog of nmd), LOC105228691 (ortholog of GstS1), LOC105231122 (ortholog of Trf), LOC105231990 (ortholog of Cdc42), LOC105222459 (ortholog of Cds), LOC105229443 (ortholog of Taf6), LOC105225988 (ortholog of Sas-4), LOC105227509 (ortholog of dia), LOC105226443 (ortholog of cue), LOC105227904 (ortholog of fbl), LOC105232639 (ortholog of bol), LOC105223271 (ortholog of pelo), LOC105229678 (ortholog of blw), LOC105234234 (ortholog of eff), LOC105227019 (ortholog of heph), LOC105232375 (ortholog of poe), LOC105222183 (ortholog of Hsp60B), LOC105222322 (ortholog of Iswi), LOC105224089 (ortholog of Klp3A), LOC105228758 (ortholog of Syx5), LOC105228161 (ortholog of tsr), LOC105225859 (ortholog of wts), LOC105232186 (ortholog of ctp), LOC105232277 (ortholog of Ance), LOC105227220 (ortholog of Trl), LOC105231354 (ortholog of ProtA), LOC105230566 (ortholog of l(2)dtl), LOC105230129 (ortholog of Arf6), LOC105223185 (ortholog of Rnp4F), LOC105230217 (ortholog of noi), LOC105229472 (ortholog of Ddx1), LOC105233718 (ortholog of eIF4E1), LOC105228415 (ortholog of Shc), LOC105227302 (ortholog of spag), LOC105226495 (ortholog of ara), LOC105222118 (ortholog of csul), LOC105232104 (ortholog of mael), LOC105224720 (ortholog of fz2), LOC105231839 (ortholog of Stat92E), LOC105231386 (ortholog of qm), LOC105232267 (ortholog of Smg5), LOC105222565 (ortholog of ATPsyngamma), LOC105226447 (ortholog of stet), LOC105230351 (ortholog of Ran), LOC105225577 (ortholog of Pdkl), LOC105223334 (ortholog of asun), LOC 1 05231149 (ortholog of eRF3), LOC105234160 (ortholog of Osbp), LOC105228306 (ortholog of Trxr-1), LOC105232723 (ortholog of chb), LOC105223739 (ortholog of Nup154), LOC105230581 (ortholog of Vps28), LOC105234052 (ortholog of Nle), LOC105230945 (ortholog of Hel89B), LOC105228308 (ortholog of Mnt), LOC105227158 (ortholog of zpg), LOC105226787 (ortholog of Dlc90F), LOC105227072 (ortholog of Bin1), LOC105225129 (ortholog of fws), LOC109579180 (ortholog of Kap-alphal), LOC109579586 (ortholog of Cpes), LOC105232124 (ortholog of Torsin), LOC105228848 (ortholog of Rtca), LOC105222319 (ortholog of Inos), LOC105223700 (ortholog of mei-P26), LOC105229463 (ortholog of SAK), LOC105224480 (ortholog of Drone), LOC105222663 (ortholog of Grip84), LOC105225668 (ortholog of Grip75), LOC105224994 (ortholog of Grip128), LOC105222107 (ortholog of Taz), LOC105230237 (ortholog of CSN5), LOC105222824 (ortholog of mino), LOC105222096 (ortholog of ball), LOC105226656 (ortholog of Kpc2), LOC105231532 (ortholog of Ack), LOC105232162 (ortholog of hfp), LOC105231852 (ortholog of Fmr1), LOC105225199 (ortholog of slmo), LOC105225561 (ortholog of wuho), LOC105232018 (ortholog of Pink1), LOC105230365 (ortholog of ATP7), LOC105228313 (ortholog of shop), LOC105230401 (ortholog of Naa20A), LOC105229265 (ortholog of shv), LOC105223584 (ortholog of Pex12), LOC105229281 (ortholog of papi), LOC105234242 (ortholog of rau), LOC105226707 (ortholog of Kdm5), LOC105223723 (ortholog of Rab30), LOC105228754 (ortholog of gudu), LOC105222269 (ortholog of Uspl14), LOC105223287 (ortholog of Bug22), LOC105232346 (ortholog of spag4), LOC105230730 (ortholog of Yipldl), LOC105223890 (ortholog of Rsph1), LOC105233586 (ortholog of Prosalpha6T), LOC105223507 (ortholog of ssp3), LOC105221883 (ortholog of COX4L), LOC105225479 (ortholog of Pngl), LOC105223055 (ortholog of U2A), LOC105228378 (ortholog of CG13202), LOC105228930 (ortholog of rho-7), LOC105230005 (ortholog of Prp8), LOC105228433 (ortholog of mip120), LOC105222860 (ortholog of S-Lap7), LOC105228443 (ortholog of eIF3m), LOC105223960 (ortholog of Hr51), LOC105225428 (ortholog of clu), LOC105224675 (ortholog of Dcr-2), LOC105227738 (ortholog of Dnaaf3), LOC105224688 (ortholog of mip40), LOC105225343 (ortholog of Mgat1), LOC105224542 (ortholog of Rael), LOC105231561 (ortholog of nsr), LOC105231560 (ortholog of nsr), LOC105222958 (ortholog of nsr), LOC105225372 (ortholog of YME1L), LOC105223298 (ortholog of thoc5), LOC105227646 (ortholog of Rsph4a), LOC105224142 (ortholog of MAN1), LOC105226464 (ortholog of Dis312), LOC105228265 (ortholog of ttm2), LOC105233093 (ortholog of Cep290), LOC105232664 (ortholog of Pex10), LOC105225029 (ortholog of Strip), LOC105223826 (ortholog of Dpy-30L2), LOC105232710 (ortholog of Dnah3), LOC105228327 (ortholog of mmm), LOC105224796 (ortholog of Pex2), LOC105223936 (ortholog of S-Lap1), LOC105223934 (ortholog of S-Lap1), LOC105230057 (ortholog of Cyp40), LOC105230058 (ortholog of Cyp40), LOC105223962 (ortholog of wls), LOC105227399 (ortholog of Duba), LOC105224103 (ortholog of Syx13), LOC105229494 (ortholog of cmb), LOC105227201 (ortholog of CG5048), LOC105233254 (ortholog of ATPsynbetaL), LOC105224012 (ortholog of Wdr92), LOC105233405 (ortholog of HipHop), LOC105224288 (ortholog of ms(3)76Cc), LOC105224286 (ortholog of wnd), LOC105225947 (ortholog of ms(3)76Ca), LOC105227195 (ortholog of Pex16), LOC105225593 (ortholog of eIF2A), LOC105231058 (ortholog of aux), LOC105222042 (ortholog of sac), LOC105226052 (ortholog of Sym), LOC105226040 (ortholog of PSMG1), LOC105222017 (ortholog of CG8236), LOC105230850 (ortholog of ScsbetaA), LOC105232030 (ortholog of topi), LOC105229507 (ortholog of Rsbp15), LOC105229787 (ortholog of HtrA2), LOC105234110 (ortholog of Kpc1), LOC105231431 (ortholog of CG14305), LOC105227630 (ortholog of Syp), LOC105222082 (ortholog of Theg), LOC105231382 (ortholog of Gld2), LOC105231945 (ortholog of wam), LOC105231893 (ortholog of wam), LOC105223922 (ortholog of wam), LOC105231892 (ortholog of wam), LOC105224061 (ortholog of bb8), LOC105227599 (ortholog of Root), LOC105225835 (ortholog of puf), LOC105223141 (ortholog of mfrn), LOC105233695 (ortholog of Cyt-c1L), LOC105230761 (ortholog of CG12069), LOC105223976 (ortholog of klhl10), LOC105222528 (ortholog of rept), LOC105224601 (ortholog of pont), LOC105223733 (ortholog of DCTN5-p25), LOC105223670 (ortholog of Top3alpha), LOC105225495 (ortholog of Roc1b), LOC105231441 (ortholog of Mytl), LOC105231424 (ortholog of CG18675), LOC105225264 (ortholog of park), LOC105231410 (ortholog of ago), LOC105230830 (ortholog of Pkd2), LOC105233682 (ortholog of Apl), LOC105227113 (ortholog of HBS1), LOC105222313 (ortholog of Fsn), LOC105222860 (ortholog of S-Lap3), LOC105225884 (ortholog of Trel), LOC105222158 (ortholog of Liprin-alpha), LOC105228890 (ortholog of Cog7), LOC105228230 (ortholog of CG31759), LOC105229336 (ortholog of Rsph9), LOC105222860 (ortholog of S-Lap4), LOC105224896 (ortholog of SCaMC), LOC105225062 (ortholog of SCaMC), LOC105229922 (ortholog of SCaMC), LOC105223936 (ortholog of S-Lap2), LOC105223934 (ortholog of S-Lap2), LOC105225611 (ortholog of tut), LOC105228241 (ortholog of Erk7), LOC105223404 (ortholog of Gorab), LOC105227394 (ortholog of endos), LOC105231987 (ortholog of Mer), LOC105225073 (ortholog of pea), LOC105233537 (ortholog of Cyt-c-d), LOC105230958 (ortholog of psidin), LOC105222303 (ortholog of Vrp1), LOC105227104 (ortholog of gish), LOC105230613 (ortholog of Mob4), LOC105224702 (ortholog of Nop60B), LOC105227333 (ortholog of Su(var)3-3), LOC105233988 (ortholog of Rcd7), LOC105226368 (ortholog of eIF4G2), LOC105224487 (ortholog of Diap1), LOC105231023 (ortholog of bond), LOC105228430 (ortholog of Vmat), LOC105230464 (ortholog of asl), LOC105233072 (ortholog of Hr39), LOC105225992 (ortholog of Cul3), LOC105228956 (ortholog of didum), LOC105224179 (ortholog of ms(2)34Fe), LOC105233310 (ortholog of nej), LOC105231919 (ortholog of larp), LOC105230765 (ortholog of brun), LOC105231126 (ortholog of Bsg), LOC105228316 (ortholog of f), LOC105230980 (ortholog of cic), LOC105223085 (ortholog of mtsh), LOC105229784 (ortholog of SmB), LOC105222207 (ortholog of Myc), LOC105234224 (ortholog of vret), LOC105222263 (ortholog of Acn), LOC105230913 (ortholog of bel), LOC105228676 (ortholog of hts), LOC105231075 (ortholog of lost), LOC105227251 (ortholog of Dic61B), LOC105228947 (ortholog of Cyt-b5), LOC105226637 (ortholog of mgr), LOC105229794 (ortholog of Mhc), LOC105229420 (ortholog of mlt), LOC105228279 (ortholog of Prosalpha4T1), LOC105229165 (ortholog of Tfb5), LOC105232593 (ortholog of Doa), LOC105225332 (ortholog of stai), LOC105224264 (ortholog of Zmynd10), LOC105231372 (ortholog of mfr), LOC105222818 (ortholog of Sf3a1), LOC105230549 (ortholog of Topors), LOC105232924 (ortholog of JYalpha), LOC105230404 (ortholog of mil), LOC105231217 (ortholog of sau), LOC105227623 (ortholog of kl-5), LOC105224389 (ortholog of WDY), LOC105233061 (ortholog of ap), LOC105227517 (ortholog of Dhc16F), LOC105230971 (ortholog of InR), LOC105227807 (ortholog of Pall), LOC105224190 (ortholog of Rpp30), LOC105232443 (ortholog of Top2), LOC105230599 (ortholog of TER94), LOC105228951 (ortholog of Orc1), LOC105230594 (ortholog of SRPK), LOC105223335 (ortholog of bor), LOC105226943 (ortholog of dbf), LOC105225314 (ortholog of Pen), and LOC105233070 (ortholog of betaTub60D).

Non-limiting examples of orthologs of *Drosophila melanogaster* male-fertility essential genes in *Helicoverpa zea* include the following GenBank^{®} Accession Nos: LOC124634569 (ortholog of Abd-B), LOC124632586 (ortholog of ac), LOC124632795 (ortholog of ac), LOC124643972 (ortholog of nAChRbetal), LOC124630271 (ortholog of Pfas), LOC124632361 (ortholog of aop), LOC124629504 (ortholog of bru1), LOC124632948 (ortholog of asp), LOC124646155 (ortholog of ca), LOC124630776 (ortholog of ca), LOC124646045 (ortholog of ca), LOC124645652 (ortholog of chic), LOC124639697 (ortholog of Chc), LOC124641288 (ortholog of eya), LOC124635931 (ortholog of crm), LOC124632737 (ortholog of CycB), LOC124638070 (ortholog of Dab), LOC124633365 (ortholog of dor), LOC124637632 (ortholog of dx), LOC124631107 (ortholog of EcR), LOC124635072 (ortholog of emc), LOC124642466 (ortholog of exu), LOC124636240 (ortholog of Fas2), LOC124646343 (ortholog of tj), LOC124636063 (ortholog of g), LOC124640171 (ortholog of H), LOC124634883 (ortholog of hay), LOC124642625 (ortholog of Hsp83), LOC124629621 (ortholog of kl-2), LOC124642144 (ortholog of Grip91), LOC124643657 (ortholog of ifc), LOC124634960 (ortholog of CIAPIN1), LOC124632989 (ortholog of stc), LOC124634286 (ortholog of ND-B17), LOC124646027 (ortholog of Phb1), LOC124633437 (ortholog of l(2)gl), LOC124642783 (ortholog of hyd), LOC124639633 (ortholog of sti), LOC124633294 (ortholog of Lam), LOC124639970 (ortholog of lds), LOC124645534 (ortholog of m), LOC124637075 (ortholog of RpL36), LOC124635793 (ortholog of RpS3), LOC124631966 (ortholog of mal), LOC124644423 (ortholog of twe), LOC124643689 (ortholog of mago), LOC124641844 (ortholog of mle), LOC124640793 (ortholog of msl-3), LOC124634414 (ortholog of mod), LOC124636228 (ortholog of mod), LOC124637779 (ortholog of mod), LOC124631993 (ortholog of PolQ), LOC124637603 (ortholog of pbl), LOC124642172 (ortholog of Poxn), LOC124629428 (ortholog of ref(2)P), LOC124643710 (ortholog of Polr2A), LOC124642994 (ortholog of rst), LOC124643025 (ortholog of rst), LOC124642956 (ortholog of rst), LOC124645666 (ortholog of rt), LOC124638934 (ortholog of run), LOC124636246 (ortholog of sbr), LOC124632326 (ortholog of sd), LOC124646283 (ortholog of shu), LOC124643126 (ortholog of sm), LOC124640087 (ortholog of spn-E), LOC124632794 (ortholog of Btk), LOC124640864 (ortholog of ss), LOC124644423 (ortholog of stg), LOC124633825 (ortholog of sub), LOC124637419 (ortholog of su(sable)), LOC124645435 (ortholog of alphaTub84B), LOC124634933 (ortholog of tud), LOC124633969 (ortholog of RpS27A), LOC124637118 (ortholog of Ubi-p63E), LOC124635950 (ortholog of v), LOC124632819 (ortholog of y), LOC124632448 (ortholog of sc), LOC124639323 (ortholog of ct), LOC124645097 (ortholog of Rdl), LOC124645029 (ortholog of Rdl), LOC124641491 (ortholog of Rdl), LOC124638702 (ortholog of porin), LOC124632262 (ortholog of aly), LOC124631647 (ortholog of fwd), LOC124630429 (ortholog of Klp67A), LOC124640079 (ortholog of pros), LOC124629731 (ortholog of Zw10), LOC124634611 (ortholog of fru), LOC124644133 (ortholog of sim), LOC124638313 (ortholog of hop), LOC124639189 (ortholog of nmd), LOC124639213 (ortholog of GstS1), LOC124639215 (ortholog of GstS1), LOC124639214 (ortholog of GstS1), LOC124639146 (ortholog of GstS1), LOC124639216 (ortholog of GstS1), LOC124629822 (ortholog of Trf), LOC124644966 (ortholog of Cdc42), LOC124630303 (ortholog of Cds), LOC124641804 (ortholog of Taf6), LOC124639354 (ortholog of Sas-4), LOC124643666 (ortholog of dia), LOC124644062 (ortholog of fbl), LOC124631261 (ortholog of pelo), LOC124638180 (ortholog of blw), LOC124629492 (ortholog of eff), LOC124630485 (ortholog of heph), LOC124632174 (ortholog of jar), LOC124639257 (ortholog of ox), LOC124636667 (ortholog of poe), LOC124629718 (ortholog of scat), LOC124638815 (ortholog of Hsp60B), LOC124631016 (ortholog of fzo), LOC124632301 (ortholog of Iswi), LOC124640226 (ortholog of Klp3A), LOC124630026 (ortholog of Syx5), LOC124642972 (ortholog of tsr), LOC124634654 (ortholog of wts), LOC124631926 (ortholog of Ance), LOC124631881 (ortholog of Ance), LOC124629510 (ortholog of l(2)dtl), LOC124637175 (ortholog of Arf6), LOC124639549 (ortholog of Rnp4F), LOC124634604 (ortholog of noi), LOC124646167 (ortholog of tilB), LOC124639440 (ortholog of Ddx1), LOC124637670 (ortholog of Rala), LOC124632076 (ortholog of Shc), LOC124638329 (ortholog of spag), LOC124637595 (ortholog of Lis-1), LOC124644807 (ortholog of ara), LOC124644807 (ortholog of caup), LOC124640649 (ortholog of csul), LOC124636387 (ortholog of mael), LOC124644853 (ortholog of fry), LOC124634952 (ortholog of fz2), LOC124644398 (ortholog of Stat92E), LOC124631938 (ortholog of qm), LOC124637103 (ortholog of Smg5), LOC124642287 (ortholog of ATPsyngamma), LOC124633447 (ortholog of stet), LOC124631264 (ortholog of Ran), LOC124635413 (ortholog of Pdkl), LOC124633000 (ortholog of asun), LOC124645240 (ortholog of eRF3), LOC124632927 (ortholog of eRF3), LOC124632918 (ortholog of Pkn), LOC124639803 (ortholog of Osbp), LOC124643190 (ortholog of chb), LOC124631039 (ortholog of Nup154), LOC124641497 (ortholog of Vps28), LOC124637446 (ortholog of Nle), LOC124632981 (ortholog of Hel89B), LOC124641687 (ortholog of dtr), LOC124639258 (ortholog of Mnt), LOC124642954 (ortholog of sns), LOC124642895 (ortholog of sns), LOC124638739 (ortholog of Npcla), LOC124640876 (ortholog of Dlc90F), LOC124633921 (ortholog of Bin1), LOC124636790 (ortholog of fws), LOC124631661 (ortholog of Kap-alpha1), LOC124642903 (ortholog of Cpes), LOC124640333 (ortholog of Torsin), LOC124637976 (ortholog of Rtca), LOC124638864 (ortholog of Inos), LOC124632213 (ortholog of mei-P26), LOC124631301 (ortholog of SAK), LOC124630861 (ortholog of Drone), LOC124632581 (ortholog of Grip84), LOC124637947 (ortholog of Grip75), LOC124638062 (ortholog of Taz), LOC124635374 (ortholog of Pop1), LOC124631187 (ortholog of CSN5), LOC124642124 (ortholog of mino), LOC124638946 (ortholog of ball), LOC124631090 (ortholog of Ack), LOC124636783 (ortholog of hfp), LOC124634373 (ortholog of Fmrl), LOC124645476 (ortholog of slmo), LOC124640884 (ortholog of Gas8), LOC124631884 (ortholog of wuho), LOC124646233 (ortholog of Upf2), LOC124634506 (ortholog of p24-1), LOC124632656 (ortholog of shop), LOC124636168 (ortholog of Naa20A), LOC124645670 (ortholog of shv), LOC124639413 (ortholog of Pex12), LOC124639467 (ortholog of Pex12), LOC124638717 (ortholog of papi), LOC124638815 (ortholog of Hsp60C), LOC124646103 (ortholog of rau), LOC124629881 (ortholog of Kdm5), LOC124646087 (ortholog of TTLL3B), LOC124635744 (ortholog of Rab30), LOC124631552 (ortholog of gudu), LOC124644447 (ortholog of PAPLA1), LOC124638213 (ortholog of Usp14), LOC124631515 (ortholog of Bug22), LOC124634505 (ortholog of Yipldl), LOC124629828 (ortholog of Rsph1), LOC124635860 (ortholog of Prosalpha6T), LOC124642235 (ortholog of ssp3), LOC124641663 (ortholog of COX4L), LOC124638544 (ortholog of Pngl), LOC124630503 (ortholog of U2A), LOC124637169 (ortholog of rho-7), LOC124637286 (ortholog of Prp8), LOC124638911 (ortholog of mip120), LOC124634050 (ortholog of S-Lap5), LOC124634050 (ortholog of S-Lap7), LOC124632578 (ortholog of eIF3m), LOC124642404 (ortholog of Hr51), LOC124637753 (ortholog of clu), LOC124634050 (ortholog of S-Lap8), LOC124631319 (ortholog of Dnaaf3), LOC124637826 (ortholog of mip40), LOC124643418 (ortholog of Mgat1), LOC124638485 (ortholog of CG9313), LOC124641323 (ortholog of Rael), LOC124641378 (ortholog of nsr), LOC124633648 (ortholog of tous), LOC124643299 (ortholog of YME1L), LOC124637855 (ortholog of thoc5), LOC124640020 (ortholog of Rsph4a), LOC124631779 (ortholog of MAN1), LOC124635233 (ortholog of Dis312), LOC124637129 (ortholog of ttm2), LOC124641202 (ortholog of Pex10), LOC124631866 (ortholog of Strip), LOC124633123 (ortholog of Dnah3), LOC124630264 (ortholog of Pex2), LOC124644794 (ortholog of S-Lapl), LOC124641576 (ortholog of wls), LOC124635354 (ortholog of twy), LOC124635262 (ortholog of twy), LOC124640888 (ortholog of Syx13), LOC124646040 (ortholog of CG5048), LOC124644041 (ortholog of ATPsynbetaL), LOC124640035 (ortholog of Wdr92), LOC124644979 (ortholog of wnd), LOC124644813 (ortholog of Pex16), LOC124636485 (ortholog of eIF2A), LOC124630185 (ortholog of aux), LOC124636077 (ortholog of sac), LOC124636174 (ortholog of Sym), LOC124636074 (ortholog of ScsbetaA), LOC124631653 (ortholog of topi), LOC124637195 (ortholog of Rsbp15), LOC124635131 (ortholog of CG9920), LOC124641857 (ortholog of HtrA2), LOC124641539 (ortholog of Kpc1), LOC124640963 (ortholog ofAtg8b), LOC124640220 (ortholog of CG14305), LOC124634523 (ortholog of Syp), LOC124636841 (ortholog of Gld2), LOC124634606 (ortholog of bb8), LOC124634284 (ortholog of Root), LOC124640007 (ortholog of puf), LOC124641939 (ortholog of mfrn), LOC124635189 (ortholog of Cyt-c1L), LOC124634227 (ortholog of Cad99C), LOC124630088 (ortholog of klhl10), LOC124634869 (ortholog of klhl10), LOC124644646 (ortholog of rept), LOC124630436 (ortholog of pont), LOC124637455 (ortholog of DCTN5-p25), LOC124633344 (ortholog of Top3alpha), LOC124642241 (ortholog of CG18675), LOC124644941 (ortholog of ago), LOC124640595 (ortholog of HBS1), LOC124645695 (ortholog of Fsn), LOC124634050 (ortholog of S-Lap3), LOC124636251 (ortholog of gskt), LOC124635242 (ortholog of Trel), LOC124633845 (ortholog of Liprin-alpha), LOC124644070 (ortholog of Cog7), LOC124646224 (ortholog of CG31759), LOC124629956 (ortholog of Rsph9), LOC124638174 (ortholog of Mst27D), LOC124634050 (ortholog of S-Lap4), LOC124644794 (ortholog of S-Lap2), LOC124630681 (ortholog of Rsph3), LOC124641631 (ortholog of Erk7), LOC124635263 (ortholog of Gorab), LOC124645902 (ortholog of endos), LOC124643226 (ortholog of Lasp), LOC124630403 (ortholog of sif), LOC124637884 (ortholog of Myo31DF), LOC124639844 (ortholog of Mer), LOC124630876 (ortholog of pea), LOC124638477 (ortholog of snky), LOC124639587 (ortholog of psidin), LOC124636930 (ortholog of gish), LOC124631282 (ortholog of Mob4), LOC124634050 (ortholog of loopin-1), LOC124644037 (ortholog of Nop60B), LOC124633665 (ortholog of Su(var)3-3), LOC124644028 (ortholog of eIF4G2), LOC124645493 (ortholog of eIF4G2), LOC124633799 (ortholog of Diap1), LOC124630702 (ortholog of bond), LOC124630584 (ortholog of bond), LOC124630484 (ortholog of bond), LOC124630567 (ortholog of bond), LOC124644306 (ortholog of Vmat), LOC124633658 (ortholog of Pif1A), LOC124638652 (ortholog of Hr39), LOC124645604 (ortholog of Cul3), LOC124643336 (ortholog of didum), LOC124632218 (ortholog of nej), LOC124644418 (ortholog of larp), LOC124638967 (ortholog of brun), LOC124638034 (ortholog of Bsg), LOC124638986 (ortholog of f), LOC124630326 (ortholog of VhaPPA1-2), LOC124640621 (ortholog of cic), LOC124638965 (ortholog of mtsh), LOC124645653 (ortholog of SmB), LOC124635434 (ortholog of Myc), LOC124638510 (ortholog of bel), LOC124644004 (ortholog of hts), LOC124636905 (ortholog of nclb), LOC124641850 (ortholog of lost), LOC124644065 (ortholog of Brr2), LOC124633774 (ortholog of Dark), LOC124642434 (ortholog of dally), LOC124631402 (ortholog of Dic61B), LOC124631321 (ortholog of Dic61B), LOC124631483 (ortholog of Dic61B), LOC124633232 (ortholog of Cyt-b5), LOC124639390 (ortholog of Cyt-b5), LOC124634079 (ortholog of Cyt-b5), LOC124633752 (ortholog of mgr), LOC124637414 (ortholog of Mhc), LOC124636530 (ortholog of mlt), LOC124641588 (ortholog of Prosalpha4T1), LOC124646229 (ortholog of Prosalpha4T1), LOC124639015 (ortholog of Tfb5), LOC124634356 (ortholog of Doa), LOC124645943 (ortholog of stai), LOC124641320 (ortholog of Zmynd10), LOC124645318 (ortholog of mfr), LOC124634244 (ortholog of Sf3a1), LOC124646347 (ortholog of Ntl), LOC124634251 (ortholog of Topors), LOC124638975 (ortholog of sau), LOC124631768 (ortholog of lovit), LOC124642697 (ortholog of kl-3), LOC124646356 (ortholog of kl-3), LOC124640114 (ortholog of kl-5), LOC124629982 (ortholog of WDY), LOC124629983 (ortholog of WDY), LOC124629706 (ortholog of WDY), LOC124629986 (ortholog of WDY), LOC124645089 (ortholog of ap), LOC124645205 (ortholog of Dhc16F), LOC124634694 (ortholog of InR), LOC124632347 (ortholog of Pall), LOC124631622 (ortholog of Rpp30), LOC124635404 (ortholog of Top2), LOC124640411 (ortholog of knon), LOC124635308 (ortholog of knon), LOC124646164 (ortholog of shps), LOC124638021 (ortholog of TER94), LOC124638170 (ortholog of Orc1), LOC124644841 (ortholog of bor), LOC124643808 (ortholog of Pen), LOC124633479 (ortholog of zip), and LOC124641309 (ortholog of betaTub60D).

Non-limiting examples of orthologs of *Drosophila melanogaster* male-fertility essential genes in *Helicoverpa armigera* include the following GenBank^{®} Accession Nos: LOC110380081 (ortholog of Abd-B), LOC110371838 (ortholog of ac), LOC110371815 (ortholog of ac), LOC110375128 (ortholog of nAChRbeta1), LOC110378974 (ortholog of Pfas), LOC110377306 (ortholog of aop), LOC110373336 (ortholog of bru1), LOC110371349 (ortholog of asp), LOC110371117 (ortholog of ca), LOC110374566 (ortholog of ca), LOC110373096 (ortholog of ca), LOC110371924 (ortholog of chic), LOC110375631 (ortholog of Chc), LOC110379936 (ortholog of eya), LOC110380319 (ortholog of crm), LOC110374094 (ortholog of CycB), LOC110370640 (ortholog of Dab), LOC110372093 (ortholog of dor), LOC110380878 (ortholog of dx), LOC110369974 (ortholog of EcR), LOC110370093 (ortholog of emc), LOC110383198 (ortholog of exu), LOC110384470 (ortholog of Fas2), LOC110371906 (ortholog of tj), LOC110374245 (ortholog of g), LOC110376415 (ortholog of H), LOC110379631 (ortholog of hay), LOC110380968 (ortholog of Hsp83), LOC110381280 (ortholog of kl-2), LOC110383762 (ortholog of Grip91), LOC110378518 (ortholog of ifc), LOC110378066 (ortholog of CIAPIN1), LOC110378011 (ortholog of stc), LOC110378537 (ortholog of ND-B17), LOC110371093 (ortholog of Phb1), LOC126057066 (ortholog of Phb1), LOC110377893 (ortholog of l(2)gl), LOC110384044 (ortholog of hyd), LOC110370843 (ortholog of sti), LOC110377751 (ortholog of Lam), LOC110381935 (ortholog of lds), LOC110373313 (ortholog of m), LOC110384293 (ortholog of RpL36), LOC110373935 (ortholog of RpS3), LOC110374298 (ortholog of mal), LOC110380949 (ortholog of twe), LOC126054883 (ortholog of mago), LOC110370757 (ortholog of mago), LOC110373560 (ortholog of mle), LOC110376818 (ortholog of msl-3), LOC110383260 (ortholog of mod), LOC126055445 (ortholog of mod), LOC126055354 (ortholog of mod), LOC110374782 (ortholog of mod), LOC110383930 (ortholog of PolQ), LOC110369697 (ortholog of pbl), LOC110381007 (ortholog of Poxn), LOC110375127 (ortholog of Polr2A), LOC110377683 (ortholog of rst), LOC110377681 (ortholog of rst), LOC126056874 (ortholog of rst), LOC110380858 (ortholog of rst), LOC110373085 (ortholog of rt), LOC110378107 (ortholog of run), LOC110376462 (ortholog of sbr), LOC110371780 (ortholog of sd), LOC110371103 (ortholog of shu), LOC126057062 (ortholog of shu), LOC126056879 (ortholog of sm), LOC110377678 (ortholog of sm), LOC110379097 (ortholog of spn-E), LOC110377297 (ortholog of Btk), LOC110374023 (ortholog of ss), LOC110380949 (ortholog of stg), LOC110372833 (ortholog of sub), LOC110375873 (ortholog of su(sable)), LOC110379267 (ortholog of alphaTub84B), LOC110377963 (ortholog of tud), LOC110372432 (ortholog of RpS27A), LOC110383601 (ortholog of v), LOC110371853 (ortholog of y), LOC110371827 (ortholog of sc), LOC110371746 (ortholog of ct), LOC110374930 (ortholog of Rdl), LOC110378226 (ortholog of Rdl), LOC110378222 (ortholog of Rdl), LOC110382331 (ortholog of Wnt2), LOC110375794 (ortholog of porin), LOC110371765 (ortholog of aly), LOC110378156 (ortholog of fwd), LOC110370469 (ortholog of Klp67A), LOC110382301 (ortholog of bgcn), LOC110381538 (ortholog of pros), LOC110381213 (ortholog of Zw10), LOC110381776 (ortholog of Zw10), LOC126053598 (ortholog of Zw10), LOC110380888 (ortholog of fru), LOC110382506 (ortholog of sim), LOC110376354 (ortholog of hop), LOC110373228 (ortholog of nmd), LOC110373172 (ortholog of GstS1), LOC110373169 (ortholog of GstS1), LOC110373170 (ortholog of GstS1), LOC110373190 (ortholog of GstS1), LOC110373211 (ortholog of GstS1), LOC110371595 (ortholog of Trf), LOC110373859 (ortholog of Cdc42), LOC110374542 (ortholog of Cds), LOC110384142 (ortholog of Taf6), LOC110370784 (ortholog of Sas-4), LOC110378508 (ortholog of dia), LOC126057007 (ortholog of dia), LOC110380537 (ortholog of fbl), LOC110372535 (ortholog of pelo), LOC110378315 (ortholog of blw), LOC110378160 (ortholog of eff), LOC110384162 (ortholog of heph), LOC110378005 (ortholog of jar), LOC110375622 (ortholog of ox), LOC110379157 (ortholog of poe), LOC110371585 (ortholog of scat), LOC110379863 (ortholog of Hsp60B), LOC110372680 (ortholog of fzo), LOC110372291 (ortholog of Iswi), LOC110377452 (ortholog of Klp3A), LOC110371593 (ortholog of Syx5), LOC110381486 (ortholog of tsr), LOC110375348 (ortholog of wts), LOC110374328 (ortholog of Ance), LOC110374326 (ortholog of Ance), LOC110379821 (ortholog of l(2)dtl), LOC110372890 (ortholog of Arf6), LOC110370952 (ortholog of Rnp4F), LOC110378840 (ortholog of noi), LOC110376946 (ortholog of tilB), LOC110370811 (ortholog of Ddx1), LOC110383693 (ortholog of Rala), LOC110383811 (ortholog of Shc), LOC110376217 (ortholog of spag), LOC110372789 (ortholog of Lis-1), LOC110378127 (ortholog of ara), LOC110378127 (ortholog of caup), LOC110383841 (ortholog of csul), LOC110372150 (ortholog of mael), LOC110379381 (ortholog of fry), LOC110381656 (ortholog of Stat92E), LOC110381245 (ortholog of Stat92E), LOC110383989 (ortholog of qm), LOC110370382 (ortholog of Smg5), LOC126055685 (ortholog of Smg5), LOC110382102 (ortholog of ATPsyngamma), LOC110376738 (ortholog of stet), LOC110372579 (ortholog of Ran), LOC110370086 (ortholog of Pdk1), LOC110371325 (ortholog of asun), LOC110375738 (ortholog of eRF3), LOC110376117 (ortholog of eRF3), LOC110377048 (ortholog of Pkn), LOC110370769 (ortholog of Osbp), LOC110369626 (ortholog of chb), LOC110370024 (ortholog of Nup154), LOC110376673 (ortholog of Vps28), LOC110372783 (ortholog of Nle), LOC110372022 (ortholog of Hel89B), LOC110376050 (ortholog of dtr), LOC110380620 (ortholog of Mnt), LOC110371420 (ortholog of sns), LOC110376585 (ortholog of WASp), LOC110373230 (ortholog of Npcla), LOC110379890 (ortholog of Dlc90F), LOC110370277 (ortholog of Bin1), LOC110378864 (ortholog of fws), LOC110375226 (ortholog of Kap-alphal), LOC110377854 (ortholog of Cpes), LOC110374446 (ortholog of Torsin), LOC110381301 (ortholog of Rtca), LOC110376708 (ortholog of Inos), LOC110378248 (ortholog of mei-P26), LOC110369932 (ortholog of SAK), LOC110379504 (ortholog of Dronc), LOC110377331 (ortholog of Grip84), LOC110370632 (ortholog of Grip75), LOC110377908 (ortholog of Taz), LOC110380329 (ortholog of Pop1), LOC110372717 (ortholog of CSN5), LOC110373571 (ortholog of mino), LOC110376726 (ortholog of ball), LOC110372546 (ortholog of Ack), LOC110375088 (ortholog of Fmrl), LOC110371907 (ortholog of slmo), LOC110374037 (ortholog of Gas8), LOC110374232 (ortholog of wuho), LOC110373082 (ortholog of Upf2), LOC110376073 (ortholog of p24-1), LOC110380013 (ortholog of p24-1), LOC110378085 (ortholog of ATP7), LOC110380305 (ortholog of shop), LOC126053602 (ortholog of shop), LOC126053603 (ortholog of shop), LOC110379794 (ortholog of Naa20A), LOC110373130 (ortholog of shv), LOC110378695 (ortholog of Pex12), LOC110380535 (ortholog of papi), LOC110379863 (ortholog of Hsp60C), LOC110381433 (ortholog of rau), LOC110371606 (ortholog of Kdm5), LOC126057056 (ortholog of TTLL3B), LOC110371099 (ortholog of TTLL3B), LOC110383065 (ortholog of Rab30), LOC110369705 (ortholog of gudu), LOC110382180 (ortholog of PAPLA1), LOC110369884 (ortholog of Usp14), LOC110381234 (ortholog of Bug22), LOC110380002 (ortholog of Yipldl), LOC110384374 (ortholog of Rsph1), LOC110380831 (ortholog of Prosalpha6T), LOC110371151 (ortholog of ssp3), LOC110374944 (ortholog of COX4L), LOC110379735 (ortholog of Pngl), LOC110384116 (ortholog of U2A), LOC110371060 (ortholog of rho-7), LOC110383687 (ortholog of Prp8), LOC110375808 (ortholog of mip120), LOC110377888 (ortholog of S-Lap5), LOC110377888 (ortholog of S-Lap7), LOC110372330 (ortholog of eIF3m), LOC110382302 (ortholog of Hr51), LOC110375891 (ortholog of clu), LOC110377888 (ortholog of S-Lap8), LOC110374868 (ortholog of Dnaaf3), LOC110383678 (ortholog of mip40), LOC110384682 (ortholog of Mgat1), LOC110370550 (ortholog of CG9313), LOC110382886 (ortholog of Rael), LOC110379479 (ortholog of nsr), LOC110375117 (ortholog of tous), LOC110376179 (ortholog of YME1L), LOC110372779 (ortholog of thoc5), LOC110382152 (ortholog of Rsph4a), LOC110378574 (ortholog of MAN1), LOC110370058 (ortholog of Dis3l2), LOC110376900 (ortholog of ttm2), LOC110381751 (ortholog of Pex10), LOC110374172 (ortholog of Strip), LOC110378209 (ortholog of Dnah3), LOC110375225 (ortholog of Pex2), LOC110374531 (ortholog of S-Lap1), LOC110376601 (ortholog of wls), LOC110378364 (ortholog of twy), LOC110373732 (ortholog of Syx13), LOC110371901 (ortholog of CG5048), LOC110382557 (ortholog of ATPsynbetaL), LOC110381250 (ortholog of Wdr92), LOC110377574 (ortholog of wnd), LOC110377586 (ortholog of Pex16), LOC110371541 (ortholog of eIF2A), LOC110380564 (ortholog of aux), LOC110373514 (ortholog of sac), LOC110372114 (ortholog of Sym), LOC126053505 (ortholog of Sym), LOC110374628 (ortholog of ScsbetaA), LOC110370204 (ortholog of CG9920), LOC126056338 (ortholog of HtrA2), LOC110379176 (ortholog of HtrA2), LOC110383137 (ortholog of Kpc1), LOC110377214 (ortholog of Atg8b), LOC110373500 (ortholog of CG14305), LOC110375328 (ortholog of Syp), LOC110381971 (ortholog of Gld2), LOC110375085 (ortholog of bb8), LOC110375327 (ortholog of Root), LOC110382932 (ortholog of puf), LOC110380785 (ortholog of mfrn), LOC110377964 (ortholog of Cyt-c1L), LOC110381684 (ortholog of Cad99C), LOC110377969 (ortholog of klhl10), LOC110384382 (ortholog of klhl10), LOC110378635 (ortholog of rept), LOC110384069 (ortholog of pont), LOC110372222 (ortholog of DCTN5-p25), LOC110375433 (ortholog of Top3alpha), LOC110380379 (ortholog of CG18675), LOC110378181 (ortholog of ago), LOC110369593 (ortholog of HBS1), LOC126054877 (ortholog of HBS1), LOC110380367 (ortholog of Fsn), LOC110377888 (ortholog of S-Lap3), LOC110384573 (ortholog of gskt), LOC110375577 (ortholog of Tre1), LOC110372090 (ortholog of Liprin-alpha), LOC110380117 (ortholog of Cog7), LOC110375442 (ortholog of CG31759), LOC110381908 (ortholog of Rsph9), LOC110376349 (ortholog of Mst27D), LOC110377888 (ortholog of S-Lap4), LOC110374531 (ortholog of S-Lap2), LOC110377956 (ortholog of Rsph3), LOC110383071 (ortholog of Erk7), LOC110372008 (ortholog of Gorab), LOC110381829 (ortholog of endos), LOC126053587 (ortholog of endos), LOC110384640 (ortholog of Lasp), LOC110384088 (ortholog of sif), LOC110380184 (ortholog of Myo31DF), LOC110382678 (ortholog of Mer), LOC110372719 (ortholog of pea), LOC110369891 (ortholog of snky), LOC110380459 (ortholog of psidin), LOC126053434 (ortholog of gish), LOC110373847 (ortholog of gish), LOC110375696 (ortholog of Mob4), LOC110377888 (ortholog of loopin-1), LOC110382503 (ortholog of Nop60B), LOC110370308 (ortholog of Su(var)3-3), LOC110374231 (ortholog of eIF4G2), LOC110380545 (ortholog of eIF4G2), LOC110380098 (ortholog of Diap1), LOC110370496 (ortholog of bond), LOC110370512 (ortholog of bond), LOC110370488 (ortholog of bond), LOC110370479 (ortholog of bond), LOC110380111 (ortholog of Vmat), LOC110382218 (ortholog of Pif1A), LOC110379055 (ortholog of Hr39), LOC110371915 (ortholog of Cul3), LOC110378619 (ortholog of larp), LOC110375370 (ortholog of brun), LOC110377906 (ortholog of Bsg), LOC110380086 (ortholog of f), LOC110374983 (ortholog of VhaPPA1-2), LOC110379888 (ortholog of cic), LOC110373242 (ortholog of mtsh), LOC110376929 (ortholog of SmB), LOC110373683 (ortholog of Myc), LOC110378270 (ortholog of bel), LOC110382694 (ortholog of hts), LOC110369873 (ortholog of nclb), LOC110377124 (ortholog of lost), LOC126056213 (ortholog of Brr2), LOC110382358 (ortholog of Brr2), LOC110370241 (ortholog of Dark), LOC110383742 (ortholog of dally), LOC126054881 (ortholog of dally), LOC110369737 (ortholog of Dic61B), LOC110369736 (ortholog of Dic61B), LOC110369687 (ortholog of Dic61B), LOC110383100 (ortholog of Cyt-b5), LOC110380638 (ortholog of Cyt-b5), LOC110380637 (ortholog of Cyt-b5), LOC110379227 (ortholog of Mhc), LOC110384602 (ortholog of mlt), LOC110381035 (ortholog of Prosalpha4T1), LOC110373136 (ortholog of Prosalpha4T1), LOC110373225 (ortholog of Tfb5), LOC110377189 (ortholog of Doa), LOC110381192 (ortholog of stai), LOC110382061 (ortholog of Zmynd10), LOC110373002 (ortholog of mfr), LOC110380034 (ortholog of Sf3a1), LOC126057052 (ortholog of Ntl), LOC110371074 (ortholog of Ntl), LOC110378539 (ortholog of Topors), LOC110375115 (ortholog of sau), LOC110383940 (ortholog of lovit), LOC110372416 (ortholog of kl-3), LOC110377788 (ortholog of kl-3), LOC110378604 (ortholog of kl-5), LOC110376887 (ortholog of WDY), LOC110376879 (ortholog of WDY), LOC110376878 (ortholog of WDY), LOC110376876 (ortholog of WDY), LOC110373008 (ortholog of ap), LOC110374235 (ortholog of Dhc 16F), LOC110377777 (ortholog of InR), LOC110372930 (ortholog of Pall), LOC110383924 (ortholog of Rpp30), LOC110375542 (ortholog of Top2), LOC126056756 (ortholog of Top2), LOC110375576 (ortholog of knon), LOC110371916 (ortholog of shps), LOC110371209 (ortholog of TER94), LOC110383683 (ortholog of Orc1), LOC110374221 (ortholog of bor), LOC110382454 (ortholog of Pen), LOC110377858 (ortholog of zip), and LOC.110379509 (ortholog of betaTub60D).

Non-limiting examples of orthologs of *Drosophila melanogaster* male-fertility essential genes in *Diabrotica virgifera* include the following GenBank^{®} Accession Nos: LOC114346330 (ortholog of Abd-B), LOC114325414 (ortholog of nAChRbetal), LOC126885248 (ortholog of Pfas), LOC114338337 (ortholog of Pfas), LOC114349517 (ortholog of aop), LOC114330181 (ortholog of bru1), LOC114326913 (ortholog of asp), LOC114344326 (ortholog of aub), LOC114326602 (ortholog of Chc), LOC114343134 (ortholog of eya), LOC114324835 (ortholog of crm), LOC114336051 (ortholog of CycB), LOC114349376 (ortholog of Dab), LOC126888797 (ortholog of dor), LOC114326126 (ortholog of dwg), LOC114338956 (ortholog of dx), LOC114324627 (ortholog of EcR), LOC114339537 (ortholog of emc), LOC126889805 (ortholog of exu), LOC114325164 (ortholog of Fas2), LOC114336033 (ortholog of tj), LOC114339188 (ortholog of g), LOC114343548 (ortholog of H), LOC114333700 (ortholog of hay), LOC114332956 (ortholog of Hsp83), LOC114327420 (ortholog of kl-2), LOC114336524 (ortholog of Grip91), LOC126880537 (ortholog of ifc), LOC114336071 (ortholog of CIAPIN1), LOC114334239 (ortholog of stc), LOC126883390 (ortholog ofND-B17), LOC114330199 (ortholog of Phb1), LOC114326415 (ortholog of l(2)gl), LOC114325347 (ortholog of hyd), LOC114332329 (ortholog of sti), LOC114342154 (ortholog of Lam), LOC126887350 (ortholog of lds), LOC126890278 (ortholog of lds), LOC114326376 (ortholog of lz), LOC126884417 (ortholog of m), LOC126880543 (ortholog of RpL36), LOC114339445 (ortholog of RpS3), LOC126883294 (ortholog of mal), LOC114330796 (ortholog of twe), LOC114330132 (ortholog of mago), LOC114344426 (ortholog of mle), LOC126884810 (ortholog of msl-3), LOC126887281 (ortholog of PolQ), LOC126880490 (ortholog of pbl), LOC126891443 (ortholog of Poxn), LOC114328271 (ortholog of prd), LOC114349455 (ortholog of Polr2A), LOC126892555 (ortholog of rst), LOC126888268 (ortholog of run), LOC114324824 (ortholog of sbr), LOC114328891 (ortholog of sd), LOC114330436 (ortholog of shu), LOC126890260 (ortholog of sm), LOC114333797 (ortholog of Sod1), LOC126880408 (ortholog of spn-E), LOC114327644 (ortholog of Btk), LOC126889413 (ortholog of ss), LOC126881579 (ortholog of stau), LOC114331798 (ortholog of sub), LOC126881112 (ortholog of su(sable)), LOC114332778 (ortholog of alphaTub84B), LOC114328275 (ortholog of tud), LOC114327318 (ortholog of RpS27A), LOC114335155 (ortholog of Ubi-p63E), LOC114328579 (ortholog of v), LOC126889912 (ortholog of y), LOC114330205 (ortholog of sc), LOC114332157 (ortholog of ct), LOC114334919 (ortholog of Rdl), LOC114325448 (ortholog of Wnt2), LOC114338616 (ortholog of porin), LOC114335571 (ortholog of fwd), LOC114339816 (ortholog of neb), LOC126884603 (ortholog of Klp67A), LOC114333004 (ortholog of pros), LOC126886939 (ortholog of Zw10), LOC114331254 (ortholog of fru), LOC114335011 (ortholog of sim), LOC114344367 (ortholog of hop), LOC126888811 (ortholog of nmd), LOC126881453 (ortholog of GstS1), LOC126881452 (ortholog of GstS1), LOC126881141 (ortholog of Trf), LOC126883167 (ortholog of Cdc42), LOC114337361 (ortholog of Cds), LOC114329055 (ortholog of Taf6), LOC114335788 (ortholog of Sas-4), LOC114339212 (ortholog of dia), LOC114325539 (ortholog of fbl), LOC126883978 (ortholog of bol), LOC114334968 (ortholog of pelo), LOC126884728 (ortholog of blw), LOC114334385 (ortholog of eff), LOC114326010 (ortholog of heph), LOC114331957 (ortholog of jar), LOC114328390 (ortholog of poe), LOC126880181 (ortholog of scat), LOC114325883 (ortholog of Iswi), LOC114342007 (ortholog of Iswi), LOC114332959 (ortholog of Klp3A), LOC114337347 (ortholog of Syx5), LOC126881920 (ortholog of tsr), LOC114347323 (ortholog of ctp), LOC114330466 (ortholog of Ance), LOC126893467 (ortholog of Ance), LOC114326567 (ortholog of l(2)dtl), LOC114344883 (ortholog of Arf6), LOC114338775 (ortholog of cnn), LOC114330877 (ortholog of Rnp4F), LOC126880584 (ortholog of noi), LOC114331286 (ortholog of tilB), LOC114326867 (ortholog of Ddx1), LOC114334820 (ortholog ofRala), LOC114331293 (ortholog of Shc), LOC114327934 (ortholog of spag), LOC126882408 (ortholog of Lis-1), LOC114328337 (ortholog of ara), LOC126881669 (ortholog of csul), LOC114337107 (ortholog of mael), LOC114324528 (ortholog of fry), LOC126884667 (ortholog of fz2), LOC114330294 (ortholog of Stat92E), LOC114330295 (ortholog of Stat92E), LOC114325237 (ortholog of qm), LOC114331928 (ortholog of Smg5), LOC126887208 (ortholog of ATPsyngamma), LOC114334179 (ortholog of stet), LOC126882357 (ortholog of Ran), LOC114325167 (ortholog of Pdk1), LOC114331758 (ortholog of asun), LOC114339790 (ortholog of eRF3), LOC126890378 (ortholog of Pkn), LOC114330832 (ortholog of Osbp), LOC114327638 (ortholog of Trxr-1), LOC114333789 (ortholog of chb), LOC114325659 (ortholog of Nup154), LOC114333792 (ortholog of Vps28), LOC114338498 (ortholog of Nle), LOC114331805 (ortholog of Hel89B), LOC114324515 (ortholog of dtr), LOC126888352 (ortholog of Mnt), LOC114340732 (ortholog of sns), LOC114328011 (ortholog of Npcla), LOC126886752 (ortholog of Npcla), LOC126881113 (ortholog of Npcla), LOC114329911 (ortholog of Dlc90F), LOC114329814 (ortholog of Bin1), LOC114338113 (ortholog of fws), LOC126887407 (ortholog of Kap-alphal), LOC126891936 (ortholog of Cpes), LOC114342875 (ortholog of Torsin), LOC126892876 (ortholog of Torsin), LOC114326340 (ortholog of Rtca), LOC114329653 (ortholog of Inos), LOC114327693 (ortholog of mei-P26), LOC114333398 (ortholog of SAK), LOC114329481 (ortholog of Dronc), LOC126881274 (ortholog of Grip84), LOC114340477 (ortholog of Grip75), LOC114328722 (ortholog of Grip128), LOC114338867 (ortholog of Taz), LOC126879938 (ortholog of Pop1), LOC126879528 (ortholog of CSN5), LOC114336016 (ortholog of mino), LOC114330568 (ortholog of ball), LOC126879425 (ortholog of ball), LOC126882893 (ortholog of Ack), LOC114326323 (ortholog of hfp), LOC126884364 (ortholog of Fmrl), LOC126882112 (ortholog of slmo), LOC126881222 (ortholog of Gas8), LOC126891935 (ortholog of wuho), LOC114340244 (ortholog of Pink1), LOC126890588 (ortholog of Upf2), LOC114328265 (ortholog of ATP7), LOC126884299 (ortholog of shop), LOC126884248 (ortholog of Naa20A), LOC114325965 (ortholog of shv), LOC126888432 (ortholog of Pex12), LOC114336404 (ortholog of Hsp60C), LOC114336859 (ortholog of rau), LOC126882917 (ortholog of Kdm5), LOC114331302 (ortholog of TTLL3B), LOC114331280 (ortholog of Rab30), LOC114324401 (ortholog of gudu), LOC114325118 (ortholog of PAPLA1), LOC114327784 (ortholog of Usp14), LOC114338895 (ortholog of Bug22), LOC114327612 (ortholog of Yipldl), LOC114334469 (ortholog of Rsph1), LOC114326715 (ortholog of Prosalpha6T), LOC114339332 (ortholog of ssp3), LOC114336136 (ortholog of COX4L), LOC114332183 (ortholog of COX4L), LOC114324236 (ortholog of COX4L), LOC114333829 (ortholog of Pngl), LOC126879982 (ortholog of U2A), LOC126892041 (ortholog of rho-7), LOC126889947 (ortholog of Prp8), LOC114338617 (ortholog of mip120), LOC114325906 (ortholog of eIF3m), LOC126880400 (ortholog of Hr51), LOC114349370 (ortholog of clu), LOC114325926 (ortholog of Dnaaf3), LOC114339250 (ortholog of mip40), LOC114324411 (ortholog of Mgat1), LOC114328191 (ortholog of CG9313), LOC114330379 (ortholog of Rael), LOC114324653 (ortholog of nsr), LOC126881566 (ortholog of tous), LOC126886914 (ortholog of YMEI1L), LOC126881925 (ortholog of thoc5), LOC114331561 (ortholog of Rsph4a), LOC114329627 (ortholog of Dis312), LOC114342279 (ortholog of ttm2), LOC126886628 (ortholog of ttm2), LOC126880648 (ortholog of Cep290), LOC126880169 (ortholog of Pex10), LOC114331645 (ortholog of Strip), LOC114328060 (ortholog of Dnah3), LOC114326464 (ortholog of Pex2), LOC114332621 (ortholog of wls), LOC114332620 (ortholog of wls), LOC126893218 (ortholog of Duba), LOC114329857 (ortholog of twy), LOC114331333 (ortholog of Syx13), LOC114324458 (ortholog of CG5048), LOC126880493 (ortholog of Wdr92), LOC114341196 (ortholog of wnd), LOC126884645 (ortholog of Pex16), LOC126892040 (ortholog of eIF2A), LOC126880070 (ortholog of aux), LOC114334288 (ortholog of sac), LOC126890959 (ortholog of sac), LOC114339168 (ortholog of sac), LOC114339583 (ortholog of sac), LOC126890957 (ortholog of sac), LOC114334539 (ortholog of Sym), LOC114326202 (ortholog of ScsbetaA), LOC114331418 (ortholog of ScsbetaA), LOC126888881 (ortholog of CG9920), LOC114341488 (ortholog of HtrA2), LOC126887345 (ortholog of CG14305), LOC114331372 (ortholog of Syp), LOC114325107 (ortholog of Gld2), LOC114326601 (ortholog of wam), LOC114347881 (ortholog of Root), LOC114328571 (ortholog of mfrn), LOC114334517 (ortholog of Cyt-c1L), LOC114324252 (ortholog of Cyt-c1L), LOC114332608 (ortholog of Cad99C), LOC114333034 (ortholog of klhl10), LOC114336343 (ortholog of rept), LOC114331368 (ortholog of pont), LOC126884476 (ortholog of DCTN5-p25), LOC114329427 (ortholog of Top3alpha), LOC114336425 (ortholog of Mytl), LOC126890982 (ortholog of Mytl), LOC126893024 (ortholog of Myt1), LOC126878500 (ortholog of Myt1), LOC126893030 (ortholog of Myt1), LOC114336531 (ortholog of Mytl), LOC126893019 (ortholog of Mytl), LOC114338100 (ortholog of Mytl), LOC126892575 (ortholog of Mytl), LOC114345225 (ortholog of Mytl), LOC126891846 (ortholog of CG18675), LOC126883169 (ortholog of park), LOC114349421 (ortholog of ago), LOC114333903 (ortholog of Ap1), LOC114329009 (ortholog of HBS1), LOC114332273 (ortholog of Fsn), LOC114336102 (ortholog of Trel), LOC114348805 (ortholog of Liprin-alpha), LOC114325547 (ortholog of Cog7), LOC114332396 (ortholog of Rsph9), LOC114328038 (ortholog of SCaMC), LOC126891717 (ortholog of Rsph3), LOC114349439 (ortholog of Erk7), LOC114332622 (ortholog of endos), LOC114332079 (ortholog of Lasp), LOC114326465 (ortholog of sif), LOC126879050 (ortholog of Myo31DF), LOC126880341 (ortholog of pea), LOC114325576 (ortholog of Cyt-c-d), LOC114325038 (ortholog of Cyt-c-d), LOC114331855 (ortholog of snky), LOC114333085 (ortholog of psidin), LOC114331294 (ortholog of Chd1), LOC114328683 (ortholog of gish), LOC114327639 (ortholog of Mob4), LOC114325393 (ortholog of Nop60B), LOC114331553 (ortholog of Su(var)3-3), LOC114326358 (ortholog of eIF4G2), LOC114327514 (ortholog of scny), LOC114327694 (ortholog of scny), LOC126881678 (ortholog of bond), LOC126879248 (ortholog of Vmat), LOC114335067 (ortholog of Hr39), LOC114331163 (ortholog of Cul3), LOC126890058 (ortholog of didum), LOC114342483 (ortholog of nej), LOC114340580 (ortholog of larp), LOC126887348 (ortholog of brun), LOC126883273 (ortholog of Bsg), LOC114328087 (ortholog of f), LOC114325264 (ortholog of VhaPPA1-2), LOC114335679 (ortholog of cic), LOC114328165 (ortholog of mtsh), LOC114331443 (ortholog of Myc), LOC114325955 (ortholog of Acn), LOC126890515 (ortholog of bel), LOC114328231 (ortholog of hts), LOC126883123 (ortholog of nclb), LOC114328905 (ortholog of Brr2), LOC126880520 (ortholog of Dark), LOC114340589 (ortholog of dally), LOC114335779 (ortholog of Dic61B), LOC114327319 (ortholog of Cyt-b5), LOC114324394 (ortholog of ab), LOC114328671 (ortholog of mgr), LOC114326036 (ortholog of Mhc), LOC114333289 (ortholog of pAbp), LOC114342260 (ortholog of pAbp), LOC114342350 (ortholog of pAbp), LOC114329703 (ortholog of mlt), LOC114334236 (ortholog of Prosalpha4T1), LOC114336359 (ortholog of Tfb5), LOC114347795 (ortholog of Doa), LOC114329725 (ortholog of Doa), LOC114326401 (ortholog of Zmynd10), LOC114337495 (ortholog of mfr), LOC126884388 (ortholog of Sf3a1), LOC114329471 (ortholog of Topors), LOC126893205 (ortholog of sau), LOC126889868 (ortholog of kl-3), LOC114326404 (ortholog of kl-5), LOC114332158 (ortholog of vib), LOC114330350 (ortholog of ap), LOC114334231 (ortholog of Dhc16F), LOC114341705 (ortholog of InR), LOC126890043 (ortholog of Rpp30), LOC126891975 (ortholog of Top2), LOC114340847 (ortholog of knon), LOC114331583 (ortholog of TER94), LOC114326804 (ortholog of Orc1), LOC114333089 (ortholog of SRPK), LOC114324425 (ortholog of bor), LOC114339215 (ortholog of dbf), LOC114339442 (ortholog of Pen), LOC114332917 (ortholog of zip), and LOC114335686 (ortholog of betaTub60D).

Non-limiting examples of orthologs of *Drosophila melanogaster* male-fertility essential genes in *Culex quinquefasciatus* include the following GenBank^{®} Accession Nos: CQUJHB013831 (ortholog of Abd-B), CQUJHB005281 (ortholog of ac), CQUJHB001670 (ortholog of ac), CQUJHB011937 (ortholog of nAChRbetal), CQUJHB002675 (ortholog of Pfas), CQUJHB014704 (ortholog of aop), CQUJHB009771 (ortholog of bru1), CQUJHB009839 (ortholog of aub), CQUJHB002833 (ortholog of aub), CQUJHB013970 (ortholog of aub), CQUJHB008282 (ortholog of aub), CQUJHB007955 (ortholog of aub), CQUJHB006760 (ortholog of aub), CQUJHB003509 (ortholog of aub), CQUJHB012292 (ortholog of ca), CQUJHB015738 (ortholog of ca), CQUJHB001871 (ortholog of ca), CQUJHB012914 (ortholog of chic), CQUJHB014452 (ortholog of crm), CQUJHB014680 (ortholog of CycB), CQUJHB018297 (ortholog of Dab), CQUJHB008065 (ortholog of dor), CQUJHB016486 (ortholog of dx), CQUJHB012806 (ortholog of EcR), CQUJHB008114 (ortholog of emc), CQUJHB005654 (ortholog of exu), CQUJHB009253 (ortholog of Fas2), CQUJHB018420 (ortholog of Fas3), CQUJUB017622 (ortholog of tj), CQUJHB004296 (ortholog of g), CQUJHB011818 (ortholog of H), CQUJHB017285 (ortholog of hay), CQUJHB006242 (ortholog of kl-2), CQUJHB009120 (ortholog of Grip91), CQUJHB009167 (ortholog of ifc), CQUJHB007458 (ortholog of CIAPIN1), CQUJHB003031 (ortholog of stc), CQUJHB013299 (ortholog of ND-B17), CQUJHB014647 (ortholog of Phb1), CQUJHB005188 (ortholog of l(2)gl), CQUJHB011047 (ortholog of hyd), CQUJHB005707 (ortholog of sti), CQUJHB016213 (ortholog of Lam), CQUJHB014837 (ortholog of Lam), CQUJHB018132 (ortholog of Ids), CQUJHB015184 (ortholog of lz), CQUJHB000015 (ortholog of RpL36), CQUJHB016204 (ortholog of RpS3), CQUJHB009364 (ortholog of RpS3), CQUJHB010643 (ortholog of mal), CQUJHB011474 (ortholog of mal), CQUJHB008395 (ortholog of mam), CQUJHB010155 (ortholog of twe), CQUJHB010135 (ortholog of mago), CQUJHB002452 (ortholog of mle), CQUJHB014751 (ortholog of msl-3), CQUJHB013441 (ortholog of mod), CQUJHB017589 (ortholog of mod), CQUJHB007308 (ortholog of PolQ), CQUJHB014221 (ortholog of osk), CQUJHB008825 (ortholog of pbl), CQUJHB001970 (ortholog of Poxn), CQUJHB008771 (ortholog of Polr2A), CQUJHB004674 (ortholog of rst), CQUJHB016351 (ortholog of rst), CQUJHB005882 (ortholog of rt), CQUJHB000251 (ortholog of run), CQUJHB000391 (ortholog of sbr), CQUJHB007109 (ortholog of sd), CQUJHB007683 (ortholog of shu), CQUJHB018246 (ortholog of sina), CQUJHB012604 (ortholog of sm), CQUJHB016838 (ortholog of Sod1), CQUJHB009375 (ortholog of spn-E), CQUJHB008308 (ortholog of Btk), CQUJHB015114 (ortholog of sub), CQUJHB011183 (ortholog of su(sable)), CQUJHB015268 (ortholog of tra2), CQUJHB011184 (ortholog of tra2), CQUJHB000729 (ortholog of tra2), CQUJHB006781 (ortholog of alphaTub84B), CQUJHB008755 (ortholog of alphaTub84B), CQUJHB018308 (ortholog of betaTub85D), CQUJHB016345 (ortholog of tud), CQUJHB014953 (ortholog of RpS27A), CQUJHB005902 (ortholog of Ubi-p63E), CQUJHB015567 (ortholog of v), CQUJHB010234 (ortholog of vg), CQUJHB014095 (ortholog of vls), CQUJHB011005 (ortholog of vn), CQUJHB005397 (ortholog of y), CQUJHB015105 (ortholog of ct), CQUJHB004999 (ortholog of Rdl), CQUJHB006647 (ortholog of Wnt2), CQUJHB011291 (ortholog of porin), CQUJHB006128 (ortholog of aly), CQUJHB017908 (ortholog of neb), CQUJHB002569 (ortholog of pros), CQUJHB009571 (ortholog of Zw10), CQUJHB015072 (ortholog of fru), CQUJHB002601 (ortholog of sim), CQUJHB017236 (ortholog of hop), CQUJHB009752 (ortholog of nmd), CQUJHB011964 (ortholog of GstS1), CQUJHB012486 (ortholog of Trf), CQUJHB014992 (ortholog of Cdc42), CQUJHB017354 (ortholog of Cds), CQUJHB007668 (ortholog of Taf6), CQUJHB006002 (ortholog of Sas-4), CQUJHB018464 (ortholog of dia), CQUJHB003755 (ortholog of fbl), CQUJHB010972 (ortholog of bol), CQUJHB012931 (ortholog of pelo), CQUJHB017964 (ortholog of blw), CQUJHB000401 (ortholog of eff), CQUJHB003660 (ortholog of heph), CQUJHB002587 (ortholog of jar), CQUJHB003106 (ortholog of ox), CQUJHB001029 (ortholog of poe), CQUJHB001861 (ortholog of scat), CQUJHB017961 (ortholog of Hsp60B), CQUJHB016971 (ortholog of Klp3A), CQUJHB001417 (ortholog of Syx5), CQUJHB008577 (ortholog of tsr), CQUJHB007681 (ortholog of wts), CQUJHB000776 (ortholog of Ance), CQUJHB001186 (ortholog of Ance), CQUJHB017460 (ortholog of Trl), CQUJHB012818 (ortholog of l(2)dtl), CQUJHB006724 (ortholog of Arf6), CQUJHB014548 (ortholog of cnn), CQUJHB008831 (ortholog of Rnp4F), CQUJHB009145 (ortholog of noi), CQUJHB014764 (ortholog of tilB), CQUJHB017667 (ortholog of Ddx1), CQUJHB017475 (ortholog of Rala), CQUJHB007728 (ortholog of Shc), CQUJHB009399 (ortholog of Lis-1), CQUJHB010113 (ortholog of csul), CQUJHB008892 (ortholog of mael), CQUJHB000131 (ortholog of mael), CQUJHB016836 (ortholog of mael), CQUJHB006953 (ortholog of fry), CQUJHB005793 (ortholog of fz2), CQUJHB009490 (ortholog of Stat92E), CQUJHB015365 (ortholog of qm), CQUJHB006750 (ortholog of Smg5), CQUJHB001872 (ortholog of ATPsyngamma), CQUJHB010626 (ortholog of stet), CQUJHB017884 (ortholog of Ran), CQUJHB016977 (ortholog of Pdkl), CQUJHB015327 (ortholog of asun), CQUJHB005741 (ortholog of eRF3), CQUJHB009291 (ortholog of Pkn), CQUJHB015025 (ortholog of Osbp), CQUJHB015437 (ortholog of Trxr-1), CQUJHB005134 (ortholog of Vps28), CQUJHB004185 (ortholog of Hel89B), CQUJHB013885 (ortholog of dtr), CQUJHB003925 (ortholog of Mnt), CQUJHB015408 (ortholog of zpg), CQUJHB004705 (ortholog of sns), CQUJHB012028 (ortholog of WASp), CQUJHB009074 (ortholog of Npcla), CQUJHB011142 (ortholog of Dlc90F), CQUJHB014173 (ortholog of Bin1), CQUJHB002083 (ortholog of fws), CQUJHB016540 (ortholog of Cpes), CQUJHB013410 (ortholog of Torsin), CQUJHB007011 (ortholog of Rtca), CQUJHB004320 (ortholog of Inos), CQUJHB015633 (ortholog of mei-P26), CQUJHB015681 (ortholog of SAK), CQUJHB012247 (ortholog of Drone), CQUJHB001978 (ortholog of Grip84), CQUJHB014318 (ortholog of Grip75), CQUJHB005410 (ortholog of Grip75), CQUJHB016145 (ortholog of Grip75), CQUJHB006405 (ortholog of Grip128), CQUJHB018279 (ortholog ofTaz), CQUJHB013228 (ortholog of Pop1), CQUJHB005376 (ortholog of CSN5), CQUJHB008891 (ortholog of ball), CQUJHB002774 (ortholog of Kpc2), CQUJHB003089 (ortholog of hfp), CQUJHB014999 (ortholog of Fmrl), CQUJHB000276 (ortholog of slmo), CQUJHB001023 (ortholog of Gas8), CQUJHB004058 (ortholog of wuho), CQUJHB009904 (ortholog of Pink1), CQUJHB009365 (ortholog of Upf2), CQUJHB015221 (ortholog of ATP7), CQUJHB009709 (ortholog of shop), CQUJHB010760 (ortholog of Naa20A), CQUJHB000936 (ortholog of shv), CQUJHB005303 (ortholog of Pex12), CQUJHB008074 (ortholog of papi), CQUJHB003090 (ortholog of rau), CQUJHB018066 (ortholog of Kdm5), CQUJHB014989 (ortholog of TTLL3B), CQUJHB000941 (ortholog of gudu), CQUJHB011845 (ortholog of Usp14), CQUJHB005386 (ortholog of Bug22), CQUJHB014106 (ortholog of Yipldl), CQUJHB014024 (ortholog of Rsph1), CQUJHB014629 (ortholog of Prosalpha6T), CQUJHB009256 (ortholog of ssp3), CQUJHB006685 (ortholog of COX4L), CQUJHB011670 (ortholog of Pngl), CQUJHB009034 (ortholog of CG13202), CQUJHB014292 (ortholog of rho-7), CQUM010344 (ortholog of Prp8), CQUJHB002648 (ortholog of mip120), CQUJHB014725 (ortholog of S-Lap5), CQUJHB012855 (ortholog of eIF3m), CQUJHB014927 (ortholog of Hr51), CQUJHB015451 (ortholog of clu), CQUJHB016298 (ortholog of clu), CQUJHB010144 (ortholog of clu), CQUJHB013342 (ortholog of Der-2), CQUJHB011752 (ortholog of Dnaaf3), CQUJHB018130 (ortholog of mip40), CQUJHB014264 (ortholog of CG9313), CQUJHB007825 (ortholog of Rael), CQUJHB010259 (ortholog of nsr), CQUJHB011841 (ortholog of YME1L), CQUJHB003648 (ortholog of thoc5), CQUJHB018257 (ortholog of MAN1), CQUJHB008436 (ortholog of ttm2), CQUJHB003133 (ortholog of Cep290), CQUJHB009541 (ortholog of Pex10), CQUJHB000685 (ortholog of Strip), CQUJHB012259 (ortholog of Dnah3), CQUJHB007903 (ortholog of Pex2), CQUJHB007348 (ortholog of S-Lap1), CQUJHB000295 (ortholog of S-Lap1), CQUJHB015411 (ortholog of Cyp40), CQUJHB015144 (ortholog of wls), CQUJHB010244 (ortholog of Duba), CQUJHB015300 (ortholog of twy), CQUJHB000387 (ortholog of Syx13), CQUJHB018206 (ortholog of CG5048), CQUJHB001922 (ortholog of Wdr92), CQUJHB010430 (ortholog of wnd), CQUJHB005968 (ortholog of Pex16), CQUJHB010183 (ortholog of eIF2A), CQUJHB018098 (ortholog of aux), CQUJHB004494 (ortholog of sac), CQUJHB002440 (ortholog of Sym), CQUJHB009165 (ortholog of PSMG1), CQUJHB008515 (ortholog of CG8236), CQUJHB000751 (ortholog of ScsbetaA), CQUJHB001440 (ortholog of topi), CQUJHB015537 (ortholog of Rsbp15), CQUJHB005338 (ortholog of CG9920), CQUJHB014824 (ortholog of HtrA2), CQUJHB001261 (ortholog of hmw), CQUJHB008496 (ortholog of CG14305), CQUJHB002534 (ortholog of CG14305), CQUJHB011875 (ortholog of Syp), CQUJHB002245 (ortholog of Gld2), CQUJHB018365 (ortholog of bb8), CQUJHB017352 (ortholog of Root), CQUJHB007684 (ortholog of puf), CQUJHB010916 (ortholog of mfrn), CQUJHB004314 (ortholog of Cyt-c1L), CQUJHB003041 (ortholog of Cad99C), CQUJHB008254 (ortholog of CG12069), CQUJHB007080 (ortholog of klhl10), CQUJHB012588 (ortholog of rept), CQUJHB009177 (ortholog of pont), CQUJHB000549 (ortholog of DCTN5-p25), CQUJHB010393 (ortholog of Top3alpha), CQUJHB015923 (ortholog of Mytl), CQUJHB004408 (ortholog of park), CQUJHB017981 (ortholog of Apl), CQUJHB011705 (ortholog of HBS1), CQUJHB013350 (ortholog of Fsn), CQUJHB004043 (ortholog of Tre1), CQUJHB009484 (ortholog of Liprin-alpha), CQUJHB009400 (ortholog of Cog7), CQUJHB007033 (ortholog of CG31759), CQUJHB003128 (ortholog of Rsph9), CQUJHB003793 (ortholog of Cap-D3), CQUJHB015240 (ortholog of SCaMC), CQUJHB017802 (ortholog of Gorab), CQUJHB012457 (ortholog of endos), CQUJHB007742 (ortholog of Lasp), CQUJHB010479 (ortholog of sif), CQUJHB003698 (ortholog of Myo31DF), CQUJHB001144 (ortholog of Mer), CQUJHB007994 (ortholog of psidin), CQUJHB003801 (ortholog of Mob4), CQUJHB016344 (ortholog of Nop60B), CQUJHB012973 (ortholog of Su(var)3-3), CQUJHB015731 (ortholog of eIF4G2), CQUJHB005752 (ortholog of eIF4G2), CQUJHB012531 (ortholog of Diap1), CQUJHB013395 (ortholog of bond), CQUJHB011793 (ortholog of Vmat), CQUJHB001496 (ortholog of asl), CQUJHB002279 (ortholog of Pif1A),CQUJHB009238 (ortholog of Hr39), CQUJHB014170 (ortholog of Cul3), CQUJHB010686 (ortholog of didum), CQUJHB014199 (ortholog of nej), CQUJHB015016 (ortholog of brun), CQUJHB014554 (ortholog of Bsg), CQUJHB002256 (ortholog of f), CQUJHB006577 (ortholog of cic), CQUJHB004891 (ortholog of mtsh), CQUJHB004281 (ortholog of SmB), CQUJHB015204 (ortholog of vret), CQUJHB005119 (ortholog of bel), CQUJHB003127 (ortholog of hts), CQUJHB007770 (ortholog of nclb), CQUJHB016647 (ortholog of lost), CQUJHB004289 (ortholog of Brr2), CQUJHB010099 (ortholog of Dark), CQUJHB017911 (ortholog of Dic61B), CQUJHB012962 (ortholog of Cyt-b5), CQUJHB009769 (ortholog of mgr), CQUJHB010995 (ortholog of Mhc), CQUJHB010709 (ortholog of Mhc), CQUJHB018358 (ortholog of mlt), CQUJHB004210 (ortholog of Prosalpha4T1), CQUJHB000715 (ortholog of Tfb5), CQUJHB009874 (ortholog of Doa), CQUJHB012980 (ortholog of stai), CQUJHB018013 (ortholog of Zmynd10), CQUJHB002879 (ortholog of mfr), CQUJHB001781 (ortholog of Sf3a1), CQUJHB018080 (ortholog of Ntl), CQUJHB014011 (ortholog of Topors), CQUJHB013141 (ortholog of mil), CQUJHB012261 (ortholog of sau), CQUJHB011626 (ortholog of lovit), CQUJHB007741 (ortholog of kl-3), CQUJHB002787 (ortholog of WDY), CQUJHB015762 (ortholog of Dhc16F), CQUJHB009713 (ortholog of InR), CQUJHB006384 (ortholog of Pal1), CQUJHB007235 (ortholog of Rpp30), CQUJHB005928 (ortholog of Top2), CQUJHB008870 (ortholog of Orc1), CQUJHB009419 (ortholog of SRPK), CQUJHB017465 (ortholog of bor), CQUJHB006170 (ortholog of dbf), CQUJHB000222 (ortholog of Pen), CQUJHB012358 (ortholog of zip), and CQUJHB008616 (ortholog of betaTub60D).

Non-limiting examples of orthologs of *Drosophila melanogaster* male-fertility essential genes in *Tribolium castaneum* include the following GenBank^{®} Accession Nos: TC000889 (ortholog of Abd-B), TC001432 (ortholog of nAChRbetal), TC007848 (ortholog of Pfas), TC005249 (ortholog of Pfas), TC005250 (ortholog of Pfas), TC007831 (ortholog of aop), TC012080 (ortholog of bru1), TC030691 (ortholog of asp), TC008711 (ortholog of aub), TC033072 (ortholog of chic), TC015014 (ortholog of Chc), TC008985 (ortholog of eya), TC007439 (ortholog of crm), TC014544 (ortholog of CycB), TC009426 (ortholog of Dab), TC006604 (ortholog of dor), TC007943 (ortholog of dwg), TC005760 (ortholog of dx), TC034305 (ortholog of EcR), TC000024 (ortholog of emc), TC009494 (ortholog of exu), TC007253 (ortholog of Fas2), TC033194 (ortholog of tj), TC033376 (ortholog of g), TC033790 (ortholog of H), TC003167 (ortholog of hay), TC014606 (ortholog of Hsp83), TC034873 (ortholog of kl-2), TC031238 (ortholog of kl-2), TC014466 (ortholog of Grip91), TC015856 (ortholog of ifc), TC004939 (ortholog of CIAPIN1), TC030607 (ortholog of stc), TC012859 (ortholog of ND-B17), TC013727 (ortholog of Phb1), TC015986 (ortholog of l(2)gl), TC014081 (ortholog of hyd), TC033419 (ortholog of sti), TC015134 (ortholog of Lam), TC000110 (ortholog of lds), TC034050 (ortholog of lz), TC003770 (ortholog of m), TC002985 (ortholog of RpL36), TC008261 (ortholog of RpS3), TC000626 (ortholog of mal), TC001885 (ortholog of twe), TC016112 (ortholog of mago), TC003184 (ortholog of mle), TC011005 (ortholog of msl-3), TC011380 (ortholog of PolQ), TC010404 (ortholog of pbl), TC034812 (ortholog of Poxn), TC015804 (ortholog of prd), TC014113 (ortholog of Rm62), TC009471 (ortholog of Polr2A), TC002914 (ortholog of rst), TC010429 (ortholog of rt), TC006542 (ortholog of run), TC005575 (ortholog of sbr), TC032219 (ortholog of sd), TC002148 (ortholog of shu), TC002147 (ortholog of shu), TC002855 (ortholog of sm), TC007011 (ortholog of Sod1), TC014516 (ortholog of spn-E), TC007809 (ortholog of Btk), TC011105 (ortholog of ss), TC008157 (ortholog of stau), TC013546 (ortholog of sub), TC030971 (ortholog of su(Hw)), TC034819 (ortholog of su(sable)), TC004873 (ortholog of alphaTub84B), TC009978 (ortholog of Ubi-p63E), TC008028 (ortholog of v), TC000802 (ortholog of y), TC008433 (ortholog of sc), TC015699 (ortholog of ct), TC010482 (ortholog of Rdl), TC010155 (ortholog of Wnt2), TC006408 (ortholog of porin), TC011124 (ortholog of fwd), TC013493 (ortholog of neb), TC002763 (ortholog of Klp67A), TC010596 (ortholog of pros), TC009559 (ortholog of Zw10), TC000589 (ortholog of fru), TC016205 (ortholog of sim), TC004556 (ortholog of Rb97D), TC005313 (ortholog of nmd), TC003496 (ortholog of GstS1), TC007228 (ortholog of Trf), TC013187 (ortholog of Cdc42), TC014743 (ortholog of Cds), TC013033 (ortholog of Taf6), TC015197 (ortholog of Sas-4), TC006029 (ortholog of dia), TC032012 (ortholog of fbl), TC015063 (ortholog of bol), TC001682 (ortholog of pelo), TC008728 (ortholog of blw), TC000443 (ortholog of eff), TC034449 (ortholog of heph), TC000685 (ortholog of jar), TC004400 (ortholog of poe), TC012073 (ortholog of scat), TC007199 (ortholog of Iswi), TC015915 (ortholog of Klp3A), TC001574 (ortholog of tsr), TC030986 (ortholog of wts), TC033437 (ortholog of ctp), TC011052 (ortholog of Ance), TC008061 (ortholog of l(2)dtl), TC004744 (ortholog of Arf6), TC002719 (ortholog of cnn), TC009182 (ortholog of Rnp4F), TC013335 (ortholog of mia), TC001034 (ortholog of noi), TC003526 (ortholog of tilB), TC008987 (ortholog of Ddx1), TC012218 (ortholog of Rala), TC010804 (ortholog of Shc), TC009385 (ortholog of spag), TC005496 (ortholog of Lis-1), TC031040 (ortholog of ara), TC003689 (ortholog of csul), TC008172 (ortholog of mael), TC000108 (ortholog of fry), TC003407 (ortholog of fz2), TC013218 (ortholog of Stat92E), TC004978 (ortholog of qm), TC001202 (ortholog of Smg5), TC009010 (ortholog of ATPsyngamma), TC034044 (ortholog of stet), TC012793 (ortholog of Ran), TC002973 (ortholog of Pdk1), TC006499 (ortholog of asun), TC006729 (ortholog of eRF3), TC032431 (ortholog of Pkn), TC006108 (ortholog of Osbp), TC002698 (ortholog of Trxr-1), TC007865 (ortholog of Trxr-1), TC008618 (ortholog of chb), TC000243 (ortholog of Nup154), TC010199 (ortholog of Vps28), TC004394 (ortholog of Nle), TC005832 (ortholog of Hel89B), TC015835 (ortholog of dtr), TC032336 (ortholog of sns), TC033918 (ortholog of Npcla), TC005088 (ortholog of Npcla), TC009745 (ortholog of Dlc90F), TC000183 (ortholog of Bin1), TC013924 (ortholog of fws), TC000963 (ortholog of Kap-alphal), TC034597 (ortholog of Cpes), TC000824 (ortholog of Torsin), TC010126 (ortholog of Rtca), TC030051 (ortholog of Inos), TC007693 (ortholog of mei-P26), TC001032 (ortholog of SAK), TC007942 (ortholog of Grip84), TC009163 (ortholog of Grip75), TC005288 (ortholog of Grip128), TC009822 (ortholog of Taz), TC011530 (ortholog of Pop1), TC011816 (ortholog of CSN5), TC012134 (ortholog of mino), TC012687 (ortholog of ball), TC009460 (ortholog of Kpc2), TC012222 (ortholog ofAck), TC001408 (ortholog ofAck), TC032198 (ortholog of hfp), TC003774 (ortholog of Fmr1), TC014470 (ortholog of slmo), TC011228 (ortholog of Gas8), TC013202 (ortholog of Pink1), TC002851 (ortholog of Upf2), TC030914 (ortholog of ATP7), TC000220 (ortholog of shop), TC007251 (ortholog of Naa20A), TC013491 (ortholog of shv), TC014905 (ortholog of Pex12), TC013683 (ortholog of Hsp60C), TC014715 (ortholog of rau), TC033256 (ortholog of TTLL3B), TC007212 (ortholog of Rab30), TC031742 (ortholog of gudu), TC030879 (ortholog of PAPLA1), TC009042 (ortholog of Usp14), TC008446 (ortholog of Yip1d1), TC007855 (ortholog of Rsph1), TC000258 (ortholog of Prosalpha6T), TC009337 (ortholog of ssp3), TC009512 (ortholog of COX4L), TC031208 (ortholog of Pngl), TC012554 (ortholog of U2A), TC013516 (ortholog of rho-7), TC010565 (ortholog of Prp8), TC005724 (ortholog of mip120), TC001175 (ortholog of S-Lap5), TC011022 (ortholog of eIF3m), TC009378 (ortholog of Hr51), TC003310 (ortholog of clu), TC012611 (ortholog of Dnaaf3), TC006557 (ortholog of mip40), TC009001 (ortholog of Mgat1), TC008686 (ortholog of CG9313), TC009967 (ortholog of Rae1), TC008871 (ortholog of nsr), TC005933 (ortholog of tous), TC009202 (ortholog of YME1L), TC005538 (ortholog of thoc5), TC008922 (ortholog of Rsph4a), TC030575 (ortholog of MAN1), TC034042 (ortholog of Dis312), TC006148 (ortholog of ttm2), TC032082 (ortholog of Cep290), TC032038 (ortholog of Pex10), TC010759 (ortholog of Strip), TC005485 (ortholog of Dnah3), TC032995 (ortholog of Pex2), TC004000 (ortholog of S-Lap1), TC000234 (ortholog of Cyp40), TC005345 (ortholog of wls), TC004141 (ortholog of Duba), TC002926 (ortholog of twy), TC007177 (ortholog of Syx13), TC014285 (ortholog of CG5048), TC009613 (ortholog of Wdr92), TC007629 (ortholog of Pex16), TC013517 (ortholog of eIF2A), TC012067 (ortholog of aux), TC007516 (ortholog of sac), TC007517 (ortholog of sac), TC010414 (ortholog of Sym), TC013859 (ortholog of ScsbetaA), TC031901 (ortholog of topi), TC013081 (ortholog of CG9920), TC010501 (ortholog of CG9920), TC003010 (ortholog of HtrA2), TC009447 (ortholog of CG14305), TC000185 (ortholog of Syp), TC004457 (ortholog of Gld2), TC015008 (ortholog of wam), TC015006 (ortholog of wam), TC015005 (ortholog of wam), TC033421 (ortholog of wam), TC015007 (ortholog of wam), TC031634 (ortholog of bb8), TC030673 (ortholog of tbrd-1), TC030708 (ortholog of tbrd-1), TC000358 (ortholog of Root), TC012585 (ortholog of mfrn), TC010331 (ortholog of Cyt-c1L), TC011374 (ortholog of Cad99C), TC012547 (ortholog of klhl10), TC015193 (ortholog of rept), TC012184 (ortholog of pont), TC008312 (ortholog of DCTN5-p25), TC006688 (ortholog of Top3alpha), TC004776 (ortholog of Myt1), TC007236 (ortholog of CG18675), TC013995 (ortholog of park), TC006451 (ortholog of ago), TC030696 (ortholog of Apl), TC002377 (ortholog of Apl), TC003854 (ortholog of HBS1), TC010464 (ortholog of Fsn), TC004716 (ortholog of Tre1), TC012918 (ortholog of Liprin-alpha), TC031006 (ortholog of Cog7), TC014029 (ortholog of CG31759), TC008456 (ortholog of Rsph9), TC006460 (ortholog of SCaMC), TC014371 (ortholog of Rsph3), TC009470 (ortholog of Erk7), TC003252 (ortholog of Gorab), TC012832 (ortholog of endos), TC009504 (ortholog of Lasp), TC031145 (ortholog of sif), TC005895 (ortholog of Myo31DF), TC014860 (ortholog of Mer), TC011820 (ortholog of pea), TC012775 (ortholog of snky), TC015420 (ortholog of psidin), TC012571 (ortholog of Mob4), TC010569 (ortholog of Nop60B), TC032013 (ortholog of Su(var)3-3), TC013876 (ortholog of eIF4G2), TC002792 (ortholog of scny), TC016280 (ortholog of bond), TC004499 (ortholog of Vmat), TC014986 (ortholog of Hr39), TC013922 (ortholog of Cul3), TC009935 (ortholog of didum), TC008222 (ortholog of nej), TC013624 (ortholog of larp), TC031285 (ortholog of brun), TC009413 (ortholog of Bsg), TC031283 (ortholog of f), TC004697 (ortholog of cic), TC005312 (ortholog of mtsh), TC030631 (ortholog of SmB), TC032473 (ortholog of Myc), TC014271 (ortholog of Acn), TC013328 (ortholog of bel), TC004497 (ortholog of hts), TC007893 (ortholog of nclb), TC012407 (ortholog of lost), TC031972 (ortholog of Brr2), TC000831 (ortholog of Dark), TC014566 (ortholog of dally), TC034331 (ortholog of Dic61B), TC001027 (ortholog of Cyt-b5), TC001098 (ortholog of mgr), TC034001 (ortholog of Mhc), TC005185 (ortholog of pAbp), TC007700 (ortholog of mlt), TC014574 (ortholog of Prosalpha4T1), TC001529 (ortholog of Doa), TC013090 (ortholog of stai), TC009549 (ortholog of Zmynd10), TC003976 (ortholog of mfr), TC004839 (ortholog of Sf3a1), TC014132 (ortholog of Nt1), TC030677 (ortholog of Topors), TC001762 (ortholog of sau), TC001165 (ortholog of lovit), TC034619 (ortholog of kl-3), TC008640 (ortholog of kl-5), TC006047 (ortholog of vib), TC000119 (ortholog of Dhc16F), TC009284 (ortholog of Pall), TC033113 (ortholog of Rpp30), TC003811 (ortholog of Top2), TC009174 (ortholog of TER94), TC008879 (ortholog of Orc1), TC014695 (ortholog of bor), TC015386 (ortholog of dbf), TC000505 (ortholog of Pen), TC034132 (ortholog of zip), and TC034766 (ortholog of betaTub60D).

Non-limiting examples of orthologs of *Drosophila melanogaster* male-fertility essential genes in *Lucilia cuprina* include the following GenBank^{®} Accession Nos: LOC111677203 (ortholog of abd-A), LOC111677200 (ortholog of Abd-B), LOC111676787 (ortholog of ac), LOC111687940 (ortholog of nAChRbeta1), LOC111688070 (ortholog of Pfas), LOC111687181 (ortholog of aop), LOC111686359 (ortholog of bru1), LOC111679249 (ortholog of asp), LOC111678555 (ortholog of aub), LOC111684430 (ortholog of ca), LOC111679664 (ortholog of chic), LOC111678708 (ortholog of Chc), LOC111677507 (ortholog of eya), LOC111678837 (ortholog of crm), LOC111681409 (ortholog of CycB), LOC111681115 (ortholog of Dab), LOC111680346 (ortholog of dor), LOC111687770 (ortholog of dx), LOC111678597 (ortholog of emc), LOC111675879 (ortholog of exu), LOC111678712 (ortholog of Fas2), LOC111689389 (ortholog of tj), LOC111684348 (ortholog of g), LOC111678859 (ortholog of H), LOC111677282 (ortholog of hay), LOC111679400 (ortholog of Hsp83), LOC111680689 (ortholog of kl-2), LOC111684350 (ortholog of Grip91), LOC111679645 (ortholog of ifc), LOC111679207 (ortholog of CIAPIN1), LOC111685399 (ortholog of stc), LOC111685429 (ortholog of ND-B17), LOC111686133 (ortholog of Phb1), LOC111677642 (ortholog of l(2)gl), LOC111680576 (ortholog of hyd), LOC111683483 (ortholog of sti), LOC111678384 (ortholog of Lam), LOC111684949 (ortholog of 1ds), LOC111684005 (ortholog of lz), LOC111679993 (ortholog of m), LOC111686570 (ortholog of RpL36), LOC111678069 (ortholog of RpS3), LOC111680597 (ortholog of mal), LOC111677570 (ortholog of mam), LOC111688011 (ortholog of mago), LOC111686523 (ortholog of mle), LOC111679404 (ortholog of msl-3), LOC124421188 (ortholog of mod), LOC111679205 (ortholog of mod), LOC111686176 (ortholog of mod), LOC111681207 (ortholog of PolQ), LOC111683354 (ortholog of osk), LOC111691135 (ortholog of otu), LOC111686844 (ortholog of pbl), LOC124421201 (ortholog of pbl), LOC111687308 (ortholog of Poxn), LOC111679050 (ortholog of prd), LOC111681949 (ortholog of ref(2)P), LOC111681567 (ortholog of Rm62), LOC111675262 (ortholog of Polr2A), LOC111677530 (ortholog of rst), LOC111685033 (ortholog of rt), LOC111674826 (ortholog of run), LOC111684943 (ortholog of sbr), LOC111682267 (ortholog of sd), LOC111680204 (ortholog of shu), LOC111682864 (ortholog of sm), LOC111688303 (ortholog of spn-E), LOC111679981 (ortholog of spn-E), LOC111682599 (ortholog of Btk), LOC111676534 (ortholog of ss), LOC111688226 (ortholog of stau), LOC111690485 (ortholog of stg), LOC111679724 (ortholog of sub), LOC111690510 (ortholog of su(Hw)), LOC111688354 (ortholog of su(sable)), LOC111687531 (ortholog of tra2), LOC111678415 (ortholog of alphaTub84B), LOC111678876 (ortholog of betaTub85D), LOC111684801 (ortholog of tud), LOC111689191 (ortholog of RpS27A), LOC111686161 (ortholog of Ubi-p63E), LOC111688806 (ortholog of v), LOC111675916 (ortholog of vg), LOC111681013 (ortholog of vls), LOC111690704 (ortholog of vn), LOC111686845 (ortholog of y), LOC111676788 (ortholog of sc), LOC111686284 (ortholog of ct), LOC111677776 (ortholog of Rdl), LOC111688655 (ortholog of Wnt2), LOC111681702 (ortholog of porin), LOC111686482 (ortholog of fwd), LOC111687490 (ortholog of neb), LOC124420755 (ortholog of neb), LOC124419941 (ortholog of neb), LOC124419540 (ortholog of neb), LOC111675099 (ortholog of Klp67A), LOC111675098 (ortholog of Klp67A), LOC111677034 (ortholog of bgcn), LOC111684952 (ortholog of pros), LOC111678634 (ortholog of Zw10), LOC111681546 (ortholog of fru), LOC111690477 (ortholog of sim), LOC111684468 (ortholog of hop), LOC111679155 (ortholog of piwi), LOC111681186 (ortholog of Rb97D), LOC111675643 (ortholog of upd1), LOC111689348 (ortholog of nmd), LOC111687996 (ortholog of GstS1), LOC111684480 (ortholog of Trf), LOC111680243 (ortholog of Cdc42), LOC111676884 (ortholog of Cds), LOC111677881 (ortholog of Taf6), LOC111678405 (ortholog of Sas-4), LOC111680779 (ortholog of dia), LOC111688764 (ortholog of cue), LOC111687383 (ortholog of fbl), LOC111683247 (ortholog of bol), LOC111687238 (ortholog of pelo), LOC111681410 (ortholog of blw), LOC111687512 (ortholog of eff), LOC111675656 (ortholog of heph), LOC111679494 (ortholog of jar), LOC111679138 (ortholog of ox), LOC111684479 (ortholog of poe), LOC111680949 (ortholog of scat), LOC111681434 (ortholog of Hsp60B), LOC111684639 (ortholog of Iswi), LOC111675239 (ortholog of Klp3A), LOC111680778 (ortholog of Syx5), LOC111677021 (ortholog of tsr), LOC111690629 (ortholog of wts), LOC111677527 (ortholog of Ance), LOC111677496 (ortholog of Ance), LOC111677497 (ortholog of Ance), LOC111681913 (ortholog of Trl), LOC111680072 (ortholog of l(2)dt1), LOC111677991 (ortholog of Arf6), LOC111679601 (ortholog of cnn), LOC111691124 (ortholog of Rnp4F), LOC111679367 (ortholog of noi), LOC111688433 (ortholog oftilB), LOC111682738 (ortholog of Ddx1), LOC111686275 (ortholog of eIF4E1), LOC111682282 (ortholog of Rala), LOC111682809 (ortholog of Shc), LOC111681502 (ortholog of Lis-1), LOC111689222 (ortholog of ara), LOC111689087 (ortholog of caup), LOC111680705 (ortholog of csul), LOC111677617 (ortholog of mael), LOC111688408 (ortholog of fry), LOC111682700 (ortholog of fz2), LOC111679071 (ortholog of Stat92E), LOC111688489 (ortholog of qm), LOC111675279 (ortholog of Smg5), LOC111675628 (ortholog of ATPsyngamma), LOC111674862 (ortholog of stet), LOC111685136 (ortholog of Ran), LOC111687838 (ortholog of Pdk1), LOC111688562 (ortholog of asun), LOC111677560 (ortholog of eRF3), LOC111675923 (ortholog of Pkn), LOC111681609 (ortholog of Osbp), LOC111680900 (ortholog of Trxr-1), LOC111675607 (ortholog of chb), LOC111681431 (ortholog of Nup154), LOC111678201 (ortholog of Vps28), LOC111682966 (ortholog of Nle), LOC111680174 (ortholog of Hel89B), LOC111679237 (ortholog of dtr), LOC111690008 (ortholog of Mnt), LOC111681418 (ortholog of zpg), LOC111677477 (ortholog of sns), LOC111690966 (ortholog of WASp), LOC111684329 (ortholog of Npc1a), LOC111686177 (ortholog of Dlc90F), LOC111690312 (ortholog of Bin1), LOC111681676 (ortholog of fws), LOC111683665 (ortholog of Kap-alphal), LOC111677140 (ortholog of Torsin), LOC111690630 (ortholog of Rtca), LOC111677637 (ortholog of Inos), LOC111683600 (ortholog of mei-P26), LOC111680220 (ortholog of SAK), LOC111688557 (ortholog of Dronc), LOC111677078 (ortholog of Dronc), LOC111676942 (ortholog of Grip84), LOC111681426 (ortholog of Grip75), LOC111675314 (ortholog of Grip128), LOC111679127 (ortholog of Taz), LOC124420777 (ortholog of Taz), LOC111675972 (ortholog of Pop1), LOC111680162 (ortholog of CSN5), LOC111675460 (ortholog of mino), LOC111681328 (ortholog of ball), LOC111679594 (ortholog of Kpc2), LOC111679721 (ortholog of Ack), LOC111689351 (ortholog of hfp), LOC111686955 (ortholog of Fmr1), LOC111688064 (ortholog of slmo), LOC111684962 (ortholog of Gas8), LOC111674885 (ortholog of wuho), LOC111675727 (ortholog of Upf2), LOC111679264 (ortholog of ATP7), LOC111677731 (ortholog of shop), LOC111687954 (ortholog of Naa20A), LOC111675757 (ortholog of shv), LOC111685380 (ortholog of Pex12), LOC111678643 (ortholog of papi), LOC111679656 (ortholog of Kdm5), LOC111674548 (ortholog of TTLL3B), LOC111679126 (ortholog of Rab30), LOC111685785 (ortholog of gudu), LOC111674775 (ortholog of PAPLA1), LOC111676794 (ortholog of Usp14), LOC124420579 (ortholog of Bug22), LOC111678995 (ortholog of Bug22), LOC111679579 (ortholog of spag4), LOC111687304 (ortholog of Yip1d1), LOC111687665 (ortholog of Rsph1), LOC111689921 (ortholog of Prosalpha6T), LOC111674916 (ortholog of ssp3), LOC111675743 (ortholog of COX4L), LOC111678177 (ortholog of Pngl), LOC111684900 (ortholog of U2A), LOC111678330 (ortholog of CG13202), LOC111678178 (ortholog of rho-7), LOC111685059 (ortholog of Prp8), LOC111677385 (ortholog of mip120), LOC111685964 (ortholog of S-Lap7), LOC111685965 (ortholog of S-Lap7), LOC111691231 (ortholog of eIF3m), LOC111681187 (ortholog of Hr51), LOC111681510 (ortholog of clu), LOC111680373 (ortholog of Dcr-2), LOC111675342 (ortholog of Dnaaf3), LOC111677577 (ortholog of mip40), LOC111677803 (ortholog of Mgat1), LOC111687251 (ortholog of CG9313), LOC111684640 (ortholog of Rae1), LOC111679010 (ortholog of nsr), LOC111687297 (ortholog of tous), LOC111678529 (ortholog of YME1L), LOC111677004 (ortholog of thoc5), LOC111691091 (ortholog of Rsph4a), LOC111678007 (ortholog of MAN1), LOC111686491 (ortholog of Dis3l2), LOC111681243 (ortholog of ttm2), LOC111684813 (ortholog of Cep290), LOC111682112 (ortholog of Pex10), LOC111676602 (ortholog of Strip), LOC111686279 (ortholog of ntc), LOC111682347 (ortholog of Dpy-30L2), LOC111680397 (ortholog of Dnah3), LOC111680666 (ortholog of mmm), LOC111677083 (ortholog of Pex2), LOC111680740 (ortholog of S-Lap1), LOC111687566 (ortholog of Cyp40), LOC111675102 (ortholog of wls), LOC111681893 (ortholog of Duba), LOC111682699 (ortholog of twy), LOC111682735 (ortholog of cmb), LOC111682769 (ortholog of CG5048), LOC111685866 (ortholog of Cep135), LOC111690796 (ortholog of ATPsynbetaL), LOC111681220 (ortholog of Wdr92), LOC111676647 (ortholog of ms(3)76Cc), LOC111676608 (ortholog of wnd), LOC111687731 (ortholog of ms(3)76Ca), LOC111686091 (ortholog of Pex16), LOC111688120 (ortholog of eIF2A), LOC111679692 (ortholog of aux), LOC111682311 (ortholog of sac), LOC111682684 (ortholog of Sym), LOC111687396 (ortholog of CG8236), LOC111683352 (ortholog of ScsbetaA), LOC111676121 (ortholog of topi), LOC111690943 (ortholog of Rsbp15), LOC111677649 (ortholog of CG9920), LOC111675035 (ortholog of HtrA2), LOC111678234 (ortholog of Kpc1), LOC111687617 (ortholog of CG14305), LOC111677161 (ortholog of Syp), LOC111686868 (ortholog of Theg), LOC111676012 (ortholog of Gld2), LOC111690775 (ortholog of wam), LOC111690779 (ortholog of bb8), LOC111683390 (ortholog of Root), LOC111686134 (ortholog of puf), LOC111675489 (ortholog of mfrn), LOC111683482 (ortholog of Cyt-c1L), LOC111690997 (ortholog of Cad99C), LOC111675374 (ortholog of CG12069), LOC111675288 (ortholog of klhl10), LOC111683820 (ortholog of rept), LOC111690882 (ortholog of pont), LOC111681315 (ortholog of DCTN5-p25), LOC111687825 (ortholog of Top3alpha), LOC111683298 (ortholog of Myt1), LOC111682244 (ortholog of CG18675), LOC111685242 (ortholog of park), LOC111678670 (ortholog of ago), LOC111690690 (ortholog of Apl), LOC111689645 (ortholog of HBS1), LOC111685677 (ortholog of Fsn), LOC111674536 (ortholog of Liprin-alpha), LOC111690027 (ortholog of Cog7), LOC111677870 (ortholog of CG31759), LOC111676862 (ortholog of Rsph9), LOC111681677 (ortholog of Cap-D3), LOC111677401 (ortholog of SCaMC), LOC111683336 (ortholog of tut), LOC111677614 (ortholog of Rsph3), LOC111682964 (ortholog of Erk7), LOC111682810 (ortholog of Gorab), LOC111686308 (ortholog of endos), LOC111681130 (ortholog of Lasp), LOC111690717 (ortholog of sif), LOC111678997 (ortholog of Myo31DF), LOC111687619 (ortholog of Mer), LOC111691226 (ortholog of pea), LOC111688548 (ortholog of Cyt-c-d), LOC111678640 (ortholog of snky), LOC111684095 (ortholog of psidin), LOC124420727 (ortholog of Vrp1), LOC124419285 (ortholog of gish), LOC111685419 (ortholog of Mob4), LOC111674641 (ortholog of Nop60B), LOC111681191 (ortholog of Su(var)3-3), LOC111685657 (ortholog of Rcd7), LOC111690584 (ortholog of eIF4G2), LOC111678887 (ortholog of Diap1), LOC111689988 (ortholog of Diap1), LOC111680581 (ortholog of mxc), LOC111687204 (ortholog of bond), LOC111677390 (ortholog of Vmat), LOC111678240 (ortholog of asl), LOC111690211 (ortholog of Pif1A), LOC111683076 (ortholog of Hr39), LOC111685833 (ortholog of Cul3), LOC111675961 (ortholog of didum), LOC111687634 (ortholog of ms(2)34Fe), LOC111680919 (ortholog of nej), LOC111689588 (ortholog of larp), LOC111675532 (ortholog of brun), LOC111684483 (ortholog of Bsg), LOC111677722 (ortholog of f), LOC111680198 (ortholog of cic), LOC111678998 (ortholog of SmB), LOC111690376 (ortholog of Myc), LOC111685044 (ortholog of vret), LOC111681727 (ortholog of Acn), LOC111683090 (ortholog of bel), LOC111682383 (ortholog of hts), LOC111679734 (ortholog of nclb), LOC111679133 (ortholog of lost), LOC111687652 (ortholog of Brr2), LOC111684219 (ortholog of Dark), LOC111683780 (ortholog of dally), LOC111686836 (ortholog of Dic61B), LOC111689337 (ortholog of Cyt-b5), LOC111681675 (ortholog of ab), LOC111684954 (ortholog of mgr), LOC111678371 (ortholog of Mhc), LOC111687791 (ortholog of pAbp), LOC111682325 (ortholog of mlt), LOC111686910 (ortholog of Prosalpha4T1), LOC111691213 (ortholog of Tfb5), LOC111676109 (ortholog of Doa), LOC111682667 (ortholog of Zmynd10), LOC124419576 (ortholog of Zmynd10), LOC111678977 (ortholog of mfr), LOC124420235 (ortholog of mfr), LOC124420236 (ortholog of mfr), LOC111679983 (ortholog of Sf3a1), LOC111687453 (ortholog of Ntl), LOC111686713 (ortholog of Topors), LOC111690412 (ortholog of mil), LOC111688757 (ortholog of sau), LOC111686037 (ortholog of lovit), LOC111676993 (ortholog of kl-3), LOC111675651 (ortholog of kl-5), LOC111679054 (ortholog of WDY), LOC124419851 (ortholog of vib), LOC111681480 (ortholog of Dhc16F), LOC111677709 (ortholog of InR), LOC111681954 (ortholog of Pall), LOC111686320 (ortholog of Rpp30), LOC111685432 (ortholog of Top2), LOC111690599 (ortholog of shps), LOC111684586 (ortholog of TER94), LOC111689868 (ortholog of Orc1), LOC111684580 (ortholog of SRPK), LOC111680257 (ortholog of bor), LOC111687309 (ortholog of dbf), LOC111686822 (ortholog of Pen), LOC111675361 (ortholog of zip), and LOC111678482 (ortholog of betaTub60D).

Non-limiting examples of orthologs of *Drosophila melanogaster* male-fertility essential genes in *Schistocerca gregaria* include the following GenBank^{®} Accession Nos: LOC126293357 (ortholog of abd-A), LOC126293227 (ortholog of Abd-B), LOC126272568 (ortholog of nAChRbeta1), LOC126334343 (ortholog of Pfas), LOC126281265 (ortholog of Pfas), LOC126281598 (ortholog of aop), LOC126278218 (ortholog of bru1), LOC126284413 (ortholog of asp), LOC126320477 (ortholog of asp), LOC126336384 (ortholog of ca), LOC126335476 (ortholog of ca), LOC126284213 (ortholog of ca), LOC126304760 (ortholog of ca), LOC126282514 (ortholog of ca), LOC126328814 (ortholog of ca), LOC126318611 (ortholog of ca), LOC126320760 (ortholog of ca), LOC126322375 (ortholog of ca), LOC126324053 (ortholog of chic), LOC126284984 (ortholog of Chc), LOC126278064 (ortholog of eya), LOC126282048 (ortholog of crm), LOC126322720 (ortholog of CycB), LOC126279072 (ortholog of CycB), LOC126272743 (ortholog of CycB), LOC126334735 (ortholog of dor), LOC126336029 (ortholog of dx), LOC126353835 (ortholog of EcR), LOC126354723 (ortholog of emc), LOC126266874 (ortholog of exu), LOC126291686 (ortholog of Fas2), LOC126272153 (ortholog of tj), LOC126272861 (ortholog of tj), LOC126329916 (ortholog of g), LOC126348507 (ortholog of H), LOC126354389 (ortholog of hay), LOC126284200 (ortholog of Hsp83), LOC126278873 (ortholog of kl-2), LOC126284391 (ortholog of ifc), LOC126353884 (ortholog of CIAPIN1), LOC126295019 (ortholog of stc), LOC126284745 (ortholog of ND-B17), LOC126273090 (ortholog of Phb1), LOC126335527 (ortholog of l(2)gl), LOC126336313 (ortholog of hyd), LOC126284006 (ortholog of sti), LOC126271979 (ortholog of Lam), LOC126283995 (ortholog of lds), LOC126335350 (ortholog of lz), LOC126268104 (ortholog of m), LOC126314455 (ortholog of RpS3), LOC126293317 (ortholog of mal), LOC126266688 (ortholog of twe), LOC126339912 (ortholog of mago), LOC126365821 (ortholog of mle), LOC126324271 (ortholog of msl-3), LOC126324245 (ortholog of msl-3), LOC126314444 (ortholog of mod), LOC126325475 (ortholog of mod), LOC126356167 (ortholog of mod), LOC126326577 (ortholog of sa), LOC126336904 (ortholog of PolQ), LOC126335502 (ortholog of pbl), LOC126273321 (ortholog of Poxn), LOC126272759 (ortholog of ref(2)P), LOC126334829 (ortholog of Polr2A), LOC126266784 (ortholog of rst), LOC126272214 (ortholog of rt), LOC126335349 (ortholog of run), LOC126291585 (ortholog of sbr), LOC126281832 (ortholog of sd), LOC126336383 (ortholog of shu), LOC126295107 (ortholog of Sod1), LOC126278125 (ortholog of spn-E), LOC126281657 (ortholog of Btk), LOC126299004 (ortholog of ss), LOC126344762 (ortholog of stau), LOC126334614 (ortholog of sub), LOC126298250 (ortholog of betaTub85D), LOC126354374 (ortholog of tud), LOC126313307 (ortholog ofRpS27A), LOC126315308 (ortholog of Ubi-p63E), LOC126281291 (ortholog of Ubi-p63E), LOC126282517 (ortholog of v), LOC126355835 (ortholog of y), LOC126354306 (ortholog of sc), LOC126284054 (ortholog of ct), LOC126366011 (ortholog of Rdl), LOC126272956 (ortholog of Wnt2), LOC126329181 (ortholog of porin), LOC126356265 (ortholog of aly), LOC126336772 (ortholog of fwd), LOC126272058 (ortholog of neb), LOC126266746 (ortholog of Klp67A), LOC126272627 (ortholog of bgen), LOC126304654 (ortholog of bgcn), LOC126349037 (ortholog of pros), LOC126278657 (ortholog of Zw10), LOC126278656 (ortholog of Zw10), LOC126364371 (ortholog of fru), LOC126335455 (ortholog of sim), LOC126266927 (ortholog of hop), LOC126336553 (ortholog of nmd), LOC126282422 (ortholog of GstS1), LOC126336691 (ortholog of GstS1), LOC126336692 (ortholog of GstS1), LOC126336695 (ortholog of GstS1), LOC126336693 (ortholog of GstS1), LOC126336696 (ortholog of GstS1), LOC126336697 (ortholog of GstS1), LOC126336730 (ortholog of GstS1), LOC126295392 (ortholog of Cdc42), LOC126283992 (ortholog of Cds), LOC126272179 (ortholog of Taf6), LOC126298999 (ortholog of Sas-4), LOC126284850 (ortholog of dia), LOC126272649 (ortholog of cue), LOC126357156 (ortholog of fbl), LOC126355811 (ortholog of bol), LOC126270342 (ortholog of pelo), LOC126365789 (ortholog of blw), LOC126278847 (ortholog of eff), LOC126353529 (ortholog of heph), LOC126267997 (ortholog of jar), LOC126299607 (ortholog of poe), LOC126278215 (ortholog of scat), LOC126278062 (ortholog of scat), LOC126353840 (ortholog of fzo), LOC126354756 (ortholog of Iswi), LOC126267242 (ortholog of Klp3A), LOC126278433 (ortholog of Syx5), LOC126335024 (ortholog of tsr), LOC126266638 (ortholog of wts), LOC126285366 (ortholog of ctp), LOC126293313 (ortholog of Ance), LOC126272843 (ortholog of Trl), LOC126279063 (ortholog of l(2)dtl), LOC126268232 (ortholog of Arf6), LOC126316990 (ortholog of Rnp4F), LOC126357124 (ortholog of noi), LOC126353992 (ortholog of tilB), LOC126278035 (ortholog of Ddx1), LOC126324041 (ortholog of eIF4E1), LOC126353891 (ortholog of Rala), LOC126272104 (ortholog of Shc), LOC126354036 (ortholog of spag), LOC126272653 (ortholog of Lis-1), LOC126267208 (ortholog of ara), LOC126267349 (ortholog of csul), LOC126266666 (ortholog of mael), LOC126343774 (ortholog of fry), LOC126355669 (ortholog of fz2), LOC126267038 (ortholog of Stat92E), LOC126278539 (ortholog of qm), LOC126281955 (ortholog of Smg5), LOC126285434 (ortholog of ATPsyngamma), LOC126335219 (ortholog of stet), LOC126266646 (ortholog of Ran), LOC126353207 (ortholog of Ran), LOC126354876 (ortholog of Pdk1), LOC126334521 (ortholog of asun), LOC126316689 (ortholog of eRF3), LOC126267370 (ortholog of Pkn), LOC126285403 (ortholog of Osbp), LOC126271953 (ortholog of Trxr-1), LOC126297568 (ortholog of chb), LOC126266642 (ortholog of Nup154), LOC126278118 (ortholog of Vps28), LOC126318222 (ortholog of Nle), LOC126328531 (ortholog of Hel89B), LOC126272909 (ortholog of dtr), LOC126272938 (ortholog of dtr), LOC126273277 (ortholog of dtr), LOC126284112 (ortholog of Mnt), LOC126327557 (ortholog of sns), LOC126293654 (ortholog ofNpcla), LOC126291761 (ortholog of Dlc90F), LOC126339643 (ortholog of Bin1), LOC126284134 (ortholog of fws), LOC126281139 (ortholog of Kap-alphal), LOC126271935 (ortholog of Cpes), LOC126334980 (ortholog of Torsin), LOC126334982 (ortholog of Torsin), LOC126272795 (ortholog of Rtca), LOC126273050 (ortholog of Rtca), LOC126328958 (ortholog of Inos), LOC126324058 (ortholog of Inos), LOC126281189 (ortholog of mei-P26), LOC126355013 (ortholog of SAK), LOC126281536 (ortholog of Grip84), LOC126267157 (ortholog of Grip75), LOC126336347 (ortholog of Grip128), LOC126272674 (ortholog of Taz), LOC126356310 (ortholog of Pop1), LOC126322525 (ortholog of CSN5), LOC126356100 (ortholog of mino), LOC126352566 (ortholog of ball), LOC126272033 (ortholog of Kpc2), LOC126353937 (ortholog of Ack), LOC126356280 (ortholog of hfp), LOC126273069 (ortholog of slmo), LOC126267362 (ortholog of Gas8), LOC126271951 (ortholog of wuho), LOC126295463 (ortholog of Pink1), LOC126297785 (ortholog of Upf2), LOC126365732 (ortholog of Upf2), LOC126319861 (ortholog of p24-1), LOC126285025 (ortholog of ATP7), LOC126333456 (ortholog of shop), LOC126333611 (ortholog of shop), LOC126291725 (ortholog of Naa20A), LOC126336799 (ortholog of shv), LOC126285362 (ortholog of Pex12), LOC126334963 (ortholog of papi), LOC126273192 (ortholog of rau), LOC126336664 (ortholog of TTLL3B), LOC126281624 (ortholog of Rab30), LOC126272276 (ortholog of gudu), LOC126272592 (ortholog of gudu), LOC126365914 (ortholog of PAPLA1), LOC126271870 (ortholog of Usp14), LOC126344845 (ortholog of Bug22), LOC126335949 (ortholog of spag4), LOC126279131 (ortholog of Yip1d1), LOC126278869 (ortholog of Rsph1), LOC126281973 (ortholog of Prosalpha6T), LOC126272212 (ortholog of ssp3), LOC126279027 (ortholog of COX4L), LOC126365785 (ortholog of Pngl), LOC126271892 (ortholog of U2A), LOC126336850 (ortholog of rho-7), LOC126349809 (ortholog of Prp8), LOC126329139 (ortholog of mip120), LOC126320559 (ortholog of S-Lap5), LOC126281246 (ortholog of eIF3m), LOC126267757 (ortholog of Hr51), LOC126272328 (ortholog of clu), LOC126336437 (ortholog of Dnaaf3), LOC126272601 (ortholog of mip40), LOC126298102 (ortholog of Mgat1), LOC126273258 (ortholog of CG9313), LOC126273102 (ortholog of CG9313), LOC126278694 (ortholog of Rae1), LOC126277905 (ortholog of nsr), LOC126336246 (ortholog of tous), LOC126333207 (ortholog of tous), LOC126335040 (ortholog of tous), LOC126334979 (ortholog of tous), LOC126324750 (ortholog of YME1L), LOC126334683 (ortholog of YME1L), LOC126281423 (ortholog of YME1L), LOC126278356 (ortholog of thoc5), LOC126267338 (ortholog of Rsph4a), LOC126299616 (ortholog of MAN1), LOC126355935 (ortholog of Dis3l2), LOC126281962 (ortholog of ttm2), LOC126305260 (ortholog of ttm2), LOC126299234 (ortholog of Pex10), LOC126354786 (ortholog of Strip), LOC126336262 (ortholog of Dnah3), LOC126355025 (ortholog of mmm), LOC126314431 (ortholog of t-Grip84), LOC126284129 (ortholog of Pex2), LOC126315416 (ortholog of Cyp40), LOC126298836 (ortholog of wls), LOC126297599 (ortholog of Duba), LOC126356197 (ortholog of twy), LOC126354158 (ortholog of Syx13), LOC126322110 (ortholog of CG5048), LOC126332406 (ortholog of ATPsynbetaL), LOC126348365 (ortholog of Wdr92), LOC126315288 (ortholog of wnd), LOC126311189 (ortholog of wnd), LOC126316981 (ortholog of wnd), LOC126325849 (ortholog of wnd), LOC126315289 (ortholog of wnd), LOC126268138 (ortholog of wnd), LOC126281655 (ortholog of Pex16), LOC126277985 (ortholog of eIF2A), LOC126356011 (ortholog of aux), LOC126267106 (ortholog of sac), LOC126278931 (ortholog of sac), LOC126291714 (ortholog of Sym), LOC126331235 (ortholog of ScsbetaA), LOC126295409 (ortholog of CG9920), LOC126282288 (ortholog of HtrA2), LOC126284497 (ortholog of Kpc1), LOC126267965 (ortholog of CG14305), LOC126320215 (ortholog of Syp), LOC126281235 (ortholog of Gld2), LOC126272020 (ortholog of bb8), LOC126297387 (ortholog of tbrd-1), LOC126365974 (ortholog of tbrd-1), LOC126336939 (ortholog of Root), LOC126293475 (ortholog of puf), LOC126284103 (ortholog of puf), LOC126316940 (ortholog of puf), LOC126266727 (ortholog of puf), LOC126315393 (ortholog of Cyt-c1L), LOC126279125 (ortholog of Cad99C), LOC126298732 (ortholog of klhl10), LOC126335797 (ortholog of klhl10), LOC126335765 (ortholog of klhl10), LOC126284384 (ortholog of rept), LOC126328615 (ortholog of pont), LOC126281586 (ortholog of DCTN5-p25), LOC126334534 (ortholog of Top3alpha), LOC126315376 (ortholog of Roc1b), LOC126299006 (ortholog of Myt1), LOC126336459 (ortholog of CG18675), LOC126355069 (ortholog of park), LOC126268112 (ortholog of ago), LOC126315382 (ortholog of Apl), LOC126266961 (ortholog of HBS1), LOC126335410 (ortholog of Fsn), LOC126292194 (ortholog of gskt), LOC126316708 (ortholog of Cog7), LOC126273104 (ortholog of CG31759), LOC126278920 (ortholog of Rsph9), LOC126271961 (ortholog of Mst27D), LOC126354384 (ortholog of Cap-D3), LOC126284445 (ortholog of Rsph3), LOC126279037 (ortholog of Erk7), LOC126268696 (ortholog of Gorab), LOC126272490 (ortholog of endos), LOC126298276 (ortholog of Lasp), LOC126283955 (ortholog of sif), LOC126354822 (ortholog of Myo31DF), LOC126284167 (ortholog of Mer), LOC126353756 (ortholog of pea), LOC126324509 (ortholog of Cyt-c-d), LOC126361248 (ortholog of snky), LOC126284352 (ortholog of psidin), LOC126325484 (ortholog of Chd1), LOC126330071 (ortholog of gish), LOC126314445 (ortholog of gish), LOC126354209 (ortholog of Mob4), LOC126315380 (ortholog of Nop60B), LOC126272345 (ortholog of Nop60B), LOC126284166 (ortholog of Su(var)3-3), LOC126318456 (ortholog of eIF4G2), LOC126354921 (ortholog of Diap1), LOC126354914 (ortholog of Diap1), LOC126299257 (ortholog of bond), LOC126338336 (ortholog of Vmat), LOC126297882 (ortholog of Hr39), LOC126318281 (ortholog of Cul3), LOC126298575 (ortholog of didum), LOC126297616 (ortholog of didum), LOC126356233 (ortholog of nej), LOC126282237 (ortholog of larp), LOC126334642 (ortholog of brun), LOC126271962 (ortholog of Bsg), LOC126319852 (ortholog of f), LOC126326545 (ortholog of VhaPPA1-2), LOC126297514 (ortholog of cic), LOC126272169 (ortholog of SmB), LOC126317467 (ortholog of SmB), LOC126354362 (ortholog of Myc), LOC126335298 (ortholog of Acn), LOC126326595 (ortholog of bel), LOC126322416 (ortholog of bel), LOC126338352 (ortholog of hts), LOC126284132 (ortholog of nclb), LOC126355427 (ortholog of lost), LOC126336912 (ortholog of Brr2), LOC126297728 (ortholog of Dark), LOC126336752 (ortholog of dally), LOC126361403 (ortholog of dally), LOC126278711 (ortholog of dally), LOC126278461 (ortholog of dally), LOC126278712 (ortholog of dally), LOC126336241 (ortholog of dally), LOC126336521 (ortholog of dally), LOC126344485 (ortholog of Dic61B), LOC126355504 (ortholog of Dic61B), LOC126305233 (ortholog of Cyt-b5), LOC126267354 (ortholog of mgr), LOC126297643 (ortholog of Mhc), LOC126329577 (ortholog of pAbp), LOC126291640 (ortholog of mlt), LOC126320750 (ortholog of Prosalpha4T1), LOC126335305 (ortholog of ProsalphadT1), LOC126341901 (ortholog of Doa), LOC126273183 (ortholog of stai), LOC126278527 (ortholog of Zmynd10), LOC126358244 (ortholog of mfr), LOC126293701 (ortholog of Sf3a1), LOC126272155 (ortholog of Topors), LOC126326031 (ortholog of JYalpha), LOC126326030 (ortholog of JYalpha), LOC126273201 (ortholog of sau), LOC126293211 (ortholog of lovit), LOC126285165 (ortholog of kl-3), LOC126335843 (ortholog of kl-3), LOC126335844 (ortholog of kl-3), LOC126335984 (ortholog of kl-5), LOC126278898 (ortholog of kl-5), LOC126354658 (ortholog of WDY), LOC126304735 (ortholog of WDY), LOC126278854 (ortholog of WDY), LOC126267456 (ortholog of ap), LOC126365881 (ortholog of Dhc16F), LOC126272079 (ortholog of InR), LOC126272765 (ortholog of Pall), LOC126337012 (ortholog of Rpp30), LOC126291679 (ortholog of Top2), LOC126354758 (ortholog of Top2), LOC126353942 (ortholog of knon), LOC126335010 (ortholog of shps), LOC126272161 (ortholog of TER94), LOC126356054 (ortholog of Orc1), LOC126295104 (ortholog of bor), LOC126327182 (ortholog of Pen), LOC126334615 (ortholog of zip), and LOC126298806 (ortholog of betaTub60D), LOC126278145 (ortholog of betaTub60D).In some aspects, an arthropod male-fertility essential gene comprises a nucleic acid sequence at least 80% identical to a nucleic acid sequence selected from the group consisting of SEQ ID NOs: 1 to 2879 and 2881 to 2902. In some aspects, an arthropod male-fertility essential gene comprises a nucleic acid sequence at least 85% identical to a nucleic acid sequence selected from the group consisting of SEQ ID NOs: 1 to 2879 and 2881 to 2902. In some aspects, an arthropod male-fertility essential gene comprises a nucleic acid sequence at least 90% identical to a nucleic acid sequence selected from the group consisting of SEQ ID NOs: 1 to 2879 and 2881 to 2902. In some aspects, an arthropod male-fertility essential gene comprises a nucleic acid sequence at least 91% identical to a nucleic acid sequence selected from the group consisting of SEQ ID NOs: 1 to 2879 and 2881 to 2902. In some aspects, an arthropod male-fertility essential gene comprises a nucleic acid sequence at least 92% identical to a nucleic acid sequence selected from the group consisting of SEQ ID NOs: 1 to 2879 and 2881 to 2902. In some aspects, an arthropod male-fertility essential gene comprises a nucleic acid sequence at least 93% identical to a nucleic acid sequence selected from the group consisting of SEQ ID NOs: 1 to 2879 and 2881 to 2902. In some aspects, an arthropod male-fertility essential gene comprises a nucleic acid sequence at least 94% identical to a nucleic acid sequence selected from the group consisting of SEQ ID NOs: 1 to 2879 and 2881 to 2902. In some aspects, an arthropod male-fertility essential gene comprises a nucleic acid sequence at least 95% identical to a nucleic acid sequence selected from the group consisting of SEQ ID NOs: 1 to 2879 and 2881 to 2902. In some aspects, an arthropod male-fertility essential gene comprises a nucleic acid sequence at least 96% identical to a nucleic acid sequence selected from the group consisting of SEQ ID NOs: 1 to 2879 and 2881 to 2902. In some aspects, an arthropod male-fertility essential gene comprises a nucleic acid sequence at least 97% identical to a nucleic acid sequence selected from the group consisting of SEQ ID NOs: 1 to 2879 and 2881 to 2902. In some aspects, an arthropod male-fertility essential gene comprises a nucleic acid sequence at least 98% identical to a nucleic acid sequence selected from the group consisting of SEQ ID NOs: 1 to 2879 and 2881 to 2902. In some aspects, an arthropod male-fertility essential gene comprises a nucleic acid sequence at least 99% identical to a nucleic acid sequence selected from the group consisting of SEQ ID NOs: 1 to 2879 and 2881 to 2902. In some aspects, an arthropod male-fertility essential gene comprises a nucleic acid sequence 100% identical to a nucleic acid sequence selected from the group consisting of SEQ ID NOs: 1 to 2879 and 2881 to 2902.

In some aspects, an arthropod male-fertility essential gene encodes an amino acid sequence at least 80% identical or similar to an amino acid sequence selected from the group consisting of SEQ ID NOs: 2880 and 4397 to 6493. In some aspects, an arthropod male-fertility essential gene encodes an amino acid sequence at least 85% identical or similar to an amino acid sequence selected from the group consisting of SEQ ID NOs: 2880 and 4397 to 6493. In some aspects, an arthropod male-fertility essential gene encodes an amino acid sequence at least 90% identical or similar to an amino acid sequence selected from the group consisting of SEQ ID NOs: 2880 and 4397 to 6493. In some aspects, an arthropod male-fertility essential gene encodes an amino acid sequence at least 91% identical or similar to an amino acid sequence selected from the group consisting of SEQ ID NOs: 2880 and 4397 to 6493. In some aspects, an arthropod male-fertility essential gene encodes an amino acid sequence at least 92% identical or similar to an amino acid sequence selected from the group consisting of SEQ ID NOs: 2880 and 4397 to 6493. In some aspects, an arthropod male-fertility essential gene encodes an amino acid sequence at least 93% identical or similar to an amino acid sequence selected from the group consisting of SEQ ID NOs: 2880 and 4397 to 6493. In some aspects, an arthropod male-fertility essential gene encodes an amino acid sequence at least 94% identical or similar to an amino acid sequence selected from the group consisting of SEQ ID NOs: 2880 and 4397 to 6493. In some aspects, an arthropod male-fertility essential gene encodes an amino acid sequence at least 95% identical or similar to an amino acid sequence selected from the group consisting of SEQ ID NOs: 2880 and 4397 to 6493. In some aspects, an arthropod male-fertility essential gene encodes an amino acid sequence at least 96% identical or similar to an amino acid sequence selected from the group consisting of SEQ ID NOs: 2880 and 4397 to 6493. In some aspects, an arthropod male-fertility essential gene encodes an amino acid sequence at least 97% identical or similar to an amino acid sequence selected from the group consisting of SEQ ID NOs: 2880 and 4397 to 6493. In some aspects, an arthropod male-fertility essential gene encodes an amino acid sequence at least 98% identical or similar to an amino acid sequence selected from the group consisting of SEQ ID NOs: 2880 and 4397 to 6493. In some aspects, an arthropod male-fertility essential gene encodes an amino acid sequence at least 99% identical or similar to an amino acid sequence selected from the group consisting of SEQ ID NOs: 2880 and 4397 to 6493. In some aspects, an arthropod male-fertility essential gene encodes an amino acid sequence 100% identical or similar to an amino acid sequence selected from the group consisting of SEQ ID NOs: 2880 and 4397 to 6493.

In some aspects, this disclosure provides a modified arthropod comprising a male sterile effector, wherein the male sterile effector comprises a loss-of-function mutation in at least one male-fertility essential gene positioned within a Y chromosome or within an M-locus. In some aspects, this disclosure provides a modified arthropod comprising a male sterile effector, wherein the male sterile effector comprises a loss-of-function mutation in at least one male-fertility essential gene positioned within a male sex-determining factor. In some aspects, the modified arthropod further comprises a rescue element comprising a functional allele of the at least one male-fertility essential gene. In some aspects, the modified arthropod further comprises a repressible female-lethal effector element. In some aspects, the modified arthropod comprises a male sterile effector encoding an anticas protein.

As used herein, a "loss-of-function" (or "LOF") mutation refers to a mutation that reduces or eliminates the normal function of a gene. In some aspects, a loss-of-function mutation reduces the expression of a gene as compared to a wildtype allele. In some aspects, a loss-of-function mutation eliminates the expression activity of a gene as compared to a wildtype allele. In some aspects, a loss-of-function mutation reduces the activity of a protein encoded by a gene as compared to a wildtype allele. In some aspects, a loss-of-function mutation eliminates the activity of a protein encoded by a gene as compared to a wildtype allele. In some aspects, a loss-of-function mutation comprises an insertion of at least one nucleotide as compared to a wildtype allele. In some aspects, a loss-of-function mutation comprises a deletion of at least one nucleotide as compared to a wildtype allele. In some aspects, a loss-of-function mutation comprises a substitution of at least one nucleotide as compared to a wildtype allele. In some aspects, a loss-of-function mutation comprises an inversion of at least two nucleotides as compared to a wildtype allele. In some aspects, a loss-of-function mutation removes an intron/exon splice site as compared to a wildtype allele. In some aspects, a loss-of-function mutation comprises a complete deletion of a wildtype allele. In some aspects, a loss-of-function mutation comprises an insertion into a coding sequence of a wildtype allele. In some aspects, an insertion into a coding sequence of a wildtype allele comprises the insertion of a transposon.

In some aspects, a loss-of-function mutation comprises one or more mutation types selected from the group consisting of a nonsense mutation, a missense mutation, a frameshift mutation, a splice-site mutation, and any combinations thereof. As used herein, a "nonsense mutation" refers to a mutation to a nucleic acid sequence that introduces a premature stop codon to an amino acid sequence by the nucleic acid sequence. As used herein, a "missense mutation" refers to a mutation to a nucleic acid sequence that causes a substitution within the amino acid sequence encoded by the nucleic acid sequence. As used herein, a "frameshift mutation" refers to an insertion or deletion to a nucleic acid sequence that shifts the frame for translating the nucleic acid sequence to an amino acid sequence. A "splice-site mutation" refers to a mutation in a nucleic acid sequence that causes an intron to be retained for protein translation, or, alternatively, for an exon to be excluded from protein translation. Splice-site mutations can cause nonsense, missense, or frameshift mutations.

Loss-of-function mutations in coding regions of genes (e.g., exonic mutations) can result in a truncated protein or polypeptide when a mutated messenger RNA (mRNA) is translated into a protein or polypeptide. In some aspects, this disclosure provides a loss-of-function mutation that results in the truncation of a protein or polypeptide as compared to a protein or polypeptide encoded by a wildtype allele. As used herein, a "truncated" protein or polypeptide comprises at least one fewer amino acid as compared to an endogenous control protein or polypeptide. For example, if endogenous Protein A comprises 100 amino acids, a truncated version of Protein A can comprise between 1 and 99 amino acids.

Without being limited by any scientific theory, one way to cause a protein or polypeptide truncation is by the introduction of a premature stop codon in an mRNA transcript of a male-fertility essential gene. In some aspects, this disclosure provides a mutation that results in a premature stop codon in an mRNA transcript of a male-fertility essential gene. As used herein, a "stop codon" refers to a nucleotide triplet within an mRNA transcript that signals a termination of protein translation. A "premature stop codon" refers to a stop codon positioned earlier (*e.g.,* on the 5'-side) than the normal stop codon position in a male-fertility essential gene mRNA transcript. Without being limiting, several stop codons are known in the art, including "UAG," "UAA," "UGA," "TAG," "TAA," and "TGA."

In some aspects, a loss-of-function mutation provided herein comprises a null mutation. As used herein, a "null mutation" refers to a mutation that confers a complete loss-of-function for a protein encoded by a gene comprising the mutation, or, alternatively, a mutation that confers a complete loss-of-function for a small RNA encoded by a genomic locus. A null mutation can cause lack of mRNA transcript production, a lack of small RNA transcript production, a lack of protein function, or a combination thereof.

A loss-of-function mutation provided herein can be positioned in any part of a male-fertility essential gene. In some aspects, a loss-of-function mutation provided herein is positioned within an exon of a male-fertility essential gene. In some aspects, a loss-of-function mutation provided herein is positioned within an intron of a male-fertility essential gene. In some aspects, a loss-of-function mutation provided herein is positioned within a coding sequence of the male-fertility essential gene. In some aspects, a loss-of-function mutation provided herein is positioned within a 5'-UTR of a gene. In some aspects, a loss-of-function mutation provided herein is positioned within a 3'-UTR of a male-fertility essential gene. In yet another aspect, a loss-of-function mutation provided herein is positioned within a promoter of a male-fertility essential gene. In some aspects, a mutation loss-of-function herein is positioned within a terminator of a male-fertility essential gene.

This disclosure provides an arthropod comprising any nucleic acid sequence provided herein. This disclosure provides an arthropod comprising any isolated nucleic acid molecule provided herein. This disclosure provides an arthropod comprising any amino acid sequence provided herein. This disclosure provides an arthropod comprising any male sterile provided herein. This disclosure provides an arthropod comprising any sterility system provided herein. This disclosure provides an arthropod comprising any repressible female-lethal effector provided herein. This disclosure provides an arthropod comprising any rescue element provided herein.

Any method provided herein is specifically envisioned for use with any arthropod. Any method provided herein is specifically envisioned for use with any arthropod provided herein. Any method provided herein is specifically envisioned for use with any modified arthropod provided herein. Any method provided herein is specifically envisioned for use with any insect. Any method provided herein is specifically envisioned for use with any insect provided herein. Any method provided herein is specifically envisioned for use with any modified insect provided herein. Any method provided herein is specifically envisioned for use with any arachnid. Any method provided herein is specifically envisioned for use with any arachnid provided herein. Any method provided herein is specifically envisioned for use with any modified arachnid provided herein.

As used herein, the term "modified" in reference to an arthropod (*e.g.,* any insect, any arachnid) refers to an arthropod comprising one or more genetic alterations introduced via human activity (*e.g.,* without being limiting, via introduction of nucleic acid molecules to an embryo) for certain purposes and beyond natural polymorphisms that exist in an unmodified wildtype counterpart arthropod.

In some aspects, an arthropod is a modified arthropod. In some aspects, an insect is a modified insect. In some aspects, an arachnid is a modified arachnid. It will be appreciated that any arthropod provided herein is also provided a

As used herein, "arthropod" refers to any member of the Phylum Arthropoda. In some aspects, an arthropod is an insect. Insects are members of the Class Insecta.

In some aspects, an insect is from the Order Ephemeroptera (*e.g.,* mayflies). In some aspects, an insect is from the Order Dermaptera (*e.g.,* earwigs). In some aspects, an insect is from the Order Orthoptera (*e.g.,* grasshoppers, crickets, katydids). In some aspects, an insect is from the Order Mantodea (e*.g.,* mantises). In some aspects, an insect is from the Order Blattodea (*e.g.,* cockroaches, termites). In some aspects, an insect is from the Order Psocodea (*e.g.,* book lice, bark lice, sucking lice). In some aspects, an insect is from the Order Hemiptera (*e.g.,* true bugs). In some aspects, an insect is from the Order Thysanoptera (*e.g.,* thrips). In some aspects, an insect is from the Order Hymenoptera (*e.g.,* sawflies, wasps, bees, ants). In some aspects, an insect is from the Order Coleoptera (e.g., beetles). In some aspects, an insect is from the Order Neuroptera (*e.g.,* lacewings). In some aspects, an insect is from the Order Lepidoptera (*e.g.,* butterflies, moths). In some aspects, an insect is from the Order Diptera (*e.g.*, true flies). In some aspects, an insect is from the Order Siphonaptera (e.g., fleas). In some aspects, an insect is from the order Zygentoma (*e.g.*, silverfish). In some aspects, an insect is selected from an Order selected from group consisting of Ephemeroptera, Dermaptera, Orthoptera, Mantodea, Blattodea, Psocodea, Hemiptera, Thysanoptera, Hymenoptera, Coleoptera, Neuroptera, Lepidoptera, Diptera, Siphonaptera, and Zygentoma.

In some aspects, an insect from the Order Lepidoptera is a Heliothinae species. Heliothinae is a subfamily of the Family Noctuidae and comprises the following genera: *Adisura, Aedophron, Australothis, Baptarma, Chloridea, Chazaria, Derrima, Eutricopis, Hebdomochondra, Helicoverpa, Heliocheilus, Heliolonche, Heliothis, Heliothodes, Melaporphyria, Micriantha, Microhelia, Periphanes, Protadisura, Psectrotarsia, Pyrocleptria, Pyrrhia, Rhodoecia, Schinia, Stenoecia,* and *Timora.*

In some aspects, a Dipteran insect is from the Family Culicidae (*e.g.,* mosquitoes). In some aspects, a Dipteran insect is from the Family Simuliidae (*e.g.,* black flies, buffalo gnats). In some aspects, a Dipteran insect is from the Family Ceratopogonidae (*e.g.,* biting midges). In some aspects, a Dipteran insect is from the Family Tabanidae (*e.g.,* horse flies, deer flies). In some aspects, a Dipteran insect is from the Family Muscidae (*e.g.,* house flies). In some aspects, a Dipteran insect is from the Family Scathophagidae (*e.g.,* dung flies). In some aspects, a Dipteran insect is from the Family Calliphoridae (*e.g.,* blowflies, carrion flies). In some aspects, a Dipteran insect is from the Family Oestridae (*e.g.,* botflies, warble flies). In some aspects, a Dipteran insect is from the Family Sarcophagidae (*e.g.,* flesh flies). In some aspects, a Dipteran insect is from the Family Drosophilidae (*e.g.,* fruit flies). Dipteran insects are insects of the Order Diptera.

In some aspects, a Dipteran insect is of the genus *Aedes.* In some aspects, a Dipteran insect is of the gens *Anopheles.* In some aspects, a Dipteran insect is of the genus *Culex.* In some aspects, a Dipteran insect is of the genus *Anastrepha,* insect is of the genus *Culex.* In some aspects, a Dipteran insect is of the genus *Drosophila.* In some aspects, a Dipteran insect is of the genus *Bactrocera.* In some aspects, a Dipteran insect is of the genus *Ceratitis.* In some aspects, a Dipteran insect is of the genus *Cochliomyia* In some aspects, a Dipteran insect is of the genus *Tuta.* In some aspects, a Dipteran insect is of the genus *Anastrepha.* In some aspects, a Dipteran insect is of the genus *Glossina.* In some aspects, a Dipteran is of the genus *Lytzomyia.*

In some aspects, an insect is an *Aedes aegypti* mosquito. In some aspects, an insect is a *Drosophila suzukii* fly. In some aspects, an insect is an *Aedes albopictus* mosquito. In some aspects, an insect is an *Anopheles gambiae* mosquito. In some aspects, an insect is a *Bactrocera dorsalis* fly. In some aspects, an insect is a *Ceratitis capitata* fly. In some aspects, an insect is a *Cochliomyia hominivorax* fly.In some aspects, an arthropod is an arachnid. Arachnids are members of the Class Arachnida. In some aspects, an arachnid is from the Order Acariformes (*e.g.,* mites). In some aspects, an arachnid is from the Order Parasitiformes (*e.g.,* ticks, predatory mites, parasitic mites).

In some aspects, an arachnid is *Ixodes scapularis.* In some aspects, an arachnid is *Tertanychus urticae.* In some aspects, an arachnid is *Varroa destructor.* In some aspects, an arachnid is selected from the group consisting of *Tertanychus urticae* and *Varroa destructor.* In some aspects, an arachnid is selected from the group consisting of *Ixodes scapularis, Tertanychus urticae,* and *Varroa destructor.*

In some aspects, an arthropod is selected from the group consisting of *Acyrthosiphon pisum, Adelges tsugae, Aedes aegypti, Aedes mascarensis, Aedes albopictus, Agrilus planipennis, Amyelois transitella, Anarsia lineatella, Anastrepha ludens, Anastrepha obliqua, Anastrepha suspensa, Anopheles arabiensis, Anopheles coluzii, Anopheles funestus, Anopheles gambiae, Anopheles stephensi, Anoplophora glabripennis, Anthonomus grandis, Aonidiella aurantia, Aphis gossypii, Apis mellifera scutellata, Bactrocera cucurbitae, Bactrocera dorsalis, Bactrocera oleae, Bactrocera tyroni, Bactrocera zonata, Bagrada hilaris, Bemisia tabaci, Bombyx mori, Callosobruchus maculatus, Ceratitis capitata, Ceratitis rosa, Chrysomya bezziana, Cochliomyia aldrichi, Cochliomyia hominivorax, Cochliomyia macellaria, Cochliomyia minima, Coptotermes formosanus, Culex quinquefasciatus, Cydia pomonella, Dalbulus maidis, Dendroctonus frontalis, Dermatobia hominis, Diabrotica balteata, Diabrotica barberi, Diabrotica undecimpunctata, Diabrotica virgifera, Diaphorina citri, Drosophila suzukii, Epiphyas postvittana, Ephestia elutella, Frankliniella occidentalis, Glossina austeni, Glossina brevipalpis, Glossina fusca, Glossina fuscipes, Glossina fuscipleuris, Glossina longipalpis, Glossina longipennis, Glossina morsitans, Glossina pallidipes, Glossina palpalis, Glossina swynnertoni, Glossina tabaniformis, Glossina tachinoides, Graphocephala atropunctata, Haematobia irritans, Halyomorpha halys, Helicoverpa armigera, Helicoverpa zea, Homalodisca vitripennis, Hypoderma bovis, Hypoderma lineatum, Ixodes scapularis, Leptinotarsa decemlineata, Leucinodes orbonalis, Lobesia botrana, Lucilia cuprina, Lutzomyia spp., Lycorma delicatula, Lymantria albescens, Lymantria dispar, Lymantria dispar asiatica, Lymantria dispar japonica, Lymantria postalba, Lymantria umbrosa, Musca domestica, Myzus persicae, Naupactus spp. (Graphognatus spp.), Nilaparvata lugens, Oestridae spp., Oryctes rhinoceros, Pectinophora gossypiella, Pectinophora scutigera, Pityophthorus juglandis, Planococcus ficus, Plutella xylostella, Popillia japonica, Rhagoletis cerasi, Rhodnius prolixus, Schistocerca gregaria, Selenothrips rubrocinctus, Sirex noctilio, Solenopsis invicta, Solenopsis richteri, Spodoptera frugiperda, Spodoptera litura, Spodotera exigua, Tabanus sulcifrons, Thaumatotibia leucotreta, Thrips tabaci, Tribolium castaneum, Trogoderma granarium, Tryporyza incertulas, Tuta absoluta,* and *Varroa destructor.* Table 2 provides common names for each of the arthropods listed in this paragraph.

**Table 2. Arthropod species.**

| **Latin Name** | **Common Name** | **Order** |
|---|---|---|
| *Acyrthosiphon pisum* | Pea Aphid | Hemiptera |
| *Adelges tsugae* | Hemlock Woolly Adelgid | Hemiptera |
| *Aedes aegypti* | Yellow Fever Mosquito | Diptera |
| *Aedes albopictus* | Tiger Mosquito | Diptera |
| *Aedes mascarensis* | Asian tiger mosquito | Diptera |
| *Agrilus planipennis* | Emerald Ash Borer | Coleoptera |
| *Amyelois transitella* | Navel Orange Worm | Lepidoptera |
| *Anarsia lineatella* | Peach Twig Borer | Lepidoptera |
| *Anastrepha ludens* | Mexican fruit fly | Diptera |
| *Anastrepha obliqua* | West Indian Fruit Fly | Diptera |
| *Anastrepha suspensa* | Caribbean Fruit Fly | Diptera |
| *Anopheles arabiensis* | Malaria Mosquito | Diptera |
| *Anopheles coluzii* | Malaria Mosquito | Diptera |
| *Anopheles funestus* | Malaria Mosquito | Diptera |
| *Anopheles gambiae* | African Malaria Mosquito | Diptera |
| *Anopheles stephensi* | Asian Malaria Mosquito | Diptera |
| *Anoplophora glabripennis* | Asian Longhomed Beetle | Coleoptera |
| *Anthonomus grandis* | Cotton Boll Weevil | Coleoptera |
| *Aonidiella aurantia* | Red Scale | Hemiptera |
| *Aphis gossypii* | Cotton Aphid | Hemiptera |
| *Apis mellifera scutellata* | Africanized Honeybee | Hymenoptera |
| *Bactrocera cucurbitae* | Melon Fly | Diptera |
| *Bactrocera dorsalis* | Oriental Fruit Fly | Diptera |
| *Bactrocera oleae* | Olive Fruit Fly | Diptera |
| *Bactrocera tyroni* | Queensland Fruit Fly | Diptera |
| *Bactrocera zonata* | Peach Fruit Fly | Diptera |
| *Bagrada hilaris* | Bagrada Bug | Hemiptera |
| *Bemisia tabaci* | Sweetpotato Whitefly | Hemiptera |
| *Bombyx mori* | Silkworm | Lepidoptera |
| *Callosobruchus maculatus* | Cowpea Weevil | Coleoptera |
| *Ceratitis capitata* | Medfly | Diptera |
| *Ceratitis rosa* | Natal Fruit Fly | Diptera |
| *Chrysomya bezziana* | Old World Screwworm | Diptera |
| *Cochliomyia aldrichi* | Screwworm Fly | Diptera |
| *Cochliomyia hominivorax* | New World Screwworm | Diptera |
| *Cochliomyia macellaria* | Screwworm Fly | Diptera |
| *Cochliomyia minima* | Screwworm Fly | Diptera |
| *Coptotermes formosanus* | Termite | Blattodea |
| *Culex quinquefasciatus* | Southern House Mosquito | Diptera |
| *Cydia pomonella* | Codling Moth | Lepidoptera |
| *Dalbulus maidis* | Corn Leafhopper | Hemiptera |
| *Dendroctonus frontalis* | Southern Pine Beetle | Coleoptera |
| *Dermatobia hominis* | Botfly | Diptera |
| *Diabrotica balteata* | Northern corn rootworm | Coleoptera |
| *Diabrotica barberi* | Northern corn rootworm | Coleoptera |
| *Diabrotica undecimpunctata* | Southern corn rootworm | Coleoptera |
| *Diabrotica virgifera* | Western Corn Rootworm | Coleoptera |
| *Diaphorina citri* | Asian Citrus Psyllid | Hemiptera |
| *Drosophila suzukii* | Spotted Wing Drosophila | Diptera |
| *Ephestia elutella* | Cocoa moth | Lepidoptera |
| *Epiphyas postvittana* | Light Brown Apple Moth | Lepidoptera |
| *Frankliniella occidentalis* | Western Flower Thrips | Thysanoptera |
| *Glossina spp* | Tsetse Fly | Diptera |
| *Graphocephala atropunctata* | Bluegreen Sharpshooter | Hemiptera |
| *Graphognatus spp.* | White-Fringed Beetle | Coleoptera |
| *Haematobia irritans* | Horn Fly | Diptera |
| *Halyomorpha halys* | Brown Marmorated Stink Bug | Hemiptera |
| *Helicoverpa armigera* | Cotton Bollworm | Lepidoptera |
| *Helicoverpa zea* | Corn Earworm | Lepidoptera |
| *Homalodisca vitripennis* | Glassy Winged Sharpshooter | Hemiptera |
| *Hypoderma bovis* | Warble Fly | Diptera |
| *Hypoderma lineatum* | Warble Fly | Diptera |
| *Ixodes scapularis* | Deer Tick or Black-legged Tick | Ixodida |
| *Leptinotarsa decemlineata* | Colorado Potato Beetle | Coleoptera |
| *Leucinodes orbonalis* | Fruit And Shoot Borer | Lepidoptera |
| *Lobesia botrana* | European Grapevine Moth | Lepidoptera |
| *Lucilia cuprina* | Australian Sheep Blowfly | Diptera |
| *Lutzomyia spp.* | Sandfly | Diptera |
| *Lycorma delicatula* | Spotted Lantemfly | Hemiptera |
| *Lymantria albescens* | Asian Gypsy Moth | Lepidoptera |
| *Lymantria dispar* | Gypsy Moth | Lepidoptera |
| *Lymantria dispar asiatica* | Asian Gypsy Moth | Lepidoptera |
| *Lymantria dispar japonica* | Asian Gypsy Moth | Lepidoptera |
| *Lymantria postalba* | Asian Gypsy Moth | Lepidoptera |
| *Lymantria umbrosa* | Asian Gypsy Moth | Lepidoptera |
| *Musca domestica* | House Fly | Diptera |
| *Myzus persicae* | Green Peach Aphid | Hemiptera |
| *Nilaparvata lugens* | Brown Planthopper | Lepidoptera |
| *Oestridae spp.* | Botfly | Diptera |
| *Oryctes rhinoceros* | Coconut Rhinoceros Beetle | Coleoptera |
| *Pectinophora gossypiella* | Pink Bollworm | Lepidoptera |
| *Pectinophora scutigera* | Pink-Spotted Bollworm | Lepidoptera |
| *Pityophthorus juglandis* | Walnut Twig Beetle | Coleoptra |
| *Planococcus ficus* | Vine Mealybug | Hemiptera |
| *Plutella xylostella* | Diamondback Moth | Lepidoptera |
| *Popillia japonica* | Japanese Beetle | Coleoptera |
| *Rhagoletis cerasi* | Cherry Fruit Fly | Diptera |
| *Rhodnius prolixus* | Kissing Bug | Hemiptera |
| *Schistocerca gregaria* | Desert Locust | Orthoptera |
| *Selenothrips rubrocinctus* | Redbanded thrip | Thysanoptera |
| *Sirex noctilio* | European Wood Wasp | Hymenoptera |
| *Solenopsis invicta* | Fire Ant | Hymenoptera |
| *Solenopsis richteri* | Fire Ant | Hymenoptera |
| *Spodoptera frugiperda* | Fall armyworm | Lepidoptera |
| *Spodoptera litura* | Taro Caterpillar | Lepidoptera |
| *Spodotera exigua* | Beet Armyworm | Lepidoptera |
| *Tabanus sulcifrons* | Horse Fly | Diptera |
| *Tertanychus urticae* | Two Spotted Spider Mite | Trombidiformes |
| *Thaumatotibia leucotreta* | False Codling Moth | Lepidoptera |
| *Thrips tabaci* | Onion Thrips | Thysanoptera |
| *Thysanoptera spp.* | Avocado Thrip | Thysanoptera |
| *Tribolium castaneum* | Red Flour Beetle | Coleoptera |
| *Trogoderma granarium* | Khapra Beetle | Coleoptera |
| *Tryporyza incertulas* | Rice Stem Borer | Lepidoptera |
| *Tuta absoluta* | Tomato Leafminer | Lepidoptera |
| *Varroa destructor* | Honeybee Mite | Mesostigmata |

In some aspects, an arthropod is selected from the group consisting of *Acyrthosiphon pisum, Adelges tsugae, Aedes aegypti, Aedes mascarensis, Aedes albopictus, Agrilus planipennis, Anastrepha ludens, Anastrepha obliqua, Anastrepha suspensa, Anopheles arabiensis, Anopheles coluzii, Anopheles funestus, Anopheles gambiae, Anopheles stephensi, Anoplophora glabripennis, Anthonomus grandis, Aonidiella aurantia, Aphis gossypii, Apis mellifera scutellata, Bactrocera cucurbitae, Bactrocera dorsalis, Bactrocera oleae, Bactrocera tyroni, Bactrocera zonata, Bagrada hilaris, Bemisia tabaci, Callosobruchus maculatus, Ceratitis capitata, Ceratitis rosa, Chrysomya bezziana, Cochliomyia aldrichi, Cochliomyia hominivorax, Cochliomyia macellaria, Cochliomyia minima, Coptotermes formosanus, Culex quinquefasciatus, Dalbulus maidis, Dendroctonus frontalis, Dermatobia hominis, Diabrotica balteata, Diabrotica barberi, Diabrotica undecimpunctata, Diabrotica virgifera, Diaphorina citri, Drosophila suzukii, Frankliniella occidentalis, Glossina austeni, Glossina brevipalpis, Glossina fusca, Glossina fuscipes, Glossina fuscipleuris, Glossina longipalpis, Glossina longipennis, Glossina morsitans, Glossina pallidipes, Glossina palpalis, Glossina swynnertoni, Glossina tabaniformis, Glossina tachinoides, Graphocephala atropunctata, Haematobia irritans, Halyomorpha halys, Homalodisca vitripennis, Hypoderma bovis, Hypoderma lineatum, Ixodes scapularis, Leptinotarsa decemlineata, Lucilia cuprina, Lutzomyia spp., Lycorma delicatula, Musca domestica, Myzus persicae, Naupactus spp. (Graphognatus spp.), Oestridae spp., Oryctes rhinoceros, Pityophthorus juglandis Planococcus ficus, Popillia japonica, Rhagoletis cerasi, Rhodnius prolixus, Schistocerca gregaria, Sirex noctilio, Solenopsis invicta, Solenopsis richteri, Tabanus sulcifrons, hrips tabaci, Tribolium castaneum, Trogoderma granarium,* and *Varroa destructor.*

In some aspects, this disclosure provides a cell of any arthropod provided herein. In some aspects, this disclosure provides an embryo of any arthropod provided herein.

Sex chromosomes are chromosomes that determine the sex of an individual animal, and they typically differ from autosomal chromosomes (or "autosomes") in their form, size, and behavior. Sex chromosomes are often referred to as X chromosomes and Y chromosomes. Typically, an organism with two X chromosomes is a female, and an organism with one X chromosome and one Y chromosome is a male, known as an XX/XY sex determination system. In some species the Y chromosome is a pseudo-Y chromosome or a homomorphic sex chromosome comprising a male-determining M-locus. In some species, an organism with two X chromosomes is a female, and an organism with one X chromosome and no Y chromosome is a male, known as an XX/XO sex determination system, and such sex determination systems are commonly found in Hemiptera, Orthoptera and Blattodea. In some species, there a multiple X chromosomes, and such sex determination systems are commonly found in ticks. Collectively, these may be referred to as XY sex determination systems and are found in most Diptera, Orthoptera, Coleoptera, Odonata, and Arachnids including ticks. *See,* for example, Blackmon et al., J Hered., 108:78-93 (2016). Without being limiting, the 2GEN system is compatible with XY sex determination systems.

In some aspects, an arthropod is a male arthropod. In some aspects, an arthropod cell is a male arthropod cell. Unless otherwise specified, a male arthropod (or arthropod cell) comprises one X chromosome (or m-locus) and one Y chromosome (or M-locus) in its genome, or one X chromosome (or m-locus) and no Y chromosome in its genome. In some aspects, a male arthropod comprises a male sex-determining factor. As used herein, a "male sex-determining factor" refers to a region within a genome (which can range from a single locus encoding a single transcript to a region of a chromosome encoding multiple different transcripts, to an entire chromosome) that results in the sex of an organism being male when present. Female arthropods lack a male sex-determining factor. In some aspects, a male sex-determining factor is a Y chromosome. In some aspects, a male sex-determining factor is an M-locus. In some aspects, a male-fertility essential gene is positioned within a Y chromosome. In some aspects, a male-fertility essential gene is positioned within an M-locus. In some aspects, a male-fertility essential gene is positioned within a male sex-determining factor. In some aspects, a male-fertility essential gene is positioned within an autosome. In some aspects, a male-fertility essential gene is positioned within an X chromosome.

In some aspects, an arthropod is a female arthropod. In some aspects, an arthropod cell is a female arthropod cell. Unless otherwise specified, a female arthropod (or arthropod cell) comprises one or two X chromosomes (or m-loci) in its genome and no Y chromosome. A female arthropod (or arthropod cell) does not comprise a Y chromosome (or M-locus) in its genome.

Although those of ordinary skill in the art will recognize that a Y chromosome and an M-locus are not identical structures and can comprise different sequences/genes, for the purposes of this disclosure they are treated as analogous structures that impart male characteristics to an organism when present. *See,* for example, Hall et al., Science, 348(6240):1268-1270 (2015). Similarly, an m-locus is treated as analogous to an X chromosome herein.

In some aspects, a male sterile effector is positioned within a Y chromosome. In some aspects, a male sterile effector is positioned within an M-locus. In some aspects, a male sterile effector is positioned within an autosome.

In some aspects, a male sterile effector is positioned within 0.5 centimorgans (cM) of an M-locus. In some aspects, a male sterile effector is positioned within 1 cM of an M-locus. In some aspects, a male sterile effector is positioned within 2 cM of an M-locus. In some aspects, a male sterile effector is positioned within 3 cM of an M-locus. In some aspects, a male sterile effector is positioned within 4 cM of an M-locus. In some aspects, a male sterile effector is positioned within 5 cM of an M-locus.

In some aspects, a male sterile effector is positioned within 100 kilobase pairs (kbp) of an M-locus. In some aspects, a male sterile effector is positioned within 50 kbp of an M-locus. In some aspects, a male sterile effector is positioned within 25 kbp of an M-locus. In some aspects, a male sterile effector is positioned within 10 kbp of an M-locus. In some aspects, a male sterile effector is positioned within 250 kbp of an M-locus.

In some aspects, a male sterile effector is positioned within 250 kbp of the male-determining gene *Nix* in an arthropod species selected from the group consisting of *Aedes aegypti* and *Aedes albopictus.* In some aspects, a male sterile effector is positioned within 100 kbp of the male-determining gene *Nix* in an arthropod species selected from the group consisting of *Aedes aegypti* and *Aedes albopictus.* In some aspects, a male sterile effector is positioned within 50 kbp of the male-determining gene *Nix* in an arthropod species selected from the group consisting of *Aedes aegypti* and *Aedes albopictus.* In some aspects, a male sterile effector is positioned within 25 kbp of the male-determining gene *Nix* in an arthropod species selected from the group consisting of *Aedes aegypti* and *Aedes albopictus.* In some aspects, a male sterile effector is positioned within 10 kbp of the male-determining gene *Nix* in an arthropod species selected from the group consisting of *Aedes aegypti* and *Aedes albopictus.*

In some aspects, a male sterile effector is positioned within 5 cM of the male-determining gene *Nix* in an arthropod species selected from the group consisting of*Aedes aegypti* and *Aedes albopictus.* In some aspects, a male sterile effector is positioned within 4 cM of the male-determining gene *Nix* in an arthropod species selected from the group consisting of *Aedes aegypti* and *Aedes albopictus.* In some aspects, a male sterile effector is positioned within 3 cM of the male-determining gene *Nix* in an arthropod species selected from the group consisting of *Aedes aegypti* and *Aedes albopictus.* In some aspects, a male sterile effector is positioned within 2 cM of the male-determining gene *Nix* in an arthropod species selected from the group consisting of *Aedes aegypti* and *Aedes albopictus.* In some aspects, a male sterile effector is positioned within 1 cM of the male-determining gene *Nix* in an arthropod species selected from the group consisting of *Aedes aegypti* and *Aedes albopictus.* In some aspects, a male sterile effector is positioned within 0.5 cM of the male-determining gene *Nix* in an arthropod species selected from the group consisting of *Aedes aegypti* and *Aedes albopictus.*

In some aspects, a rescue element is positioned within an X chromosome. In some aspects, a rescue element is positioned within an m-locus. In some aspects, a rescue element is positioned within an autosome.

In some aspects, a repressible female-lethal effector is positioned within an X chromosome. In some aspects, a repressible female-lethal effector is positioned within an m-locus. In some aspects, repressible female-lethal effector is positioned within an autosome.

In some aspects, a repressible female-lethal effector and a rescue element are positioned together at a single locus within an X chromosome. In some aspects, a repressible female-lethal effector and a rescue element are positioned together at a single locus within an m-locus. In some aspects, a repressible female-lethal effector and a rescue element are positioned together at a single locus within an autosome.

In some aspects, a rescue element and a male sterile effector are positioned together at a single locus within an m-locus. In some aspects, a repressible female-lethal effector and a male sterile effector are positioned together at a single locus within an autosome. In some aspects, a repressible female-lethal effector and a rescue element and a male sterile effector are positioned together at a single locus within an m-locus.

In some aspects, a rescue element is positioned within 0.5 cM of an m-locus. In some aspects, a rescue element is positioned within 1 cM of an m-locus. In some aspects, a rescue element is positioned within 2 cM of an m-locus. In some aspects, a rescue element is positioned within 3 cM of an m-locus. In some aspects, a rescue element is positioned within 4 cM of an m-locus. In some aspects, a rescue element is positioned within 5 cM of an m-locus.

In some aspects, a rescue element is positioned within 100 kbp of an m-locus. In some aspects, a rescue element is positioned within 50 kbp of an m-locus. In some aspects, a rescue element is positioned within 25 kbp of an m-locus. In some aspects, a rescue element is positioned within 10 kbp of an m-locus. In some aspects, a rescue element is positioned within 250 kbp of an m-locus.

In some aspects, an *Aedes aegypti* m-locus is positioned within 250 kbp of a locus selected from the group consisting of: AAEL019619, AAEL006712, AAEL022649, AAEL014760, AAEL020950, AAEL021928, AAEL027507, AAEL019917, AAEL006708, AAEL006707, AAEL006713, AAEL006712, AAEL019921, AAEL025609, AAEL005160, AAEL005169, AAEL009784, AAEL001236, AAEL002407, AAEL014760, AAEL028070, AAEL015064, and AAEL019619. In some aspects, an *Aedes aegypti* m-locus is positioned within 100 kbp of a locus selected from the group consisting of: AAEL019619, AAEL006712, AAEL022649, AAEL014760, AAEL020950, AAEL021928, AAEL027507, AAEL019917, AAEL006708, AAEL006707, AAEL006713, AAEL006712, AAEL019921, AAEL025609, AAEL005160, AAEL005169, AAEL009784, AAEL001236, AAEL002407, AAEL014760, AAEL028070, AAEL015064, and AAEL019619. In some aspects, an *Aedes aegypti* m-locus is positioned within 50 kbp of a locus selected from the group consisting of: AAEL019619, AAEL006712, AAEL022649, AAEL014760, AAEL020950, AAEL021928, AAEL027507, AAEL019917, AAEL006708, AAEL006707, AAEL006713, AAEL006712, AAEL019921, AAEL025609, AAEL005160, AAEL005169, AAEL009784, AAEL001236, AAEL002407, AAEL014760, AAEL028070, AAEL015064, and AAEL019619. In some aspects, an *Aedes aegypti* m-locus is positioned within 25 kbp of a locus selected from the group consisting of: AAEL019619, AAEL006712, AAEL022649, AAEL014760, AAEL020950, AAEL021928, AAEL027507, AAEL019917, AAEL006708, AAEL006707, AAEL006713, AAEL006712, AAEL019921, AAEL025609, AAEL005160, AAEL005169, AAEL009784, AAEL001236, AAEL002407, AAEL014760, AAEL028070, AAEL015064, and AAEL019619. In some aspects, an *Aedes aegypti* m-locus is positioned within 10 kbp of a locus selected from the group consisting of: AAEL019619, AAEL006712, AAEL022649, AAEL014760, AAEL020950, AAEL021928, AAEL027507, AAEL019917, AAEL006708, AAEL006707, AAEL006713, AAEL006712, AAEL019921, AAEL025609, AAEL005160, AAEL005169, AAEL009784, AAEL001236, AAEL002407, AAEL014760, AAEL028070, AAEL015064, and AAEL019619.

In some aspects, an *Aedes aegypti* m-locus is positioned within 5 cM of a locus selected from the group consisting of: AAEL019619, AAEL006712, AAEL022649, AAEL014760, AAEL020950, AAEL021928, AAEL027507, AAEL019917, AAEL006708, AAEL006707, AAEL006713, AAEL006712, AAEL019921, AAEL025609, AAEL005160, AAEL005169, AAEL009784, AAEL001236, AAEL002407, AAEL014760, AAEL028070, AAEL015064, and AAEL019619. In some aspects, an *Aedes aegypti* m-locus is positioned within 4 cM of a locus selected from the group consisting of: AAEL019619, AAEL006712, AAEL022649, AAEL014760, AAEL020950, AAEL021928, AAEL027507, AAEL019917, AAEL006708, AAEL006707, AAEL006713, AAEL006712, AAEL019921, AAEL025609, AAEL005160, AAEL005169, AAEL009784, AAEL001236, AAEL002407, AAEL014760, AAEL028070, AAEL015064, and AAEL019619. In some aspects, an *Aedes aegypti* m-locus is positioned within 3 cM of a locus selected from the group consisting of: AAEL019619, AAEL006712, AAEL022649, AAEL014760, AAEL020950, AAEL021928, AAEL027507, AAEL019917, AAEL006708, AAEL006707, AAEL006713, AAEL006712, AAEL019921, AAEL025609, AAEL005160, AAEL005169, AAEL009784, AAEL001236, AAEL002407, AAEL014760, AAEL028070, AAEL015064, and AAEL019619. In some aspects, an *Aedes aegypti* m-locus is positioned within 2 cM of a locus selected from the group consisting of: AAEL019619, AAEL006712, AAEL022649, AAEL014760, AAEL020950, AAEL021928, AAEL027507, AAEL019917, AAEL006708, AAEL006707, AAEL006713, AAEL006712, AAEL019921, AAEL025609, AAEL005160, AAEL005169, AAEL009784, AAEL001236, AAEL002407, AAEL014760, AAEL028070, AAEL015064, and AAEL019619. In some aspects, an *Aedes aegypti* m-locus is positioned within 1 cM of a locus selected from the group consisting of: AAEL019619, AAEL006712, AAEL022649, AAEL014760, AAEL020950, AAEL021928, AAEL027507, AAEL019917, AAEL006708, AAEL006707, AAEL006713, AAEL006712, AAEL019921, AAEL025609, AAEL005160, AAEL005169, AAEL009784, AAEL001236, AAEL002407, AAEL014760, AAEL028070, AAEL015064, and AAEL019619. In some aspects, an *Aedes aegypti* m-locus is positioned within 0.5 cM of a locus selected from the group consisting of: AAEL019619, AAEL006712, AAEL022649, AAEL014760, AAEL020950, AAEL021928, AAEL027507, AAEL019917, AAEL006708, AAEL006707, AAEL006713, AAEL006712, AAEL019921, AAEL025609, AAEL005160, AAEL005169, AAEL009784, AAEL001236, AAEL002407, AAEL014760, AAEL028070, AAEL015064, and AAEL019619.

In some aspects, a *Drosophila suzukii* m-locus is positioned within 250 kbp of a locus selected from the group consisting of: LOC108015366 and LOC108016595. In some aspects, a *Drosophila suzukii* m-locus is positioned within 100 kbp of a locus selected from the group consisting of: LOC108015366 and LOC108016595. In some aspects, a *Drosophila suzukii* m-locus is positioned within 50 kbp of a locus selected from the group consisting of: LOC108015366 and LOC108016595. In some aspects, a *Drosophila suzukii* m-locus is positioned within 25 kbp of a locus selected from the group consisting of: LOC108015366 and LOC108016595. In some aspects, a *Drosophila suzukii* m-locus is positioned within 10 kbp of a locus selected from the group consisting of: LOC108015366 and LOC108016595.

In some aspects, a *Drosophila suzukii* m-locus is positioned within 5 cM of a locus selected from the group consisting of: LOC108015366 and LOC108016595. In some aspects, a *Drosophila suzukii* m-locus is positioned within 4 cM of a locus selected from the group consisting of: LOC108015366 and LOC108016595. In some aspects, a *Drosophila suzukii* m-locus is positioned within 3 cM of a locus selected from the group consisting of: LOC108015366 and LOC108016595. In some aspects, a *Drosophila suzukii* m-locus is positioned within 2 cM of a locus selected from the group consisting of: LOC108015366 and LOC108016595. In some aspects, a *Drosophila suzukii* m-locus is positioned within 1 cM of a locus selected from the group consisting of: LOC108015366 and LOC108016595. In some aspects, a *Drosophila suzukii* m-locus is positioned within 0.5 cM of a locus selected from the group consisting of: LOC108015366 and LOC108016595.

Insects develop through several life stages before reaching sexually mature adults capable of mating and producing progeny (*e.g.,* eggs). Most insects begin their development as an egg, and undergo several stages of metamorphosis before reaching an adult stage capable of sexual reproduction. Some insects are holometabolous insects that develop through distinct egg, larval, pupal, and adult stages. In some aspects, holometabolous insects include Coleoptera (beetles), Hymenoptera (ants, wasps and sawflies), Lepidoptera (butterflies and moths) and Diptera (flies and mosquitoes). Other insects are ametabolous insects that undergo incomplete metamorphosis, while others are hemimetabolous insects that undergo incomplete metamorphosis, developing through juvenile stages called nymphs. In some aspects, hemimetabolous insects include Hemiptera (e.g. true bugs and aphids), Thysanoptera (thrips), and Orthoptera (e.g. grasshoppers). A minority of insects are viviparous that do not lay eggs, such as aphids. Other insects are ovoviviparous that produce eggs that hatch inside the mother, such as Tsetse flies (Glossina spp.). *See,* for example, Truman and Riddiford, Philosophical Transactions of the Royal Society B, 374:20190070 (2019).

Arachnids typically have a three-stage life cycle. Like insects, arachnids begin their development as an embryo within in egg. After hatching into a spiderling or nymph, the arachnid molts several times as they grow before reaching adult stage, where the arachnid is capable of reproducing.

In some aspects, an arthropod is an embryonic arthropod. It will be appreciated that an embryonic arthropod can be encased in an egg. Therefore, an egg comprises an embryonic arthropod. In some aspects, an arthropod is a larval arthropod. In some aspects, an arthropod is a pupal arthropod. In some aspects, an arthropod is an adult arthropod. In some aspects, an arthropod is a nymph arthropod.

In some aspects, an arthropod is an egg-laying arthropod. In some aspects, an arthropod is an ovoviviparous arthropod. In some aspects, an arthropod is a holometabolous insect. In some aspects, an arthropod is a hemimetabolous insect. In some aspects, an arthropod is an ametabolous insect.

In some aspects, an insect is an embryonic insect. In some aspects, an insect is a larval insect. In some aspects, an insect is a pupal insect. In some aspects, an insect is an adult insect. In some aspects, an insect is selected from the group consisting of an embryonic insect, a larval insect, a pupal insect, and an adult insect.

In some aspects, an arachnid is an embryonic arachnid. In some aspects, an arachnid is a nymph arachnid. In some aspects, an arachnid is an adult arachnid. In some aspects, an arachnid is selected from the group consisting of an embryonic arachnid, a nymph arachnid, and an adult arachnid.

In some aspects, an arthropod is a wildtype arthropod. In some aspects, an arthropod is a wildtype insect. In some aspects, an arthropod is a wildtype arachnid. In contrast to modified arthropods, wildtype arthropods are arthropods that have not been subjected to the modification processes that generate modified arthropods (*e.g.,* they lack the modifications present in corresponding modified arthropods). In some aspects, a wildtype arthropod is an adult arthropod. In some aspects, a wildtype female arthropod is an adult arthropod.

In some aspects, this disclosure provides a method of generating a population of progeny arthropods, the method comprising: (a) providing a male modified arthropod comprising any sterility system provided herein; and (b) allowing the male modified arthropod to mate with a wildtype female arthropod of the same species to produce the population of progeny arthropods, wherein the population of progeny arthropods comprises: (i) at least one sterile male progeny arthropod; (ii) at least one inviable female progeny arthropod; or (iii) both (i) and (ii). In some aspects, the method further comprises: (c) selecting the at least one sterile male progeny arthropod. In some aspects, the method further comprises: (d) allowing the at least one sterile male progeny arthropod selected in step (c) to mate with a wildtype female arthropod of the same species. In some aspects, the method further comprises: (c) allowing the at least one sterile male progeny arthropod to mate with a wildtype female arthropod of the same species.

In some aspects, this disclosure provides a method of generating a population of progeny arthropods, the method comprising: (a) providing a male modified arthropod comprising any sterility system provided herein; (b) providing a wildtype arthropod of the same species to the male modified arthropod; and (c) providing conditions which allow the male modified arthropod to mate with the wildtype female arthropod to produce the population of progeny arthropods, wherein the population of progeny arthropods comprises: (i) at least one sterile male progeny arthropod; (ii) at least one inviable female progeny arthropod; or (iii) both (i) and (ii). In some aspects, the method further comprises: (d) selecting the at least one sterile male progeny arthropod. In some aspects, the method further comprises: (e) allowing the at least one sterile male progeny arthropod selected in step (d) to mate with a wildtype female arthropod of the same species. In some aspects, the method further comprises: (d) allowing the at least one sterile male progeny arthropod to mate with a wildtype female arthropod of the same species.

In some aspects, this disclosure provides a method of generating a population of progeny arthropods, the method comprising: (a) providing a male modified arthropod comprising any sterility system provided herein; and (b) allowing the male modified arthropod to mate with a wildtype female arthropod of the same species to produce the population of progeny arthropods, wherein the population of progeny comprises a subpopulation of male progeny arthropods and a subpopulation of female arthropods, wherein at least 25% of the male progeny arthropods are sterile and wherein at least 25% of the female progeny arthropods are inviable. In some aspects, the method further comprises: (c) selecting the at least one sterile male progeny arthropod. In some aspects, the method further comprises: (d) allowing the at least one sterile male progeny arthropod selected in step (c) to mate with a wildtype female arthropod of the same species. In some aspects, the method further comprises: (c) allowing the at least one sterile male progeny arthropod to mate with a wildtype female arthropod of the same species.

In some aspects, this disclosure provides a method of generating a population of progeny arthropods, the method comprising: (a) providing a male modified arthropod comprising any sterility system provided herein; (b) providing a wildtype arthropod of the same species to the male modified arthropod; and (c) providing conditions which allow the male modified arthropod to mate with the wildtype female arthropod to produce the population of progeny arthropods, wherein the population of progeny arthropods comprises a subpopulation of male progeny and a subpopulation of female progeny, wherein at least 25% of the male progeny are sterile and wherein at least 25% of the female progeny are inviable. In some aspects, the method further comprises: (d) selecting the at least one sterile male progeny arthropod. In some aspects, the method further comprises: (e) allowing the at least one sterile male progeny arthropod selected in step (d) to mate with a wildtype female arthropod of the same species. In some aspects, the method further comprises: (d) allowing the at least one sterile male progeny arthropod to mate with a wildtype female arthropod of the same species.

In some aspects, this disclosure provides a method comprising: (a) obtaining any arthropod provided herein, wherein the arthropod is a male; and (b) allowing the modified male arthropod to mate with a modified female arthropod of the same species, wherein the modified female arthropod is homozygous for the recoded rescue element on an X chromosome or m-locus, to produce a progeny population, wherein the progeny population comprises at least one fertile modified male arthropod comprising the male sterility effector and the recoded rescue element. In some aspects, the method further comprises a progeny population comprising at least one fertile female arthropod. In some aspects, the method further comprises providing the modified female arthropod with a repressor of the repressible female lethal effector. In some aspects, the repressor is tetracycline or doxycycline. In some aspects, a repressor is an analog of tetracycline or an analog of doxycycline.

In some aspects, this disclosure provides a method comprising: (a) obtaining any arthropod provided herein, wherein the arthropod is a male; (b) allowing the modified male arthropod to mate with at least one wildtype female arthropod of the same species to produce a progeny population; and (c) selecting at least one sterile male arthropod from the progeny population.

In some aspects, this disclosure provides a method comprising: (a) providing any arthropod provided herein, wherein the arthropod is a male and at least one wildtype female arthropod of the same species; (b) obtaining a progeny population produced by the modified male arthropod and the at least one wildtype female arthropod; and (c) selecting at least one sterile male arthropod from the progeny population.

In some aspects a sterile male progeny arthropod is an embryonic arthropod. In some aspects a sterile male progeny arthropod is a larval arthropod. In some aspects a sterile male progeny arthropod is a nymph arthropod. In some aspects a sterile male progeny arthropod is a pupal arthropod. In some aspects a sterile male progeny arthropod is an adult arthropod.

In some aspects, a wildtype female arthropod is an insect. In some aspects, a male modified arthropod is an insect. In some aspects, a wildtype female arthropod is an arachnid. In some aspects, a male modified arthropod is an arachnid.

As used herein, the term "population" refers to any positive whole number of individual arthropods of the same species. In some aspects, a population comprises 1 individual. In some aspects, a population comprises at least 1 individual. In some aspects, a population comprises at least 10 individuals. In some aspects, a population comprises at least 100 individuals. In some aspects, a population comprises at least 250 individuals. In some aspects, a population comprises at least 500 individuals. In some aspects, a population comprises at least 750 individuals. In some aspects, a population comprises at least 1000 individuals. In some aspects, a population comprises at least 2500 individuals. In some aspects, a population comprises at least 5000 individuals. In some aspects, a population comprises at least 7500 individuals. In some aspects, a population comprises at least 10,000 individuals. In some aspects, a population comprises at least 25,000 individuals. In some aspects, a population comprises at least 50,000 individuals. In some aspects, a population comprises at least 75,000 individuals. In some aspects, a population comprises at least 100,000 individuals. In some aspects, a population comprises at least 500,000 individuals. In some aspects, a population comprises at least 1,000,000 individuals. In some aspects, a population comprises at least 5,000,000 individuals. In some aspects, a population comprises at least 10,000,000 individuals. In some aspects, a population comprises at least 50,000,000 individuals. In some aspects, a population comprises at least 100,000,000 individuals. In some aspects, a population comprises at least 500,000,000 individuals. In some aspects, a population comprises at least 1,000,000,000 individuals. In some aspects, a population comprises at least 5,000,000,000 individuals. In some aspects, a population comprises at least 10,000,000,000 individuals. In some aspects, a population comprises at least 50,000,000,000 individuals. In some aspects, a population comprises at least 100,000,000,000 individuals. In some aspects, a population comprises at least 500,000,000,000 individuals. In some aspects, a population comprises at least 1,000,000,000,000 individuals. In some aspects, a population comprises between 1 and 1,000,000,000,000 individuals. In some aspects, a population comprises between 1 and 100,000,000,000 individuals. In some aspects, a population comprises between 1 and 10,000,000,000 individuals. In some aspects, a population comprises between 1 and 1,000,000,000 individuals. In some aspects, a population comprises between 1 and 100,000,000 individuals. In some aspects, a population comprises between 1 and 10,000,000 individuals. In some aspects, a population comprises between 1 and 1,000,000 individuals. In some aspects, a population comprises between 1 and 500,000 individuals. In some aspects, a population comprises between 1 and 100,000 individuals. In some aspects, a population comprises between 1 and 50,000 individuals. In some aspects, a population comprises between 1 and 10,000 individuals. In some aspects, a population comprises between 1 and 5000 individuals. In some aspects, a population comprises between 1 and 1000 individuals. In some aspects, a population comprises between 1 and 500 individuals. In some aspects, a population comprises between 1 and 100 individuals. In some aspects, a population comprises between 1 and 50 individuals. In some aspects, a population comprises between 1 and 10 individuals. In some aspects, a population comprises between 100 and 5000 individuals. In some aspects, a population comprises between 100 and 10,000 individuals. In some aspects, a population comprises between 100 and 100,000 individuals. In some aspects, a population comprises between 1000 and 5,000,000 individuals. In some aspects, a population comprises between 1,000,000 and 50,000,000 individuals.

In some aspects, a male population comprises 1 male individual. In some aspects, a male population comprises at least 1 male individual. In some aspects, a male population comprises at least 10 male individuals. In some aspects, a male population comprises at least 100 male individuals. In some aspects, a male population comprises at least 250 male individuals. In some aspects, a male population comprises at least 500 male individuals. In some aspects, a male population comprises at least 750 male individuals. In some aspects, a male population comprises at least 1000 male individuals. In some aspects, a male population comprises at least 2500 male individuals. In some aspects, a male population comprises at least 5000 male individuals. In some aspects, a male population comprises at least 7500 male individuals. In some aspects, a male population comprises at least 10,000 male individuals. In some aspects, a male population comprises at least 25,000 male individuals. In some aspects, a male population comprises at least 50,000 male individuals. In some aspects, a male population comprises at least 75,000 male individuals. In some aspects, a male population comprises at least 100,000 male individuals. In some aspects, a male population comprises at least 500,000 male individuals. In some aspects, a male population comprises at least 1,000,000 male individuals. In some aspects, a male population comprises at least 5,000,000 male individuals. In some aspects, a male population comprises at least 10,000,000 male individuals. In some aspects, a male population comprises at least 50,000,000 male individuals. In some aspects, a male population comprises at least 100,000,000 male individuals. In some aspects, a male population comprises at least 500,000,000 male individuals. In some aspects, a male population comprises at least 1,000,000,000 male individuals. In some aspects, a male population comprises at least 5,000,000,000 male individuals. In some aspects, a male population comprises at least 10,000,000,000 male individuals. In some aspects, a male population comprises at least 50,000,000,000 male individuals. In some aspects, a male population comprises at least 100,000,000,000 male individuals. In some aspects, a male population comprises at least 500,000,000,000 male individuals. In some aspects, a male population comprises at least 1,000,000,000,000 male individuals. In some aspects, a male population comprises between 1 and 1,000,000,000,000 male individuals. In some aspects, a male population comprises between 1 and 100,000,000,000 male individuals. In some aspects, a male population comprises between 1 and 10,000,000,000 male individuals. In some aspects, a male population comprises between 1 and 1,000,000,000 male individuals. In some aspects, a male population comprises between 1 and 100,000,000 male individuals. In some aspects, a male population comprises between 1 and 10,000,000 male individuals. In some aspects, a male population comprises between 1 and 1,000,000 male individuals. In some aspects, a male population comprises between 1 and 500,000 male individuals. In some aspects, a male population comprises between 1 and 100,000 male individuals. In some aspects, a male population comprises between 1 and 50,000 male individuals. In some aspects, a male population comprises between 1 and 10,000 male individuals. In some aspects, a male population comprises between 1 and 5000 male individuals. In some aspects, a male population comprises between 1 and 1000 male individuals. In some aspects, a male population comprises between 1 and 500 male individuals. In some aspects, a male population comprises between 1 and 100 male individuals. In some aspects, a male population comprises between 1 and 50 male individuals. In some aspects, a male population comprises between 1 and 10 male individuals. In some aspects, a male population comprises between 100 and 5000 male individuals. In some aspects, a male population comprises between 100 and 10,000 male individuals. In some aspects, a male population comprises between 100 and 100,000 male individuals. In some aspects, a male population comprises between 1000 and 5,000,000 male individuals. In some aspects, a male population comprises between 1,000,000 and 50,000,000 male individuals.

As used herein, a "subpopulation" refers to any fragment of a whole population that is at least one less than the size of the whole population. As a non-limiting example, if a whole population comprises 100 individuals, a subpopulation of the whole could comprise anywhere between 1 individual and 99 individuals. As another non-limiting example, if a population of 100 individuals comprises 48 males and 52 females, the male subpopulation would have 48 individuals, and the female subpopulation would have 52 individuals.

As used herein, a "male population" refers to a population consisting of only male arthropods. Similarly, a "female population" refers to a population consisting of only female arthropods. A "wildtype population" refers to a population comprising wildtype females or wildtype females and wildtype males.

As used herein, "progeny" refers to offspring produced by a mating between a male arthropod and a female arthropod.

As used herein, "sterile" or "sterility" refers to an inability to produce viable progeny, or an inability to produce a normal number of viable progeny of one or both sexes relative to a wild type (*e.g.,* non-sterile) male. Without being limiting, sterility can be caused by many factors and have a variety of mechanisms. Typically, sterile males are capable of progressing through life stages (*e.g.,* embryonic, larval, pupal, adult stages) normally. As adults, sterile males can mate with females, but (a) no viable progeny are produced by the mating, (b) viable progeny are produced in significantly reduced numbers or (c) viable progeny disproportionately of one sex are produced relative to a mating involving a wild type (non-sterile) male; in some aspects a sterile male mating with a female yields any combination of (a), (b), and (c). When viable progeny are produced in significantly reduced numbers, the male can be said to be "partially" sterile. This disclosure intends for partially sterile arthropods to be encompassed by the terms "sterile" and "sterility." For clarity, a partially sterile arthropod is considered a sterile arthropod for the purposes of this disclosure. Without being limited by any scientific theory, partially sterility can occur when only a fraction of targeted cells comprise and/or express a male sterility effector or any component of a sterility system provided herein.

In some aspects, a sterile male produces no viable progeny after mating with a wildtype female. In some aspects, a sterile male produces a statistically significant reduced number of viable progeny after mating with a wildtype female as compared to the number of viable progeny produced by a wildtype male mating with a wildtype female. In some aspects, a sterile male produces statistically significant more male progeny after mating with a wildtype female as compared to the number of male progeny produced by a wildtype male mating with a wildtype female. In some aspects, a sterile male produces statistically significant more female progeny after mating with a wildtype female as compared to the number of female progeny produced by a wildtype male mating with a wildtype female. As used herein, the phrase "statistically significant" refers to a *p*-value of <0.05 for an appropriate statistical comparison (*e.g.,* without being limiting, a Student's t-test, ANOVA, Mann-Whitney test, Kruskal-Wallis test, Wilcoxon signed-rank test, Kolmogorov-Smirnov test, Fisher's Exact Test, Chi-square test, etc.).

In some aspects, a female lays unfertilized eggs after mating with a sterile male. In some aspects, a female lays inviable fertilized eggs after mating with a sterile male. In some aspects, a female lays sex-biased fertilized eggs after mating with a sterile male. In some aspects, a female lays male-biased fertilized eggs after mating with a sterile male (e.g., X-shredding systems). In some aspects, a female lays fertilized eggs wherein only the female fertilized eggs are inviable after mating with a sterile male (e.g. X-poisoning systems, Y-linked editor systems).

As used herein, "inviable" refers to being incapable of being able to survive to adulthood. In some aspects, inviable female progeny die during the embryo stage. In some aspects, inviable female progeny die during the larval stage. In some aspects, inviable female progeny die during the pupal stage. In some aspects, inviable female progeny are incapable of hatching from an egg. In some aspects, inviable female progeny die as a zygote.

As used herein, "conditions which allow" a male and female arthropod to mate refers to providing environmental conditions (*e.g.,* without being limiting, temperature, humidity, oxygen level, nutritional requirements) which are permissive for an adult male and an adult female arthropod to successfully mate. Those of ordinary skill in the art recognize and understand conditions needed for a species of interest, and that that such conditions will differ based on which species is being used.

In some aspects, this disclosure provides a method comprising: (a) obtaining at least one modified male arthropod comprising any sterility system provided herein; and (b) releasing the at least one arthropod outdoors in the vicinity of at least one wildtype female arthropod of the same species. In some aspects, this disclosure provides a method comprising: (a) obtaining any modified arthropod provided herein, wherein the modified arthropod is a male arthropod; and (b) releasing the modified arthropod outdoors in the vicinity of at least one wildtype female arthropod of the same species.

In some aspects, this disclosure provides a method comprising: (a) obtaining a male population of any modified arthropod provided herein; and (b) releasing the male population outdoors in the vicinity of a wildtype population of the arthropod that comprises at least one wildtype female arthropod of the same species, wherein at least one member of the male population of the modified arthropod mates with the at least one wildtype female arthropod, and wherein the at least one wildtype female arthropod produces (i) at least one sterile male progeny, (ii) at least one inviable female progeny, or (iii) both (i) and (ii).

In some aspects, this disclosure provides a method comprising: (a) obtaining at least one modified male arthropod comprising any sterility system provided herein; and (b) releasing the at least one arthropod in a controlled environment in the vicinity of at least one wildtype female arthropod of the same species. In some aspects, this disclosure provides a method comprising: (a) obtaining any modified arthropod provided herein, wherein the modified arthropod is a male arthropod; and (b) releasing the modified arthropod in a controlled environment in the vicinity of at least one wildtype female arthropod of the same species.

In some aspects, this disclosure provides a method comprising: (a) obtaining a male population of any modified arthropod provided herein; and (b) releasing the male population in a controlled environment in the vicinity of a wildtype population of the arthropod that comprises at least one wildtype female arthropod of the same species, wherein at least one member of the male population of the modified arthropod mates with the at least one wildtype female arthropod, and wherein the at least one wildtype female arthropod produces (i) at least one sterile male progeny, (ii) at least one inviable female progeny, or (iii) both (i) and (ii).

As used herein, "releasing" refers to allowing a modified arthropod provided herein free, unrestrained movement in a location where (a) it is desired to reduce the naturally-occurring (*e.g.,* non-modified) population of the same arthropod species or (b) where it is desired to prevent natural occurrence of the same arthropod species at the release location. A modified arthropod is "released" via purposeful direct or indirect human action (*e.g.,* without being limiting, by placing eggs, larvae, pupae, or adult modified arthropods at the release point). In some aspects, an arthropod is released within a building. In some aspects, an arthropod is released within a controlled environment. In some aspects, an arthropod is released within a greenhouse. In some aspects, an arthropod is released outdoors. In some aspects, an arthropod is released within a container that is open to the local environment (*e.g.*, without being limiting, a greenhouse, the outdoors). It will be appreciated that released modified arthropods have the ability to interact with, comingle with, and/or mate with naturally-occurring (*e.g.,* non-modified) arthropods of the same species at any point in their life cycle. In contrast, modified arthropods that have not been "released" are unable to interact with, comingle with, and/or mate with naturally-occurring (*e.g.,* non-modified) arthropods of the same species due to various containment strategies.

In some aspects, releasing comprises a single release. As used herein, a "single release" refers to releasing a population in the same location within a single 24-hour period, regardless of the size of the population or how many containers of arthropods are released.

In some aspects, releasing comprises multiple releases. As used herein, "multiple releases" refers to releasing a population at different locations within a single 24-hour period or releasing a population at the same location during two or more, non-overlapping 24-hour periods, regardless of the size of the population or how many containers of arthropods are released.

In some aspects, multiple releases are performed at different locations within a single 24-hour period. In some aspects, multiple releases are performed at the same location during two or more non-overlapping 24-hour periods. In some aspects, multiple releases are performed at different locations during two or more non-overlapping 24-hour periods. As used herein, the "same location" refers to a release within 500 meters of an initial release, and "different locations" refers to releases greater than 500 meters of an initial release.

In some aspects, multiple releases occur over a period of between 48 hours and 365 days. In some aspects, multiple releases occur over a period of between 48 hours and 180 days. In some aspects, multiple releases occur over a period of between 48 hours and 90 days. In some aspects, multiple releases occur over a period of between 48 hours and 60 days. In some aspects, multiple releases occur over a period of between 48 hours and 30 days. In some aspects, multiple releases occur over a period of between 48 hours and 15 days. In some aspects, multiple releases occur over a period of between 48 hours and 10 days. In some aspects, multiple releases occur over a period of between 48 hours and 5 days.

In some aspects, a first different location is within 1 kilometer of a second different location. In some aspects, a first different location is within 2 kilometers of a second different location. In some aspects, a first different location is within 3 kilometers of a second different location. In some aspects, a first different location is within 4 kilometers of a second different location. In some aspects, a first different location is within 5 kilometers of a second different location. In some aspects, a first different location is within 6 kilometers of a second different location. In some aspects, a first different location is within 7 kilometers of a second different location. In some aspects, a first different location is within 8 kilometers of a second different location. In some aspects, a first different location is within 9 kilometers of a second different location. In some aspects, a first different location is within 10 kilometers of a second different location. In some aspects, a first different location is within 25 kilometers of a second different location. In some aspects, a first different location is within 50 kilometers of a second different location. In some aspects, a first different location is within 75 kilometers of a second different location. In some aspects, a first different location is within 100 kilometers of a second different location.

In some aspects, multiple releases occur at a single location. In some aspects, multiple releases occur at between 2 and 100 different locations. In some aspects, multiple releases occur at between 2 and 75 different locations. In some aspects, multiple releases occur at between 2 and 50 different locations. In some aspects, multiple releases occur at between 2 and 25 different locations. In some aspects, multiple releases occur at between 2 and 10 different locations. In some aspects, multiple releases occur at between 2 and 5 different locations.

In some aspects, multiple releases occur at least 2 times at a single location. In some aspects, multiple releases occur at least 3 times at a single location. In some aspects, multiple releases occur at least 4 times at a single location. In some aspects, multiple releases occur at least 5 times at a single location. In some aspects, multiple releases occur at least 10 times at a single location. In some aspects, multiple releases occur between 2 times and 100 times at a single location. In some aspects, multiple releases occur between 2 times and 75 times at a single location. In some aspects, multiple releases occur between 2 times and 50 times at a single location. In some aspects, multiple releases occur between 2 times and 25 times at a single location. In some aspects, multiple releases occur between 2 times and 10 times at a single location. In some aspects, multiple releases occur between 2 times and 5 times at a single location.

In some aspects, a released arthropod is an adult arthropod. In some aspects, a released arthropod is a pupal arthropod. In some aspects, a released arthropod is a nymph arthropod. In some aspects, a released arthropod is a larval arthropod. In some aspects, a released arthropod is an embryonic arthropod. Unless otherwise specified, a released embryonic arthropod is released as an egg.

In some aspects, a male population of arthropods comprises at least one adult arthropod. In some aspects, a male population of arthropods comprises at least one pupal arthropod. In some aspects, a male population of arthropods comprises at least one nymph arthropod. In some aspects, a male population of arthropods comprises at least one larval arthropod. In some aspects, a male population of arthropods comprises at least one embryonic arthropod.

As used herein, "outdoors" refers to any area outside of enclosed buildings or other fully-enclosing shelters (*e.g.,* outdoors is an open air environment). Non-limiting examples of "outdoor" areas for release include fields, forests, open-air sheds, open-air barns, poultry houses, deserts, lawns, parks, ponds, lakes, rivers, marshes, swamps, fens, bogs, open-air parking structures, public sewer systems, silos, grain bins, grasslands, savannas, scrublands, wetlands, beaches, oases, caves, sinkholes, wastewater treatment areas, tidal flats, sandbars, ditches, canals, pastures, orchards, cultivated farm fields, sand dunes, tidepools, mangroves, shorelines, transit line, etc.

As used herein, "in the vicinity of" refers to a distance that can be covered by the natural movements of an adult male arthropod, an adult female arthropod, or both, such that they could come into contact with each other within 720 hours of release. In some aspects, a modified male arthropod is released within 1 meter of a wildtype female arthropod of the same species. In some aspects, a modified male arthropod is released within 2.5 meters of a wildtype female arthropod of the same species. In some aspects, a modified male arthropod is released within 5 meters of a wildtype female arthropod of the same species. In some aspects, a modified male arthropod is released within 7.5 meters of a wildtype female arthropod of the same species. In some aspects, a modified male arthropod is released within 10 meters of a wildtype female arthropod of the same species. In some aspects, a modified male arthropod is released within 25 meters of a wildtype female arthropod of the same species. In some aspects, a modified male arthropod is released within 50 meters of a wildtype female arthropod of the same species. In some aspects, a modified male arthropod is released within 100 meters of a wildtype female arthropod of the same species. In some aspects, a modified male arthropod is released within 250 meters of a wildtype female arthropod of the same species. In some aspects, a modified male arthropod is released within 500 meters of a wildtype female arthropod of the same species. In some aspects, a modified male arthropod is released within 750 meters of a wildtype female arthropod of the same species. In some aspects, a modified male arthropod is released within 1 kilometer of a wildtype female arthropod of the same species. In some aspects, a modified male arthropod is released within 2.5 kilometers of a wildtype female arthropod of the same species. In some aspects, a modified male arthropod is released within 5 kilometers of a wildtype female arthropod of the same species. In some aspects, a modified male arthropod is released within 10 kilometers of a wildtype female arthropod of the same species.

In some aspects, a method provided herein occurs outdoors. In some aspects, at least one step of a method provided herein occurs outdoors. In some aspects, at least two steps of a method provided herein occur outdoors.

In some aspects, a method provided herein occurs in a controlled environment. In some aspects, at least one step of a method occurs in a controlled environment. In some aspects, at least two steps of a method occur in a controlled environment.

As used herein, a "controlled environment" refers to an enclosed environment where humans are capable of manipulating and/or setting environmental conditions such as, without being limiting, air temperature, humidity, light cycle, enclosure size, the number of arthropods present, water availability, food availability, oxygen content of the air, air movement, etc. In some aspects, a controlled environment comprises a non-modified arthropod population (*e.g.,* a wildtype population) that is desired to be reduced via a release of any modified arthropods provided herein. In some aspects, a controlled environment comprises a non-modified arthropod population (*e.g.,* a wildtype population) that is useful for rearing and maintaining a population of modified arthropods, and for which a reduction in population size is not desired.

In some aspects, a controlled environment comprises a container or cage to house arthropods. In some aspects, a controlled environment comprises a greenhouse. In some aspects, a controlled environment comprises a vertical farming factory. In some aspects, a controlled environment is positioned within a laboratory. In some aspects, a controlled environment comprises a room inside a building. In some aspects, a controlled environment is a condominium. In some aspects, a controlled environment is an apartment. In some aspects, a controlled environment is an apartment building. In some aspects, a controlled environment is a house. In some aspects, a controlled environment is a residential building. In some aspects, a controlled environment is a commercial building. In some aspects, a controlled environment is a hospital. In some aspects, a controlled environment is a clinic. In some aspects, a controlled environment is a medical building. In some aspects, a controlled environment is a grocery store or supermarket. In some aspects, a controlled environment is a food-processing building. In some aspects, a controlled environment is a hotel. In some aspects, a controlled environment is a hostel. In some aspects, a controlled environment is an office building.

In some aspects, a controlled environment comprises an air temperature of between 5°C and 40°C. In some aspects, a controlled environment comprises an air temperature of between 10°C and 40°C. In some aspects, a controlled environment comprises an air temperature of between 15°C and 40°C. In some aspects, a controlled environment comprises an air temperature of between 15°C and 30°C.In some aspects, a controlled environment comprises an air temperature of between 20°C and 40°C. In some aspects, a controlled environment comprises an air temperature of between 25°C and 40°C. In some aspects, a controlled environment comprises an air temperature of between 20°C and 30°C. In some aspects, a controlled environment comprises an air temperature of between 30°C and 40°C.

In some aspects, a controlled environment comprises a humidity of between 10% and 100% relative humidity. In some aspects, a controlled environment comprises a humidity of between 20% and 100% relative humidity. In some aspects, a controlled environment comprises a humidity of between 30% and 100% relative humidity. In some aspects, a controlled environment comprises a humidity of between 40% and 100% relative humidity. In some aspects, a controlled environment comprises a humidity of between 50% and 100% relative humidity. In some aspects, a controlled environment comprises a humidity of between 60% and 100% relative humidity. In some aspects, a controlled environment comprises a humidity of between 70% and 100% relative humidity. In some aspects, a controlled environment comprises a humidity of between 80% and 100% relative humidity. In some aspects, a controlled environment comprises a humidity of between 90% and 100% relative humidity. In some aspects, a controlled environment comprises a humidity of between 40% and 80% relative humidity. In some aspects, a controlled environment comprises a humidity of between 40% and 60% relative humidity. In some aspects, a controlled environment comprises a humidity of between 60% and 80% relative humidity.

In some aspects, a controlled environment comprises a 24-hour photoperiod comprising 12 hours of light and 12 hours of darkness. In some aspects, a controlled environment comprises a 24-hour photoperiod comprising between 10 and 14 hours of light and between 10 and 14 hours of darkness. In some aspects, a controlled environment comprises a 24-hour photoperiod comprising at least 12 hours of light. In some aspects, a controlled environment comprises a 24-hour photoperiod comprising at least 12 hours of darkness.

In some aspects, a controlled environment comprises an enclosure having an area of less than 0.1 square meters. In some aspects, a controlled environment comprises an enclosure having an area of less than 0.25 square meters. In some aspects, a controlled environment comprises an enclosure having an area of less than 0.5 square meters. In some aspects, a controlled environment comprises an enclosure having an area of less than 0.75 square meters. In some aspects, a controlled environment comprises an enclosure having an area of less than 1 square meter. In some aspects, a controlled environment comprises an enclosure having an area of less than 2 square meters. In some aspects, a controlled environment comprises an enclosure having an area of less than 5 square meters. In some aspects, a controlled environment comprises an enclosure having an area of less than 10 square meters. In some aspects, a controlled environment comprises an enclosure having an area of between 0.05 square meters and 50 square meters. In some aspects, a controlled environment comprises an enclosure having an area of between 0.05 square meters and 25 square meters. In some aspects, a controlled environment comprises an enclosure having an area of between 0.05 square meters and 10 square meters. In some aspects, a controlled environment comprises an enclosure having an area of between 0.05 square meters and 5 square meters. In some aspects, a controlled environment comprises an enclosure having an area of between 0.05 square meters and 1 square meter.

In some aspects, this disclosure provides a method comprising: (a) introducing any male sterile effector provided herein to at least one arthropod cell; and (b) obtaining an adult arthropod from the at least one arthropod cell, wherein the adult arthropod comprises the male sterile effector. In some aspects, this disclosure provides a method comprising: (a) introducing any sterility system provided herein to at least one arthropod cell; and (b) obtaining an adult arthropod from the at least one arthropod cell, wherein the adult arthropod comprises the sterility system. In some aspects, this disclosure provides a method comprising: (a) introducing any isolated nucleic acid molecule provided herein to at least one arthropod cell; and (b) obtaining an adult arthropod from the at least one arthropod cell, wherein the adult arthropod comprises the isolated nucleic acid molecule. In some aspects, this disclosure provides a method comprising: (a) introducing any plasmid provided herein to at least one arthropod cell; and (b) obtaining an adult arthropod from the at least one arthropod cell, wherein the adult arthropod comprises the male sterile effector. In some aspects, the plasmid comprises a nucleic acid sequence at least 80% identical to a nucleic acid sequence selected from the group consisting of SEQ ID NOs: 4012 to 4249, 4335 to 4337, 4391 to 4396, and 6832 to 6850.

In some aspects, this disclosure provides a method comprising: (a) introducing a recoded rescue element into a genome of a first arthropod cell, wherein the recoded rescue element encodes a male-fertility essential gene; (b) further introducing a loss-of-function mutation in an endogenous allele of the male-fertility essential gene into the genome; and (c) obtaining a male arthropod comprising the recoded rescue element and the loss-of-function mutation. In some aspects, this disclosure provides a method comprising: (a) introducing a loss-of-function mutation in an endogenous allele of a male-fertility essential gene in at least one arthropod cell, wherein the at least one arthropod cell further comprises a recoded recue element encoding a functional copy of the male-fertility essential gene; and (b) obtaining an adult male arthropod from the at least one arthropod cell, wherein the adult male arthropod comprises the loss-of-function mutation and the recoded rescue element.

This disclosure provides an arthropod produced by, or obtained from, any method provided herein.

Those of ordinary skill in the art are aware of myriad methods to "introduce" nucleic acid molecules (e.g., a male sterile effector, a sterility system, an isolated nucleic acid molecule, a plasmid) into an arthropod cell. Any method known in the art for introducing nucleic acid molecules into arthropod cells is envisioned for use with the methods provided herein. In some aspects, a nucleic acid molecule is electroporated into at least one arthropod cell. In some aspects, a nucleic acid molecule is microinjected into at least one arthropod cell. In some aspects, a nucleic acid molecule is chemically transformed into at least one arthropod cell.

In some aspects, a nucleic acid molecule is introduced to an embryonic arthropod cell. In some aspects, a nucleic acid molecule is introduced into a male embryonic arthropod cell. In some aspects, a nucleic acid molecule is introduced into a female embryonic arthropod cell. In some aspects, a nucleic acid molecule is introduced into an arthropod gametic cell. n some aspects, a nucleic acid molecule is introduced into an arthropod zygotic cell.

Methods for genetic transformation by embryo microinjection are well established, and include: (1) The collection of embryos between the period of embryo fertilization to cellularization, typically within 1 to 3 hours of oviposition; (2) Embryos may need to be desiccated or further treated to remove wax layers that may preclude microinjection, and in some cases the Gené's organ can be removed to prevent wax formation; (3) Embryos must be carefully manipulated to allow access to the posterior pole, where the germline will form, typically by transferring to a glass slide using a fine paint brush; (4) For some species it is necessary to submerge the embryos in water, buffer, or oil to avoid further desiccation. In some instances, and particularly for *Aedes aegypti* mosquitoes, Halocarbon-27 oil (a medium molecular weight polymer of chlorotrifluoroethylene) is used, often as an emulsion with water; (5) Glass needles may need to be prepared for microinjection, typically made from glass, preferably borosilicate or quartz. These may be prepared by pulling glass capillaries using a Sutter Instruments^{®} P-2000 laser-based micropipette puller; (6) Injection mixtures are prepared to contain a mixture of nucleic acids, typically in combination with a microinjection buffer or water alone. The transgene (e.g., without being limiting, male sterile effector, rescue element, or repressible female lethal effector) can be provided on a plasmid as DNA, optionally flanked by inverted repeats or homology arms to facility entry into the genome. Plasmids are typically injected at a final concentration between 100 nanograms (ng) and 1000 ng/microliter (µL) each, most preferably between 200 ng/µL and 400 ng/µL each. "Helper" proteins or nucleic acids can be included in the mixture to facilitate integration within the arthropod genome and include transposase *(e.g.,* piggyBac, minos), integrase *(e.g.,* phiC31 integrase), or Cas9 and gRNA. Alternatively, injections can be performed into transgenic strains expressing helper proteins; (7) Microinjection is performed into the posterior pole of the embryo using a microinjector (e.g. Eppendorf^{™} FemtoJet^{™} microinjector) and micromanipulator (e.g. Eppendorf^{™} InjectMan^{®} 4 or Narishige MN-153 Micromanipulator). Microinjections must generally occur before cellularization to ensure access of injected nucleic acids to the polar nuclei that will form later the germline. Typically this encompasses a period within two hours of oviposition, sometimes within one hour of oviposition, and not usually sooner than 15 minutes post oviposition to avoid destroying delicate embryos; and (8) Injected embryos are allowed to recover, reared to adults, mated to wild-type, and allowed to produce progeny. Progeny are screened for integration of nucleic acids, typically by inspection of a visual marker such as the fluorescent proteins GFP, DsRed, or CFP that can be viewed in first generation (G1) transgenics under a fluorescence microscope. *See,* for example, Harrell II, "Mosquito Embryo Microinjection," Cold Spring Harb Protoc, 2024*;* and Sharma et al., iScience, 25(3):103781 (2022).

For additional information on methods of introducing a nucleic acid molecule to an arthropod cell is via microinjection using transposable elements such as *piggyBac* or the *P* element, *see,* for example, Gregory et al., Insect Molecular Biology, 25(3):259-271 (2016) and Rubin and Spradling, Science, 218:348-353 (1982), respectively. Additional transformation methods using other transposons *(e.g., Minos, mariner, hobo)* as well as viral vectors is also known in the art. *See,* for example, Handler, Insect Biochemistry and Molecular Biology, 31:111-128 (2001). Additional transformation methods using site-specific integrases such as ΦC31 integrase or Cre recombinase can be used to secondarily modify attP or LoxP sites, respectively. *See,* for example, Volohonsky et al., G3, 5(6):1151-1163 (2015). Additional transformation methods include by homology-directed repair (HDR) such as CRISPR-mediated HDR or CRISPR-mediated cassette exchange (CRIMCE). *See,* for example, Morianou et al., CRISPR J., (6):868-876 (2022).

The following examples of non-limiting embodiments are specifically envisioned:
1. A male sterile effector comprising:
   (a) a first promoter operably linked to a first nucleic acid molecule encoding an RNA-guided nuclease; and
   (b) a second promoter operably linked to a second nucleic acid molecule encoding a guide RNA (gRNA), wherein the gRNA comprises a nucleic acid sequence at least 90% identical or complementary to at least 17 consecutive nucleotides of a first arthropod male-fertility essential gene.
2. A male sterile effector comprising a first promoter operably linked to a first nucleic acid molecule encoding a microRNA (miRNA) molecule or a short hairpin RNA (shRNA) molecule comprising a nucleic acid sequence at least 90% identical to at least 17 consecutive nucleotides of a first arthropod male-fertility essential gene messenger RNA molecule.
3. The male sterile effector of embodiment 1, wherein the male sterile effector further comprises:
   (c) a third promoter operably linked to a third nucleic acid molecule encoding a microRNA (miRNA) molecule or a short hairpin RNA (shRNA) molecule comprising a sequence at least 90% identical to at least 19 consecutive nucleotides of a second arthropod male-fertility essential gene messenger RNA.
4. The male sterile effector of any one of embodiments 1 to 3, wherein the first promoter is selected from the group consisting of a germline-preferred promoter, a ubiquitous promoter, and an inducible promoter.
5. The male sterile effector of any one of embodiments 1, 3, or 4, wherein the second promoter is selected from the group consisting of a germline-preferred promoter, a ubiquitous promoter, and an inducible promoter.
6. The male sterile effector of any one of embodiments 3 to 5, wherein the third promoter is selected from the group consisting of a germline-preferred promoter, a ubiquitous promoter, and an inducible promoter.
7. The male sterile effector of any one of embodiments 4 to 6, wherein the germline-preferred promoter is selected from the group consisting of: a *nanos* promoter, a *vasa* promoter, an *exu* promoter, a *zpg* promoter, and a *β2-tubulin* promoter.
8. The male sterile effector of any one of embodiments 4 to 6, wherein the germline-preferred promoter comprises a nucleic acid sequence at least 90% identical to a nucleic acid sequence selected from the group consisting of SEQ ID NOs: 4250 to 4254 and 4257 to 4274, or a functional thereof.
9. The male sterile effector of any one of embodiments 4 to 6, wherein the ubiquitous promoter is selected from the group consisting of: a *Nup50* promoter, an *hsp83* promoter, and a *ubiquitin* promoter.
10. The male sterile effector of any one of embodiments 4 to 6, wherein the ubiquitous promoter comprises a nucleic acid sequence at least 90% identical to a nucleic acid sequence selected from the group consisting of SEQ ID NOs: 4309 to 4312 and 4386, or a functional fragment thereof.
11. The male sterile effector of any one of embodiments 4 to 6, wherein the inducible promoter comprises (a) a response element selected from the group consisting of: UAS, QUAS, tetO, and LexO; (b) *Hs70Bb,* or (c) both (a) and (b).
12. The male sterile effector of any one of embodiments 4 to 6, wherein the inducible promoter comprises a nucleic acid sequence at least 90% identical to a nucleic acid sequence selected from the group consisting of SEQ ID NOs: 4275 to 4289, or a functional fragment thereof.
13. The male sterile effector of embodiment 1 or embodiment 3, wherein the first promoter, the second promoter, and the third promoter are independently selected from the group consisting of a *β2-tubulin* promoter, an *Nup50* promoter, a *nanos* promoter, a *ubiquitin* promoter, a *vasa* promoter, and an *exu* promoter.
14. The male sterile effector of embodiment 1 or embodiment 3, wherein the first promoter is selected from the group consisting of a *β2-tubulin* promoter, a *protamine* promoter, a *Dnah3* promoter, a *Loopin-1* promoter, an *S-Lap1* promoter, an *S-Lap2* promoter, an *S-Lap3* promoter, an *S-Lap4* promoter, an *S-Lap5* promoter, an *S-Lap7* promoter, and an S-*Lap8* promoter.
15. The male sterile effector of embodiment 1 or embodiment 3, wherein the first promoter is selected from the group consisting of: *a β2-tubulin* promoter, a *protamine* promoter, a *dnah3* promoter, a *loopin-1* promoter, an *s-lap1* promoter, an *s-lap2* promoter, an *s-lap3* promoter, an *s-lap4* promoter, an *s-lap5* promoter, an *s-lap7* promoter, an *s-lap8* promoter, *a vasa* promoter, a *nanos* promoter, an *exu* promoter, a *fas3* promoter, a *squid* promoter, a *belle* promoter, a *geminin* promoter, an *sds3* promoter, a *racGAP1* promoter, an *aurora kinase* promoter, a *shu* promoter, a *zpg* promoter, an *argonaute* 3 promoter, an *aubergine* promoter, an *eif4E-5* promoter, a *bgcn* promoter, a *bam* promoter, a *piwi* promoter, a *pumilio* promoter, and a *spol* promoter.
16. The male sterile effector of embodiment 1 or embodiment 3, wherein the first promoter is selected from the group consisting of a *β2-tubulin* promoter, an *exu* promoter, a *nanos* promoter, and a *vasa* promoter.
17. The male sterile effector of embodiment 1 or embodiment 3, wherein the second promoter and the third promoter are independently selected from the group consisting of a *U6* promoter, a *7SK* promoter, a U3 promoter, an *H1* promoter, a *ubiquitin* promoter, and a *tRNA* promoter.
18. The male sterile effector of any one of embodiments 7 or 13 to 16, wherein the *β2-tubulin* promoter comprises a nucleic acid sequence at least 90% identical to a nucleic acid sequence selected from the group consisting of SEQ ID NOs: 4271 to 4274, or a functional fragment thereof.
19. The male sterile effector of any one of embodiments 7 or 13 to 16, wherein the *nanos* promoter comprises a nucleic acid sequence at least 90% identical to a nucleic acid sequence selected from the group consisting of SEQ ID NOs: 4253, 4254, and 4258, or a functional fragment thereof.
20. The male sterile effector of any one of embodiments 7 or 13 to 16, wherein the *exu* promoter comprises a nucleic acid sequence at least 90% identical to a nucleic acid sequence selected from the group consisting of SEQ ID NOs: 4251 and 4252, or a functional fragment thereof.
21. The male sterile effector of any one of embodiments 7 or 13 to 16, wherein the *vasa* promoter comprises a nucleic acid sequence at least 90% identical to a nucleic acid sequence selected from the group consisting of SEQ ID NOs: 4263 and 4267, or a functional fragment thereof.
22. The male sterile effector of embodiment 9 or embodiment 13, wherein the *Nup50* promoter comprises a nucleic acid sequence at least 90% identical to a nucleic acid sequence of SEQ ID NO: 4310, or a functional fragment thereof.
23. The male sterile effector of embodiment 17, wherein the *U6* promoter comprises a nucleic acid sequence at least 90% identical to a nucleic acid sequence selected from the group consisting of SEQ ID NOs: 4295 to 4307, or a functional fragment thereof.
24. The male sterile effector of any one of embodiments 1 to 23, wherein the first promoter, the second promoter, or the third promoter comprises a nucleic acid sequence at least 90% identical to a nucleic acid sequence independently selected from the group consisting of: SEQ ID NOs: 4250 to 4254, 4258 to 4307, 4309 to 4312, 4386, and 6848 to 6850.
25. The male sterile effector of any one of embodiments 1 or 3 to 24, wherein the RNA-guided nuclease is a Cas9.
26. The male sterile effector of embodiment 25, wherein the Cas9 nuclease is a *Streptococcus pyogenes* Cas9.
27. The male sterile effector of any one of embodiments 1 to 26, wherein the first arthropod male-fertility essential gene and the second arthropod male-fertility essential gene are independently selected from the group consisting of: *betaTub85D, ory, zpg, 28S rDNA, S-Lap3, blw, ary, kl-2, kl-3, kl-5, snky, knon, blanks, sa, ddbt, WDY, S-Lap1, S-Lap2, S-Lap4, S-Lap5, S-Lap7, S-Lap8, fzo, exu, bel, shu, CG12069, Cyt-clL, Hsp60B, Kpcl, Kpc2, loopin-1, mmm, sac, Red7, Rsbp15, Rsphl, Rsph3, Rsph4a, Rsph9, wam, bam, bb8, bond, wupA, RpS6, RpS10, RpS19a, RpL35, RpL37, muc14a,* AAEL028070, *atlas, aust, Mst27D, nht, run, spag4, pp1-Y1, pp1-Y2, soti, eIF4E5, Dic61B, maca, can, bgcn, Dpy-30L2, ms(2)34Fe, ms(2)35Ci, ms(3)76Ba, ms(3)76Cc, ms(3)K81, nsr, Ntl, Pif1A, Prosalpha4T1, Prosalpha6T, PRY, shps, salto, tbrd-2, ttm2, xmas, ATPsynbetaL, 14-3-3epsilon, ab, abd-A, Abd-B, ac, Ack, Acly, Acn, ago, alphaTub84B, aly, aop, ap, Apl, ara, Arf6, asl, asp, asun, Atg8b, ATP7, ATPsyngamma, aub, aux, ball, betaTub60D, Bin1, bol, bor, Brca2, Brr2, bru1, brun, Bsg, Btk, Bug22, Bx, ca, Cad99C, Cap-D3, Cap-H2, caup, Cdc42, Cds, Cep135, Cep290, CG13202, CG18675, CG31759, CG8236, CG9313, CG9920, chb, Chc, Chd1, chic, CIAPIN1, cic, clu, cmb, cn, cnn, Cog7, COX4L, Cpes, crm, CSN5, csul, ct, ctp, cue, Cul3, CycB, Cyp40, Cyt-b5, Cyt-c-d, Dab, dally, dany, Dark, dbf, Dcr-2, DCTN5-p25, Ddx1, del, Dhc16F, dia, Diap1, didum, Diedel, Dis3l2, dj, Dlc90F, Dnaaf3, Dnaaf5, Dnaaf6, Dnah3, Doa, dor, Dronc, dsx, dtr, Duba, dwg, dx, EcR, eff, eIF2A, eIF3m, eIF4E1, eIF4E3, eIF4G2, emc, EndoGI, endos, eRF3, Erk7, eya, f, Fas2, Fas3, fbl, Fmrl, fru, fry, Fsn, fwd, fws, fz2, g, Gas8, gdrd, gish, Gld2, Gorab, Grip128, Grip75, Grip84, Grip91, gskt, GstS1, gudu, H, hay, HBS1, Hel89B, heph, hfp, HipHop, hmw, hop, HPle, Hr39, Hr51, Hsp60C, Hsp83, HtrA2, hts, hyd, Ibf2, ifc, Inos, InR, Iswi, jar, JYalpha, Kap-alpha1, Kdm5, klhl10, Klp3A, Klp67A, l(2)dtl, l(2)gl, Lam, larp, Lasp, lds, Liprin-alpha, Lis-1, IncRNA:CR45362, lncRNA:Hsromega, IncRNA:iab4, IncRNA:iab8, lost, lovit, lz, m, mael, mago, mal, mam, MAN1, mei-P26, mei-S332, Mer, mfr, mfrn, Mgat1, mgr, Mhc, mia, mil, mino, mip120, mip40, mle, mlt, Mob4, mod, mod(mdg4), msl-3, Mst77F, mt:CoI, mt:Cyt-b, mtsh, mxc, Myc, Myt1, Naa20A, nAChRbeta1, nclb, ND-B17, neb, nej, Nle, nmd, noi, Nop60B, Npc1a, ntc, Nup154, nw, orb2, Orc1, Osbp, osk, otu, ox, p24-1, pAbp, Pall, papi, PAPLA1, park, pbl, Pde1c, Pdkl, pea, pelo, Pen, Pex10, Pex12, Pex16, Pex2, Pfas, Phb1, Pih1D1, Pink1, piwi, Pkd2, Pkn, Pngl, poe, PolQ, Polr2A, pont, Popl, porin, Poxn, prd, pros, Prp8, psidin, PSMG1, puf, qm, Rab30, Rae1, Rala, Ran, rau, Rb97D, Rdl, ref(2)P, rept, rho-7, Rm62, rn, Rnp4F, Roc1b, Root, RpL36, Rpp30, RpS27A, RpS3, rst, rt, Rtca, rux, SAK, Sas-4, sau, sbr, sc, SCaMC, scat, scny, ScsbetaA, sd, Sf3a1, Shc, shop, shv, sif, sim, sina, slmo, sm, SmB, Smg5, snRNA:U7, sns, Sod1, spag, spd-2, spn-E, SRPK, ss, ssp3, stai, Stat92E, stau, stet, stg, sti, Strip, su(Hw), su(sable), Su(var)3-3, sub, Sxl, Sym, Syp, Syx13, Syx5, Taf6, Taz, tbrd-1, TER94, Tfb5, t-Grip84, Theg, thoc5, tilB, tj, tomb, Top2, Top3alpha, topi, Topors, Torsin, tous, tra, tra2, Trel, Trf, Trl, tRNA:Leu-CAA-1-1, tRNA:Tyr-GTA-1-3, Trxr1, tsr, Tssk, TTLL3B, tud, tut, twe, twy, U2A, Ubi-p63E, Ulp1, upd1, upd3, Upj2, Usp14, v, vg, VhaPPAl-2, vib, vis, Vmat, vn, Vps28, vret, Vrp1, Vtld, WASp, Wdr92, wls, wnd, Wnt2, wts, wuho, y, Yipldl, YMEIL, zip, Zmynd10,* and *Zw10,* or an ortholog thereof.
28. The male sterile effector of any one of embodiments 1 to 26, wherein the first arthropod male-fertility essential gene and the second arthropod male-fertility essential gene independently comprise a nucleic acid sequence at least 90% identical to a nucleic acid sequence selected from the group consisting of SEQ ID NOs: 1 to 2879 and 2881 to 2902.
29. The male sterile effector of any one of embodiments 1 to 26, wherein the first arthropod male-fertility essential gene is selected from the group consisting of *betaTub85D, ory, zpg, 28S rDNA, S-Lap3, blw, ary, kl-2, kl-3, kl-5, snky, knon, blanks, sa, ddbt, WDY, S-Lap1, S-Lap2, S-Lap4, S-Lap5, S-Lap7, S-Lap8, fzo, exu, bel, shu, CG12069, Cyt-x1L, Hsp60B, Kpcl, Kpc2, loopin-1, mmm, sac, Red7, Rsbpl5, Rsphl, Rsph3, Rsph4a, Rsph9, wam, bam, bb8, bond, wupA, RpS6, RpS10, RpS19a, RpL35, RpL37, muc14a,* AAEL028070, *atlas, aust, Mst27D, nht, run, spag4, pp1-Y1, pp1-Y2, soti, eIF4E5, Dic61B, maca, can, bgcn, Dpy-30L2, ms(2)34Fe, ms(2)35Ci, ms(3)76Ba, ms(3)76Cc, ms(3)K81, nsr, Ntl, Pif1A, Prosalpha4T1, Prosalpha6T, PRY, shps, salto, tbrd-2, ttm2, xmas, and ATPsynbetaL,* or an ortholog thereof.
30. The male sterile effector of any one of embodiments 1 to 29, wherein the gRNA comprises a nucleic acid sequence at least 90% identical or complementary to a nucleic acid sequence selected from the group consisting of SEQ ID NOs: 2903 to 4011, 4342, 4343, and 4345 to 4385.
31. The male sterile effector of any one of embodiments 2 to 29, wherein the miRNA or the shRNA comprises a nucleic acid sequence at least 90% identical or complementary to at least 18 consecutive nucleotides of a nucleic acid sequence selected from the group consisting of SEQ ID NOs: 1 to 2879 and 2881 to 2902.
32. The male sterile effector of any one of embodiments 1, 3, or 4, wherein the first promoter is a PolII promoter.
33. The male sterile effector of any one of embodiments 1, 3, or 4, wherein the second promoter is selected from the group consisting of a PolII promoter and a PolIII promoter.
34. The male sterile effector of embodiment 2, wherein the first promoter is selected from the group consisting of a PolII promoter and a PolIII promoter.
35. The male sterile effector of embodiment 3, wherein the third promoter is selected from the group consisting of a PolII promoter and a PolIII promoter.
36. The male sterile effector of embodiment 2, wherein the first promoter is selected from the group consisting of a *U6* promoter, a 7SK promoter, a U3 promoter, an *H1* promoter, a *ubiquitin* promoter, and a *tRNA* promoter.
37. The male sterile effector of embodiment 2, wherein the first promoter is selected from the group consisting of a germline-preferred promoter, a ubiquitous promoter, and an inducible promoter.
38. One or more isolated nucleic acid molecules encoding the male sterile effector of any one of embodiments 1 to 37.
39. A sterility system comprising:
   (a) the male sterile effector of any one of embodiments 1 to 37; and
   (b) a repressible female-lethal effector.
40. A sterility system comprising:
   (a) the male sterile effector of any one of embodiments 1 to 37; and
   (b) a rescue element comprising a fourth promoter operably linked to a nucleic acid molecule encoding at least one of: an anticas protein, a functional copy of the first arthropod male-fertility essential gene, and a functional copy of the second arthropod male-fertility essential gene.
41. A sterility system comprising:
   (a) the male sterile effector of any one of embodiments 1 to 37;
   (b) a repressible female-lethal effector; and
   (c) a rescue element comprising a fourth promoter operably linked to a nucleic acid molecule encoding at least one of: an anticas protein, a functional copy of the first arthropod male-fertility essential gene, and a functional copy of the second arthropod male-fertility essential gene.
42. The sterility system of embodiment 39 or 41, wherein the repressible female-lethal effector is operably linked to a drug-repressible promoter.
43. The sterility system of embodiment 39, wherein the drug-repressible promoter is a tetracycline-repressible promoter.
44. The sterility system of any one of embodiments 40 to 43, wherein the anticas protein is an AcrIIA4 protein.
45. The sterility system of embodiment 44, wherein the AcrIIA4 protein comprises an amino acid sequence at least 95% identical or similar to the amino acid sequence of SEQ ID NO: 6494 or 6495.
46. The sterility system of any one of embodiments 40 to 45, wherein the nucleic acid molecule encoding the anticas protein comprises a nucleic acid sequence at least 90% identical to SEQ ID NO: 4314 or 4314.
47. The sterility system of any one of embodiments 40 to 43, wherein the functional copy of the first arthropod male-fertility essential gene is selected from the group consisting of a wildtype allele of the first arthropod male-fertility essential gene and a codon-scrambled allele of the first arthropod male-fertility essential gene.
48. The sterility system of embodiment 47, wherein the codon-scrambled allele of the first arthropod male-fertility essential gene comprises a nucleic acid sequence at least 95% identical to a nucleic acid sequence selected from the group consisting of SEQ ID NOs: 4338 to 4340.
49. The sterility system of any one of embodiments 40 to 42 or 47, wherein the functional copy of the second arthropod male-fertility essential gene is selected from the group consisting of a wildtype allele of the second arthropod male-fertility essential gene and a codon-scrambled allele of the second arthropod male-fertility essential gene.
50. The sterility system of embodiment 49, wherein the codon-scrambled allele of the second arthropod male-fertility essential gene comprises a nucleic acid sequence at least 95% identical to a nucleic acid sequence selected from the group consisting of SEQ ID NOs: 4338 to 4340.
51. The sterility system of any one of embodiments 40 to 50, wherein the rescue element encodes an anticas protein, and wherein the anticas protein is operably linked to at least one QUAS element.
52. The sterility system of any one of embodiments 40 to 50, wherein the male sterility effector further comprises a fifth promoter operably linked to a transactivator, and wherein the first, second, third, or fourth promoter comprises at least one copy of an upstream activation sequence compatible with the transactivator.
53. The sterility system of embodiment 52, wherein the transactivator is selected from the group consisting of Gal4, QF, QF2, and QF2^{w}.
54. The sterility system of embodiment 52 or embodiment 53, wherein the upstream activation sequence is selected from the group consisting of UAS and QUAS.
55. The sterility system of any one of embodiments 52 to 54, wherein any promoter comprises at least three copies of the upstream activation sequence.
56. One or more isolated nucleic acid molecules encoding the sterility system of any one of embodiments 39 to 55 or 180 to 192.
57. An isolated nucleic acid molecule comprising a nucleic acid sequence at least 95% identical to a nucleic acid sequence selected from the group consisting of SEQ ID NOs: 1 to 2879 2881 to 4396, and 6832 to 6487.
58. The isolated nucleic acid molecule of embodiment 57, wherein the nucleic acid sequence is operably linked to a promoter.
59. The isolated nucleic acid molecule of embodiment 58, wherein the promoter comprises a nucleic acid sequence at least 95% identical to a nucleic acid sequence selected from the group consisting of SEQ ID NOs: 4250 to 4254, 4258 to 4307, 4309 to 4312, 4386, and 6848 to 6850.
60. An isolated nucleic acid molecule comprising a non-naturally occurring codon-scrambled nucleic acid sequence that encodes an amino acid sequence at least 90% identical to an amino acid sequence selected from the group consisting of SEQ ID NOs: 2880 and 4397 to 6493.
61. The isolated nucleic acid molecule of embodiment 60, wherein the nucleic acid sequence is operably linked to a promoter.
62. The isolated nucleic acid molecule of embodiment 61, wherein the promoter comprises a nucleic acid sequence at least 95% identical to a nucleic acid sequence selected from the group consisting of SEQ ID NOs: 4250 to 4254, 4258 to 4307, 4309 to 4312, 4386, and 6848 to 6850.
63. A modified arthropod comprising the isolated nucleic acid molecule of any one of embodiments 57 to 62.
64. A modified arthropod comprising the male sterile effector of any one of embodiments 1 to 31.
65. A modified arthropod comprising the one or more isolated nucleic acid molecules of embodiment 38.
66. A modified arthropod comprising the sterility system of any one of embodiments 39 to 55 or 180 to 192.
67. A modified arthropod comprising the one or more isolated nucleic acid molecules of embodiment 56.
68. A modified arthropod comprising a male sterile effector, wherein the male sterile effector comprises a loss-of-function mutation in at least one male-fertility essential gene positioned within a Y chromosome or within an M-locus.
69. The modified arthropod of embodiment 68, wherein the modified arthropod further comprises a rescue element comprising a functional allele of the at least one male-fertility essential gene.
70. The modified arthropod of embodiment 68 or 69, wherein the modified arthropod further comprises a repressible female-lethal effector.
71. The modified arthropod of embodiment 64, wherein part (a) of the male sterile effector is positioned within a Y chromosome or an M-locus of the modified arthropod.
72. The modified arthropod of embodiment 64 or 71, wherein part (b) of the male sterile effector is positioned within a Y chromosome or an M-locus of the modified arthropod.
73. The modified arthropod of embodiment 64, wherein part (a) of the male sterile effector is positioned within an autosome of the modified arthropod.
74. The modified arthropod of embodiment 64 or embodiment 73, wherein part (b) of the male sterile effector is positioned within an autosome of the modified arthropod.
75. The modified arthropod of any one of embodiments 64 or 71 to 74, wherein part (c) of the male sterile effector is positioned within a Y chromosome or an M-locus of the modified arthropod.
76. The modified arthropod of any one of embodiments 64 or 71 to 74, wherein part (c) of the male sterile effector is positioned within an autosome of the modified arthropod.
77. The modified arthropod of embodiment 66, wherein part (a) of the male sterile effector is positioned within a Y chromosome or an M-locus of the modified arthropod.
78. The modified arthropod of embodiment 66 or embodiment 77, wherein part (b) of the male sterile effector is positioned within a Y chromosome or an M-locus of the modified arthropod.
79. The modified arthropod of embodiment 66, wherein part (a) of the male sterile effector is positioned within an autosome of the modified arthropod.
80. The modified arthropod of embodiment 66 or embodiment 79, wherein part (b) of the male sterile effector is positioned within an autosome of the modified arthropod.
81. The modified arthropod of any one of embodiments 66 to 80, wherein the rescue element is positioned within an X chromosome or an m-locus of the modified arthropod.
82. The modified arthropod of any one of embodiments 66, 67, or 70 to 81, wherein the repressible female-lethal effector is positioned within an X chromosome, an m-locus, or an autosome.
83. The modified arthropod of any one of embodiments 63 to 82, wherein the modified arthropod is an insect.
84. The modified arthropod of embodiment 83, wherein the insect is selected from an Order selected from group consisting of Ephemeroptera, Dermaptera, Orthoptera, Mantodea, Blattodea, Psocodea, Hemiptera, Thysanoptera, Hymenoptera, Coleoptera, Neuroptera, Lepidoptera, Diptera, Siphonaptera, and Zygentoma.
85. The modified arthropod of embodiment 84, wherein the insect from the Order Lepidoptera is a Heliothinae species.
86. The modified arthropod of embodiment 79, wherein the insect from the Order Diptera is of a genus selected from the group consisting *of Aedes, Anopheles, Culex,* and *Drosophila.*
87. The modified arthropod of embodiment 83, wherein the insect is selected from the group consisting of *Acyrthosiphon pisum, Adelges tsugae, Aedes aegypti, Aedes albopictus, Agrilus planipennis, Amyelois transitella, Anarsia lineatella, Anastrepha ludens, Anastrepha obliqua, Anastrepha suspensa, Anopheles arabiensis, Anopheles coluzii, Anopheles funestus, Anopheles gambiae, Anopheles stephensi, Anoplophora glabripennis, Anthonomous grandis, Anthonomus grandis, Aonidiella aurantia, Aphis gossypii, Apis mellifera scutellata, Bactrocera cucurbitae, Bactrocera dorsalis, Bactrocera oleae, Bactrocera tyroni, Bactrocera zonata, Bagrada hilaris, Bemisia tabaci, Bombyx mori, Callosobruchus maculatus, Ceratitis capitata, Ceratitis rosa, Chrysomya bezziana, Cochliomyia aldrichi, Cochliomyia hominivorax, Cochliomyia macellaria, Cochliomyia minima, Coptotermes formosanus, Culex quinquefasciatus, Cydia pomonella, Cydia pomonella, Dalbulus maidis, Dendroctonus frontalis, Dermatobia hominis, Diabrotica balteata, Diabrotica barberi, Diabrotica undecimpunctata, Diabrotica virgifera, Diaphorina citri, Drosophila suzukii, Epiphyas postvittana, Frankliniella occidentalis, Glossina austeni, Glossina brevipalpis, Glossina fusca, Glossina fuscipes, Glossina fuscipleuris, Glossina longipalpis, Glossina longipennis, Glossina morsitans, Glossina pallidipes, Glossina palpalis, Glossina swynnertoni, Glossina tabaniformis, Glossina tachinoides, Graphocephala atropunctata, Haematobia irritans, Halyomorpha halys, Helicoverpa armigera, Helicoverpa zea, Homalodisca vitripennis, Hypoderma bovis, Hypoderma lineatum, Leptinotarsa decemlineata, Leucinodes orbonalis, Lobesia botrana, Lucilia cuprina, Lucilia cuprina, Lytzomyia spp., Lycorma delicatula, Lymantria albescens, Lymantria dispar, Lymantria dispar asiatica, Lymantria dispar japonica, Lymantria postalba, Lymantria umbrosa, Musca domestica, Myzus persicae, Naupactus spp. (Graphognatus spp.), Nilaparvata lugens, Oestridae spp., Oryctes rhinoceros, Pectinophora gossypiella, Pectinophora scutigera, Pityophthorus juglandis, Planococcus ficus, Plutella xylostella, Popillia japonica, Rhagoletis cerasi, Rhodnius prolixus, Schistocerca gregaria, Selenothrips rubrocinctus, Sirex noctilio, Solenopsis invicta, Solenopsis richteri, Spodoptera litura, Spodotera exigua, Tabanus sulcifrons, Thaumatotibia leucotreta, Thrips tabaci, Tribolium castaneum, Trogoderma granarium, Tryporyza incertulas, Tuta absoluta,* and *Varroa destructor.*
88. The modified arthropod of embodiment 83, wherein the insect is an *Aedes aegypti* mosquito.
89. The modified arthropod of embodiment 83, wherein the insect is a *Drosophila suzukii* fly.
90. The modified arthropod of any one of embodiments 63 to 82, wherein the modified arthropod is an arachnid.
91. The modified arthropod of embodiment 90, wherein the arachnid is of an Order selected from the group consisting of Acariformes and Parasitiformes.
92. The modified arthropod of embodiment 90, wherein the arachnid is selected from a group consisting of *Ixodes scapularis, Ixodes pacificus, Amblyomma americanum, Amblyomma maculatum, Dermacentor variabilis, Haemaphysalis longicornis, Rhipicephalus microplus, Rhipicephalus annulatus, and Rhipicephalus sanguineus.*
93. The modified arthropod of embodiment 90, wherein the arachnid is selected from the group consisting of *Tertanychus urticae* and *Varroa destructor.*
94. The modified arthropod of any one of embodiments 63 to 93, wherein the modified arthropod is a male arthropod.
95. The modified arthropod of any one of embodiments 63 to 93, wherein the modified arthropod is a female arthropod.
96. A method of generating a population of progeny arthropods, the method comprising:
   (a) providing a male modified arthropod comprising the sterility system of any one of embodiments 39 to 55 or 180 to 192; and
   (b) allowing the male modified arthropod to mate with a wildtype female arthropod of the same species to produce the population of progeny arthropods, wherein the population of progeny arthropods comprises:
      (i) at least one sterile male progeny arthropod,
      (ii) at least one inviable female progeny arthropod, or
      (iii) both (i) and (ii).
97. The method of embodiment 96, wherein the method further comprises: (c) selecting the at least one sterile male progeny arthropod.
98. The method of embodiment 97, wherein the method further comprises: (d) allowing the at least one sterile male progeny arthropod selected in step (c) to mate with a wildtype female arthropod of the same species.
99. The method of embodiment 96, wherein the method further comprises: (c) allowing the at least one sterile male progeny arthropod to mate with a wildtype female arthropod of the same species.
100. A method of generating a population of progeny arthropods, the method comprising:
   (a) providing a male modified arthropod comprising the sterility system of any one of embodiments 39 to 55 or 180 to 192; and
   (b) allowing the male modified arthropod to mate with a wildtype female arthropod of the same species to produce the population of progeny arthropods, wherein the population of progeny comprises a subpopulation of male progeny arthropods and a subpopulation of female progeny arthropods, wherein at least 25% of the male progeny arthropods are sterile and wherein at least 25% of the female progeny arthropods are inviable.
101. The method of embodiment 100, wherein the method further comprises: (c) selecting at least one sterile arthropod from the subpopulation of male progeny arthropods.
102. The method of embodiment 101, wherein the method further comprises: (d) allowing the at least one sterile arthropod from the subpopulation of male progeny arthropods selected in step (c) to mate with a wildtype female arthropod of the same species.
103. The method of embodiment 100, wherein the method further comprises: (c) allowing at least one sterile arthropod from the subpopulation of male progeny arthropods to mate with a wildtype female arthropod of the same species.
104. A method of generating a population of progeny arthropods, the method comprising:
   (a) providing a male modified arthropod comprising the sterility system of any one of embodiments 39 to 55 or 180 to 192;
   (b) providing a wildtype female arthropod of the same species to the male modified arthropod; and
   (c) providing conditions which allow the male modified arthropod to mate with the wildtype female arthropod to produce the population of progeny arthropods, wherein the population of progeny comprises:
      (i) at least one sterile male progeny arthropod,
      (ii) at least one inviable female progeny arthropod, or
      (iii) both (i) and (ii).
105. The method of embodiment 104, wherein the method further comprises: (d) selecting the at least one sterile male progeny arthropod.
106. The method of embodiment 105, wherein the method further comprises: (e) allowing the at least one sterile male progeny arthropod selected in step (d) to mate with a wildtype female arthropod of the same species.
107. The method of embodiment 104, wherein the method further comprises: (d) allowing the at least one sterile male progeny arthropod to mate with a wildtype female arthropod of the same species.
108. A method of generating a population of progeny arthropods, the method comprising:
   (a) providing a male modified arthropod comprising the sterility system of any one of embodiments 39 to 55 or 180 to 192;
   (b) providing a wildtype female arthropod of the same species to the male modified arthropod; and
   (c) providing conditions which allow the male modified arthropod to mate with the wildtype female arthropod to produce the population of progeny arthropods, wherein the population of progeny arthropods comprises a subpopulation of male progeny and a subpopulation of female progeny, wherein at least 25% of the male progeny are sterile and wherein at least 25% of the female progeny are inviable.
109. A method comprising:
   (a) obtaining at least one modified male arthropod comprising the sterility system of any one of embodiments 39 to 55 or 180 to 192; and
   (b) releasing the at least one male modified male arthropod outdoors in the vicinity of at least one wildtype female arthropod of the same species.
110. A method comprising:
   (a) obtaining the modified arthropod of any one of embodiments 63 to 95, wherein the modified arthropod is a male arthropod; and
   (b) releasing the modified arthropod outdoors in the vicinity of at least one wildtype female arthropod of the same species.
111. The method of embodiment 108, wherein the method further comprises: (d) selecting at least one sterile arthropod from the subpopulation of male progeny arthropods.
112. The method of embodiment 111, wherein the method further comprises: (e) allowing the at least one sterile arthropod from the subpopulation of male progeny arthropods selected in step (c) to mate with a wildtype female arthropod of the same species.
113. The method of embodiment 108, wherein the method further comprises: (d) allowing at least one sterile arthropod from the subpopulation of male progeny arthropods to mate with a wildtype female arthropod of the same species.
114. The method of any one of embodiments 96 to 108 or 111 to 113, wherein the method occurs outdoors.
115. The method of any one of embodiments 96 to 108 or 111 to 113, wherein at least one step of the method occurs outdoors.
116. The method of any one of embodiments 96 to 108 or 111 to 113, wherein the method occurs in a controlled environment.
117. The method of any one of embodiments 96 to 108 or 111 to 113 or 115, wherein at least one step of the method occurs in a controlled environment.
118. The method of any one of embodiments 96 to 117, wherein the male modified arthropod is an adult arthropod.
119. The method of any one of embodiments 96 to 117, wherein the male modified arthropod is a larval arthropod.
120. The method of any one of embodiments 96 to 117, wherein the male modified arthropod is an embryonic arthropod.
121. The method of any one of embodiments 96 to 117, wherein the male modified arthropod is nymph arthropod.
122. The method of any one of embodiments 96 to 117, wherein the male modified arthropod is a pupal arthropod.
123. The method of any one of embodiments 96 to 122, wherein the wildtype female arthropod is an adult arthropod.
124. The method of embodiment 97, wherein the at least one sterile male progeny arthropod selected in step (c) is selected from the group consisting of an embryonic arthropod, a larval arthropod, a pupal arthropod, and an adult arthropod.
125. The method of embodiment 101, wherein the at least one sterile arthropod selected in step (c) is selected from the group consisting of an embryonic arthropod, a larval arthropod, a pupal arthropod, and an adult arthropod.
126. The method of embodiment 105, wherein the at least one sterile male progeny arthropod selected in step (d) is selected from the group consisting of an embryonic arthropod, a larval arthropod, a pupal arthropod, and an adult arthropod.
127. The method of embodiment 111, wherein the at least one sterile arthropod selected in step (d) is selected from the group consisting of an embryonic arthropod, a larval arthropod, a pupal arthropod, and an adult arthropod.
128. The method of any one of embodiments 96 to 127, wherein the male modified arthropod and the wildtype female arthropod are insects.
129. The method of embodiment 128, wherein the insects are selected from an Order selected from group consisting of Ephemeroptera, Dermaptera, Orthoptera, Mantodea, Blattodea, Psocodea, Hemiptera, Thysanoptera, Hymenoptera, Coleoptera, Neuroptera, Lepidoptera, Diptera, Siphonaptera and Zygentoma.
130. The method of embodiment 129, wherein the insects from the Order Lepidoptera are a Heliothinae species.
131. The method of embodiment 129, wherein the insects from the Order Diptera are of a genus selected from the group consisting of *Aedes, Anopheles, Culex,* and *Drosophila.*
132. The method of embodiment 128, wherein the insects are selected from the group consisting of *Acyrthosiphon pisum, Adelges tsugae, Aedes aegypti, Aedes albopictus, Agrilus planipennis, Amyelois transitella, Anarsia lineatella, Anastrepha ludens, Anastrepha obliqua, Anastrepha suspensa, Anopheles arabiensis, Anopheles coluzii, Anopheles funestus, Anopheles gambiae, Anopheles stephensi, Anoplophora glabripennis, Anthonomous grandis, Anthonomus grandis, Aonidiella aurantia, Aphis gossypii, Apis mellifera scutellata, Bactrocera cucurbitae, Bactrocera dorsalis, Bactrocera oleae, Bactrocera tyroni, Bactrocera zonata, Bagrada hilaris, Bemisia tabaci, Bombyx mori, Callosobruchus maculatus, Ceratitis capitata, Ceratitis rosa, Chrysomya bezziana, Cochliomyia aldrichi, Cochliomyia hominivorax, Cochliomyia macellaria, Cochliomyia minima, Coptotermes formosanus, Culex quinquefasciatus, Cydia pomonella, Cydia pomonella, Dalbulus maidis, Dendroctonus frontalis, Dermatobia hominis, Diabrotica balteata, Diabrotica barberi, Diabrotica undecimpunctata, Diabrotica virgifera, Diaphorina citri, Drosophila suzukii, Epiphyas postvittana, Frankliniella occidentalis, Glossina austeni, Glossina brevipalpis, Glossina fusca, Glossina fuscipes, Glossina fuscipleuris, Glossina longipalpis, Glossina longipennis, Glossina morsitans, Glossina pallidipes, Glossina palpalis, Glossina swynnertoni, Glossina tabaniformis, Glossina tachinoides, Graphocephala atropunctata, Haematobia irritans, Halyomorpha halys, Helicoverpa armigera, Helicoverpa zea, Homalodisca vitripennis, Hypoderma bovis, Hypoderma lineatum, Leptinotarsa decemlineata, Leucinodes orbonalis, Lobesia botrana, Lucilia cuprina, Lucilia cuprina, Lutzomyia spp., Lycorma delicatula, Lymantria albescens, Lymantria dispar, Lymantria dispar asiatica, Lymantria dispar japonica, Lymantria postalba, Lymantria umbrosa, Musca domestica, Myzus persicae, Naupactus spp. (Graphognatus spp.), Nilaparvata lugens, Oestridae spp., Oryctes rhinoceros, Pectinophora gossypiella, Pectinophora scutigera, Pityophthorus juglandis, Planococcus ficus, Plutella xylostella, Popillia japonica, Rhagoletis cerasi, Rhodnius prolixus, Schistocerca gregaria, Selenothrips rubrocinctus, Sirex noctilio, Solenopsis invicta, Solenopsis richteri, Spodoptera litura, Spodotera exigua, Tabanus sulcifrons, Thaumatotibia leucotreta, Thrips tabaci, Tribolium castaneum, Trogoderma granarium, Tryporyza incertulas, Tuta absoluta,* and *Varroa destructor.*
133. The method of embodiment 128, wherein the insects are *Aedes aegypti.*
134. The method of embodiment 128, wherein the insects are *Drosophila suzukii.*
135. The method of any one of embodiments 96 to 118, 120, or 123, wherein the modified arthropod is an arachnid.
136. The method of embodiment 135, wherein the arachnid is of an Order selected from the group consisting of Acariformes and Parasitiformes.
137. The method of embodiment 135, wherein the arachnid is selected from the group consisting of *Tertanychus urticae* and *Varroa destructor.*
138. The method of embodiment 135, wherein the arachnid is *Ixodes scapularis.*
139. A method comprising:
   (a) obtaining a male population of the modified arthropod of any one of embodiments 63 to 95; and
   (b) releasing the male population outdoors in the vicinity of a wildtype population of the arthropod that comprises at least one wildtype female arthropod of the same species,
   wherein at least one member of the male population of the modified arthropod mates with the at least one wildtype female arthropod, and wherein the at least one wildtype female arthropod produces (i) at least one sterile male progeny, (ii) at least one inviable female progeny, or (iii) both (i) and (ii).
140. The method of embodiment 139, wherein the modified arthropod is selected from the group consisting of *Drosophila suzukii* and *Aedes aegypti.*
141. The method of embodiment 139 or 140, wherein the male population of the modified arthropod comprises between 1 male arthropod and 1,000,000,000,000 male arthropods.
142. The method of any one of embodiments 139 to 141, wherein the releasing comprises a single release.
143. The method of any one of embodiments 139 to 141, wherein the releasing comprises multiple releases.
144. The method of embodiment 143, wherein the multiple releases occur on the same day.
145. The method of embodiment 143, wherein the multiple releases each occur on different days.
146. The method of any one of embodiments 139 to 145, wherein the male population released in step (b) comprises at least one adult male arthropod.
147. The method of any one of embodiments 139 to 146, wherein the male population released in step (b) comprises at least one male arthropod embryo.
148. The method of any one of embodiments 139 to 147, wherein the male population released in step (b) comprises at least one male larval arthropod.
149. The method of any one of embodiments 139 to 148, wherein the male population released in step (b) comprises at least one male pupal arthropod.
150. A method comprising:
   (a) introducing:
      (i) the male sterile effector of any one of embodiments 1 to 31; or
      (ii) the sterility system of any one of embodiments 39 to 55 or 180 to 192, to at least one arthropod cell; and
   (b) obtaining an adult arthropod from the at least one arthropod cell, wherein the adult arthropod comprises the male sterility effector or the sterility system.
151. A method comprising:
   (a) introducing:
      (i) the one or more isolated nucleic acid molecules of embodiment 38;
      (ii) the one or more isolated nucleic acid molecules of embodiment 56;
      (iii) the isolated nucleic acid molecule of any one of embodiments 57 to 59; or
      (iv) the isolated nucleic acid molecule of any one of embodiments 60 to 62 to at least one arthropod cell; and
   (b) obtaining an adult arthropod from the at least one arthropod cell, wherein the adult arthropod comprises at least one of the isolated nucleic acid molecules.
152. The method of embodiment 150 or 151, wherein the at least one arthropod cell is selected from the group consisting of a *Drosophila suzukii* cell and an *Aedes aegypti* cell.
153. An arthropod produced by the method of any one of embodiments 150 to 152.
154. A sterility system comprising:
   (a) a male sterile effector comprising a loss-of-function mutation in at least one male-fertility essential gene; and
   (b) a repressible female-lethal effector.
155. A sterility system comprising:
   (a) a male sterile effector comprising a loss-of-function mutation in at least one male-fertility essential gene; and
   (b) a rescue element comprising a first promoter operably linked to a functional copy of the at least one male-fertility essential gene.
156. A sterility system comprising:
   (a) a male sterile effector comprising a loss-of-function mutation in at least one male-fertility essential gene;
   (b) a rescue element comprising a first promoter operably linked to a functional copy of the at least one male-fertility essential gene; and
   (c) a repressible female-lethal effector.
157. The sterility system of any one of embodiments 154 to 156, wherein the functional copy of the at least one male-fertility essential gene comprises a codon-scrambled allele of the at least one male-fertility essential gene.
158. The sterility system of any one of embodiments 155 to 157, wherein the first promoter comprises at least a functional fragment of the endogenous promoter of the at least one male-fertility essential gene.
159. One or more isolated nucleic acid molecules encoding the sterility system of any one of embodiments 154 to 158.
160. An arthropod comprising the sterility system of any one of embodiments 154 to 158.
161. The arthropod of embodiment 160, wherein the arthropod is selected from the group consisting *of Aedes aegypti* and *Drosophila suzukii.*
162. The arthropod of embodiment 160 or 161, wherein the functional copy of the at least one male-fertility essential gene comprises a naturally-occurring ortholog of the at least one male-fertility essential gene from another arthropod species.
163. The arthropod of embodiment 162, wherein the naturally-occurring ortholog is from another arthropod species in the same Genus as the arthropod.
164. The arthropod of embodiment 162, wherein the naturally-occurring ortholog is from another arthropod species in the same Family as the arthropod.
165. The arthropod of embodiment 162, wherein the naturally-occurring ortholog is from another arthropod species in the same Order as the arthropod.
166. The arthropod of any one of embodiments 160 to 165, wherein the male sterile effector is positioned within a Y chromosome or an M-locus of the arthropod.
167. The arthropod of any one of embodiments 160 to 165, wherein the male sterile effector is positioned within an autosome of the arthropod.
168. The arthropod of any one of embodiments 160 to 167, wherein the rescue element is positioned within an X chromosome or an m-locus of the arthropod.
169. A method comprising:
   (a) introducing a recoded rescue element into a genome of a first arthropod cell, wherein the recoded rescue element encodes a male-fertility essential gene;
   (b) further introducing a loss-of-function mutation in an endogenous allele of the male-fertility essential gene into the genome; and
   (c) obtaining a male arthropod comprising the recoded rescue element and the loss-of-function mutation.
170. A method comprising:
   (a) introducing a loss-of-function mutation in an endogenous allele of a male-fertility essential gene in at least one arthropod cell, wherein the at least one arthropod cell further comprises a recoded recue element encoding a functional copy of the male-fertility essential gene; and
   (b) obtaining an adult male arthropod from the at least one arthropod cell, wherein the adult male arthropod comprises the loss-of-function mutation and the recoded rescue element.
171. The method of embodiment 169 or 170, wherein the endogenous allele of the male-fertility essential gene is positioned within a Y chromosome or an M-locus.
172. The method of any one of embodiments 169 to 171, wherein the recoded rescue element is positioned within an X chromosome or an m-locus.
173. A method comprising:
   (a) obtaining the modified male arthropod of any one of embodiments 160 to 167;
   (b) allowing the modified male arthropod to mate with a modified female arthropod of the same species, wherein the modified female arthropod is homozygous for the recoded rescue element on an X chromosome or m-locus, to produce a progeny population,
   wherein the progeny population comprises at least one fertile modified male arthropod comprising the male sterility effector and the recoded rescue element.
174. The method of embodiment 173, wherein the progeny population further comprises at least one fertile modified female arthropod.
175. The method of embodiment 173 or 174, wherein the modified male arthropod and the modified female arthropod both further comprise the repressible female-lethal effector on an X chromosome, an m-locus, or an autosome.
176. The method of embodiment 175, wherein step (b) further comprises providing the modified female arthropod a repressor of the repressible female lethal effector.
177. The method of embodiment 176, wherein the repressor is tetracycline or doxycycline.
178. A method comprising:
   (a) obtaining the modified male arthropod of any one of embodiments 160 to 167;
   (b) allowing the modified male arthropod to mate with at least one wildtype female arthropod of the same species to produce a progeny population; and
   (c) selecting at least one sterile male arthropod from the progeny population.
179. A method comprising:
   (a) providing the modified male arthropod of any one of embodiments 160 to 167 and at least one wildtype female arthropod of the same species;
   (b) obtaining a progeny population produced by the modified male arthropod and the at least one wildtype female arthropod; and
   (c) selecting at least one sterile male arthropod from the progeny population.
180. The method of any one of embodiments 173 to 179, wherein the modified male arthropod is a modified male *Aedes aegypti* mosquito or a modified male *Drosophila suzukii* fly.
181. The sterility system of any one of embodiments 39, 41 to 55, 154, or 156 to 158, wherein the repressible female-lethal effector is operably linked to a promoter.
182. The sterility system of any one of embodiments 39, 41 to 55, 154, 156 to 158, or 181, wherein the promoter is selected from the group consisting of a TRE promoter, an *hsp83* promoter, and a *ubiquitin* promoter.
183. The sterility system of any one of embodiments 39, 41 to 55, 154, 156 to 158, 181, or 182 wherein the repressible female-lethal effector is selected from the group consisting of: *Hid, grim, reaper, NipplDm,* calmodulin, *Michelob-X,* tTA, and tTAV, or variants thereof.
184. The sterility system of any one of embodiments 39, 41 to 55, 154, 156 to 158, 181, or 182 wherein the repressible female-lethal effector is a Class IV effector.
185. The sterility system of any one of embodiments 39, 41 to 55, 154, 156 to 158, 181, or 182 wherein the repressible female-lethal effector is a proapoptotic gene.
186. The sterility system of any one of embodiments 39, 41 to 55, 154, 156 to 158, or 181 to 185, wherein the repressible female-lethal effector comprises a female-specific splice module.
187. The sterility system of embodiment 186, wherein the female-specific splice module is selected from the group consisting of a *tra* female-specific splice module, a *dsx* female-specific splice module, and an *actin-4* female-specific splice module.
188. The sterility system of any one of embodiments 39, 41 to 55, 154, 156 to 158, or 181, wherein the promoter is a TRE promoter and the female-lethal effector is selected from the group consisting of tTA, and tTAV, or variants thereof.
189. The sterility system of any one of embodiments 39, 41 to 55, 154, 156 to 158, or 181, wherein the promoter is an *hsp83* promoter and the female-lethal effector is selected from the group consisting of tTA, and tTAV, or variants thereof.
190. The sterility system of any one of embodiments 182 to 188, wherein the TRE promoter comprises at least one copy of a tetO sequence operably linked to a minimal promoter.
191. The sterility system of embodiment 190, wherein the TRE promoter comprises at least 7 copies of the tetO sequence.
192. The sterility system of embodiment 190 or 191, wherein the minimal promoter is selected from the group consisting of an *hsp70* minimal promoter, a *DSCP* minimal promoter, a *pgd* minimal promoter, and an *sry-a* minimal promoter.
193. The sterility system of any one of embodiments 39, 41 to 55, 154, 156 to 158, or 181 to 192, wherein the sterility system further comprises a second female-lethal effector.
194. The modified arthropod of any one of the preceding embodiments, wherein the male sterile effector is positioned within 1 centimorgan (cM) of an M-locus.
195. The modified arthropod of any one of the preceding embodiments, wherein the male sterile effector is positioned within 50 kilobase pairs of the male-determining gene *Nix,* and wherein the modified arthropod is a modified *Aedes aegypti* or a modified *Aedes albopictus.*
196. The modified arthropod of any one of the preceding embodiments, wherein the rescue element is positioned within 1 cM of an m-locus.
197. The modified arthropod of embodiment 196, wherein the modified arthropod is a modified *Aedes aegypti* arthropod and the m-locus is positioned within 50 kilobase pairs of a locus selected from the group consisting of AAEL019619, AAEL006712, AAEL022649, AAEL014760, AAEL020950, AAEL021928, AAEL027507, AAEL019917, AAEL006708, AAEL006707, AAEL006713, AAEL006712, AAEL019921, AAEL025609, AAEL005160, AAEL005169, AAEL009784, AAEL001236, AAEL002407, AAEL014760, AAEL028070, AAEL015064, and AAEL019619.
198. The modified arthropod of embodiment 196, wherein the modified arthropod is a modified *Drosophila suzukii* arthropod and the m-locus is positioned within 50 kilobase pairs of a locus selected from the group consisting of LOC108015366 and LOC108016595.

Having now generally described the disclosure, the same will be more readily understood through reference to the following examples that are provided by way of illustration, and are not intended to be limiting of the present disclosure, unless specified.

### EXAMPLES

### Example 1. 2GEN System

The 2GEN system for arthropod population control is limited to two generations of persistence in the environment, possesses in-built sex-sorting technology, and enables greater than 10x efficiency as compared to previously known sterile male technologies. Other advantages of the 2GEN system as compared to prior technologies include a reduction in arthropod release frequency, no genetic introgression of trains, and freedom of selection of which arthropod life stage is released.

The 2GEN system comprises three main components: a male sterility effector, a repressible female-lethal effector, and a rescue element. *See* FIG. 1 and FIGs. 2A and 2B. The male sterility effector is positioned within a Y chromosome, an M-locus, or is homozygous on an autosome. The repressible female-lethal effector is positioned within an X chromosome, an m-locus, or an autosome. The rescue element is positioned within an X chromosome or m-locus in males and is homozygous on the X chromosome or m-locus in females.

In a typical, but non-limiting, rearing situation, females are provided that are homozygous on X chromosomes for both the repressible female-lethal effector and the rescue element, and the females are provided with a compound (e.g., tetracycline) to repress the female-lethal effector, and males are provided that possess the male sterility effector on a Y chromosome, and both the repressible female-lethal effector and the rescue element on the X chromosome. Females remain viable and fertile when reared in the presence of the compound that represses the female-lethal effector.

Prior to release, the compound that represses the female-lethal effector is removed. This causes the females to die without impacting the males. The males are then released to mate with wildtype females. In the first generation following this mating, female progeny inherit the repressible female-lethal effector, which kills the female progeny (e.g., they are inviable). First generation male progeny from this mating inherit the male sterility effector, but not the rescue element, leading to sterile males. The sterile males are then able to mate with wildtype females to produce a second generation, which is entirely inviable.

The 2GEN system is made possible by the innovation of an X-linked rescue element that allows a sterile male effector to be present on the Y chromosome (or homozygous on an autosome). In this way, sterile males can be reared at scale under laboratory conditions, with both transgenes in homozygosity to obviate the need for screening or sex-sorting. To generate sterile males that will bring about a population suppression, the male sterile effector must be released from its rescue by mating: by virtue of meiotic sex chromosome segregation, males carrying both the male sterile and rescue elements will transmit the male sterile effector to all their male progeny, and the rescue element only to their female progeny, thus rendering all male progeny sterile. This solves a significant issue in the field of insect-based control, which is a simple system to create sterile males that does not involve rearing independent transgenic strains (as in the pgSIT system), nor uses radiation that can severely impact fitness during the sterilization procedure. This approach also enables the use of male sterile effectors that generate partial sterility such as sex-ratio distortion systems that bias progeny towards males (including X-shredders) and X-poisoning systems and Y-linked editor systems that can produce mutations that selectively kill females (such as LOF mutations in haploinsufficient essential genes on the X chromosome).

The second innovation of the 2GEN system is the addition of the repressible female-lethal effector. This element allows arthropods to be reared under permissive conditions (e.g., when tetracycline is provided to a tetracycline-inducible repressor) and then sex-sort under release or natural environmental conditions by removing the repressor, thus activating the female-lethal effector.

A fourth innovation of the 2GEN system is the use of bi-partite expression systems to ensure that a rescue element is only active in the presence of a repressible female lethal effector or male sterility effector. This prevents rescue elements (e.g. antiCRISPR proteins) from interacting with other genetic control elements that may also be released into the environment.

The 2GEN system thus allows the creation of a scalable sterile male technology and a two generation-limited population control system that restricts the introgression of genetic traits into the environment.

### Example 2. Modeling the 2GEN System

The 2GEN system also allows for the release of sterile males that will persist for a single generation following release or fertile males that will produce sterile male progeny and inviable female progeny (e.g., that persists for two generations).

To model the 2GEN system, the most broadly applicable design criteria is assumed, i.e. one that does not require integration of the male sterile effector onto a sex chromosome, or the presence of Y-linked male germline-essential genes. The model assumes up to five independently segregating loci: a male-determining m-factor on an M locus (locus L1), an anticas-based rescue element on an X/m-locus (locus L2), a repressible female lethal effector on an autosome (locus L3), a male sterile effector on homozygous an autosome (locus L4), and a haplosufficient essential male fertility target gene on an autosome (locus L5). The model assumes the male determining M-locus is at locus L1, with varying linkage to the L2 locus. The model assumes the male sterile effector targets a male-fertility essential gene at another independently segregating location (locus L5), and that the gene is haplosufficient and required only for male fertility (e.g. a Class I target gene). The model assumes in the idealized case: 0% Female Pupation Success [FPS], equivalent to 100% female-specific pupal lethality; 0% Chromosomal Recombination Rate [CRR] between the X/m sex determining locus (locus L1) and the male determining M-locus (locus L1), corresponding to a distance of 0 centimorgan (cM); 0% Male Fertility [MF], corresponding to 100% cleavage at the target gene (locus L5) and no resistant allele generation (i.e. cleavage invariable results in a null mutation in the male-fertility essential gene). Finally, the model assumes 0% cleavage at the target gene (locus L5), and therefore 100% anticas fertility [AF], in males carrying the rescue element, irrespective of the male sterile effector. The idealized case is equivalent to a system wherein: 1) the male sterile effector is present on an autosome or on a Y chromosome; 2) the rescue is present on an X chromosome unable to recombine with the Y chromosome; and 3) the repressible female lethal element is present homozygous on an autosome or an X chromosome. Simulations are performed assuming a single release rate (RR) of 0.55 modified "2GEN" males compared to wild-type males in the target population, chosen on the basis of a Bisection Optimization Analysis over the same release period (6 months), release frequency (7 days), and target suppression (95% of fertile females). A series of simulations are performed assuming variable FPS (0, 0.001, 0.005, 0.01, 0.05), CRR (0, 0.005, 0.01, 0.05, 0.15), MF (0, 0.01, 0.05), AF (0.7, 0.95, 1). The model is based upon the MGDrivE framework *(See,* for example, Wu et al., PLoS Comput Biol., 17(5):e1009030 (2021), modified to include an inheritance cube compatible with the various configurations of the 2GEN system. To compare the 2GEN system against other genetic control technologies, the model assumes: 1) an optimal fsRIDL system wherein the genetic control element is equivalent to the optimal repressible female lethal effector alone (0% FPS); and 2) an optimal SIT / pgSIT system wherein a genetic control element is equivalent to the male sterile effector alone (0% MF), each introduced with the same release schema (0.55 RR, 7 day release frequency, 6 month release period). Further comparisons are performed for different release rates, release frequency, target suppression, and release period. The model allows total population, male population, female population, and individual genotypes to be tracked over time, pre- and post- release.

Modeling shows that the 2GEN system provides the greatest efficiency compared to optimal SIT and fsRIDL technologies in terms of speed of suppression and minimal total release number. *See* FIGs. 3A, 3B, and 3C.

Additional modeling is performed to investigate the impact of suboptimal performance for each of male sterility, female lethality, and rescue of male sterility, along with the rate of recombination between X and Y chromosomes (or M- and m-loci). The modeling is performed across two areas: the total number of released insects to generate strong levels of population suppression and the persistence of genetic traits in the target population after releases have stopped. *See* FIG. 4.

The 2GEN system is most sensitive to the repressible female lethal effector, wherein female lethality below 99% can allow long-term transgene persistence in a closed environment lacking migration. This reduces the long-term efficacy of suppression if release rates are kept constant. However, it is apparent that population full suppression can be achieved even with values of female lethality far below 99%, by increasing the release rate to remove fertile males from the subsequent generations. It is widely known that traits imposing even small fitness costs are rapidly selected out of natural populations. Thus, it is also apparent that long-term transgene persistence in a wild-population would be minimal for any configuration where the rescue element is genetically linked to the female lethal element, for any level of female lethality greater than 5%.

The other parameter which the 2GEN system is most sensitive to is the level of recombination between the X (or m-locus) and Y (or M-locus) chromosomes, whereby a recombination rate of 15% (the lower end explored) reduces the suppression to 80% (compared to 95% in the optimal scenario), and recombination rates above 0.5% lead to a small but perduring transgenic population post-release. Reduced suppression results from perdurance of the rescue element, and can be mitigated by linking the female lethal and rescue elements to promote its removal from the population. Nevertheless, full suppression can still be achieved by increasing the release rate to remove viable females in the subsequent population.

On the other hand, ineffective male sterility and rescue of male fertility leads to only a modest reduction in overall efficacy, essentially equivalent to fsRIDL in the worst case where males are not sterile. Similarly, the level of rescue of male sterility is not essential for efficacy, but has a directly proportional detrimental impact wherein the worst case of no rescue is essentially SIT, although a non-functional rescue would render the system impossible to rear.

With respect to the repressible female-lethal effector, the 2GEN system can achieve high levels of population suppression (>90%) for any rate of female killing greater than 99%. ***See*** **FIG. 4****, top row.** Decreasing the efficacy of the repressible female lethal effector leads to a small reduction in the speed and maximum level of suppression for a given release rate and time horizon, leading to an ineffective system for rates of female survival exceeding 5%.

The 2GEN genetic traits show increasing environmental persistence for decreasing repressible female lethal efficacy, for example, an increased perdurance from approximately 100 days to greater than 200 days after the final release if female survival increases from 0% to 0.5% is observed.

With respect to the male sterile effector, the 2GEN system can achieve high levels of population suppression (>90%) for any rate of male sterility greater than 95%. ***See*** **FIG. 4****, middle row.** In general, high rates of male sterility are not essential for the 2GEN system to drive robust population suppression or to limit trait persistence. Rather, male sterility is responsible for improvements in suppression potential over and above a repressible lethal effector on its own *(i.e.,* fsRIDL), and the worst case, where males are not sterile, results in a system essentially equivalent to fsRIDL.

With respect to the male sterile rescue, the 2GEN system can achieve high levels of population suppression for any rate of male sterility rescue greater than 70%. Greater than 90% suppression for any rate of male sterility greater than 95% using a release rate 5x lower than the threshold value for fsRIDL is observed. *See* **FIG. 4****, bottom row.** An ineffective rescue will turn the system in a classical sterile male technology, and thus will show efficacy equivalent to SIT, and any level of rescue will increase relative efficacy linearly towards that of the optimal 2GEN system.

### Example 3. Identification of Targets for Male Sterility Effectors

All methods designed to suppress a population of arthropods are susceptible to resistance whereby targeted arthropods are no longer suppressed. For example, resistance to insecticides has developed many times in various species to various insecticides. Similarly, it may be possible for targeted arthropods to develop resistance to male sterility effectors via a variety of mechanisms.

As discussed elsewhere herein, some embodiments of male sterile effectors include guide RNAs to direct an RNA-guided nuclease to cleave a target sequence or a microRNA molecule to guide cleavage of a target sequence. In order to mitigate resistance to male sterility effectors from occurring in a population, several methods can be used. Without being limiting, these methods include: targeting highly conserved sequences that show little-to-no sequence variation in target populations; targeting highly constrained sequences that may be unable to tolerate protein or nucleotide changes without incurring a strong fitness cost; targeting multiple sequences within a single gene; targeting multi-copy genes; and targeting multiple different genes simultaneously. Additionally, a loss-of-function mutation of a Y-linked fertility gene can also mitigate the development of resistance. Even if the mutated Y-linked fertility gene underwent a spontaneous reversion to a functional copy, the reversion would need to occur in a majority of released sterile males to significantly reduce the efficacy of the male sterility effector in the population.

Potential targets of male sterility factors are categorized into different classes. *See* FIG. 5. "Class I" target genes are target genes that are only required to function in the male germline *(e.g.,* without being limiting, *s-lap* genes, *β2-tubulin, ory, WDY, snky),* and their expression can be disrupted in non-male germline cells without impacting mating potential of the organism. Class I target genes can be further categorized based on whether they are Y-linked ("Class Ia") or autosomal ("Class Ib") genes. "Class II" target genes are essential for both male fertility and female fertility *(e.g.,* without being limiting, *vasa, zpg),* so their disruption must be restricted to males so as to not impact upon female viability and fertility during mass rearing of organisms. "Class III" target genes are target sequences where male sterility factor expression must be tightly restricted to the male germline (or another male reproductive tissue) to avoid impacting upon male fitness and mating potential. Class III target genes can be further categorized based on whether they are: fertility essential genes whose disruption would affect a one or more non-target tissues that are not essential for viability but could be required for mating competitiveness ("Class IIIa"; e.g., without being limiting, *mmm, sac, tous, hmw)* fertility-essential male sex determination genes whose disruption would affect a broad range of sexually dimorphic tissues in males but should have no impact in females ("Class IIIb"; *e.g.,* without being limiting, *yobl, nix);* housekeeping genes that are broadly expressed and essential in the male germline ("Class IIIc", *e.g.,* without being limiting, *bel, blw);* high copy number sequences whose disruption would cause programmed cell death ("Class IIId"; e.g., without being limiting, rDNA, transposable elements); X-linked high copy number sequences whose disruption during spermatogenesis would cause a male sex-bias in mature spermatozoa ("Class IIIe"; *e.g.,* without being limiting, *28S rDNA* in *Anopheles gambiae,* transposable elements); or X-linked haploinsufficient / haplolethal genes whose disruption during spermatogenesis would cause lethality in female progeny ("Class IIIf"; *e.g.,* without being limiting, *wupA, RpS6, Rps5a, Rps19a, Rps35).* "Class IV" effectors include cell-lethal toxins (such as, without being limiting, the pro-apoptotic genes *bax, hid, grim, reaper, fasl,* and *caspases* or toxins such as tTa, diptheria toxin, and ricin toxin) for which expression should be limited to the male germline or another male reproductive tissue not required for mating.

With the above considerations in mind, a cross-species whole genome analysis is performed to identify genes that have a putative essential role in male fertility in arthropods. The search involves literature review, examining FlyBase *(see* Öztürk-Çolak et al., Genetics, 227(1):iyad211 (2024); Jenkins et al., Methods Mol. Biol., 2540:1-34 (2022)), and using bioinformatics approaches based on gene expression profiles.

FlyBase is searched for all genes associated with a mutant allele that produces a sterile phenotype in *Drosophila melanogaster,* and identified alleles are further inspected to determine which have strong evidence for having a role in fertility, but do not associate with other reduced fitness phenotypes. The identified genes are classified into different groups depending on whether the phenotype is sex-specific, if the phenotype is embryo lethal or adult sterile, and whether the gene is known to be involved in meiosis. Results are provided in **FIG. 6****.**

Each identified gene from FIG. 6 is further inspected for its presence amongst highly abundant sperm proteins (*e.g.*, structural proteins expressed at late stages of spermatogenesis). Putative functional orthologs are identified in the mosquito species *Aedes aegypti* and *Anopheles gambiae* using Flybase, VectorBase (see Alvarez-Jarreta et al., Nucleic Acids Research, 52(D1):D808-D816 (2023)), and OrthoDB *(see* Kuznetsov et al., Nucleic Acids Research, 51(D1):D445-D451 (2023)). Identified *Anopheles* orthologs are examined to determine if they comprise any ultraconserved elements that could be targeted to mitigate resistance. *See,* for example, O'Loughlin et al., G3, 11(6):jkab086 (2021).

In total, 1168 genes associated with a sterile allele are identified in *D. melanogaster.* Of these genes, 478 are associated with male sterility, 67 are male sterile with no other associated phenotypes (male sterile only, MSO), 170 are associated with both male and female sterility (male female sterile, MFS), and 61 are associated with male sterility or a male sterile paternal effect in 50% to 99% of well-described alleles (male sterile only enriched, MSOE). A further 16 are associated with embryonic lethality post-fertilization (paternal effect lethal, PEL).

The 67 MSO genes are: *kl-2, kl-3, kl-5, sa, can, Hsp60B, mia, tRNA:Leu-CAA-1-1, fzo, tRNA:Tyr-GTA-1-3, Inos, topi, Ubi-p63E, PSMG1, Roclb, Pex12, JYalpha, S-Lap3, mmm, gudu, Gld2, knon, Theg, Dnah3, tomb, t-Grip84, Dpy-30L2, sac, asun, ssp3, S-Lap7, CG8236, S-Lap4, spag4, nsr, S-Lap8, loopin-1, CG12069, tbrd-1, dj, ms(3)76Ba, Rcd7, ms(3)76Cc, Vtld, fws, cmb, Kpc1, ttm2, tut, S-Lap2, tbrd-2, papi, dany, SCaMC, salto, S-Lap5, Rsbp15, Rsph3, Rsphl, Rsph4a, Cyp40, Kpc2, lncRNA:CR45362, eIF4E5, Tssk, WDY, Atg8b.*

The 61 MSOE genes are: *betaTub85D, Dlc90F, mei-S332, mlt, ms(2)34Fe, Mst77F, eIF4G2, aly, Rb97D, Cyt-c-d, dbf, pelo, Trf, Cds, eIF4E3, blanks, zpg, stet, Pdelc, rau, bond, Uspl4, ntc, mfr, thoc5, brun, Cep135, gdrd, Dis3l2, Dnaaf3, aust, nht, Cap-H2, soti, klhl10, ATPsynbetaL, Vrpl, Prosalpha6T, mfrn, Pex10, mtsh, Osbp, Ntl, PiflA, Cyt-clL, mil, ScsbetaA, VhaPPAl-2, CG9920, bb8, Duba, Prosalpha4T1, xmas, HBS1, S-Lap1, Rsph9, wam, Erk7, maca, COX4L, Mst27D.*

The 16 PEL gene are: *zip, ms(3)K81, snky, loqs, l(2)gl, HP1e, Hsc70-1, Hsc70-2, Hsc70-3, Hsc70-5, Hsc70-4, Caf1-55, Cyt-clL, ddbt, shop, Hyls1.*

The 8 RNAi sterile only genes are: *loqs, Hsc70-1, Hsc70-2, Hsc70-3, Hsc70-5, Hsc70-4, Caf1-55, Hylsl.*

The 170 MFS genes are: *14-3-3epsilon, abd-A, Abd-B, ac, Acn, aop, ap, ara, asl, asp, aub, aux, ball, bam, bel, bgen, bond, Brca2, brul, ca, Cad99C, caup, chb, Chdl, chic, CIAPIN1, cic, clu, cn, cnn, crm, CSN5, csul, ct, ctp, CycB, Cyt-b5, Dab, dally, Dark, DCTN5-p25, Ddxl, del, dia, Diap1, Dnaaf5, Doa, Dronc, dsx, EcR, eff, eIF4E1, endos, exu, eya, f, fbl, fry, fz2, gish, Gorab, Grip128, Grip75, Grip91, hay, hfp, hop, Hr39, Hr51, hts, hyd, InR, Iswi, Kdm5, Klp3A, l(2)gl, Lam, larp, lds, Lis-1, lncRNA:iab4, IncRNA:iab8, lz, m, mael, mago, mam, mei-P26, mino, mip120, mip40, mod(mdg4), mxc, Myc, neb, noi, Nop60B, Nup154, nw, Orc1, osk, otu, pAbp, park, pbl, Pdkl, pelo, Pen, Pfas, Phb1, piwi, Pngl, Polr2A, qm, Ran, Rm62, rn, Rpp30, RpS27A, RpS3, rst, rt, SAK, sbr, sc, scny, Sf3a1, Shc, shu, sim, sina, sm, SmB, snRNA:U7, spd-2, spn-E, SRPK, Stat92E, stau, stet, stg, sti, su(Hw), Su(var)3-3, sub, Sxl, Sym, Syx13, Taf6, tj, Top2, Top3alpha, tra, tra2, Trel, Trl, tsr, tud, twe, U2A, upd1, v, vg, vls, Vmat, vret, wls, wts, zpg, Zwl.*

A literature review of genes known to play an essential role in spermatogenesis confirms the suitability of the database mining described above, and identified several additional male-fertility essential genes.

Several genes known to play a role exclusively in male fertility are identified, including: Y-linked fertility factors involved with sperm flagellum structure and function *(e.g., kl-2, kl-3, kl-5, wdy, ory, ppl-Y1);* autosomal flagellum proteins (radial spoke head proteins: Rsph1, Rsph3, Rsph4a); cilia-related proteins *(mmm, sac, tous, hmw);* sperm-leucylaminopeptidases (S-LAPs: *loopin-1, s-lap2, s-lap3, s-lap4, s-lap7);* protein constituents of sperm tail paracrystalline material *(bb8);* sperm nuclear basic proteins (SNBPs: *prtl99C, ddbt, Mst77F);* sperm-expressed tubulins *(betaTub85D*/*β2-tubulin, betaTub60D);* TBP-associated factor proteins required for the coordinated expression of more than 1,000 genes during spermatid differentiation (TAFs: *mia, can, sa, nht, rya).* Also present are genes of various structure and function wherein disruption in males leads to the production of inviable embryos post-fertilization, known as paternal effect lethal genes (PELs: *snky, ddbt, Mst-77F).*

Cilia-related proteins may have additional roles in hearing that could impact male fitness if targeted outside the male germline and thus, may be better classified as Class IIIb target genes. Radial spoke head proteins, TAFs, and some SNBPs are fast evolving genes that may not have orthologs outside of the most closely related species. However, S-LAPs (SEQ ID NO: 2173 to 2196, 5930, and 6147 to 6154), some SNBPs (SEQ ID NO: 2173 to 2196, 5930, and 6147 to 6154), *snky* (SEQ ID NO: 2209 to 2573 and 6160 to 6342), *bel* (SEQ ID NO: 142 to 784 and 5298 to 5617) and *knon* (SEQ ID NO: 1539 to 1674, and 5848 to 5914) are identified as being well-conserved families that are critical only for male fertility, expressed at high levels in testes and developing sperm across Arthropod species, and thus may be appropriate targets for male sterility effectors throughout pest and vector species.

Literature review also reveals a further 31 genes are known to confer male sterility upon RNAi knockdown, of which 8 are not associated with a sterile allele (RNAi sterile only). *See,* for example, Ibaraki et al., G3, 11(8):jkab183 (2021).

The 8 RNAi sterile only genes are: *loqs, Hsc70-1, Hsc70-2, Hsc70-3, Hsc70-5, Hsc70-4, Caf1-55, Hyls1*.The set of *D. melanogaster* alleles is also reviewed to identify alleles associated with potentially non-sterile phenotypes (e.g., alleles associated with the terms visible, auditory, behavior, female, and larval phenotypes). Gene expression is also examined in both male and female reproductive tissues and cell types.

Orthologs identified using MetazoaEnsembl reveal a subset of male fertility essential genes that are highly conserved genes between three species separated by more than 500 million years of evolution, *Drosophila melanogaster* (Diptera, Arthropod), *Penaeus vannamei* (Pacific white shrimp, Decapoda, Arthropod), and *Caenorhabditis elegans* (Nematode). These include 177 genes: *abd-A, Abd-B, ac, ap, ara, Arf6, ATPsyngamma, aux, ball, bel, betaTub85D, Bin1, blanks, blw, bol, brul, brun, Bsg, Btk, Cad99C, Cap-D3, Cdc42, CG31759, CG9920, chic, CIAPIN1, cic, COX4L, crm, CSN5, ct, ctp, cue, CycB, Cyt-clL, Cyt-c-d, Dab, DCTN5-p25, Ddx1, didum, Doa, dor, Duba, EcR, eIF2A, eIF3m, eIF4G2, Erk7, f, Fsn, fwd, fws, fz2, g, Gld2, GstS1, gudu, hay, HBS1, heph, hfp, Hr39, Hr51, Hsp83, hts, ifc, jar, Kdm5, knon, l(2)dtl, l(2)gl, Lam, larp, lds, Lis-1, mago, mal, Mer, mfrn, Mgat1, mgr, mino, mip120, mle, mlt, Mob4, msl-3, Myo31DF, Mytl, nAChRbeta1, nclb, ND-B17, nej, Nle, nmd, Nop60B, Npc1a, Nup154, Orc1, park, pbl, pelo, Pex10, Pex12, Pex2, Pfas, Phb1, Pkn, poe, PolQ, Polr2A, pont, Popl, prd, pros, Prosalpha4T1, Prosalpha6T, Prp8, psidin, puf, Rab30, Rala, Ran, rau, Rb97D, ref(2)P, rept, rho-7, Rnp4F, Root, RpL36, RpS3, rst, run, sbr, SCaMC, scat, scny, ScsbetaA, sd, Sf3a1, shu, shv, slmo, sm, SmB, sns, spag, ss, ssp3, Stat92E, stc, Strip, Su(var)3-3, sub, Sym, Syx5, Taz, tbrd-1, tj, Top2, Top3alpha, Torsin, tra2, tsr, ttm2, U2A, Uspl4, v, VhaPPA1-2, Vmat, Vps28, wls, wts, wuho, Yipldl,* and *zw10.*

Amongst the conserved male fertility genes are several putative Class I male-fertility essential genes known or predicted to have an exclusive role in male fertility, and could be ideal targets for extension of the 2GEN approach throughout Arthropods: *betaTub85D, mlt, eIF4G2, Rb97D, Cyt-c-d, pelo, blanks, rau, Usp14, brun, Prosalpha6T, mfrn, Pex10, Cyt-x1L, ScsbetaA, VhaPPA1-2, CG9920, Duba, Prosalpha4T1, HBS1, Erk7, COX4L, Pex12, gudu, Gld2, knon, ssp3, tbrd-1, fws, ttm2,* and *SCaMC.*

Across arthropods we find 194 male fertility essential genes conserved between *Drosophila melanogaster* (Fruit fly, Diptera), *Drosophila suzukii* (Fruit fly, Diptera), *Lucilia cuprina* (Australian sheep blowfly, Diptera), *Anopheles gambiae* (mosquito, Diptera), *Aedes aegypti* (mosquito, Diptera), *Schistocerca gregaria* (Grasshoppers, Orthoptera), *Bombyx mori* (Domestic silkworm, Lepidoptera), *Diabrotica virgifera virgifera* (Western corn rootworm, Coleoptera), *Tribolium castaneum* (Red flour beetle, Coleoptera), *Apis mellifera* (Honey bee, Hymenoptera), *Parasteatoda tepidariorum* (Common house spider, Araneae), *Tetranychus urticae* (Two-spotted spider mite, Acari), *Daphnia pulex* (Common water flea, Cladocera), *Penaeus vannamei* (Pacific white shrimp, Decapoda), *Limulus polyphemus* (Atlantic horseshoe crab, Xiphosura). These include 194 genes: *abd-A, Abd-B, ac, ap, ara, Arf6, ATPsyngamma, aux, ball, bel, betaTub85D, Bin1, blanks, blw, bol, brul, brun, Bsg, Btk, Cad99C, Cap-D3, Cdc42, CG31759, CG9920, chic, CL4PIN1, cic, COX4L, crm, CSN5, ct, ctp, cue, CycB, Cyt-x1L, Cyt-c-d, Dab, DCTN5-p25, Ddx1, didum, Doa, dor, Duba, EcR, eIF2A, eIF3m, eIF4G2, Erk7, f, Fsn, fwd, fws, fz2, g, Gld2, GstS1, gudu, hay, HBS1, heph, hfp, Hr39, Hr51, Hsp83, hts, ifc, jar, Kdm5, knon, l(2)dtl, l(2)gl, Lam, larp, lds, Lis-1, mago, mal, Mer, mfrn, Mgatl, mgr, mino, mip120, mle, mlt, Mob4, msl-3, Myo31DF, Mytl, nAChRbeta1, nclb, ND-B17, nej, Nle, nmd, Nop60B, Npc1a, Nup154, Orc1, park, pbl, pelo, Pex10, Pex12, Pex2, Pfas, Phb1, Pkn, poe, PolQ, Polr2A, pont, Popl, prd, pros, Prosalpha4T1, Prosalpha6T, Prp8, psidin, puf, Rab30, Rala, Ran, rau, Rb97D, ref(2)P, rept, rho-7, Rnp4F, Root, RpL36, RpS3, rst, run, sbr, SCaMC, scat, scny, ScsbetaA, sd, Sf3a1, shu, shv, slmo, sm, SmB, sns, spag, ss, ssp3, Stat92E, stc, Strip, Su(var)3-3, sub, Sym, Syx5, Taz, tbrd-1, tj, Top2, Top3alpha, Torsin, tra2, tsr, ttm2, U2A, Uspl4, v, VhaPPAl-2, Vmat, Vps28, wls, wts, wuho, Yipldl,* and*zw10*.

Amongst these are several putative Class I male-fertility essential genes known or predicted to have an exclusive role in male fertility: *Dlc90F, mlt, stet, Usp14, brun, Prosalpha6T, Pex10, Prosalpha4T1, HBS1, COX4L, Pex12, Gld2, fws, ttm2.*

A non-limiting list of putative male-fertility essential gene names together with their short names and accession numbers is provided: abdominal A (abd-A, FBgn0000014), Abdominal B (Abd-B, FBgn0000015), achaete (ac, FBgn0000022), nicotinic Acetylcholine Receptor beta1 (nAChRbeta1, FBgn0000038), Phosphoribosylformylglycinamidine synthase (Pfas, FBgn0000052), anterior open (aop, FBgn0000097), bruno 1 (bru1, FBgn0000114), abnormal spindle (asp, FBgn0000140), aubergine (aub, FBgn0000146), bag of marbles (bam, FBgn0000158), claret (ca, FBgn0000247), chickadee (chic, FBgn0000308), Clathrin heavy chain (Che, FBgn0000319), eyes absent (eya, FBgn0000320), cinnabar (cn, FBgn0000337), cramped (crm, FBgn0000376), Cyclin B (CycB, FBgn0000405), Dab adaptor protein (Dab, FBgn0000414), deep orange (dor, FBgn0000482), doublesex (dsx, FBgn0000504), deformed wings (dwg, FBgn0000520), deltex (dx, FBgn0000524), Ecdysone receptor (EcR, FBgn0000546), extra macrochaetae (emc, FBgn0000575), exuperantia (exu, FBgn0000615), Fasciclin 2 (Fas2, FBgn0000635), Fasciclin 3 (Fas3, FBgn0000636), traffic jam (tj, FBgn0000964), garnet (g, FBgn0001087), Hairless (H, FBgn0001169), haywire (hay, FBgn0001179), Heat shock protein 83 (Hsp83, FBgn0001233), male fertility factor kl2 (kl-2, FBgn0001313), Gamma-tubulin ring protein 91 (Grip91, FBgn0001612), infertile crescent (ifc, FBgn0001941), Cytokine induced apoptosis inhibitor 1 (CIAPIN1, FBgn0001977), shuttle craft (stc, FBgn0001978), NADH dehydrogenase (ubiquinone) B17 subunit (ND-B17, FBgn0001989), Prohibitin 1 (Phb1, FBgn0002031), lethal (2) giant larvae (l(2)gl, FBgn0002121), hyperplastic discs (hyd, FBgn0002431), sticky (sti, FBgn0002466), Lamin (Lam, FBgn0002525), lodestar (lds, FBgn0002542), lozenge (lz, FBgn0002576), miniature (m, FBgn0002577), Ribosomal protein L36 (RpL36, FBgn0002579), Ribosomal protein S3 (RpS3, FBgn0002622), maroon-like (mal, FBgn0002641), mastermind (mam, FBgn0002643), twine (twe, FBgn0002673), meiotic from via Salaria 332 (mei-S332, FBgn0002715), mago nashi (mago, FBgn0002736), maleless (mle, FBgn0002774), male-specific lethal 3 (msl-3, FBgn0002775), modulo (mod, FBgn0002780), modifier of mdg4 (mod(mdg4), FBgn0002781), male sterile (3) K81 (ms(3)K81, FBgn0002838), spermatocyte arrest (sa, FBgn0002842), DNA polymerase theta (PolQ, FBgn0002905), oskar (osk, FBgn0003015), ovarian tumor (otu, FBgn0003023), pebble (pbl, FBgn0003041), Pox neuro (Poxn, FBgn0003130), paired (prd, FBgn0003145), refractory to sigma P (ref(2)P, FBgn0003231), Rm62 (Rm62, FBgn0003261), RNA polymerase II subunit A (Polr2A, FBgn0003277), roughest (rst, FBgn0003285), rotated abdomen (rt, FBgn0003292), runt (run, FBgn0003300), roughex (rux, FBgn0003302), small bristles (sbr, FBgn0003321), scalloped (sd, FBgn0003345), shutdown (shu, FBgn0003401), seven in absentia (sina, FBgn0003410), smooth (sm, FBgn0003435), Superoxide dismutase 1 (Sod1, FBgn0003462), spindle E (spn-E, FBgn0003483), Bruton tyrosine kinase (Btk, FBgn0003502), spineless (ss, FBgn0003513), staufen (stau, FBgn0003520), string (stg, FBgn0003525), subito (sub, FBgn0003545), suppressor of Hairy wing (su(Hw), FBgn0003567), suppressor of sable (su(sable), FBgn0003575), transformer (tra, FBgn0003741), transformer 2 (tra2, FBgn0003742), alpha-Tubulin at 84B (alphaTub84B, FBgn0003884), beta-Tubulin at 85D (betaTub85D, FBgn0003889), tudor (tud, FBgn0003891), Ribosomal protein S27A (RpS27A, FBgn0003942), Ubiquitin-63E (Ubip63E, FBgn0003943), vermilion (v, FBgn0003965), vestigial (vg, FBgn0003975), valois (vls, FBgn0003978), vein (vn, FBgn0003984), yellow (y, FBgn0004034), scute (sc, FBgn0004170), cut (ct, FBgn0004198), Resistant to dieldrin (Rdl, FBgn0004244), Wnt oncogene analog 2 (Wnt2, FBgn0004360), porin (porin, FBgn0004363), always early (aly, FBgn0004372), four wheel drive (fwd, FBgn0004373), nebbish (neb, FBgn0004374), Kinesin-like protein at 67A (Klp67A, FBgn0004379), benign gonial cell neoplasm (bgcn, FBgn0004581), prospero (pros, FBgn0004595), Zeste-white 10 (zw10, FBgn0004643), fruitless (fru, FBgn0004652), single-minded (sim, FBgn0004666), hopscotch (hop, FBgn0004864), P-element induced wimpy testis (piwi, FBgn0004872), Ribonuclear protein at 97D (Rb97D, FBgn0004903), unpaired 1 (upd1, FBgn0004956), no mitochondrial derivative (nmd, FBgn0005322), Glutathione S transferase S1 (GstS1, FBgn0010226), TBP-related factor (Trf, FBgn0010287), Cell division cycle 42 (Cdc42, FBgn0010341), CDP-diacylglycerol synthase (Cds, FBgn0010350), TBP-associated factor 6 (Taf6, FBgn0010417), Spindle assembly abnormal 4 (Sas-4, FBgn0011020), diaphanous (dia, FBgn0011202), cueball (cue, FBgn0011204), fumble (fbl, FBgn0011205), boule (bol, FBgn0011206), pelota (pelo, FBgn0011207), bellwether (blw, FBgn0011211), effete (eff, FBgn0011217), hephaestus (heph, FBgn0011224), jaguar (jar, FBgn0011225), oxen (ox, FBgn0011227), purity of essence (poe, FBgn0011230), scattered (scat, FBgn0011232), male sterile (2) 35Ci (ms(2)35Ci, FBgn0011239), Heat shock protein 60B (Hsp60B, FBgn0011244), cannonball (can, FBgn0011569), fuzzy onions (fzo, FBgn0011596), Imitation SWI (Iswi, FBgn0011604), Kinesin-like protein at 3A (Klp3A, FBgn0011606), Syntaxin 5 (Syx5, FBgn0011708), twinstar (tsr, FBgn0011726), warts (wts, FBgn0011739), cut up (ctp, FBgn0011760), Angiotensin converting enzyme (Ance, FBgn0012037), Trithorax-like (Trl, FBgn0013263), Protamine A (protA, FBgn0013300), lethal-(2)-denticleless (l(2)dtl, FBgn0013548), ADP ribosylation factor 6 (Arf6, FBgn0013750), centrosomin (cnn, FBgn0013765), RNA-binding protein 4F (Rnp4F, FBgn0014024), meiosis I arrest (mia, FBgn0014342), noisette (noi, FBgn0014366), touch insensitive larva B (tilB, FBgn0014395), Dead-box-1 (Ddx1, FBgn0015075), eukaryotic translation initiation factor 4E1 (eIF4E1, FBgn0015218), Ras-like protein A (Rala, FBgn0015286), SHC-adaptor protein (Shc, FBgn0015296), spaghetti (spag, FBgn0015544), Lissencephaly-1 (Lis-1, FBgn0015754), araucan (ara, FBgn0015904), caupolican (caup, FBgn0015919), capsuleen (csul, FBgn0015925), maelstrom (mael, FBgn0016034), furry (fry, FBgn0016081), frizzled 2 (fz2, FBgn0016797), Signal-transducer and activator of transcription protein at 92E (Stat92E, FBgn0016917), quemao (qm, FBgn0019662), don juan (dj, FBgn0019828), Smg5 nonsense mediated mRNA decay factor (Smg5, FBgn0019890), ATP synthase, gamma subunit (ATPsyngamma, FBgn0020235), stem cell tumor (stet, FBgn0020248), Ran (Ran, FBgn0020255), Phosphoinositide-dependent kinase 1 (Pdk1, FBgn0020386), asunder (asun, FBgn0020407), eukaryotic translation release factor 3 (eRF3, FBgn0020443), Protein kinase N (Pkn, FBgn0020621), Oxysterol binding protein (Osbp, FBgn0020626), Thioredoxin reductase 1 (Trxr1, FBgn0020653), chromosome bows (chb, FBgn0021760), Nucleoporin 154kD (Nup154, FBgn0021761), Vacuolar protein sorting 28 (Vps28, FBgn0021814), Notchless (Nle, FBgn0021874), Helicase 89B (Hel89B, FBgn0022787), defective transmitter release (dtr, FBgn0023090), Mnt (mnt, FBgn0023215), zero population growth (zpg, FBgn0024177), sticks and stones (sns, FBgn0024189), WASp (WASp, FBgn0024273), Niemann-Pick type C-1a (Npc1a, FBgn0024320), Dynein light chain 90F (Dlc90F, FBgn0024432), Bicoid interacting protein 1 (Bin1, FBgn0024491), four way stop (fws, FBgn0024689), karyopherin alpha1 (Kap-alpha1, FBgn0024889), Ceramide phosphoethanolamine synthase (Cpes, FBgn0025335), Torsin (Torsin, FBgn0025615), RNA 3'-terminal phosphate cyclase (Rtca, FBgn0025630), myo-inositol-1-phosphate synthase (Inos, FBgn0025885), meiotic P26 (mei-P26, FBgn0026206), Sak kinase (SAK, FBgn0026371), Death regulator Nedd2-like caspase (Dronc, FBgn0026404), Gamma-tubulin ring protein 84 (Grip84, FBgn0026430), Grip75 (Grip75, FBgn0026431), Grip128 (Grip128, FBgn0026433), Tafazzin (Taz, FBgn0026619), POP1 ribonuclease P/MRP subunit (Pop1, FBgn0026702), COP9 signalosome subunit 5 (CSN5, FBgn0027053), spindle defective 2 (spd-2, FBgn0027500), minotaur (mino, FBgn0027579), Ulp1 (Ulp1, FBgn0027603), ballchen (ball, FBgn0027889), Kip1 ubiquitination-promoting complex subunit 2 (Kpc2, FBgn0028372), Activated Cdc42 kinase (Ack, FBgn0028484), Endonuclease G inhibitor (EndoGI, FBgn0028515), half pint (hfp, FBgn0028577), Fragile X messenger ribonucleoprotein 1 (Fmr1, FBgn0028734), slowmo (slmo, FBgn0029161), Growth arrest specific protein 8 (Gas8, FBgn0029667), wuho (wuho, FBgn0029857), PTEN-induced putative kinase 1 (Pink1, FBgn0029891), Upf2, (Upf2, FBgn0029992), p24-related-1 (p24-1, FBgn0030341), ATP7 (ATP7, FBgn0030343), shopper (shop, FBgn0030966), N(alpha)-acetyltransferase 20 A (Naa20A, FBgn0031043), shriveled (shv, FBgn0031256), Peroxin 12 (Pex12, FBgn0031282), papi (papi, FBgn0031401), tombola (tomb, FBgn0031715), Heat shock protein 60C (Hsp60C, FBgn0031728), rau (rau, FBgn0031745), Lysine demethylase 5 (Kdm5, FBgn0031759), Tubulin tyrosine ligase-like 3B (TTLL3B, FBgn0031853), Rab30 (Rab30, FBgn0031882), gudu (gudu, FBgn0031905), Phosphatidic Acid Phospholipase A1 (PAPLA1, FBgn0031990), australin (aust, FBgn0032104), Ubiquitin specific protease 14 (Usp14, FBgn0032216), Basal body up regulated gene 22 (Bug22, FBgn0032248), sperm-associated antigen 4 (spag4, FBgn0032368), PIH1 domain containing 1 (Pih1D1, FBgn0032455), Yip1 domain-containing 1 (Yip1d1, FBgn0032465), Radial spoke head protein 1 (Rsph1, FBgn0032478), Proteasome alpha6 subunit, Testis-specific (Prosalpha6T, FBgn0032492), short spindle 3 (ssp3, FBgn0032723), Cytochrome c oxidase subunit 4-like (COX4L, FBgn0033020), PNGase-like (Pngl, FBgn0033050), U2A (U2A, FBgn0033210), CG13202 (CG13202, FBgn0033623), rhomboid-7 (rho-7, FBgn0033672), pre-mRNA processing factor 8 (Prp8, FBgn0033688), Myb-interacting protein 120 (mip120, FBgn0033846), Sperm-Leucylaminopeptidase 5 (S-Lap5, FBgn0033860), Sperm-Leucylaminopeptidase 7 (S-Lap7, FBgn0033868), eukaryotic translation initiation factor 3 subunit m (eIF3m, FBgn0033902), Hormone receptor 51 (Hr51, FBgn0034012), clueless (clu, FBgn0034087), Sperm-Leucylaminopeptidase 8 (S-Lap8, FBgn0034132), Dicer-2 (Dcr-2, FBgn0034246), Dynein axonemal assembly factor 3 (Dnaaf3, FBgn0034352), testis-specifically expressed bromodomain containing protein-2 (tbrd-2, FBgn0034423), Myb-interacting protein 40 (mip40, FBgn0034430), Mannosyl (alpha-1,3-)-glycoprotein beta-1,2-N-acetylglucosaminyltransferase (Mgat1, FBgn0034521), CG9313 (CG9313, FBgn0034566), Rae1 (Rae1, FBgn0034646), novel spermatogenesis regulator (nsr, FBgn0034740), testes of unusual size (tous, FBgn0034745), YME1 like ATPase (YME1L, FBgn0034792), atlas (atlas, FBgn0034841), thoc5 (thoc5, FBgn0034939), Radial spoke head protein 4a (Rsph4a, FBgn0034957), MAN1 (MAN1, FBgn0034962), Dis3 like 3'-5' exoribonuclease 2 (Dis3l2, FBgn0035111), tiny tim 2 (ttm2, FBgn0035124), Centrosomal protein 290kDa (Cep290, FBgn0035168), Peroxin 10 (Pex10, FBgn0035233), Striatin interacting protein (Strip, FBgn0035437), nutcracker (ntc, FBgn0035461), Dpy-30-like 2 (Dpy-30L2, FBgn0035491), dynein, axonemal, heavy chain 3 (Dnah3, FBgn0035581), blanks (blanks, FBgn0035608), missing minor mitochondria (mmm, FBgn0035799), testis-specific gamma-tubulin ring protein 84 (t-Grip84, FBgn0035800), eukaryotic translation initiation factor 4E5 (eIF4E5, FBgn0035823), Peroxin 2 (Pex2, FBgn0035876), Sperm-Leucylaminopeptidase 1 (S-Lap1, FBgn0035915), Cyclophilin 40 (Cyp40, FBgn0036020), wntless (wls, FBgn0036141), Deubiquitinating enzyme A (Duba, FBgn0036180), twitchy (twy, FBgn0036206), Syntaxin 13 (Syx13, FBgn0036341), combover (cmb, FBgn0036365), Dynein axonemal assembly factor 6 (Dnaaf6, FBgn0036437), goddard (gdrd, FBgn0036438), Centrosomal protein 135kDa (Cep135, FBgn0036480), ATP synthase, beta subunit-like (ATPsynbetaL, FBgn0036568), WD repeat domain 92 (Wdr92, FBgn0036771), HP1-HOAP-interacting protein (HipHop, FBgn0036815), male sterile (3) 76Ba (ms(3)76Ba, FBgn0036868), male sterile (3) 76Cc (ms(3)76Cc, FBgn0036895), wallenda (wnd, FBgn0036896), Viral tegument-like deubiquitinase (Vtld, FBgn0036905), Peroxin 16 (Pex16, FBgn0037019), eukaryotic translation initiation factor 2A (eIF2A, FBgn0037135), auxilin (aux, FBgn0037218), sterile affecting ciliogenesis (sac, FBgn0037254), Symplekin (Sym, FBgn0037371), Proteasome assembly chaperone 1 (PSMG1, FBgn0037378), CG8236 (CG8236, FBgn0037626), Succinyl-coenzyme A synthetase beta subunit, ADP-forming (ScsbetaA, FBgn0037643), Insulator binding factor 2 (Ibf2, FBgn0037669), Heterochromatin Protein 1e (HP1e, FBgn0037675), matotopetli (topi, FBgn0037751), Chromosome associated protein H2 (Cap-H2, FBgn0037831), Radial spoke binding protein 15 (Rsbp15, FBgn0038000), CG9920 (CG9920, FBgn0038200), scotti (soti, FBgn0038225), HTRA2-related serine protease (HtrA2, FBgn0038233), Kip1 ubiquitination-promoting complex subunit 1 (Kpc1, FBgn0038296), maca (maca, FBgn0038345), Autophagy-related 8b (Atg8b, FBgn0038539), hemingway (hmw, FBgn0038607), Testis-specific serine/threonine kinase (Tssk, FBgn0038630), Syncrip (Syp, FBgn0038826), Testicular haploid expressed gene (Theg, FBgn0038921), GLD2 poly(A) polymerase (Gld2, FBgn0038934), wampa (wam, FBgn0039070), big bubble 8 (bb8, FBgn0039071), testis-specifically expressed bromodomain containing protein-1 (tbrd-1, FBgn0039124), Rootletin (Root, FBgn0039152), puffyeye (puf, FBgn0039214), mitoferrin (mfrn, FBgn0039561), Cytochrome c1-like (Cyt-c1L, FBgn0039651), Diedel (Diedel, FBgn0039666), Cadherin 99C (Cad99C, FBgn0039709), CG12069 (CG12069, FBgn0039796), kelch like family member 10 (klhl10, FBgn0040038), reptin (rept, FBgn0040075), pontin (pont, FBgn0040078), Dynactin 5, p25 subunit (DCTN5-p25, FBgn0040228), Topoisomerase 3alpha (Top3alpha, FBgn0040268), Regulator of cullins 1b (Roc1b, FBgn0040291), Myt1 (Myt1, FBgn0040298), CG18675 (CG18675, FBgn0040696), parkin (park, FBgn0041100), no hitter (nht, FBgn0041103), archipelago (ago, FBgn0041171), Polycystic kidney disease 2 (Pkd2, FBgn0041195), Apollo (Apl, FBgn0042178), HBS1 (HBS1, FBgn0042712), F-box synaptic protein (Fsn, FBgn0043010), Sperm-Leucylaminopeptidase 3 (S-Lap3, FBgn0045770), gasket (gskt, FBgn0046332), Trapped in endoderm 1 (Tre1, FBgn0046687), Liprin-alpha (Liprin-alpha, FBgn0046704), BRCA2, DNA repair associated (Brca2, FBgn0050169), distal antenna-young (dany, FBgn0050401), Component of oligomeric golgi complex 7 (Cog7, FBgn0051040), CG31759 (CG31759, FBgn0051759), Radial spoke head protein 9 (Rsph9, FBgn0051803), Male specific transcript 27D (Mst27D, FBgn0051907), Chromosome associated protein D3 (Cap-D3, FBgn0051989), Sperm-Leucylaminopeptidase 4 (S-Lap4, FBgn0052064), Short Calcium-binding Mitochondrial Carrier (SCaMC, FBgn0052103), Sperm-Leucylaminopeptidase 2 (S-Lap2, FBgn0052351), tumorous testis (tut, FBgn0052364), Radial spoke head protein 3 (Rsph3, FBgn0052392), Extracellularly regulated kinase 7 (Erk7, FBgn0052703), Golgin, RAB6 interacting (Gorab, FBgn0053052), unpaired 3 (upd3, FBgn0053542), salto (salto, FBgn0061197), endosulfine (endos, FBgn0061515), Lasp (Lasp, FBgn0063485), still life (sif, FBgn0085447), deadlock (del, FBgn0086251), Myosin 31DF (Myo31DF, FBgn0086347), Merlin (Mer, FBgn0086384), peanuts (pea, FBgn0086895), Cytochrome c distal (Cyt-c-d, FBgn0086907), Male-specific transcript 77F (Mst77F, FBgn0086915), sneaky (snky, FBgn0086916), phagocyte signaling impaired (psidin, FBgn0243511), Verprolin 1 (Vrp1, FBgn0243516), Chromodomain-helicase-DNA-binding protein 1 (Chd1, FBgn0250786), gilgamesh (gish, FBgn0250823), MOB kinase activator 4 (Mob4, FBgn0259483), loopin-1 (loopin-1, FBgn0259795), Nucleolar protein at 60B (Nop60B, FBgn0259937), Suppressor of variegation 3-3 (Su(var)3-3, FBgn0260397), Reduction in Cnn dots 7 (Rcd7, FBgn0260461), eukaryotic translation initiation factor 4G2 (eIF4G2, FBgn0260634), Death-associated inhibitor of apoptosis 1 (Diap1, FBgn0260635), multi sex combs (mxc, FBgn0260789), scrawny (scny, FBgn0260936), james bond (bond, FBgn0260942), Vesicular monoamine transporter (Vmat, FBgn0260964), asterless (asl, FBgn0261004), PFTAIRE-interacting factor 1A (Pif1A, FBgn0261015), Hormone receptor-like in 39 (Hr39, FBgn0261239), Cullin 3 (Cul3, FBgn0261268), dilute class unconventional myosin (didum, FBgn0261397), male sterile (2) 34Fe (ms(2)34Fe, FBgn0261529), nejire (nej, FBgn0261617), La related protein (larp, FBgn0261618), brunelleschi (brun, FBgn0261787), Basigin (Bsg, FBgn0261822), forked (f, FBgn0262111), Vacuolar H[+] ATPase PPA1 subunit 2 (VhaPPA1-2, FBgn0262514), capicua (cic, FBgn0262582), mitoshell (mtsh, FBgn0262598), Small ribonucleoprotein particle protein SmB (SmB, FBgn0262601), Myc (Myc, FBgn0262656), vreteno (vret, FBgn0263143), Acinus (Acn, FBgn0263198), belle (bel, FBgn0263231), hu li tai shao (hts, FBgn0263391), no child left behind (nclb, FBgn0263510), lost (lost, FBgn0263594), Brr2 U5 snRNP complex subunit (Brr2, FBgn0263599), Death-associated APAF1-related killer (Dark, FBgn0263864), division abnormally delayed (dally, FBgn0263930), Dynein intermediate chain at 61B (Dic61B, FBgn0263988), Sex lethal (Sxl, FBgn0264270), Cytochrome b5 (Cyt-b5, FBgn0264294), orb2 (orb2, FBgn0264307), abrupt (ab, FBgn0264442), merry-go-round (mgr, FBgn0264694), Myosin heavy chain (Mhc, FBgn0264695), Phosphodiesterase 1c (Pde1c, FBgn0264815), eukaryotic translation initiation factor 4E3 (eIF4E3, FBgn0265089), poly(A) binding protein (pAbp, FBgn0265297), mulet (mlt, FBgn0265512), Beadex (Bx, FBgn0265598), Proteasome alpha4 subunit, Testis-specific 1 (Prosalpha4T1, FBgn0265606), Transcription factor B5 (Tfb5, FBgn0265968), Darkener of apricot (Doa, FBgn0265998), stathmin (stai, FBgn0266521), Zinc finger MYND-type containing 10 (Zmynd10, FBgn0266709), misfire (mfr, FBgn0266757), Splicing factor 3a subunit 1 (Sf3a1, FBgn0266917), Neurotransmitter transporter-like (Ntl, FBgn0267326), rotund (rn, FBgn0267337), Topoisomerase I-interacting protein (Topors, FBgn0267351), JYalpha (JYalpha, FBgn0267363), milkah (mil, FBgn0267366), sauron (sau, FBgn0267378), loss of visual transmission (lovit, FBgn0267429), male fertility factor kl3 (kl-3, FBgn0267432), male fertility factor kl5 (kl-5, FBgn0267433), WD40 Y (WDY, FBgn0267449), vibrator (vib, FBgn0267975), apterous (ap, FBgn0267978), Dynein heavy chain at 16F (Dhc16F, FBgn0283476), Insulin-like receptor (InR, FBgn0283499), narrow (nw, FBgn0283508), Peptidyl-alpha-hydroxyglycine-alpha-amidating lyase 1 (Pal1, FBgn0283510), Ribonuclease P/MRP subunit p30 (Rpp30, FBgn0283652), Topoisomerase 2 (Top2, FBgn0284220), deadbeat (ddbt, FBgn0284444), knotted onions (knon, FBgn0285943), sheepish (shps, FBgn0286199), Transitional endoplasmic reticulum 94 (TER94, FBgn0286784), Origin recognition complex subunit 1 (Orc1, FBgn0286788), xmas (xmas, FBgn0286809), SR Protein Kinase (SRPK, FBgn0286813), belphegor (bor, FBgn0287225), doublefault (dbf, FBgn0287630), Pendulin (Pen, FBgn0287720), zipper (zip, FBgn0287873), beta-Tubulin at 60D (betaTub60D, FBgn0288686), Loquacious (loqs, FBgn0032515), Heat shock protein 70 cognate 1 (Hsc70-1, FBgn0001216), Heat shock protein 70 cognate 2 (Hsc70-2, FBgn0001217), Heat shock protein 70 cognate 3 (Hsc70-3, FBgn0001218), Heat shock protein 70 cognate 4 (Hsc70-4, FBgn0266599), Heat shock protein 70 cognate 5 (Hsc70-5, FBgn0001220), Chromatin assembly factor 1, p55 subunit (Caf1-55, FBgn0263979), Hyls1 centriolar and ciliogenesis associated (Hyls1, FBgn0087041).

Based on the above criteria, a subset of genes is identified that may be ideal targets for male sterility effectors in the 2GEN system. ***See* Table 3.**

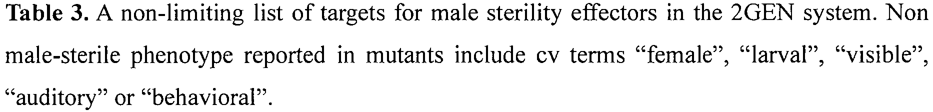

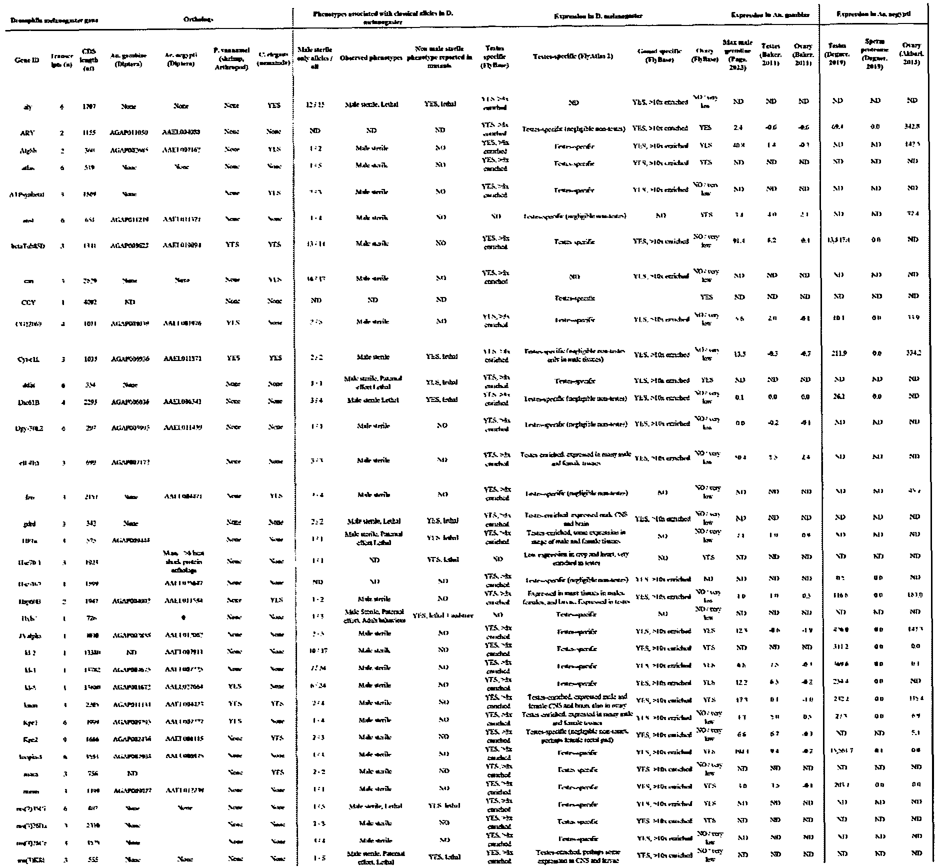

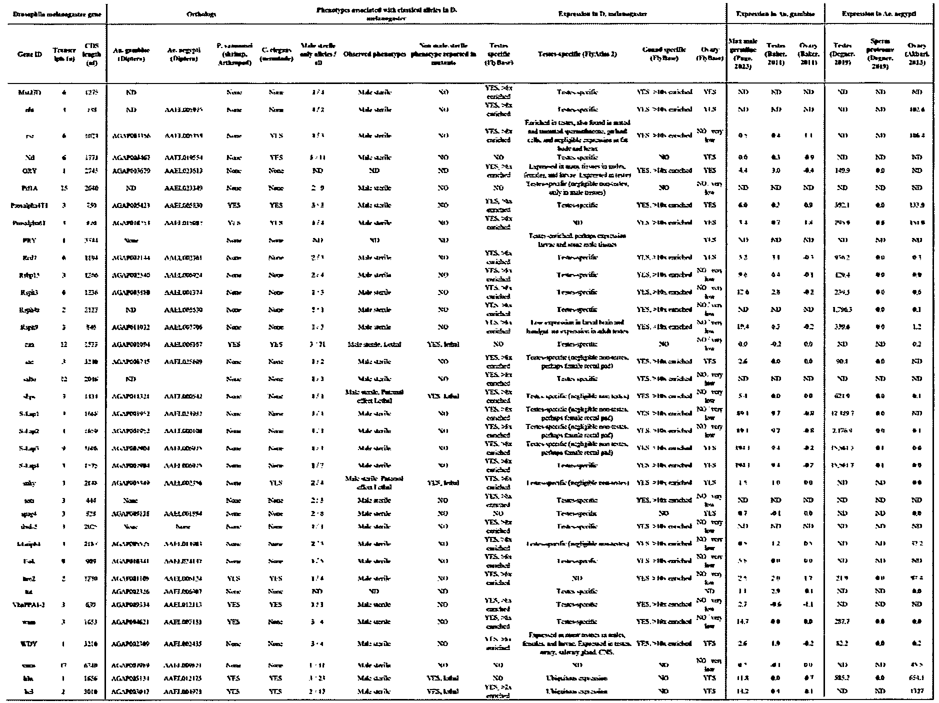

The strongest candidates are expressed exclusively, or almost exclusively, in the male germline. Such genes make up a significant proportion of the sperm proteome and contain protein domains consistent with known sperm proteins in *D. melanogaster.*

Identification of orthologous and novel male germline essential genes in other species is performed by searching for genes with similar expression profiles as the identified *D. melanogaster* genes. This approach identifies RSHPs and Y-fertility genes as some of the most highly expressed genes in sperm across arthropod species, even when the protein and nucleic acid sequences are highly diverged or independently evolved.

### Example 4. Identification of Conserved Target Sequences

Highly conserved sequences that can be targeted by any nuclease or RNAi method are identified for each of *28S rRNA, β2-tubulin (betaTub85D), belle (bel),* and *bellwether (blw).* First, orthologs are identified by mining literature and publicly available sequence databases (*e.g.*, NCBI, orthoDB) using both protein (if appropriate) and nucleotide BLAST^{™} searches. Transcript and coding sequences are identified, aligned using Clustal Omega (Sievers et al., Molecular Systems Biology, 7:539 (2011)), and a consensus sequence is generated.

A per-nucleotide conservation score is calculated by quantifying the number of species containing the consensus sequence in each position. Invariant nucleotides are identified if present in all species reporting a nucleotide at the consensus sequence. A total conservation percentage is determined by comparing the entire sequence of each species to the consensus sequence and finding the average percentage match across the entire length of the compared region. ***See*** **FIGs. 7A, 7B, 7C, and 7D****.**

To determine potential CRISPR target sites, a sliding window analysis is performed to generate every possible 23-nucleotide subsequence along the length of the consensus sequences, and inspected for the presence of "NGG" or "CCN" sequences that would constitute the canonical SpCas9 protospacer adjacent motif (PAM). Each CRISPR target is given a "conservation score" on the basis of mean per-nucleotide conservation score across the 20-nucleotide protospacer and the 2-nucleotide requirement of the PAM. Each target is then scored on the basis of invariant nucleotides present at the protospacer *(i.e.,* the gRNA binding site) and the PAM. Because the PAM is an essential requirement for targeting by CRISPR, it was given priority in selecting conserved sites over and above the protospacer for which some degeneracy is tolerated (typically 1 to 3 nucleotides). Target sites are selected on the basis of the highest conservation score and, where possible, PAM invariance, and finally protospacer invariance. Without being limiting, invariant nucleotides may represent essential binding sites required for proper gene expression, splicing, or secondary RNA-structure formation.

Identified target site sequences are compared to the full dataset to determine the number of species for which the target is fully conserved, conserved with a single mismatch, conserved with two mismatches, conserved with three mismatches, and so on. Putative gRNAs are then evaluated for potential off-target effects using publicly available software such as CHOPCHOP (Labun et al., Nucleic Acids Research, 47(W1):W171-W174 (2019)).

Ultraconserved sequences that could be targeted are identified in *bel* from *Drosophila, Aedes,* and *Anopheles* (SEQ ID NO: 782 to 784). gRNA target sites are identified in *bel* (SEQ ID NO: 3024 to 3330) including several target sites conserved throughout Diptera (SEQ ID NO: 3577 to 3580), and in *Aedes aegypti snky* (SEQ ID NO: 3602 to 3744).

### Example 5. Identification of Putative Target Sites in Bellwether

The *D. melanogaster* gene *Bellwether (blw)* is an essential gene that has a highly conserved protein sequence across many species. Homologs of *blw* are identified in 321 arthropod species. Analysis to identify putative target sites in *blw* is performed as described in Example 4 using 321 *blw* protein sequences (SEQ ID NO: 6511-6831) and 312 *blw* transcript sequences (SEQ ID NO: 797-1112). ***See*** **FIGs. 8A****,** **8B****,** **8C****,** **8D****, and 5E.**

Several putative CRISPR target sites are identified that show great than 90% average sequence conservation over the entire length of the transcript and sequence invariance at greater than 50% of nucleotide positions (SEQ ID NO: 3577 and 3578), and gRNAs targeting those sequences (SEQ ID NO: 3579 and 3580). Approximately 20% of the nucleotides are invariant across the 312 species examined. gRNAs targeting the coding sequence *of Aedes aegypti blw* are identified (SEQ ID NO: 3024 to 3330).

### Example 6. Identification of Putative Target Sites in β2-tubulin

*β2-tubulin* is an essential and major component of sperm. *β-tubulin* family sequences are compared from species in the Kingdoms Amoebozoa, Plantae, Protista, and Animalia. Between 68% and 98% protein sequence identity is observed across these kingdoms. ***See* Table 4**

**Table 4. Percent sequence identity of β-tubulin proteins across eukaryotic Kingdoms.**

| Specie s | *Sacchar omyces cerivisia e* | *Dictyos telium discoid eum* | *Arabido psis thalian a* | *Plasmodi um falciparu m* | *Caenorh αbditis elegans* | *Drosop hila melano gaster* | *Danio rerio* | *Mus muscul us* | *Xenopus laevis* |
|---|---|---|---|---|---|---|---|---|---|
| NCBI Access ion No. | NP_116 616.1 | XP_646 162.1 | NP_568 959.1 | XP_0013 47369.1 | NP_497 806.1 | NP_524 290.2 | NP_0010 19593.1 | NP_076 205.1 | XP_0181 23241.1 |
| *S*. *cerivis iae* | 100 | 68.22 | 71.33 | 72.58 | 73.11 | 73.21 | 73.99 | 74.32 | 74.27 |
| *D. discoid eum* | 68.22 | 100 | 74 | 75.73 | 73.78 | 73.44 | 74.22 | 74.55 | 74.27 |
| *A. thalian a* | 71.33 | 74 | 100 | 86.74 | 79.78 | 84.15 | 83.86 | 85.81 | 85.78 |
| *P*. *falcipa rum* | 72.58 | 75.73 | 86.74 | 100 | 83.6 | 85.55 | 86.26 | 87.61 | 86.91 |
| *C*. *elegan s* | 73.11 | 73.78 | 79.78 | 83.6 | 100 | 86.16 | 88.34 | 88.96 | 88.49 |
| *D. melan ogster* | 73.21 | 73.44 | 84.15 | 85.55 | 86.16 | 100 | 92.58 | 95.49 | 95.7 |
| *D. rerio* | 73.99 | 74.22 | 83.86 | 86.26 | 88.34 | 92.58 | 100 | 95.05 | 94.58 |
| *M*. *muscul us* | 74.32 | 74.55 | 85.81 | 87.61 | 88.96 | 95.49 | 95.7 | 100 | 98.65 |
| *X*. *laevis* | 74.27 | 74.27 | 85.78 | 86.91 | 88.49 | 95.7 | 94.58 | 98.65 | 100 |

The amino acid sequence identity across species within Kingdom Animalia is greater than 86%, and some Orders (e.g., Diptera and Lepidoptera) have up to 100% sequence identity between species. *See* **FIG. 9****.**

*β2-tubulin* nucleic acid sequences are identified in 52 Arthropods (SEQ ID NO: 2824 to 2877). In order to determine a consensus sequence across arthropods, *β2-tubulin* amino acid sequences (SEQ ID NO: 6467 to 6487) are compared across 21 species and *β2-tubulin* nucleic acid sequences are compared across 16 species as described in Example 4. *See* FIG. 10.

*β2-tubulin* protein sequences range in length from 427 to 446 amino acids, of which 374 amino acid residues are invariable in the first 427 amino acid residues (87.5%). Moreover, pairwise alignment percentage identities exhibit even greater conservation, with 20 species pairs showing 100% conservation, 64 pairs showing greater than 99%, and 290 pairs showing greater than 95%. The lowest percentage identity is between *Drosophila melanogaster* and the common house spider *(Parasteatoda tepidariorum)* at 93.58%.

Notably, *β-tubulin* protein variation can be greater between species belonging to a single Family compared to the those in the most divergent Arthropod groups. As a non-limiting example, *Aedes* mosquitoes share approximately 95% identity with *Anopheles* and *Culex* mosquitoes (divergence ~75 mya), but also similar identity greater than 95% to the *Penaeus* shrimp (divergence ~500 mya) and *Ixodida* ticks (divergence ~550 mya). Thus, it appears that protein sequence constraints to *β-tubulin* are strict and conserved.

Remarkable coding-sequence conservation is observed in *β2-tubulin* genes across 16 representative Dipteran species (including Calliphoridae, Tephritidae, Culicidae, and Drosophilidae), with more than 60% of nucleotides being invariant (816 of 1338 to 1344 nucleotides). Pairwise comparisons show greater than 70% sequence identity between any two species, and greater than 80% within a family. ***See*** **FIG. 11****.**

Additional comparisons within only Drosophilidae find 65.5% (879 of 1341 nucleotide positions) invariant nucleotide positions across 38 species. Across all 703 pairwise comparisons there is an average of 86.7% sequence identity. ***See*** **FIG. 12****.**

Based on these findings, the most highly conserved CRISPR target sites are identified for use as targets of male sterility effectors (SEQ ID NO: 3747 to 3834). CRISPR target sites are identified throughout the coding sequence of *β2-tubulin* of *Aedes aegypti* (SEQ ID NO: 3835 to 4011).

### Example 7. Identification of Putative Target Sites in 28S rRNA

28S rRNA is a structural component of the large ribosomal subunit in eukaryotic cells. An alignment and analysis of *28S rRNA* nucleic acid sequences (SEQ ID NO: 1 to 24) from diverse eukaryotic species to identify consensus and target sequences is performed as described in Example 4. ***See*** **FIGs. 13A****,** **13B, and 13C****.**

The analysis identifies two segments of 341- and 205-nucleotides long of 28S rDNA that are show extremely low variation across taxa, for which synthetic consensus sequences have be identified (SEQ ID NO: 25 and 26), that comprises a target site for the I-PpoI endonuclease and two SpCas9 target sites that are invariant across all examined Eukaryotes (SEQ ID NO: 3005 and 3007). SpCas9 gRNAs spacers and target sites are identified throughout the ultra-conserved regions (SEQ ID NO: 2903 to 3020). The 341-nucleotide long segment also comprises a 49-nucleotide long subsequence that is completely conserved across examined multicellular eukaryotes (SEQ ID NO: 3016). CRISPR target sites are identified throughout the two conserved segments based on synthetic consensus sequences (SEQ ID NO: 2903 to 3020), including two gRNA targets and associated spacer sequences that are conserved throughout Arthropods (SEQ ID NO: 3001 to 3004).

Given the ultra-conserved nature of this essential gene, it is believed that 49-nucleotide long subsequence can be targeted by a male sterility effector in virtually any arthropod species to cause male sterility.

### Example 8. Identification of Y-linked fertility genes in Drosophila suzukii

No published genome for *Drosophila suzukii* includes the Y chromosome.

Sequence fragments putatively belonging to the *D. suzukii* Y chromosome are assembled using PacBio long read sequences from male fly samples using Canu software. *See* Koren et al., Genome Research, 27:722-736 (2017).

The fragments are assembled into 3275 contigs with a final genome size of approximately 313 megabase pairs (the expected size for *D. suzukii*)*.* The fragments are searched for the presence of known *D. melanogaster* Y-fertility genes or fragments thereof using BLAST^{™}. Several matches are identified for exons of the genes *kl-2* (SEQ ID NO: 2885 to 2888), *kl-3* (SEQ ID NO: 2890), *kl-5* (SEQ ID NO: 2891), *ary* (SEQ ID NO: 2882), *ory* (SEQ ID NO: 2898), *pp1-Y1*, and *ppl-Y2* (SEQ ID NO: 2899). Several translated protein sequences are identified for *kl-3* (SEQ ID NO: 6489), *kl-5* (SEQ ID NO: 6490), and *ppl-Y2* (SEQ ID NO: 6492). ***See* Table 5.**

**Table 5. Y-linked fertility genes identified in D. suzukii**

| **Gene Name** | **Subregion ID** | **Query Length** | **HSP Location** | **HSP Score** | **HSP Bits** | **HSP e-value** | **Match length** | **Gaps** | **Match coverage** | **Start** | **End** |
|---|---|---|---|---|---|---|---|---|---|---|---|
| kl-2 | 1 | 1849 | tig00001376 | 796 | 1471 | 0 | 1509 | 25 | 81.61% | 3729 | 1886 |
| kl-2 | 2 | 1600 | tig00002455 | 903 | 1668.64 | 0 | 1373 | 8 | 85.81% | 18255 | 19859 |
| kl-2 | 3 | 1580 | tig00002455 | 820 | 1515.37 | 0 | 1319 | 2 | 83.48% | 19861 | 21427 |
| kl-3 | 1 | 2213 | tig00004524 | 761 | 1406.42 | 0 | 1062 | 1 | 47.99% | 14673 | 13463 |
| kl-3 | 1 | 2213 | tig00004523 | 729 | 1347.33 | 0 | 1057 | 11 | 47.76% | 5924 | 4716 |
| kl-3 | 3 | 2876 | tig00004406 | 1726 | 3188.44 | 0 | 2494 | 4 | 86.72% | 31195 | 34069 |
| kl-3 | 3 | 2876 | tig00001077 | 625 | 1155.28 | 0 | 885 | 4 | 30.77% | 9913 | 8903 |
| kl-5 | 0 | 2760 | tig00004476 | 1470 | 2715.7 | 0 | 2329 | 10 | 84.38% | 27715 | 24965 |
| ARY | 0 | 1382 | tig00001806 | 334 | 617.9 | 4.95E-175 | 659 | 13 | 47.68% | 51074 | 51885 |
| ORY | 0 | 644 | tig00001886 | 406 | 750.86 | 0 | 565 | 1 | 87.73% | 8607 | 9249 |
| Pp1-Y1 | 0 | 954 | tig00002260 | 508 | 939.21 | 0 | 785 | 2 | 82.29% | 10215 | 9294 |
| Pp1-Y2 | 0 | 942 | tig00003223 | 551 | 1018.62 | 0 | 811 | 3 | 86.09% | 7579 | 8515 |

Primers are designed to amplify the putative genes identified in Table 8. The sequences are amplified in males, but not females, indicating that they are Y-linked sequences. Primers are also designed to attempt to isolate larger fragments of the identified genes. Sequences for the entirety of *pp1-Y1,* approximately half of *pp1-Y2,* three of eight exons of *ory,* and fragments comprising complete exons of each of *kl-2*, *kl-3*, are *kl-5* are obtained.gRNAs are designed against each of *kl-2* (SEQ ID NO: 3581 to 3585), *kl-3* (SEQ ID NO: 3588 and 3589), *ory* (SEQ ID NO: 3594 and 3595), *pp1-Y1* (SEQ ID NO: 3596 to 3599), and *ppl-Y2* (SEQ ID NO: 3600 and 3601).

### Example 9. Rescue Elements

A sterility system can comprise a rescue element that blocks the activity of a male sterile effector or compensates for the disruption of a sequence targeted by a male sterility effector in cells or tissues where expression or activity of the targeted gene is required for normal function.

Without being limiting, several examples of rescue elements are shown in **FIG. 14****.**

Using rescue elements in male sterility systems presents several challenges that must be overcome.

First, it is unknown whether suitable expression levels of the rescue element can be achieved. It is known that promoter elements designed to function in the male germline often fail to recapitulate their endogenous expression when inserted outside their native locus in the genome. *See* White-Cooper, Spermatogenesis, 2(1):11-22 (2012).

Second, key male fertility genes that are targets for male sterile effectors are typically amongst the most highly expressed genes in the male germline. This means that both sufficient disruption of the target gene and sufficient expression of the rescue element could be difficult to achieve. This problem can be exacerbated by the ability of male germline cells to share RNAs and proteins through cytoplasmic bridges that form a spermatogenic syncytium for much of their development from a stem cell progenitor through to mature sperm. Thus, a population of unaffected or resistant cells could compensate for otherwise targeted cells.

Expression of rescue elements comprising codon-scrambled alleles of a male sterility effector target is achieved using the endogenous promoter for the codon-scrambled gene. However, for most other rescue elements a ubiquitous, germline specific, or male germline specific promoter is used.

A split-rescue system is also used in conjunction with rescue elements. A codon-scrambled allele or another rescue element (e.g., an anticas protein) is expressed under the control of a binary expression system such as the Q-system. *See* Potter et al., Cell, 141(3):536-548 (2010); Riabinina and Potter, Methods Mol Biol, 1478:53-78 (2016).

Binary expression systems comprise a transactivator protein (e.g., Gal4, QF2) that regulates expression of another RNA or protein (termed the "responder") under the control of upstream activation sequences (e.g., UAS for Gal4, QUAS for QF2). The magnitude of responder expression is modulated by changing the number of upstream activation sequences operably linked to the responder. Relative expression of the responder increases as the number of upstream activation sequences increases.

Using a binary expression system, expression of a rescue element can be carefully modulated by operably linking the rescue element to the same promoter used to express a corresponding male sterile effector, ensuring complete overlap between cells targeted and cells rescued; operably linking a rescue element to an alternative promoter, for example and without being limiting, a promoter that drives stronger expression, earlier expression, and expression in a greater number of cells to mitigate potential fitness effects of nonspecific male sterile effector expression; or by altering the number of upstream activation sequences. A split-rescue system offers other advantages too, as neither component of a split-rescue system will be sufficient to express a functional rescue individually, mitigating potential resistance.

A guide for the selection of an appropriate male sterile effector/rescue element pair is provided in **FIG. 5****.**

### Example 10. Identification of Germline Promoters

Selection of the proper promoter(s) for each component of a sterility system is vital to the sterility system's efficacy. *See* **FIG. 15****.** Several genes are identified as being expected to have promoters which drive robust expression at various stages of male germline development in diverse insect species. *See* **Table 6.**

**Table 6. Genes with promoters active during male germline development in insects. D. refers to Drosophila; An. refers to Anopheles; and Ae. refers to Aedes. For GenBank, see Benson et al., Nucleic Acids Research, 41:D36-D42 (2013).**

| **Gene** | **Primary onset of expression** | **GenBank Gene Accession Number** | | | |
|---|---|---|---|---|---|
| | | ***D. melanogaster*** | ***D. suzukii*** | ***An. gambiae*** | ***Ae. aegypti*** |
| vas | Hub cell | FBgn0283442 | LOC108018430 | AGAP008578 | AAEL004978 |
| Fas3 | Hub cell | FBgn0000636 | LOC108018875 | AGAP029564 | AAEL019406 |
| sqd | Hub cell | FBgn0263396 | LOC108010991 | AGAP000399 | AAEL005515 |
| bel | Hub cell | FBgn0263231 | LOC108007982 | AGAP003047 | AAEL004472 |
| geminin | Germline Stem Cell | FBgn0033081 | LOC108014321 | AGAP000496 | AAEL009773 |
| CG14220 (sds3) | Germline Stem Cell | FBgn0031036 | LOC108015386 | AGAP000738 | AAEL002084 |
| exu | Germline Stem Cell | FBgn0000615 | LOC108009852 | AGAP007365 | AAEL010097 |
| RacGAP84C | Germline Stem Cell | FBgn0045843 | LOC108011277 | AGAP008912 | AAEL010342 |
| nanos | Germline Stem Cell / Primary Spermatogonia | FBgn0002962 | LOC108017556 | AGAP006098 | AAEL012107 |
| aurA | Primary Spermatogonia | FBgn0000147 | LOC108007369 | AGAP007855 | AAEL009880 |
| shu | Primary Spermatogonia | FBgn0003401 | LOC108008450 | AGAP011458 | AAEL011632 |
| zpg | Primary Spermatogonia / Late spermatogonia (known role in hub cell) | FBgn0024177 | LOC108013401 | AGAP006241 | AAEL006726 |
| AGO3 | Late Spermatogonia / Primary Spermatocytes | FBgn0250816 | LOC108018563 | AGAP008862 | Many PIWI |
| aub | Late Spermatogonia / Primary Spermatocytes | FBgn0000146 | LOC108021436 | AGAP011204 | Many PIWI |
| betaTub85D | Spermatocytes (male-specific) | FBgn0003889 | LOC108015852 | AGAP008622 | AAEL019894 |
| eIF4E5 | Spermatocytes (male-specific) | FBgn0035823 | LOC108006846 | AGAP007172 | AAEL001916 |
| mei-W68 (spo11) | Spermatocytes | FBgn0002716 | LOC108008966 | AGAP010898 | AAEL013702 |
| ProtA | Late Spermatids (male-specific) | FBgn0013300 | 3 paralogs | AGAP028569 | AAEL003177 |
| Dnah3 | Late Spermatids (male-specific) | FBgn0035581 | LOC108014446 | AGAP007675 | Many dynein heavy chain |
| Ubi-p63E | Ubiquitous (germline expressed) | FBgn0003943 | LOC108006803 | AGAP001971 | AAEL000795 |
| Nup50 | Ubiquitous (germline expressed) | FBgn0033264 | LOC108019711 | AGAP008747 | AAEL005635 |
| Hsp83 | Ubiquitous (germline expressed) | FBgn0001233 | LOC108007143 | AGAP006958 | AAEL014843 |

Some promoters (*e.g.*, without being limiting, a *β2-tubulin* promoter, a *don juan* promoter, a *protamine* promoter, an *s-lap* promoter) are suitable for driving expression of Class I, II, III, and IV male sterile effectors. Other promoters (*e.g*., without being limiting, *a vasa* promoter, a *nanos* promoter, an *exu* promoter, a *zpg* promoter) are best suited for driving expression of Class I male sterile effectors (or other Classes if inserted into a Y chromosome or M-locus). Still other promoters (*e.g.*, without being limiting, a *ubiquitin* promoter) that are expressed broadly in germline and non-germline tissues can be suitable for expressing Class I male sterile effectors, but are more suitable for expressing rescue elements to ensure neutralization of the rescue element target in all tissues.

### Example 11. Design of Compatible Male Sterile Effectors and Rescue Elements for Aedes aegypti and Drosophila suzukii

An array of sterility systems are generated to utilize the 2GEN system against*Aedes aegypti* and *Drosophila melanogaster. See* **FIGs. 16A, 16B, 16C****, and** **16D****.**

Male sterility effectors are designed to target several different genes. The targeted genes include the Class Ia genes *ory* and *kl-2* in *D. suzukii*; the Class Ib gene *β2-tubulin* in *A. aegypti* and *D. suzukii*; the Class II gene *zpg* in *D. suzukii*; a novel *A. aegypti* gene called *msp* that is believed to be essential for spermatogenesis on the basis of very high transcript and protein abundance in the male germline of both mosquitoes and flies. However, *msp* also exhibits broader expression in other cell and tissue types, suggesting it is likely a Class IIIa gene. The Class IIId/IIIe ultraconserved *28S rRNA* gene is also targeted in both *A. aegypti* and *D. suzukii.*

Male sterility effectors also encode the fluorescent marker DsRed to aid in identification, while rescue elements also encode the fluorescent marker Green Fluorescence Protein (GFP) to aid in identification.

**Table 7** provides a summary of the male sterility effector designs, and **Table 8** provides a summary of the rescue element designs.

**Table 7. Male sterility effector construct designs. Aa refers to Aedes aegypti; Ds refers to Drosophila suzukii; gRNA refers to guide RNA; miRNA refers to microRNA; LOF refers to loss-of-function; n/a refers to not applicable; 2x and 3xrefers to two or three different gRNA or miRNA (as appropriate) sequences being present.**

| **Strain ID** | **First component** | **Second component** | **Third component** | **Construct (SEQ ID NOs:)** |
|---|---|---|---|---|
| AaMS1 | AaNup50 promoter::Cas9 | AaU6 promoter::3x β2-tubulin gRNA | n/a | 4182 |
| AaMS2 | AaNup50 promoter::Cas9 | AaU6 promoter:: 3x β2-tubulin gRNA | AaUbi promoter::3x β2-tubulin miRNA | 4243 |
| AaMS3 | Aaβ2-tubulin promoter::Cas9 | AaU6 promoter::2x 28S rDNA guide RNA | n/a | 4230 |
| AaMS4 | AaNup50 promoter::Cas9 | AaU6 promoter::2x msp guide RNA | n/a | 4179 |
| AaMS5 | Aaβ2-tubulin promoter::Cas9 | DsU6 promoter::3x β2-tubulin gRNA | n/a | 4182 |
| DsMS1 | Dsβ2-tubulin promoter::Cas9 | DsU6 promoter::3x zpg gRNA | n/a | 4180 |
| DsMS2 | Dsβ2-tubulin promoter::Cas9 | DsU6 promoter::2x 28S rDNA guide RNA | n/a | 4235 |
| DsMS3 | DsNos promoter::Cas9 | DsU6 promoter::3x ory gRNA | n/a | |
| DsMS4 | DsNos promoter::Cas9 | DsU6 promoter::3x kl-2 gRNA | n/a | 4211 |
| DsMS5 | LOF *ory* mutation | n/a | n/a | |
| AaMSSR1 | AaNup50 promoter::Cas9 | AaU6 promoter::3x β2-tubulin gRNA | AaUbi promoter::QF2 | |
| AaMSSR2 | Aaβ2-tubulin promoter::Cas9 | AaU6 promoter::3x 28S rDNA guide RNA | AaUbi promoter::QF2 | 4240, 4241, 4242 |
| DsMSSR1 | DsNos promoter::Cas9 | DsU6 promoter::3x β2-tubulin gRNA | DsUbi promoter::QF2 | 4239 |
| DsMSSR2 | Dsβ2-tubulin promoter::Cas9 | DsU6 promoter::3x 28S rDNA guide RNA | DsUbi promoter::QF2 | 4238 |

**Table 8. Rescue element construct designs. Aa refers to Aedes aegypti; Ds refers to Drosophila suzukii; recoded refers to a codon-scrambled allele**

| **Strain ID** | **Construct** | **SEQ ID NOs:** |
|---|---|---|
| AaRB2T1 to 3 | Aaβ2-tubulin promoter::recoded Aaβ2-tubulin | 4090, 4339 |
| DsRB2T1 | Dsβ2-tubulin promoter::recoded Dsβ2-tubulin | 4103, 4104, 4100, 4101, 4340 |
| AaRACR1 to 3 | AaUbi promoter::AcrIIA4 | 4023, 4024, 4045, 4047 |
| DsRACR1 | DsUbi promoter::AcrIIA4 | 4315, 4041, 4044, 4046, 4048, 4050 |
| DsRORY1 | *Ds ory* promoter::recoded *ory* | 4338, 4388, |
| AaSRACR1 to 3 | QUAS::AcrIIA4 | 4049, 4053 |
| DsSRACR1 | QUAS::AcrIIA4 | 4049, 4051, 4052 |

### Example 12. Generation of modified insects

Various strains of modified *Aedes aegypti* mosquitos are generated via embryo injection of various plasmids comprising male sterile effectors (SEQ ID NO: 4171, 4173 to 4174, 4176 to 4179, 4181, 4183, 4186 to 4187, 4191 to 4199, 4202 to 4209, 4212 to 4220, 4223 to 4225, 4226 to 4230, 4233 to 4234, 4236 to 4237, 4240 to 4247), each strain comprising one of AaMS1, AaMS2, AaMS3, AaMS4, AaMS5, AaMSSR1, and AaMSSR2. Each of these strains is either co-injected with or crossed to independent strains comprising one of AaRACR1, AaRACR2, AaRACR3, AaSRACR1, AaSRACR2, or AaSRACR3. Strains comprising AaMS1, AaMS2, or AaMS5 are either co-injected with or crossed to independent strains comprising one of AaRB2T1, AaRB2T2, or AaRB2T3.

Various strains of modified *Drosophila suzukii* flies are generated via embryo injection of various plasmids comprising male sterile effectors (SEQ ID NO: 4172, 4175, 4180, 4182, 4184 to 4185, 4188 to 4190, 4200 to 4201, 4210 to 4211, 4221 to 4222, 4231 to 4232, 4235, 4238 to 4239, and 4248), each strain comprising one of DsMS1, DsMS2, DsMS3, DsMS4, DsMS5, DsMSSR1, or DsMSSR2. Each of these strains, except DsMS5, is either co-injected with or crossed to independent strains comprising one of DsRACR1, or DsSRACR1. The DsMS3 strain or DsMS5 strains are either co-injected with or crossed to a strain comprising DsRORY1. The DsMSSR1 strain is either co-injected with or crossed to a strain comprising DsMSSR1.

### Example 13. Design of codon-scrambled β2-tubulin sequences

Although *β2-tubulin* is a highly conserved gene (*see* Example 6), it is unknown and unpredictable whether a mutated allele of *β2-tubulin* can be complemented by a synthetic copy, and if so, whether the synthetic copy can be a codon-scrambled allele. The high conservation of both the nucleotide and amino acid sequences across taxa strongly suggests that even small modifications to the gene may adversely alter its function or expression. Additionally, it is unknown and unpredictable whether sufficient transcription of*β2-tubulin* can occur when it is placed outside of its native locus.

Codon-scrambled alleles of *β2-tubulin* are generated to function in *Aedes aegypti* and *Drosophila suzukii* (SEQ ID NOs: 4339, 4344 and 4340). To minimize any potential impact of recoding on normal function of the gene, the *β2-tubulin* transcript sequences of closely related species (*Drosophila melanogaster* and *Aedes albopictus*) are used as staring points, where natural nucleotide diversity eliminates a fraction of potential target sites for *A. aegypti-* and *D. suzukii-specific* gRNAs .

*β2-tubulin* genes share 88.64% sequence identity between *Drosophila suzukii* and *Drosophila melanogaster,* and in basing a codon-scrambled rescue element on the *Drosophila melanogaster* sequence, 140 nucleotides of intron sequence unique to *Drosophila suzukii* is removed.

*β2-tubulin* genes share 87.24% sequence identity between *Aedes aegypti* and *Aedes albopictus*, and in basing a codon-scrambled rescue element on the *Aedes albopictus* sequence, an intron that is substantially different from its homolog in *Aedes aegypti* is introduced. However, because of the conserved nature of the intended targets, additional nucleotide variation is introduced in ultra-conserved sequences that do not show variation in arthropods. Thus, the codon-scrambled *β2-tubulin* sequences comprise only 79.78% and 82.64% sequence identity with their endogenous counterparts in *Drosophila suzukii* and *Aedes aegypti,* respectively. *See* **Table 9.**

**Table 9. Percent identities between Drosophila (D.) and Aedes (A.) β2-tubulin nucleic acid sequences.**

| | ***D. suzukii*** | ***D. melanogaster*** | ***D*. codon-scrambled** | ***A. aegypti*** | ***A*. *albopictus*** | ***A*. codon-scrambled** |
|---|---|---|---|---|---|---|
| ***D. suzukii*** | 100 | 88.64 | 82.64 | 77.56 | 75.86 | 76.21 |
| ***D. melanogaster*** | 88.64 | 100 | 93.29 | 77.18 | 76.38 | 76.38 |
| ***D*. codon-scrambled** | 82.64 | 93.29 | 100 | 76.59 | 76D.53 | 76.01 |
| ***A. aegypti*** | 77.56 | 77.18 | 76.59 | 100 | | 79.78 |
| ***A. albopictus*** | 75.86 | 76.38 | 76.53 | 87.24 | 100 | 92.29 |
| ***A*. codon-scrambled** | 76.21 | 76.38 | 76.01 | 79.78 | 92.29 | 100 |

### Example 14. Generation of Male Sterile Aedes aegypti

Independent construct integrations (strains) are generated from embryo injections in *Aedes aegypti* for each constructs described in Example 11: AaMS1 (strains 101, 201, 202, 203, 204, 205, 206, 207, 208, 301, 302, and 401), AaMS2 (strains 701, 702, 703, and 704), AsMS3 (strain 801), AaMS4 (strain 501), and AaMS5 (strain 601). *See* **FIG. 17A** and Harrell II. Three gRNA sequences are used in AaMS1 strains (SEQ ID NO: 3748, 3752, 3754). Three miRNA sequences are used AaMS1s strains (SEQ ID NO: 6832 to 6834), comprising miRNA spacer sequences specific to β2-tubulin (SEQ ID NO: 6835 to 6837).

A rapid screen approach is used to narrow down single integration events giving rise to high levels of sterility. This is measured by counting total larval progeny in group matings.

Each founding group of isolated modified mosquitoes can contain one or more integration events. Thus, male-specific sterility is evaluated in sibling groups over several generations (up to generation 4), wherein separate groups of modified males and modified females are maintained through sibling females mated to wildtype males, and sterility is evaluated in sibling males (presumably carrying the same integration event) mated to wildtype females.

The number of individuals tested varies based on availability of modified mosquitoes. For strains producing large numbers of larval progeny that cannot be easily counted, full fertility is assumed and the strain is not further investigated if fertility counts remain high over two successive generations.

Reduced fertility is observed in several groups, including those targeting *msp* (13% relative fertility) and *β2-tubulin* (0% to 100% relative fertility). Relative fertility is calculated by determining the average number of larval progeny produced per female per gonotrophic cycle, followed by normalization to a wild-type control reference (defined as 65.3 larvae per wild-type female per gonotrophic cycle). Wild-type control data are obtained from both contemporaneous and non-contemporaneous experiments, including assays using individual and pooled females, with no statistically significant difference observed in larval output across these methods. The reference value of 65.3 larvae per female is derived from multiple fertility metrics: approximately 80% of females lay, with an average of 92.4 eggs per laying female and an 88.7% egg hatch rate, resulting in a mean larval output of 65.3. As an illustrative example, if 2,000 total larval progeny are recovered from 400 females over the course of two gonotrophic cycles, the average larval output per female per cycle is 2.5. When normalized to the wild-type reference, the relative fertility is calculated as 2.5 divided by 65.3, yielding a relative fertility of approximately 0.038, or *3.8%.* ***See* Table 10.**

**Table 10. Median fertility rates for Aedes aegypti comprising male sterility effector constructs. Median and average fertility rates are relative to wildtype. Gen. refers to generation; - means not measured**

| **Construct** | **Strain** | **Males Examined (total)** | **%** | | | | | |
|---|---|---|---|---|---|---|---|---|
| | | | **Median Fertility** | **Average Fertility** | **Gen. 1** | **Gen. 2** | **Gen. 3** | **Gen 4.** |
| AaMS1 with gRNAs 1 and 2 | 101 | 78 | 51.3 | 51.3 | - | 21.2 | 81.3 | - |
| | 201 | 100 | 100 | 79.8 | 39.4 | 100 | 100 | - |
| | 202 | 24 | 54.3 | 54.3 | 8.7 | 100 | - | - |
| | 203 | 12 | 46 | 46 | 46 | - | - | - |
| AaMS1 with gRNAs 1, 2, and 3 | 204 | 246 | 100 | 66.7 | 0 | 100 | 100 | - |
| | 205 | 132 | 0.2 | 33.4 | - | 0 | 100 | 0.2 |
| | 206 | 175 | 100 | 66.7 | - | 0.2 | 100 | 100 |
| | 207 | 66 | 4.9 | 6.3 | 14.1 | 0 | | - |
| | 208 | 103 | 2.3 | 2.3 | 0.5 | - | 4.1 | - |
| | 209 | 226 | 0 | 0.3 | - | 0 | 0 | 0.9 |
| AaMS1 with gRNAs 1, 2, and 4 | 301 | 42 | 43.4 | 43.4 | 0.7 | 86.1 | - | - |
| | 302 | 33 | 4 | 4 | 0.4 | 7.6 | - | - |
| AaMS1 with gRNAs 1, 5, and 6 | 401 | 106 | 66.3 | 66.3 | 58.1 | 74.4 | - | - |
| AaMS4 with gRNAs 1, 2, and 3 | 501 | 11 | 13.1 | 13.1 | 13.1 | - | - | - |
| AaMS5 with gRNAs 1, 2, and 3 | 601 | 13 | 71.8 | 71.8 | 61.2 | 82.4 | - | - |
| AaMS2 with gRNAs 1, 2, and 3 and miRNAs 1, 2, and 3 | 701 | 3 | 47.9 | 47.9 | 47.9 | - | - | - |
| | 702 | 51 | 83.7 | 83.7 | 67.3 | 100 | - | - |
| | 703 | 20 | 62.7 | 62.7 | 99 | 26.4 | - | - |
| | 704 | 69 | 0 | 0 | 0.1 | 0 | 0 | - |
| | 705 | 240 | 0 | 0.1 | - | 0 | 0 | 0.3 |

Two promising male sterile strains are isolated, 207 and 704, each of which exhibit an inheritance pattern consistent with a single integration in the genome. The two strains each target *β2-tubulin*, either using three gRNAs in a CRISPR system (strain 207), or the same three gRNAs in a CRISPR system combined with three miRNAs also targeting *β2-tubulin. See* **FIG. 17B****.**

To further evaluate AaMS1 and AaMS2, fertility assays are carried out on modified males and females from the fourth generation onwards. *See* **Table 11.**

Groups of approximately 40 to 50 males of each strain, or wildtype control males, are mated to an equivalent number of wildtype females and the entire larval progenies are counted. The experiment is repeated over several gonotrophic cycles and across a minimum of two generations. No differences in trait penetrance are observed. Equivalent matings using modified females are performed, and no clear indication of a sterile phenotype is observed.

Consistent with the initial round of testing, both AaMS1 and AaMS2 exhibit high levels of male sterility on their own (99.7% and 99.85% sterility, respectively) compared to wildtype. In each case, more than 200 modified males are allowed to mate with an equivalent number of wildtype females, but fewer than 100 larvae are recovered across all replicates, blood meals, and generations of testing despite an expectation of more than 30,000 larvae for each. Experiments are repeated across several replicates, across generations, and across multiple gonotrophic cycles to determine the stability of the phenotype. No notable deviation is observed across the experiments, indicating that the phenotype is stable.

### Example 15. Rescue of AaMS1 and AaMS2 male sterility effectors in Aedes aegypti.

To establish a rescue system for male sterility, two approaches are pursued. The first makes use of a codon-scrambled *β2-tubulin* allele (*see* Example 13; SEQ ID NO: 4339 and 4344; AaRB2T) operably linked to a *β2-tubulin* promoter and inserted within a transformation plasmid (SEQ ID NO: and 4098), and the second makes use of a nucleic acid sequence encoding a AcrIIA4 anticas protein (SEQ ID NO: 4313 and 4314) operably linked to either the 421-nucleotide long ubiquitin (GenBank Accession No. AAEL006511) promoter (SEQ ID NO: 4311) inserted within a transformation plasmid (SEQ ID NO: 4042) or a QUAS promoter (AaRACR) (SEQ ID NO: 4278), inserted within a transformation plasmid (SEQ ID NO: 4053) . *See* **FIG. 14A.**

Rescue strains are generated using quasi-random integration into the *Aedes aegypti* genome using the piggyBac transposase leading to three separate autosomal integrations each (AaRACR1; AaRACR2; AaRACR3; AaRB2Tl; AaRB2T2; AaRB2T3). Each integrated plasmid also encodes a green fluorescent (GFP) protein.

### Example 16. Rescue using AaRACR

To evaluate the anticas rescue approach, females of the AaMS1 and AaMS2 strains are crossed with each of the three AaRACR strains to generate transheterozygous male progeny carrying both the male sterile effector and the rescue element (identified by presence of both red (DsRed; AaMS1 or AaMS2) and green (GFP; AaRACR) fluorescent markers. Fertility is assessed as described in Example 14. *See* **FIG. 18B** and **Table 11.** Complete restoration of fertility in AaMS1 individuals containing AaRACR1 or AaRACR2 rescue elements (100-108% relative fertility) is observed, but no clear restoration of fertility is observed with AaRACR3 (presumably due to insufficiency in its expression). As anticipated, the anticas-based rescue is effective against AaMS 1 (AaRACR1), but does not restore fertility in the AaMS2 strain (2.2% relative fertility), indicating that, without being limited by any scientific theory, the second mode of action based on miRNA technology adds a layer of redundancy that can improve sterile trait penetrance and help combat target site resistance at the CRISPR target site.

Next, sterility is tested in AaMS1 and AaMS2 strains that do not contain a rescue element, but are exposed to a paternal source of AcrIIA4 in the parental cross (e.g. AaMS1 (pAaRACR3), AaMS2 (pAaRACR1)). Stable sterility is observed across all combinations.

**Table 11. Summary of fertility assays on Aedes aegypti male sterile strains comprising AaMS1 or AaMS2 with and without AaRACR and AaRB2T rescue elements. Gen. refers to generation.**

| **Strain** | **Replicate s** | **Last Gen. Teste d** | **Total Examined** | | **Total larval progeny recovere d** | **Larva e per female** | **Total larval progen y expecte d** | **% Fertility** | | |
|---|---|---|---|---|---|---|---|---|---|---|
| | | | **Mal e** | **Femal e** | | | | **Total** | **Low** | **High** |
| AaMS1 | 8 | 4 | 226 | 215 | 91 | 0.2 | 30,399 | 0.3 | 0 | 0.92 |
| AaMS1 (pAaRACR1) | 4 | 6 | 117 | 120 | 351 | 1.35 | 16,998 | 2.07 | 0 | 10.74 |
| AaMS1 (pAaRACR2) | 4 | 6 | 118 | 120 | 6 | 0.02 | 20,920 | 0.03 | 0 | 0.03 |
| AaMS1 (pAaRACR3) | 3 | 5 | 9 | 18 | 0 | 0 | 1700 | 0 | 0 | 0 |
| AaMS1 (pAaRB2T1) | 3 | 6 | 33 | 30 | 0 | 0 | 5884 | 0 | 0 | 0 |
| AaMS1 (pAaRB2T2) | 3 | 6 | 35 | 30 | 0 | 0 | 5884 | 0 | 0 | 0 |
| AaMS1 (pAaRB2T3) | 2 | 7 | 30 | 30 | 143 | 2.38 | 3923 | 3.65 | 3.65 | 3.65 |
| AaMS2 | 7 | 4 | 240 | 280 | 46 | 0.1 | 30,073 | 0.15 | 0 | 0.31 |
| AaMS2 (pAaRACR1) | 4 | 5 | 38 | 55 | 467 | 3.74 | 8172 | 5.71 | 3.73 | 16.14 |
| AaMS2 (pAaRB2T1) | 3 | 6 | 23 | 30 | 9 | 0.1 | 5884 | 0.15 | 0.15 | 0.15 |
| AaMS2 (pAaRB2T2) | 3 | 6 | 35 | 30 | 61 | 0.68 | 5884 | 1.04 | 1.04 | 1.04 |
| AaMS2 (pAaRB2T3) | 3 | 7 | 30 | 30 | 0 | 0 | 5884 | 0 | 0 | 0 |
| AaMS1/AaRAC R1 | 2 | 6 | 115 | 120 | 7869 | 65.58 | 7845 | 100.3 1 | 87.9 2 | 125.0 9 |
| AaMS1/AaRAC R2 | 4 | 6 | 44 | 46 | 4110 | 70.86 | 3792 | 108.3 9 | 70.9 6 | 125.2 4 |
| AaMS1/AaRAC R3 | 3 | 5 | 40 | 38 | 0 | 0 | 3007 | 0 | 0 | 0 |
| AaMS1/AaRB2T 1 | 3 | 6 | 33 | 30 | 3671 | 40.79 | 5884 | 62.39 | 62.3 9 | 62.3 |
| AaMS1/AaRB2T 2 | 3 | 6 | 35 | 30 | 3507 | 38.97 | 5884 | 59.6 | 59.6 | 59.6 |
| AaMS1/AaRB2T 3 | 2 | 7 | 30 | 30 | 2179 | 36.32 | 3923 | 55.55 | 55.5 5 | 55.5 |
| AaMS2/AaRAC R1 | 3 | 4 | 27 | 35 | 149 | 1.42 | 6864 | 2.17 | 2.17 | 2.17 |
| AaMS2/AaRB2T 1 | 3 | 6 | 29 | 30 | 3670 | 40.78 | 5884 | 62.38 | 62.3 8 | 62.38 |
| AaMS2/AaRB2T 2 | 3 | 6 | 35 | 30 | 2892 | 32.13 | 5884 | 49.15 | 49.1 5 | 49.15 |
| AaMS2/AaRB2T 3 | 1 | 7 | 30 | 30 | 1736 | 57.87 | 1961 | 88.51 | 88.5 1 | 88.51 |

### Example 17. Rescue using AaRB2T

Three *β2-tubulin* codon-scrambled rescue stains (AaRB2T1-3) are crossed with each of the AaMS1 and AaMS2 strains to generate transheterozygous male progeny carrying both the male sterile effector and the rescue element (identified by presence of both red (DsRed; AaMS1 or AaMS2) and green (GFP; AaRACR) fluorescent markers. Fertility is assessed as described in Example 14. *See* **FIG. 18B** and **Table 11.**

A strong but potentially incomplete restoration of fertility (55% to 88% relative fertility) is observed. It is unclear if these rescues are unable to fully restore fertility, or if there is inherent variability in fertility between experiments and replicates. As expected, no restoration of fertility in male sterile strains exposed to a paternal source of the codon-scrambled rescue elements.

Observing rescue in transheterozygous individuals confirms that the codon-scrambled element can compensate for mutagenesis of the endogenous target in the germline.

To maintain a mass rearing colony, it is important that the codon-scrambled rescue element can also restore fertility to individuals carrying stable loss-of-function (LOF) mutations, as the AaMS1 and AaMS2 male sterility effectors continue to mutate the endogenous *β2-tubulin* gene, leading to homozygous LOF *β2 tubulin* alleles in the rearing colony.

To this end, colonies of AaMS1 and AaMS2 are maintained with each of the three recoded rescues and the colonies are healthy. Notably, the sterile phenotypes have remained consistently strong after seven generations.

### Example 18. Generation of Male Sterile Drosophila suzukii

Male sterility effector constructs targeting each of *β2-tubulin*, *zpg*, *kl-2*, *kl-3*, *ory*, and *rDNA* are tested in *Drosophila suzukii.* Independent strains are generated via embryo injection by means of piggyBac transposition using various transformation plasmids comprising male sterile effectors (SEQ ID NO: 4172, 4175, 4180, 4182, 4184 to 4185, 4188 to 4190, 4200 to 4201, 4210 to 4211, 4221 to 4222, 4231 to 4232, 4235, 4238 to 4239, 4248) and gRNA-expression vectors targeting Y-linked male fertility essential genes (SEQ ID NO: 4130 to 4131, 4136, 4141 to 4145, and 4149 to 4150), and adult flies are obtained. Strains are also created with plasmids comprising Cas9 expressed under various germline-active promoters (including *nanos, shu, ubiquitin,* and *β2-tubulin*) (SEQ ID NO: 4075 to 4078, 4082 to 4084). Strains carrying loss-of-function (LOF) mutations in Y-linked fertility genes are created by mating Cas9-expressing strains to gRNA-expressing strains, and stable LOF-carrying strains isolated by first introducing a recoded rescue element (SEQ ID NO: 4338 and 4388). Strains are also created by injecting plasmids comprising the DsRACR1 rescue element that encodes an anticas protein, amongst others (SEQ ID NO: 4041, 4044, 4046, 4048, and 4050 to 4052). Further strains are created with both male sterile effector and rescue elements by mating rescue strain males with male sterile strain males. *See* **FIG. 19A** for an example related to *zpg.*

Two independent male sterile strains targeting zpg with three anticas rescue strains are evaluated. Males carrying each of the male sterility effector constructs (SEQ ID NO: 4180) are mated individual to three wildtype females, with or without an accompanying rescue element, and allowed to produce progeny in two replicate egg laying opportunities (*e.g.*, "flips" into fresh food media) of five days each.

A male sterile strain carrying DsMS1, which targets *zpg* and has Cas9 expressed under a *D. suzukii β2-tubulin* promoter (SEQ ID NO: 4274), is examined for sterility. A significant reduction in fertility is observed in DsMS1 heterozygous males (9/22 fertile crosses, 60% sterile; p-value <0.001) compared to wildtype control crosses (30/30 fertile crosses, 0% sterile). Sterility is apparent also by reductions in the total number of viable progeny produced. DsMS1 homozygous males exhibited complete sterility (0/11 fertile crosses, 100% sterile; p-value <0.001). *See* **FIG. 19B****.**

When the DsRACR1 rescue element (SEQ ID NO: 4315), transformed using plasmid p446 (SEQ ID NO: 4050), is introduced to DsMS1 individuals males heterozygous for DsMS1 are no longer sterile (12/14 fertile crosses, 14% sterile). Additionally, males homozygous for DsMS1 also exhibit some fertility (2/5 fertile crosses; 60% sterility) when DsRACR1 is present. The presence of DsRACR1 also improves total fertility of heterozygous DsMS1 males, with average number of progeny rising from 14 progeny without DsRACR1 to 53 progeny (equivalent to wildtype) in the presence of DsRACR1.

### Example 19. Targeting ory in Drosophila suzukii

Guide RNA-expressing constructs targeting *ory* operably linked to a *D. suzukii U6* promoter are introduced to *D. suzukii* via embryo injection by means of piggyBac transposition using plasmid p403 (SEQ ID NO: 4150). Three strains are obtained (DsORY-B, DsORY-D, and DsORY-E). These strains are crossed to either DsDFS1 (a strain using the early germline-expressed *nanos* promoter to drive the expression of Cas9) or the DsMS1 strain to provide a source of Cas9 for each DsORY strain. *See* **FIG. 20A****.**

Fertility assays are carried out as described in Example 14. The DsDFS1 strain does not exhibit sterility in males on its own, but does exhibit low fertility in males also carrying DsORY (1/10 to 2/6 fertile crosses for two DsORY strains). Flies heterozygous for DsMS1 generate approximately 50% fertile males on their own, but fertility drops to between 0% and 17% with DsORY present as well (0/5 to 1/6 fertile crosses for two DsORY strains). *See* **FIG. 20B****.**

Taken together, these results indicate that *ory* is critical for male fertility in *D. suzukii.* This is confirmed via PCR and sequence of *ory* in sterile and wildtype males, which shows frameshift mutations in *ory* in the majority of sterile males.

*ory* is thus confirmed as a Class Ia target gene, and *zpg* is confirmed as a Class II target gene (*see* Example 18) in *D. suzukii.* It is also confirmed that anticas can rescue male fertility in *D. suzukii.*

### Example 20. Targeting the m-locus in Aedes aegypti

Rescue plasmids comprising a recoded male-germline essential gene (SEQ ID NO: 4087 to 4099) or an AcrIIA4 (SEQ ID NO: 4042, 4043, 4045, 4047, 4049 and 4053) are introduced into the m-locus of *Aedes aegypti* by embryo microinjection. Specific regions of the m-locus are modified by homology-directed repair in and around known m-linked genes including, mucin-3A (AAEL019619), aurora kinase B (AAEL006712), AAEL022649 , and AAEL014760. gRNAs and target sites to stimulate HDR of rescue elements are identified in mucin-3A(SEQ ID NO: 4382 to 4385) and aurora kinase B (SEQ ID NO: 4360 to 4381). gRNAs targeting the m-linked genes are provided as helper plasmids in the injected transformation mixture (SEQ ID NO: 4106, 4107, and 4163 to 4169). Other genes that could be targeted on the m-locus include AAEL020950, AAEL021928, AAEL027507, AAEL019917, AAEL006708, AAEL006707, AAEL006713, AAEL006712, AAEL019921, AAEL025609, AAEL005160, AAEL005169, AAEL009784, AAEL001236, AAEL002407, AAEL014760, AAEL028070, AAEL015064, AAEL019619.

### Example 21. Targeting the X-chromosome in Drosophila suzukii

Transformation plasmids comprising a recoded male-germline essential gene (SEQ ID NO: 4088, 4092 to 4093, 4096 to 4097, 4100 to 4103) or an AcrIIA4 (SEQ ID NO: 4012 to 4013, 4041, 4044, 4046, 4048, 4050 to 4052) are introduced into the X-chromosome of *Drosophila suzukii* by embryo microinjection, mediated by piggyBac transposase or homology-directed repair. Approximately 1/3 of integrations into the genome of *Drosophila suzukii* using piggyBac transposes land on the X-chromosome by chance. Specific regions of the X-chromosome are modified by homology-directed repair in and around known X-linked genes including, *hexA*, *Futsch*, by co-injecting one or more helper plasmids expressing Cas9 (SEQ ID NO: 4075 to 4078, 4082 to 4084) and one or more gRNAs. gRNAs and target sites to stimulate HDR of rescue elements are identified in *hexA* (SEQ ID NO: 4356 to 4359) and *Futsch* (SEQ ID NO: 4348 to 4355). Specific regions of the X-chromosome are also targeted by secondarily modifying a first transgene by site-specific recombination or CRIMCE.

## Claims

1. A male sterile effector comprising:
(a) a first promoter operably linked to a first nucleic acid molecule encoding an RNA-guided nuclease; and
(b) a second promoter operably linked to a second nucleic acid molecule encoding a guide RNA (gRNA), wherein the gRNA comprises a nucleic acid sequence at least 90% identical or complementary to at least 17 consecutive nucleotides of a first arthropod male-fertility essential gene.

2. A male sterile effector comprising a first promoter operably linked to a first nucleic acid molecule encoding a microRNA (miRNA) molecule or a short hairpin RNA (shRNA) molecule comprising a nucleic acid sequence at least 90% identical to at least 17 consecutive nucleotides of a first arthropod male-fertility essential gene messenger RNA molecule.

3. The male sterile effector of any one of claims 1 or 2, wherein the first arthropod male-fertility essential gene and the second arthropod male-fertility essential gene are independently selected from the group consisting of: *betaTub85D, ory, zpg, 28S rDNA, S-Lap3, blw, ary, kl-2, kl-3, kl-5, snky, knon, blanks, sa, ddbt, WDY, S-Lap1, S-Lap2, S-Lap4, S-Lap5, S-Lap7, S-Lap8, fzo, exu, bel, shu, CG12069, Cyt-c1L, Hsp60B, Kpc1, Kpc2, loopin-1, mmm, sac, Red7, Rsbp15, Rsphl, Rsph3, Rsph4a, Rsph9, wam, bam, bb8, bond, wupA, RpS6, RpS10, RpS19a, RpL35, RpL37, muc14a,* AAEL028070, *atlas, aust, Mst27D, nht, run, spag4, pp1-Y1, ppl-Y2, soti, eIF4E5, Dic61B, maca, can, bgcn, Dpy-30L2, ms(2)34Fe, ms(2)35Ci, ms(3)76Ba, ms(3)76Cc, ms(3)K81, nsr, Ntl, Pif1A, Prosalpha4T1, Prosalpha6T, PRY, shps, salto, tbrd-2, ttm2, xmas, ATPsynbetaL, loqs, Hsc70-1, Hsc70-2, Hsc70-3, Hsc70-5, Hsc70-4, Caf1-55, Hyls1, 14-3-3epsilon, ab, abd-A, Abd-B, ac, Ack, Acly, Acn, ago, alphaTub84B, aly, aop, ap, Apl, ara, Arf6, asl, asp, asun, Atg8b, ATP7, ATPsyngamma, aub, aux, ball, betaTub60D, Bin1, bol, bor, Brca2, Brr2, brul, brun, Bsg, Btk, Bug22, Bx, ca, Cad99C, Cap-D3, Cap-H2, caup, Cdc42, Cds, Cep135, Cep290, CG13202, CG18675, CG31759, CG8236, CG9313, CG9920, chb, Chc, Chdl, chic, CIAPIN1, cic, clu, cmb, cn, cnn, Cog7, COX4L, Cpes, crm, CSN5, csul, ct, ctp, cue, Cul3, CycB, Cyp40, Cyt-b5, Cyt-c-d, Dab, dally, dany, Dark, dbf, Dcr-2, DCTN5-p25, Ddx1, del, Dhc16F, dia, Diap1, didum, Diedel, Dis3l2, dj, Dlc90F, Dnaaf3, Dnaaf5, Dnaaf6, Dnah3, Doa, dor, Dronc, dsx, dtr, Duba, dwg, dx, EcR, eff, eIF2A, eIF3m, eIF4E1, eIF4E3, eIF4G2, emc, EndoGI, endos, eRF3, Erk7, eya, f, Fas2, Fas3, fbl, Fmr1, fru, fry, Fsn, fwd, fws, fz2, g, Gas8, gdrd, gish, Gld2, Gorab, Grip128, Grip75, Grip84, Grip9l, gskt, GstS1, gudu, H, hay, HBS1, Hel89B, heph, hfp, HipHop, hmw, hop, HPle, Hr39, Hr51, Hsp60C, Hsp83, HtrA2, hts, hyd, Ibf2, ifc, Inos, InR, Iswi, jar, JYalpha, Kap-alpha1, Kdm5, klhl10, Klp3A, Klp67A, l(2)dtl, l(2)gl, Lam, larp, Lasp, lds, Liprin-alpha, Lis-1, IncRNA:CR45362, lncRNA:Hsromega, IncRNA:iab4, IncRNA:iab8, lost, lovit, lz, m, mael, mago, mal, mam, MAN1, mei-P26, mei-S332, Mer, mfr, mfrn, Mgat1, mgr, Mhc, mia, mil, mino, mip120, mip40, mle, mlt, Mob4, mod, mod(mdg4), msl-3, Mst77F, mt:CoI, mt:Cyt-b, mtsh, mxc, Myc, Myt1, Naa20A, nAChRheta1, nclb, ND-B17, neb, nej, Nle, nmd, noi, Nop60B, Npc1a, ntc, Nup154, nw, orb2, Orc1, Osbp, osk, otu, ox, p24-1, pAbp, Pall, papi, PAPLA1, park, pbl, Pdelc, Pdkl, pea, pelo, Pen, Pex10, Pex12, Pex16, Pex2, Pfas, Phbl, Pih1D1, Pinkl, piwi, Pkd2, Pkn, Pngl, poe, PolQ, Polr2A, pont, Pop1, porin, Poxn, prd, pros, Prp8, psidin, PSMG1, puf, qm, Rab30, Rae1, Rala, Ran, rau, Rb97D, Rdl, ref(2)P, rept, rho-7, Rm62, rn, Rnp4F, Roc1b, Root, RpL36, Rpp30, RpS27A, RpS3, rst, rt, Rtca, rux, SAK, Sas-4, sau, sbr, sc, SCaMC, scat, scny, ScsbetaA, sd, Sf3a1, Shc, shop, shv, sif, sim, sina, slmo, sm, SmB, Smg5, snRNA:U7, sns, Sod1, spag, spd-2, spn-E, SRPK, ss, ssp3, stai, Stat92E, stau, stet, stg, sti, Strip, su(Hw), su(sable), Su(var)3-3, sub, Sxl, Sym, Syp, Syx13, Syx5, Taf6, Taz, tbrd-1, TER94, Tfb5, t-Grip84, Theg, thoc5, tilB, tj, tomb, Top2, Top3alpha, topi, Topors, Torsin, tous, tra, tra2, Trel, Trf, Trl, tRNA:Leu-CAA-1-1, tRNA:Tyr-GTA-1-3, Trxrl, tsr, Tssk, TTLL3B, tud, tut, twe, twy, U2A, Ubi-p63E, Ulpl, updl, upd3, Upf2, Usp14, v, vg, VhaPPAl-2, vib, vls, Vmat, vn, Vps28, vret, Vrp1, Vtld, WASp, Wdr92, wls, wnd, Wnt2, wts, wuho, y, Yipldl, YMEIL, zip, Zmynd10,* and *Zw10,* or an ortholog thereof.

4. A sterility system comprising:
(a) the male sterile effector of any one of claims 1 to 3; and
(b) a repressible female-lethal effector.

5. A sterility system comprising:
(a) the male sterile effector of any one of claims 1 to 3; and
(b) a rescue element comprising a fourth promoter operably linked to a nucleic acid molecule encoding at least one of: an anticas protein, a functional copy of the first arthropod male-fertility essential gene, and a functional copy of the second arthropod male-fertility essential gene.

6. A sterility system comprising:
(a) the male sterile effector of any one of claims 1 to 3;
(b) a repressible female-lethal effector; and
(c) a rescue element comprising a fourth promoter operably linked to a nucleic acid molecule encoding at least one of: an anticas protein, a functional copy of the first arthropod male-fertility essential gene, and a functional copy of the second arthropod male-fertility essential gene.

7. One or more isolated nucleic acid molecules encoding the male sterile effector of any one of claims 1 to 3 or the sterility system of any one of claims 4 to 6.

8. A modified arthropod comprising the male sterile effector of any one of claims 1 to 3.

9. A modified arthropod comprising the sterility system of any one of claims 5 to 7.

10. The modified arthropod of claim 8 or 9, wherein the arthropod is selected from the group consisting of *Acyrthosiphon pisum, Adelges tsugae, Aedes aegypti, Aedes albopictus, Agrilus planipennis, Amyelois transitella, Anarsia lineatella, Anastrepha ludens, Anastrepha obliqua, Anastrepha suspensa, Anopheles arabiensis, Anopheles coluzii, Anopheles funestus, Anopheles gambiae, Anopheles stephensi, Anoplophora glabripennis, Anthonomous grandis, Anthonomus grandis, Aonidiella aurantia, Aphis gossypii, Apis mellifera scutellata, Bactrocera cucurbitae, Bactrocera dorsalis, Bactrocera oleae, Bactrocera tyroni, Bactrocera zonata, Bagrada hilaris, Bemisia tabaci, Bombyx mori, Callosobruchus maculatus, Ceratitis capitata, Ceratitis rosa, Chrysomya bezziana, Cochliomyia aldrichi, Cochliomyia hominivorax, Cochliomyia macellaria, Cochliomyia minima, Coptotermes formosanus, Culex quinquefasciatus, Cydia pomonella, Cydia pomonella, Dalbulus maidis, Dendroctonus frontalis, Dermatobia hominis, Diabrotica balteata, Diabrotica barberi, Diabrotica undecimpunctata, Diabrotica virgifera, Diaphorina citri, Drosophila suzukii, Epiphyas postvittana, Frankliniella occidentalis, Glossina austeni, Glossina brevipalpis, Glossina fusca, Glossina fuscipes, Glossina fuscipleuris, Glossina longipalpis, Glossina longipennis, Glossina morsitans, Glossina pallidipes, Glossina palpalis, Glossina swynnertoni, Glossina tabaniformis, Glossina tachinoides, Graphocephala atropunctata, Haematobia irritans, Halyomorpha halys, Helicoverpa armigera, Helicoverpa zea, Homalodisca vitripennis, Hypoderma bovis, Hypoderma lineatum, Leptinotarsa decemlineata, Leucinodes orbonalis, Lobesia botrana, Lucilia cuprina, Lucilia cuprina, Lutzomyia spp., Lycorma delicatula, Lymantria albescens, Lymantria dispar, Lymantria dispar asiatica, Lymantria dispar japonica, Lymantria postalba, Lymantria umbrosa, Musca domestica, Myzus persicae, Naupactus spp. (Graphognatus spp.), Nilaparvata lugens, Oestridae spp., Oryctes rhinoceros, Pectinophora gossypiella, Pectinophora scutigera, Pityophthorus juglandis, Planococcus ficus, Plutella xylostella, Popillia japonica, Rhagoletis cerasi, Rhodnius prolixus, Schistocerca gregaria, Selenothrips rubrocinctus, Sirex noctilio, Solenopsis invicta, Solenopsis richteri, Spodoptera litura, Spodotera exigua, Tabanus sulcifrons, Thaumatotibia leucotreta, Thrips tabaci, Tribolium castaneum, Trogoderma granarium, Tryporyza incertulas, Tuta absoluta,* and *Varroa destructor.*

11. A method comprising:
(a) obtaining the modified arthropod of any one of claims 8 to 10, wherein the modified arthropod is a male arthropod; and
(b) releasing the modified arthropod outdoors in the vicinity of at least one wildtype female arthropod of the same species.

12. A method comprising:
(a) obtaining a male population of the modified arthropod of any one of claims 8 to 10; and
(b) releasing the male population outdoors in the vicinity of a wildtype population of the arthropod that comprises at least one wildtype female arthropod of the same species,
wherein at least one member of the male population of the modified arthropod mates with the at least one wildtype female arthropod, and wherein the at least one wildtype female arthropod produces (i) at least one sterile male progeny, (ii) at least one inviable female progeny, or (iii) both (i) and (ii).

13. A method comprising:
(a) introducing the one or more isolated nucleic acid molecules of claim 7 to at least one arthropod cell; and
(b) obtaining an adult arthropod from the at least one arthropod cell, wherein the adult arthropod comprises at least one of the isolated nucleic acid molecules.

14. A sterility system comprising:
(a) a male sterile effector comprising a loss-of-function mutation in at least one male-fertility essential gene; and
(b) a rescue element comprising a first promoter operably linked to a functional copy of the at least one male-fertility essential gene.

15. A method comprising:
(a) introducing a loss-of-function mutation in an endogenous allele of a male-fertility essential gene in at least one arthropod cell, wherein the at least one arthropod cell further comprises a recoded recue element encoding a functional copy of the male-fertility essential gene; and
(b) obtaining an adult male arthropod from the at least one arthropod cell, wherein the adult male arthropod comprises the loss-of-function mutation and the recoded rescue element.
